(19) Europäisches Patentamt
European Patent Office
Office européen des brevets

(11) EP 2 771 470 B1

(12) # FASCICULE DE BREVET EUROPEEN

(45) Date de publication et mention de la délivrance du brevet:
**28.12.2016 Bulletin 2016/52**

(21) Numéro de dépôt: **12794398.3**

(22) Date de dépôt: **29.10.2012**

(51) Int Cl.:
***C12N 15/85*** *(2006.01)* ***C07K 16/28*** *(2006.01)*
***C12P 21/00*** *(2006.01)*

(86) Numéro de dépôt international:
**PCT/FR2012/052496**

(87) Numéro de publication internationale:
**WO 2013/061010 (02.05.2013 Gazette 2013/18)**

(54) **UNITES DE TRANSCRIPTION ET LEUR UTILISATION DANS DES VECTEURS D'EXPRESSION**

TRANSKRIPTIONSEINHEIT UND VERWENDUNG DAVON IN EXPRESSIONSVEKTOREN

TRANSCRIPTION UNIT AND USE THEREOF IN EXPRESSION VECTORS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.10.2011 FR 1159864**

(43) Date de publication de la demande:
**03.09.2014 Bulletin 2014/36**

(73) Titulaire: **Laboratoire Français du Fractionnement et des Biotechnologies**
**91958 Courtaboeuf Cedex (FR)**

(72) Inventeurs:
- **FONTAYNE, Alexandre**
**59110 La Madeleine (FR)**
- **COUTARD, François**
**30100 Ales (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
**EP-A1- 1 405 908** **WO-A1-2004/050879**
**WO-A1-2011/110864** **WO-A2-01/77181**
**WO-A2-03/106658** **WO-A2-2006/022944**
**WO-A2-2009/042971**

- **FOECKING M K ET AL: "Powerful and versatile enhancer-promoter unit for mammalian expression vectors", GENE, ELSEVIER, AMSTERDAM, NL, vol. 45, no. 1, 1 janvier 1986 (1986-01-01), pages 101-105, XP025688566, ISSN: 0378-1119, DOI: 10.1016/0378-1119(86)90137-X [extrait le 1986-01-01]**
- **LIU H ET AL: "Genomic organization and characterization of promoter function of the human CDK9 gene", GENE, ELSEVIER, AMSTERDAM, NL, vol. 252, no. 1-2, 11 juillet 2000 (2000-07-11), pages 51-59, XP004210154, ISSN: 0378-1119, DOI: 10.1016/S0378-1119(00)00215-8 cité dans la demande**
- **LIU H ET AL: "Isolation and characterization of the human cyclin T1 promoter", GENE, ELSEVIER, AMSTERDAM, NL, vol. 252, no. 1-2, 11 juillet 2000 (2000-07-11), pages 39-49, XP004210153, ISSN: 0378-1119, DOI: 10.1016/S0378-1119(00)00214-6**
- **GIULIA DE FALCO ET AL: "Cdk9/Cyclin T1 complex: A key player during the activation/differentiation process of normal lymphoid B cells", JOURNAL OF CELLULAR PHYSIOLOGY, vol. 215, no. 1, 1 avril 2008 (2008-04-01) , pages 276-282, XP055051946, ISSN: 0021-9541, DOI: 10.1002/jcp.21311**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 771 470 B1

- R. E. RHOADS: "Internal Initiation of Translation Directed by the 5'-Untranslated Region of the mRNA for eIF4G, a Factor Involved in the Picornavirus-induced Switch from Cap-dependent to Internal Initiation", JOURNAL OF BIOLOGICAL CHEMISTRY, vol. 271, no. 2, 12 janvier 1996 (1996-01-12), pages 623-626, XP055028850, ISSN: 0021-9258, DOI: 10.1074/jbc.271.2.623
- YOO E M ET AL: "Myeloma expression systems", JOURNAL OF IMMUNOLOGICAL METHODS, ELSEVIER SCIENCE PUBLISHERS B.V.,AMSTERDAM, NL, vol. 261, no. 1-2, 1 mars 2002 (2002-03-01), pages 1-20, XP004341264, ISSN: 0022-1759

SEQUENCE LISTING

<110> LFB BIOTECHNOLOGIES

<120> UNITE DE TRANSCRIPTION ET LEUR UTILISATION DANS DES VECTEURS

<130> WOB 11 CO LFB UTRA

<150> FR1159864
<151> 2011-10-28

<160> 71

<170> PatentIn version 3.5

<210> 1
<211> 306
<212> DNA
<213> Artificial Sequence

<220>
<223> CMV

<400> 1

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatgg                                                                306
```

<210> 2
<211> 352
<212> DNA
<213> Artificial Sequence

<220>
<223> CDK9

<400> 2

```
catgcagcgg gacgcgccac cccgagcccc agctccggcg ccccggctcc ccgcgccccc      60
gatcggggcc gccgctagta gtggcggcgg cggaggcggg ggcagcggcg gcggcggcgg     120
aggcgcctct gcagctccgg ctccccctgg cctctcggga actacaagtc ccaggggggcc    180
tggcggtggg cggcgggcgg aagaggcggg gtcggcgccg cgaggccgga agtggccgtg     240
gaggcggaag tggcgcggcc gcggaggggc ctggagtgcg gcggcggcgg gacccggagc     300
aggagcggcg gcagcagcga ctgggggcgg cggcggcgcg ttggaggcgg cc             352
```

<210> 3
<211> 267
<212> DNA

<213> Artificial Sequence

<220>
<223> U1

<400> 3

```
ggctcgcatc tctccttcac gcgcccgccg ccctacctga ggccgccatc cacgccggtt      60
gagtcgcgtt ctgccgcctc ccgcctgtgg tgcctcctga actgcgtccg ccgtctaggt     120
aagtttaaag ctcaggtcga gaccgggcct ttgtccggcg ctcccttgga gcctacctag     180
actcagccgg ctctccacgc tttgcctgac cctgcttgct caactctacg tctttgtttc     240
gttttctgtt ctgcgccgtt acagatc                                         267
```

<210> 4
<211> 653
<212> DNA
<213> Artificial Sequence

<220>
<223> U2

<400> 4

```
cagagtaatg acatggttcc ttccatcctc caaaggtgac caataatagt ttgtaagtat      60
cattatgaac taatgaattt tcaacatatt tgatatattt caatccattg ccatcattgt     120
tcttatcgat atttgagttg gctcactttg ccagtaagag tctattcaaa ttggcttctg     180
agtccatttg acacaacacc tttgatcttt gacagtttcc ttggttttag gtgctagatg     240
atttctcagg ctcaccttag acatttcctg ccacagactt agaatcagcc atttctctaa     300
ggaccctgat tccatttcat gagaaatgat agagaccaca atcaaaacaa gtcatgaatt     360
tatactgata ttttcaattc aaattaaaga tgaggttttt gctaaatttt tttgagttta     420
tatttgtatg tcttatgctg aaaaatcttg tttcctaatt agtaacataa ttattcattt     480
gatgggtaaa tattttaggg ccgattcttt ggttttatag ccaagatacc ctgttgataa     540
agtcttgtgg gagcaattat aagactggct tattttgaag ctttttaaaa aagacatcct     600
tacctgtttt aactgtagat tatattaact taaataggta cagcccacgc ttg            653
```

<210> 5
<211> 314
<212> DNA
<213> Artificial Sequence

<220>
<223> U3

<400> 5

```
gctggtgggt agggatgagg gagggagggg cattgtgatg tacagggctg ctctgtgaga        60
tcaagggtct cttaagggtg ggagctgggg cagggactac gagagcagcc agatgggctg       120
aaagtggaac tcaaggggtt tctggcacct acctacctgc ttcccgctgg ggggtgggga       180
gttggcccag agtcttaaga ttggggcagg gtggagaggt gggctcttcc tgcttcccac       240
tcatcttata gctttctttc cccagatccg aattcgagat ccaaaccaag gaggaaagga       300
tatcacagag gaga                                                         314
```

<210> 6
<211> 933
<212> DNA
<213> Artificial Sequence

<220>
<223> U1U2

<400> 6

```
ggctcgcatc tctccttcac gcgcccgccg ccctacctga ggccgccatc cacgccggtt        60
gagtcgcgtt ctgccgcctc ccgcctgtgg tgcctcctga actgcgtccg ccgtctaggt       120
aagtttaaag ctcaggtcga gaccgggcct ttgtccggcg ctcccttgga gcctacctag       180
actcagccgg ctctccacgc tttgcctgac cctgcttgct caactctacg tctttgtttc       240
gttttctgtt ctgcgccgtt acagatcact agtgtttaaa cagagtaatg acatggttcc       300
ttccatcctc caaaggtgac caataatagt ttgtaagtat cattatgaac taatgaattt       360
tcaacatatt tgatatattt caatccattg ccatcattgt tcttatcgat atttgagttg       420
gctcactttg ccagtaagag tctattcaaa ttggcttctg agtccatttg acacaacacc       480
tttgatcttt gacagtttcc ttggttttag gtgctagatg atttctcagg ctcaccttag       540
acatttcctg ccacagactt agaatcagcc atttctctaa ggaccctgat tccatttcat       600
gagaaatgat agagaccaca atcaaaacaa gtcatgaatt tatactgata ttttcaattc       660
aaattaaaga tgaggttttt gctaaatttt tttgagttta tatttgtatg tcttatgctg       720
aaaaatcttg tttcctaatt agtaacataa ttattcattt gatgggtaaa tattttaggg       780
ccgattcttt ggttttatag ccaagatacc ctgttgataa agtcttgtgg gagcaattat       840
aagactggct tattttgaag ctttttaaaa aagacatcct tacctgtttt aactgtagat       900
tatattaact taaataggta cagcccacgc ttg                                    933
```

<210> 7
<211> 591
<212> DNA
<213> Artificial Sequence

<220>
<223> U1U3

<400> 7

```
ggctcgcatc tctccttcac gcgcccgccg ccctacctga ggccgccatc cacgccggtt      60
gagtcgcgtt ctgccgcctc ccgcctgtgg tgcctcctga actgcgtccg ccgtctaggt     120
aagtttaaag ctcaggtcga gaccgggcct ttgtccggcg ctcccttgga gcctacctag     180
actcagccgg ctctccacgc tttgcctgac cctgcttgct caactctacg tctttgtttc     240
gttttctgtt ctgcgccgtt acagatcact agttaacgct ggtgggtagg gatgagggag     300
ggaggggcat tgtgatgtac agggctgctc tgtgagatca agggtctctt aagggtggga     360
gctggggcag ggactacgag agcagccaga tgggctgaaa gtggaactca aggggtttct     420
ggcacctacc tacctgcttc ccgctggggg gtggggagtt ggcccagagt cttaagattg     480
gggcagggtg gagaggtggg ctcttcctgc ttcccactca tcttatagct ttctttcccc     540
agatccgaat tcgagatcca aaccaaggag gaaaggatat cacagaggag a              591
```

<210> 8
<211> 977
<212> DNA
<213> Artificial Sequence

<220>
<223> U2U3

<400> 8

```
cagagtaatg acatggttcc ttccatcctc caaaggtgac caataatagt ttgtaagtat        60
cattatgaac taatgaattt tcaacatatt tgatatattt caatccattg ccatcattgt       120
tcttatcgat atttgagttg gctcactttg ccagtaagag tctattcaaa ttggcttctg       180
agtccatttg acacaacacc tttgatcttt gacagtttcc ttggttttag gtgctagatg       240
atttctcagg ctcaccttag acatttcctg ccacagactt agaatcagcc atttctctaa       300
ggaccctgat tccatttcat gagaaatgat agagaccaca atcaaaacaa gtcatgaatt       360
tatactgata ttttcaattc aaattaaaga tgaggttttt gctaaatttt tttgagttta       420
tatttgtatg tcttatgctg aaaaatcttg tttcctaatt agtaacataa ttattcattt       480
gatgggtaaa tattttaggg ccgattcttt ggttttatag ccaagatacc ctgttgataa       540
agtcttgtgg gagcaattat aagactggct tattttgaag ctttttaaaa aagacatcct       600
tacctgtttt aactgtagat tatattaact taaataggta cagcccacgc ttgactagtt       660
aacgctggtg ggtagggatg agggagggag gggcattgtg atgtacaggg ctgctctgtg       720
agatcaaggg tctcttaagg gtgggagctg gggcagggac tacgagagca gccagatggg       780
ctgaaagtgg aactcaaggg gtttctggca cctacctacc tgcttcccgc tgggggggtgg      840
ggagttggcc cagagtctta agattggggc agggtggaga ggtgggctct tcctgcttcc       900
cactcatctt atagctttct ttccccagat ccgaattcga gatccaaacc aaggaggaaa       960
ggatatcaca gaggaga                                                      977
```

<210> 9
<211> 1257
<212> DNA
<213> Artificial Sequence

<220>
<223> U1U2U3

<400> 9

```
ggctcgcatc tctccttcac gcgcccgccg ccctacctga ggccgccatc cacgccggtt      60
gagtcgcgtt ctgccgcctc ccgcctgtgg tgcctcctga actgcgtccg ccgtctaggt     120
aagtttaaag ctcaggtcga gaccgggcct ttgtccggcg ctcccttgga gcctacctag     180
actcagccgg ctctccacgc tttgcctgac cctgcttgct caactctacg tctttgtttc     240
gttttctgtt ctgcgccgtt acagatcact agtgtttaaa cagagtaatg acatggttcc     300
ttccatcctc caaaggtgac caataatagt ttgtaagtat cattatgaac taatgaattt     360
tcaacatatt tgatatattt caatccattg ccatcattgt tcttatcgat atttgagttg     420
gctcactttg ccagtaagag tctattcaaa ttggcttctg agtccatttg acacaacacc     480
tttgatcttt gacagtttcc ttggttttag gtgctagatg atttctcagg ctcaccttag     540
acatttcctg ccacagactt agaatcagcc atttctctaa ggaccctgat tccatttcat     600
gagaaatgat agagaccaca atcaaaacaa gtcatgaatt tatactgata ttttcaattc     660
aaattaaaga tgaggttttt gctaaatttt tttgagttta tatttgtatg tcttatgctg     720
aaaaatcttg tttcctaatt agtaacataa ttattcattt gatgggtaaa tattttaggg     780
ccgattcttt ggttttatag ccaagatacc ctgttgataa agtcttgtgg gagcaattat     840
aagactggct tattttgaag ctttttaaaa aagacatcct tacctgtttt aactgtagat     900
tatattaact taaataggta cagcccacgc ttgactagtt aacgctggtg ggtagggatg     960
agggagggag gggcattgtg atgtacaggg ctgctctgtg agatcaaggg tctcttaagg    1020
gtgggagctg ggcagggac tacgagagca gccagatggg ctgaaagtgg aactcaaggg     1080
gtttctggca cctacctacc tgcttcccgc tggggggtgg ggagttggcc cagagtctta    1140
agattggggc agggtggaga ggtgggctct tcctgcttcc cactcatctt atagctttct    1200
ttccccagat ccgaattcga gatccaaacc aaggaggaaa ggatatcaca gaggaga       1257
```

<210> 10
<211> 939
<212> DNA
<213> Artificial Sequence

<220>
<223> EF1A

<400> 10

```
gtaagtgccg tgtgtggttc ccgcgggcct ggcctcttta cgggttatgg cccttgcgtg      60
ccttgaatta cttccacctg gctgcagtac gtgattcttg atcccgagct tcgggttgga     120
agtgggtggg agagttcgag gccttgcgct taaggagccc cttcgcctcg tgcttgagtt     180
```

gaggcctggc ctgggcgctg gggccgccgc gtgcgaatct ggtggcacct tcgcgcctgt 240

ctcgctgctt tcgataagtc tctagccatt taaaattttt gatgacctgc tgcgacgctt 300

tttttctggc aagatagtct tgtaaatgcg ggccaagatc tgcacactgg tatttcggtt 360

tttggggccg cgggcggcga cggggcccgt gcgtcccagc gcacatgttc ggcgaggcgg 420

ggcctgcgag cgcggccacc gagaatcgga cgggggtagt ctcaagctgg ccggcctgct 480

ctggtgcctg gcctcgcgcc gccgtgtatc gccccgccct gggcggcaag gctggcccgg 540

tcggcaccag ttgcgtgagc ggaaagatgg ccgcttcccg gccctgctgc agggagctca 600

aaatggagga cgcggcgctc gggagagcgg gcgggtgagt cacccacaca aaggaaaagg 660

gcctttccgt cctcagccgt cgcttcatgt gactccacgg agtaccgggc gccgtccagg 720

cacctcgatt agttctcgag cttttggagt acgtcgtctt taggttgggg ggaggggttt 780

tatgcgatgg agtttcccca cactgagtgg gtggagactg aagttaggcc agcttggcac 840

ttgatgtaat tctccttgga atttgccctt tttgagtttg gatcttggtt cattctcaag 900

cctcagacag tggttcaaag tttttttctt ccatttcag 939

<210> 11
<211> 351
<212> DNA
<213> Artificial Sequence

<220>
<223> MROSA

<400> 11

gtaggggatc gggactctgg cgggagggcg gcttggtgcg tttgcgggga tgggcggccg 60

cggcaggccc tccgagcgtg gtggagccgt tctgtgagac agccgggtac gagtcgtgac 120

gctggaaggg gcaagcgggt ggtgggcagg aatgcggtcc gccctgcagc aaccggaggg 180

ggagggagaa gggagcggaa aagtctccac cggacgcggc catggctcgg gggggggggg 240

gcagcggagg agcgcttccg gccgacgtct cgtcgctgat tggcttcttt tcctcccgcc 300

gtgtgtgaaa acacaattgt actaaccttc ttctctttcc tctcctgaca g 351

<210> 12
<211> 288
<212> DNA
<213> Artificial Sequence

<220>
<223> HTLV-1

<400> 12

ggctcgcatc tctccttcac gcgcccgccg ccctacctga ggccgccatc cacgccggtt 60

gagtcgcgtt ctgccgcctc ccgcctgtgg tgcctcctga actgcgtccg ccgtctaggt 120

aagtttaaag ctcaggtcga gaccgggcct ttgtccggcg ctcccttgga gcctacctag 180

actcagccgg ctctccacgc tttgcctgac cctgcttgct caactctacg tctttgtttc 240

gttttctgtt ctgcgccgtt acagatccaa gctgtgaccg gcgcctac 288

<210> 13
<211> 133
<212> DNA
<213> Artificial Sequence

<220>
<223> PCINEO

<400> 13

gtaagtatca aggttacaag acaggtttaa ggagaccaat agaaactggg cttgtcgaga 60

cagagaagac tcttgcgttt ctgataggca cctattggtc ttactgacat ccactttgcc 120

tttctctcca cag 133

<210> 14
<211> 951
<212> DNA
<213> Artificial Sequence

<220>
<223> séquence artificielle

<400> 14

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aaacggctcg catctctcct tcacgcgccc gccgccctac     720
ctgaggccgc catccacgcc ggttgagtcg cgttctgccg cctcccgcct gtggtgcctc     780
ctgaactgcg tccgccgtct aggtaagttt aaagctcagg tcgagaccgg gcctttgtcc     840
ggcgctccct tggagcctac ctagactcag ccggctctcc acgctttgcc tgaccctgct     900
tgctcaactc tacgtctttg tttcgttttc tgttctgcgc cgttacagat c              951
```

<210> 15
<211> 1336
<212> DNA
<213> Artificial Sequence

<220>
<223> séquence artificielle

<400> 15

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aaacagagta atgacatggt tccttccatc ctccaaaggt     720
gaccaataat agtttgtaag tatcattatg aactaatgaa ttttcaacat atttgatata     780
tttcaatcca ttgccatcat tgttcttatc gatatttgag ttggctcact ttgccagtaa     840
gagtctattc aaattggctt ctgagtccat ttgacacaac acctttgatc tttgacagtt     900
tccttggttt taggtgctag atgatttctc aggctcacct tagacatttc ctgccacaga     960
cttagaatca gccatttctc taaggaccct gattccattt catgagaaat gatagagacc    1020
acaatcaaaa caagtcatga atttatactg atattttcaa ttcaaattaa agatgaggtt    1080
tttgctaaat ttttttgagt ttatatttgt atgtcttatg ctgaaaaatc ttgtttccta    1140
attagtaaca taattattca tttgatgggt aaatatttta gggccgattc tttggtttta    1200
tagccaagat accctgttga taaagtcttg tgggagcaat tataagactg gcttattttg    1260
aagcttttta aaaaagacat ccttacctgt tttaactgta gattatatta acttaaatag    1320
gtacagccca cgcttg                                                    1336
```

<210> 16
<211> 997
<212> DNA
<213> Artificial Sequence

<220>
<223> séquence artificielle

<400> 16

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc aggggcctg gcggtgggcg gcgggcggaa gaggcggggt cggcgccgcg 540
aggccggaag tggccgtgga ggcggaagtg gcgcggccgc ggagggggcct ggagtgcggc 600
ggcggcggga cccggagcag gagcggcggc agcagcgact gggggcggcg gcggcgcgtt 660
ggaggcggcc ggatccgttt aacgctggtg ggtagggatg agggagggag gggcattgtg 720
atgtacaggg ctgctctgtg agatcaaggg tctcttaagg gtgggagctg gggcagggac 780
tacgagagca gccagatggg ctgaaagtgg aactcaaggg gtttctggca cctacctacc 840
tgcttcccgc tggggggtgg ggagttggcc cagagtctta agattggggc agggtggaga 900
ggtgggctct tcctgcttcc cactcatctt atagctttct ttccccagat ccgaattcga 960
gatccaaacc aaggaggaaa ggatatcaca gaggaga 997

<210> 17
<211> 1617
<212> DNA
<213> Artificial Sequence

<220>
<223> séquence artificielle

<400> 17

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480

```
tacaagtccc aggggggcctg gcggtgggcg gcgggcggaa gaggcggggt cggcgccgcg      540
aggccggaag tggccgtgga ggcggaagtg gcgcggccgc ggaggggcct ggagtgcggc      600
ggcggcggga cccggagcag gagcggcggc agcagcgact gggggcggcg gcggcgcgtt      660
ggaggcggcc ggatccgttt aaacggctcg catctctcct tcacgcgccc gccgccctac      720
ctgaggccgc catccacgcc ggttgagtcg cgttctgccg cctcccgcct gtggtgcctc      780
ctgaactgcg tccgccgtct aggtaagttt aaagctcagg tcgagaccgg gcctttgtcc      840
ggcgctccct tggagcctac ctagactcag ccggctctcc acgctttgcc tgaccctgct      900
tgctcaactc tacgtctttg tttcgttttc tgttctgcgc cgttacagat cactagtgtt      960
taaacagagt aatgacatgg ttccttccat cctccaaagg tgaccaataa tagtttgtaa     1020
gtatcattat gaactaatga attttcaaca tatttgatat atttcaatcc attgccatca     1080
ttgttcttat cgatatttga gttggctcac tttgccagta agagtctatt caaattggct     1140
tctgagtcca tttgacacaa cacctttgat ctttgacagt ttccttggtt ttaggtgcta     1200
gatgatttct caggctcacc ttagacattt cctgccacag acttagaatc agccatttct     1260
ctaaggaccc tgattccatt tcatgagaaa tgatagagac cacaatcaaa acaagtcatg     1320
aatttatact gatattttca attcaaatta aagatgaggt ttttgctaaa ttttttttgag     1380
tttatatttg tatgtcttat gctgaaaaat cttgtttcct aattagtaac ataattattc     1440
atttgatggg taaatatttt agggccgatt ctttggtttt atagccaaga taccctgttg     1500
ataaagtctt gtgggagcaa ttataagact ggcttatttt gaagcttttt aaaaaagaca     1560
tccttacctg ttttaactgt agattatatt aacttaaata ggtacagccc acgcttg        1617
```

<210> 18
<211> 1275
<212> DNA
<213> Artificial Sequence

<220>
<223> séquence artificielle

<400> 18

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420

cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aaacggctcg catctctcct tcacgcgccc gccgccctac     720
ctgaggccgc catccacgcc ggttgagtcg cgttctgccg cctcccgcct gtggtgcctc     780
ctgaactgcg tccgccgtct aggtaagttt aaagctcagg tcgagaccgg gcctttgtcc     840
ggcgctccct tggagcctac ctagactcag ccggctctcc acgctttgcc tgaccctgct     900
tgctcaactc tacgtctttg tttcgttttc tgttctgcgc cgttacagat cactagttaa     960
cgctggtggg tagggatgag ggagggaggg gcattgtgat gtacagggct gctctgtgag    1020
atcaagggtc tcttaagggt gggagctggg gcagggacta cgagagcagc cagatgggct    1080
gaaagtggaa ctcaaggggt ttctggcacc tacctacctg cttcccgctg gggggtgggg    1140
agttggccca gagtcttaag attggggcag ggtggagagg tgggctcttc ctgcttccca    1200
ctcatcttat agctttcttt ccccagatcc gaattcgaga tccaaaccaa ggaggaaagg    1260
atatcacaga ggaga                                                     1275
```

<210> 19
<211> 1660
<212> DNA
<213> Artificial Sequence

<220>
<223> séquence artificielle

<400> 19

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacagagta atgacatggt tccttccatc ctccaaaggt 720

gaccaataat agtttgtaag tatcattatg aactaatgaa ttttcaacat atttgatata 780
tttcaatcca ttgccatcat tgttcttatc gatatttgag ttggctcact ttgccagtaa 840
gagtctattc aaattggctt ctgagtccat ttgacacaac acctttgatc tttgacagtt 900
tccttggttt taggtgctag atgatttctc aggctcacct tagacatttc ctgccacaga 960
cttagaatca gccatttctc taaggaccct gattccattt catgagaaat gatagagacc 1020
acaatcaaaa caagtcatga atttatactg atattttcaa ttcaaattaa agatgaggtt 1080
tttgctaaat ttttttgagt ttatatttgt atgtcttatg ctgaaaaatc ttgtttccta 1140
attagtaaca taattattca tttgatgggt aaatatttta gggccgattc tttggtttta 1200
tagccaagat accctgttga taaagtcttg tgggagcaat tataagactg gcttattttg 1260
aagcttttta aaaaagacat ccttacctgt tttaactgta gattatatta acttaaatag 1320
gtacagccca cgcttgacta gttaacgctg gtgggtaggg atgagggagg gaggggcatt 1380
gtgatgtaca gggctgctct gtgagatcaa gggtctctta agggtgggag ctggggcagg 1440
gactacgaga gcagccagat gggctgaaag tggaactcaa ggggtttctg gcacctacct 1500
acctgcttcc cgctgggggg tggggagttg gcccagagtc ttaagattgg ggcagggtgg 1560
agaggtgggc tcttcctgct tcccactcat cttatagctt tctttcccca gatccgaatt 1620
cgagatccaa accaaggagg aaaggatatc acagaggaga 1660

<210> 20
<211> 1941
<212> DNA
<213> Artificial Sequence

<220>
<223> séquence artificielle

<400> 20

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
```

ggcggcggga cccggagcag gagcggcggc agcagcgact gggggcggcg gcggcgcgtt 660

ggaggcggcc ggatccgttt aaacggctcg catctctcct tcacgcgccc gccgccctac 720

ctgaggccgc catccacgcc ggttgagtcg cgttctgccg cctcccgcct gtggtgcctc 780

ctgaactgcg tccgccgtct aggtaagttt aaagctcagg tcgagaccgg gcctttgtcc 840

ggcgctccct tggagcctac ctagactcag ccggctctcc acgctttgcc tgaccctgct 900

tgctcaactc tacgtctttg tttcgttttc tgttctgcgc cgttacagat cactagtgtt 960

taaacagagt aatgacatgg ttccttccat cctccaaagg tgaccaataa tagtttgtaa 1020

gtatcattat gaactaatga attttcaaca tatttgatat atttcaatcc attgccatca 1080

ttgttcttat cgatatttga gttggctcac tttgccagta agagtctatt caaattggct 1140

tctgagtcca tttgacacaa cacctttgat ctttgacagt ttccttggtt ttaggtgcta 1200

gatgatttct caggctcacc ttagacattt cctgccacag acttagaatc agccatttct 1260

ctaaggaccc tgattccatt tcatgagaaa tgatagagac cacaatcaaa acaagtcatg 1320

aatttatact gatattttca attcaaatta aagatgaggt ttttgctaaa ttttttttgag 1380

tttatatttg tatgtcttat gctgaaaaat cttgtttcct aattagtaac ataattattc 1440

atttgatggg taaatatttt agggccgatt ctttggtttt atagccaaga taccctgttg 1500

ataaagtctt gtgggagcaa ttataagact ggcttatttt gaagcttttt aaaaaagaca 1560

tccttacctg ttttaactgt agattatatt aacttaaata ggtacagccc acgcttgact 1620

agttaacgct ggtgggtagg gatgagggag ggaggggcat tgtgatgtac agggctgctc 1680

tgtgagatca agggtctctt aagggtggga gctggggcag ggactacgag agcagccaga 1740

tgggctgaaa gtggaactca aggggtttct ggcacctacc tacctgcttc ccgctggggg 1800

gtggggagtt ggcccagagt cttaagattg gggcagggtg gagaggtggg ctcttcctgc 1860

ttcccactca tcttatagct ttctttcccc agatccgaat tcgagatcca aaccaaggag 1920

gaaaggatat cacagaggag a 1941

<210> 21
<211> 1643
<212> DNA
<213> Artificial Sequence

<220>
<223> séquence artificielle

<400> 21

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct cccccctggcc tctcgggaac    480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccacta gtcgggtttg ccgccagaac acaggtaagt gccgtgtgtg     720
gttcccgcgg gcctggcctc tttacgggtt atggcccttg cgtgccttga attacttcca     780
cctggctgca gtacgtgatt cttgatcccg agcttcgggt tggaagtggg tgggagagtt     840
cgaggccttg cgcttaagga gccccttcgc ctcgtgcttg agttgaggcc tggcctgggc     900
gctggggccg ccgcgtgcga atctggtggc accttcgcgc ctgtctcgct gctttcgata     960
agtctctagc catttaaaat ttttgatgac ctgctgcgac gctttttttc tggcaagata    1020
gtcttgtaaa tgcgggccaa gatctgcaca ctggtatttc ggtttttggg gccgcgggcg    1080
gcgacggggc ccgtgcgtcc cagcgcacat gttcggcgag gcggggcctg cgagcgcggc    1140
caccgagaat cggacggggg tagtctcaag ctggccggcc tgctctggtg cctggcctcg    1200
cgccgccgtg tatcgccccg ccctgggcgg caaggctggc ccggtcggca ccagttgcgt    1260
gagcggaaag atggccgctt cccggccctg ctgcagggag ctcaaaatgg aggacgcggc    1320
gctcgggaga gcgggcgggt gagtcaccca cacaaaggaa aagggccttt ccgtcctcag    1380
ccgtcgcttc atgtgactcc acggagtacc gggcgccgtc caggcacctc gattagttct    1440
cgagcttttg gagtacgtcg tctttaggtt gggggggaggg gttttatgcg atggagtttc   1500
cccacactga gtgggtggag actgaagtta ggccagcttg gcacttgatg taattctcct    1560
tggaatttgc cctttttgag tttggatctt ggttcattct caagcctcag acagtggttc    1620
aaagtttttt tcttccattt cag                                            1643
```

<210> 22
<211> 1051
<212> DNA
<213> Artificial Sequence

<220>
<223> séquence artificielle

<400> 22

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccacta gttcagagag cctcggctag gtaggggatc gggactctgg     720
cgggagggcg gcttggtgcg tttgcgggga tgggcggccg cggcaggccc tccgagcgtg     780
gtggagccgt tctgtgagac agccgggtac gagtcgtgac gctggaaggg gcaagcgggt     840
ggtgggcagg aatgcggtcc gccctgcagc aaccggaggg ggagggagaa gggagcggaa     900
aagtctccac cggacgcggc catggctcgg gggggggggg gcagcggagg agcgcttccg     960
gccgacgtct cgtcgctgat tggcttcttt tcctcccgcc gtgtgtgaaa acacaattgt    1020
actaaccttc ttctctttcc tctcctgaca g                                   1051
```

<210> 23
<211> 978
<212> DNA
<213> Artificial Sequence

<220>
<223> séquence artificielle

<400> 23

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc aggggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccacta gtcttcgagg ggctcgcatc tctccttcac gcgcccgccg     720
ccctacctga ggccgccatc cacgccggtt gagtcgcgtt ctgccgcctc ccgcctgtgg     780
tgcctcctga actgcgtccg ccgtctaggt aagtttaaag ctcaggtcga gaccgggcct     840
ttgtccggcg ctcccttgga gcctacctag actcagccgg ctctccacgc tttgcctgac     900
cctgcttgct caactctacg tctttgtttc gttttctgtt ctgcgccgtt acagatccaa     960
gctgtgaccg gcgcctac                                                   978
```

<210> 24
<211> 844
<212> DNA
<213> Artificial Sequence

<220>
<223> séquence artificielle

<400> 24

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccacta gtgcagaagt tggtcgtgag gcactgggca ggtaagtatc     720
aaggttacaa gacaggttta aggagaccaa tagaaactgg gcttgtcgag acagagaaga     780
ctcttgcgtt tctgataggc acctattggt cttactgaca tccactttgc ctttctctcc     840
acag                                                                  844
```

<210> 25
<211> 1918
<212> DNA
<213> Artificial Sequence

<220>
<223> séquence artificielle

<400> 25

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc aggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagtcgg 960
gtttgccgcc agaacacagg taagtgccgt gtgtggttcc cgcgggcctg gcctctttac 1020
gggttatggc ccttgcgtgc cttgaattac ttccacctgg ctgcagtacg tgattcttga 1080
tcccgagctt cgggttggaa gtgggtggga gagttcgagg ccttgcgctt aaggagcccc 1140
ttcgcctcgt gcttgagttg aggcctggcc tgggcgctgg ggccgccgcg tgcgaatctg 1200
gtggcacctt cgcgcctgtc tcgctgcttt cgataagtct ctagccattt aaaatttttg 1260
atgacctgct gcgacgcttt ttttctggca agatagtctt gtaaatgcgg gccaagatct 1320
gcacactggt atttcggttt ttggggccgc gggcggcgac ggggcccgtg cgtcccagcg 1380
cacatgttcg gcgaggcggg gcctgcgagc gcggccaccg agaatcggac gggggtagtc 1440
tcaagctggc cggcctgctc tggtgcctgg cctcgcgccg ccgtgtatcg ccccgccctg 1500
ggcggcaagg ctggcccggt cggcaccagt tgcgtgagcg gaaagatggc cgcttcccgg 1560
ccctgctgca gggagctcaa aatggaggac gcggcgctcg ggagagcggg cgggtgagtc 1620
acccacacaa aggaaaaggg cctttccgtc ctcagccgtc gcttcatgtg actccacgga 1680
gtaccgggcg ccgtccaggc acctcgatta gttctcgagc ttttggagta cgtcgtcttt 1740
aggttggggg gaggggtttt atgcgatgga gtttccccac actgagtggg tggagactga 1800
agttaggcca gcttggcact tgatgtaatt ctccttggaa tttgcccttt ttgagtttgg 1860
atcttggttc attctcaagc ctcagacagt ggttcaaagt ttttttcttc catttcag 1918

<210> 26
<211> 1326
<212> DNA
<213> Artificial Sequence

<220>
<223> séquence artificielle

<400> 26

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc aggggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aaacggctcg catctctcct tcacgcgccc gccgccctac     720
ctgaggccgc catccacgcc ggttgagtcg cgttctgccg cctcccgcct gtggtgcctc     780
ctgaactgcg tccgccgtct aggtaagttt aaagctcagg tcgagaccgg gcctttgtcc     840
ggcgctccct tggagcctac ctagactcag ccggctctcc acgctttgcc tgaccctgct     900
tgctcaactc tacgtctttg tttcgttttc tgttctgcgc cgttacagat cactagttca     960
gagagcctcg gctaggtagg ggatcgggac tctggcggga gggcggcttg gtgcgtttgc    1020
ggggatgggc ggccgcggca ggccctccga gcgtggtgga gccgttctgt gagacagccg    1080
ggtacgagtc gtgacgctgg aaggggcaag cgggtggtgg gcaggaatgc ggtccgccct    1140
gcagcaaccg gagggggagg gagaagggag cggaaaagtc tccaccggac gcggccatgg    1200
ctcggggggg ggggggcagc ggaggagcgc ttccggccga cgtctcgtcg ctgattggct    1260
tcttttcctc ccgccgtgtg tgaaaacaca attgtactaa ccttcttctc tttcctctcc    1320
tgacag                                                               1326
```

<210> 27
<211> 1263
<212> DNA
<213> Artificial Sequence

<220>
<223> séquence artificielle

<400> 27

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccacta ggatccgttt aaacggctcg catctctcct tcacgcgccc     720
gccgccctac ctgaggccgc catccacgcc ggttgagtcg cgttctgccg cctcccgcct     780
gtggtgcctc ctgaactgcg tccgccgtct aggtaagttt aaagctcagg tcgagaccgg     840
gcctttgtcc ggcgctccct tggagcctac ctagactcag ccggctctcc acgctttgcc     900
tgaccctgct tgctcaactc tacgtctttg tttcgttttc tgttctgcgc cgttacagat     960
cactagtctt cgaggggctc gcatctctcc ttcacgcgcc cgccgcccta cctgaggccg    1020
ccatccacgc cggttgagtc gcgttctgcc gcctcccgcc tgtggtgcct cctgaactgc    1080
gtccgccgtc taggtaagtt taaagctcag gtcgagaccg ggcctttgtc cggcgctccc    1140
ttggagccta cctagactca gccggctctc cacgctttgc ctgaccctgc ttgctcaact    1200
ctacgtcttt gtttcgtttt ctgttctgcg ccgttacaga tccaagctgt gaccggcgcc    1260
tac                                                                  1263
```

<210> 28
<211> 1119
<212> DNA
<213> Artificial Sequence

<220>
<223> séquence artificielle

<400> 28

```
gcgttacata acttacggta aatggcccgc ctggctgacc gcccaacgac ccccgcccat         60
tgacgtcaat aatgacgtat gttcccatag taacgccaat agggactttc cattgacgtc        120
aatgggtgga gtatttacgg taaactgccc acttggcagt acatcaagtg tatcatatgc        180

caagtacgcc ccctattgac gtcaatgacg gtaaatggcc cgcctggcat tatgcccagt        240
acatgacctt atgggacttt cctacttggc agtacatcta cgtattagtc atcgctatta        300
ccatggcccg ggtcgcgaca tgcagcggga cgcgccaccc cgagccccag ctccggcgcc        360
ccggctcccc gcgcccccga tcggggccgc cgctagtagt ggcggcggcg gaggcggggg        420
cagcggcggc ggcggcggag gcgcctctgc agctccggct ccccctggcc tctcgggaac        480
tacaagtccc agggggcctg gcggtgggcg gcgggcggaa gaggcggggt cggcgccgcg        540
aggccggaag tggccgtgga ggcggaagtg gcgcggccgc ggaggggcct ggagtgcggc        600
ggcggcggga cccggagcag gagcggcggc agcagcgact gggggcggcg gcggcgcgtt        660
ggaggcggcc ggatccgttt aaacggctcg catctctcct tcacgcgccc gccgccctac        720
ctgaggccgc catccacgcc ggttgagtcg cgttctgccg cctcccgcct gtggtgcctc        780
ctgaactgcg tccgccgtct aggtaagttt aaagctcagg tcgagaccgg gcctttgtcc        840
ggcgctccct tggagcctac ctagactcag ccggctctcc acgctttgcc tgaccctgct        900
tgctcaactc tacgtctttg tttcgttttc tgttctgcgc cgttacagat cactagtgca        960
gaagttggtc gtgaggcact gggcaggtaa gtatcaaggt tacaagacag gtttaaggag       1020
accaatagaa actgggcttg tcgagacaga gaagactctt gcgtttctga taggcaccta       1080
ttggtcttac tgacatccac tttgcctttc tctccacag                              1119
```

<210> 29
<211> 2309
<212> DNA
<213> Artificial Sequence

<220>
<223> séquence artificielle

<400> 29

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
```

ggcggcggga cccggagcag gagcggcggc agcagcgact gggggcggcg gcggcgcgtt 660

ggaggcggcc ggatccacta gtgtttaaac agagtaatga catggttcct tccatcctcc 720

aaaggtgacc aataatagtt tgtaagtatc attatgaact aatgaatttt caacatattt 780

gatatatttc aatccattgc catcattgtt cttatcgata tttgagttgg ctcactttgc 840

cagtaagagt ctattcaaat tggcttctga gtccatttga cacaacacct ttgatctttg 900

acagtttcct tggttttagg tgctagatga tttctcaggc tcaccttaga catttcctgc 960

cacagactta gaatcagcca tttctctaag gaccctgatt ccatttcatg agaaatgata 1020

gagaccacaa tcaaaacaag tcatgaattt atactgatat tttcaattca aattaaagat 1080

gaggtttttg ctaaattttt ttgagtttat atttgtatgt cttatgctga aaaatcttgt 1140

ttcctaatta gtaacataat tattcatttg atgggtaaat attttagggc cgattctttg 1200

gttttatagc caagataccc tgttgataaa gtcttgtggg agcaattata agactggctt 1260

attttgaagc tttttaaaaa agacatcctt acctgtttta actgtagatt atattaactt 1320

aaataggtac agcccacgct tgactagtcg ggtttgccgc cagaacacag gtaagtgccg 1380

tgtgtggttc ccgcgggcct ggcctcttta cgggttatgg cccttgcgtg ccttgaatta 1440

cttccacctg gctgcagtac gtgattcttg atcccgagct tcgggttgga agtgggtggg 1500

agagttcgag gccttgcgct taaggagccc cttcgcctcg tgcttgagtt gaggcctggc 1560

ctgggcgctg gggccgccgc gtgcgaatct ggtggcacct tcgcgcctgt ctcgctgctt 1620

tcgataagtc tctagccatt taaaattttt gatgacctgc tgcgacgctt tttttctggc 1680

aagatagtct tgtaaatgcg ggccaagatc tgcacactgg tatttcggtt tttggggccg 1740

cgggcggcga cggggcccgt gcgtcccagc gcacatgttc ggcgaggcgg ggcctgcgag 1800

cgcggccacc gagaatcgga cgggggtagt ctcaagctgg ccggcctgct ctggtgcctg 1860

gcctcgcgcc gccgtgtatc gccccgccct gggcggcaag gctggcccgg tcggcaccag 1920

ttgcgtgagc ggaaagatgg ccgcttcccg gccctgctgc agggagctca aaatggagga 1980

cgcggcgctc gggagagcgg gcgggtgagt cacccacaca aaggaaaagg gcctttccgt 2040

cctcagccgt cgcttcatgt gactccacgg agtaccgggc gccgtccagg cacctcgatt 2100

agttctcgag cttttggagt acgtcgtctt taggttgggg ggaggggttt tatgcgatgg 2160

agtttcccca cactgagtgg gtggagactg aagttaggcc agcttggcac ttgatgtaat 2220

tctccttgga atttgccctt tttgagtttg gatcttggtt cattctcaag cctcagacag 2280

tggttcaaag ttttttctt ccatttcag 2309

<210> 30
<211> 1717
<212> DNA

<213> Artificial Sequence

<220>
<223> séquence artificielle

<400> 30

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc aggggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccacta gtgtttaaac agagtaatga catggttcct tccatcctcc     720
aaaggtgacc aataatagtt tgtaagtatc attatgaact aatgaatttt caacatattt     780
gatatatttc aatccattgc catcattgtt cttatcgata tttgagttgg ctcactttgc     840
cagtaagagt ctattcaaat tggcttctga gtccatttga cacaacacct ttgatctttg     900
acagtttcct tggttttagg tgctagatga tttctcaggc tcaccttaga catttcctgc     960
cacagactta gaatcagcca tttctctaag gaccctgatt ccatttcatg agaaatgata    1020
gagaccacaa tcaaaacaag tcatgaattt atactgatat tttcaattca aattaaagat    1080
gaggtttttg ctaaattttt ttgagtttat atttgtatgt cttatgctga aaaatcttgt    1140
ttcctaatta gtaacataat tattcatttg atgggtaaat attttagggc cgattctttg    1200
gttttatagc caagataccc tgttgataaa gtcttgtggg agcaattata agactggctt    1260
attttgaagc tttttaaaaa agacatcctt acctgtttta actgtagatt atattaactt    1320
aaataggtac agcccacgct tgactagttc agagagcctc ggctaggtag gggatcggga    1380
ctctggcggg agggcggctt ggtgcgtttg cggggatggg cggccgcggc aggccctccg    1440
agcgtggtgg agccgttctg tgagacagcc gggtacgagt cgtgacgctg gaaggggcaa    1500
gcgggtggtg ggcaggaatg cggtccgccc tgcagcaacc ggagggggag ggagaaggga    1560
gcggaaaagt ctccaccgga cgcggccatg gctcgggggg gggggggcag cggaggagcg    1620
cttccggccg acgtctcgtc gctgattggc ttcttttcct cccgccgtgt gtgaaaacac    1680
aattgtacta accttcttct ctttcctctc ctgacag                            1717
```

<210> 31
<211> 1625
<212> DNA

<213> Artificial Sequence

<220>
<223> séquence artificielle

<400> 31

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc cagagtaatg acatggttcc ttccatcctc caaaggtgac caataatagt     720
ttgtaagtat cattatgaac taatgaattt tcaacatatt tgatatattt caatccattg     780
ccatcattgt tcttatcgat atttgagttg gctcactttg ccagtaagag tctattcaaa     840
ttggcttctg agtccatttg acacaacacc tttgatcttt gacagtttcc ttggttttag     900
gtgctagatg atttctcagg ctcaccttag acatttcctg ccacagactt agaatcagcc     960
atttctctaa ggaccctgat tccatttcat gagaaatgat agagaccaca atcaaaacaa    1020
gtcatgaatt tatactgata ttttcaattc aaattaaaga tgaggttttt gctaaatttt    1080
tttgagttta tatttgtatg tcttatgctg aaaaatcttg tttcctaatt agtaacataa    1140
ttattcattt gatgggtaaa tattttaggg ccgattcttt ggttttatag ccaagatacc    1200
ctgttgataa agtcttgtgg gagcaattat aagactggct tattttgaag ctttttaaaa    1260
aagacatcct tacctgtttt aactgtagat tatattaact taaataggta cagcccacgc    1320
ttgactagtc ttcgaggggc tcgcatctct ccttcacgcg cccgccgccc tacctgaggc    1380
cgccatccac gccggttgag tcgcgttctg ccgcctcccg cctgtggtgc ctcctgaact    1440
gcgtccgccg tctaggtaag tttaaagctc aggtcgagac cgggcctttg tccggcgctc    1500
ccttggagcc tacctagact cagccggctc tccacgcttt gcctgaccct gcttgctcaa    1560
ctctacgtct ttgtttcgtt ttctgttctg cgccgttaca gatccaagct gtgaccggcg    1620
```

cctac 1625

<210> 32
<211> 1491
<212> DNA
<213> Artificial Sequence

<220>
<223> séquence artificielle

<400> 32

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc aggggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc cagagtaatg acatggttcc ttccatcctc caaaggtgac caataatagt     720
ttgtaagtat cattatgaac taatgaattt tcaacatatt tgatatattt caatccattg     780
ccatcattgt tcttatcgat atttgagttg gctcactttg ccagtaagag tctattcaaa     840
ttggcttctg agtccatttg acacaacacc tttgatcttt gacagtttcc ttggttttag     900
gtgctagatg atttctcagg ctcaccttag acatttcctg ccacagactt agaatcagcc     960
atttctctaa ggaccctgat tccatttcat gagaaatgat agagaccaca atcaaaacaa    1020
gtcatgaatt tatactgata ttttcaattc aaattaaaga tgaggttttt gctaaatttt    1080
tttgagttta tatttgtatg tcttatgctg aaaaatcttg tttcctaatt agtaacataa    1140
ttattcattt gatgggtaaa tattttaggg ccgattcttt ggttttatag ccaagatacc    1200
ctgttgataa agtcttgtgg gagcaattat aagactggct tattttgaag ctttttaaaa    1260
aagacatcct tacctgtttt aactgtagat tatattaact taaataggta cagcccacgc    1320
ttgactagtg cagaagttgg tcgtgaggca ctgggcaggt aagtatcaag gttacaagac    1380
aggtttaagg agaccaatag aaactgggct tgtcgagaca gagaagactc ttgcgtttct    1440
gataggcacc tattggtctt actgacatcc actttgcctt tctctccaca g              1491
```

<210> 33
<211> 1964
<212> DNA
<213> Artificial Sequence

<220>
<223> séquence artificielle

<400> 33

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aacgctggtg ggtagggatg agggagggag gggcattgtg 720
atgtacaggg ctgctctgtg agatcaaggg tctcttaagg gtgggagctg gggcagggac 780
tacgagagca gccagatggg ctgaaagtgg aactcaaggg gtttctggca cctacctacc 840
tgcttcccgc tggggggtgg ggagttggcc cagagtctta agattggggc agggtggaga 900
ggtgggctct tcctgcttcc cactcatctt atagctttct ttccccagat ccgaattcga 960
gatccaaacc aaggaggaaa ggatatcaca gaggagagct agtcgggttt gccgccagaa 1020
cacaggtaag tgccgtgtgt ggttcccgcg ggcctggcct ctttacgggt tatggccctt 1080
gcgtgccttg aattacttcc acctggctgc agtacgtgat tcttgatccc gagcttcggg 1140
ttggaagtgg gtgggagagt tcgaggcctt gcgcttaagg agccccttcg cctcgtgctt 1200
gagttgaggc ctggcctggg cgctggggcc gccgcgtgcg aatctggtgg caccttcgcg 1260
cctgtctcgc tgctttcgat aagtctctag ccatttaaaa tttttgatga cctgctgcga 1320
cgcttttttt ctggcaagat agtcttgtaa atgcgggcca agatctgcac actggtattt 1380
cggtttttgg ggccgcgggc ggcgacgggg cccgtgcgtc ccagcgcaca tgttcggcga 1440
ggcggggcct gcgagcgcgg ccaccgagaa tcggacgggg gtagtctcaa gctggccggc 1500
ctgctctggt gcctggcctc gcgccgccgt gtatcgcccc gccctgggcg gcaaggctgg 1560
cccggtcggc accagttgcg tgagcggaaa gatggccgct tcccggccct gctgcaggga 1620

```
gctcaaaatg gaggacgcgg cgctcgggag agcgggcggg tgagtcaccc acacaaagga     1680
aaagggcctt tccgtcctca gccgtcgctt catgtgactc cacggagtac cgggcgccgt     1740
ccaggcacct cgattagttc tcgagctttt ggagtacgtc gtctttaggt tggggggagg     1800
ggttttatgc gatggagttt ccccacactg agtgggtgga gactgaagtt aggccagctt     1860
ggcacttgat gtaattctcc ttggaatttg ccctttttga gtttggatct tggttcattc     1920
tcaagcctca gacagtggtt caaagttttt ttcttccatt tcag                      1964
```

<210> 34
<211> 1372
<212> DNA
<213> Artificial Sequence

<220>
<223> séquence artificielle

<400> 34

```
gcgttacata acttacggta aatggcccgc ctggctgacc gcccaacgac ccccgcccat        60
tgacgtcaat aatgacgtat gttcccatag taacgccaat agggactttc cattgacgtc       120
aatgggtgga gtatttacgg taaactgccc acttggcagt acatcaagtg tatcatatgc       180
caagtacgcc ccctattgac gtcaatgacg gtaaatggcc cgcctggcat tatgcccagt       240
acatgacctt atgggacttt cctacttggc agtacatcta cgtattagtc atcgctatta       300
ccatggcccg ggtcgcgaca tgcagcggga cgcgccaccc cgagccccag ctccggcgcc       360
ccggctcccc gcgcccccga tcggggccgc cgctagtagt ggcggcggcg gaggcggggg       420
cagcggcggc ggcggcggag gcgcctctgc agctccggct ccccctggcc tctcgggaac       480
tacaagtccc agggggcctg gcggtgggcg gcgggcggaa gaggcggggt cggcgccgcg       540
aggccggaag tggccgtgga ggcggaagtg gcgcggccgc ggaggggcct ggagtgcggc       600
ggcggcggga cccggagcag gagcggcggc agcagcgact gggggcggcg gcggcgcgtt       660
ggaggcggcc ggatccgttt aacgctggtg ggtagggatg agggagggag gggcattgtg       720
atgtacaggg ctgctctgtg agatcaaggg tctcttaagg gtgggagctg gggcagggac       780
tacgagagca gccagatggg ctgaaagtgg aactcaaggg gtttctggca cctacctacc       840
tgcttcccgc tgggggggtgg ggagttggcc cagagtctta agattggggc agggtggaga       900
ggtgggctct tcctgcttcc cactcatctt atagctttct ttccccagat ccgaattcga       960
gatccaaacc aaggaggaaa ggatatcaca gaggagagct agttcagaga gcctcggcta      1020
ggtaggggat cgggactctg gcgggagggc ggcttggtgc gtttgcgggg atgggcggcc      1080
gcggcaggcc ctccgagcgt ggtggagccg ttctgtgaga cagccgggta cgagtcgtga      1140
cgctggaagg ggcaagcggg tggtgggcag gaatgcggtc cgccctgcag caaccggagg      1200
gggagggaga agggagcgga aaagtctcca ccggacgcgg ccatggctcg gggggggggg      1260
ggcagcggag gagcgcttcc ggccgacgtc tcgtcgctga ttggcttctt ttcctcccgc      1320
cgtgtgtgaa aacacaattg tactaacctt cttctctttc ctctcctgac ag              1372
```

<210> 35
<211> 1302
<212> DNA
<213> Artificial Sequence

<220>
<223> séquence artificielle

<400> 35

```
gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc aggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccacta gttaacgctg gtgggtaggg atgagggagg gaggggcatt 720
gtgatgtaca gggctgctct gtgagatcaa gggtctctta agggtgggag ctggggcagg 780
gactacgaga gcagccagat gggctgaaag tggaactcaa ggggtttctg gcacctacct 840
acctgcttcc cgctgggggg tggggagttg gcccagagtc ttaagattgg ggcagggtgg 900
agaggtgggc tcttcctgct tcccactcat cttatagctt tctttcccca gatccgaatt 960
cgagatccaa accaaggagg aaaggatatc acagaggaga gctagtcttc gaggggctcg 1020
catctctcct tcacgcgccc gccgccctac ctgaggccgc catccacgcc ggttgagtcg 1080
cgttctgccg cctcccgcct gtggtgcctc ctgaactgcg tccgccgtct aggtaagttt 1140
aaagctcagg tcgagaccgg gcctttgtcc ggcgctccct tggagcctac ctagactcag 1200
ccggctctcc acgctttgcc tgaccctgct tgctcaactc tacgtctttg tttcgttttc 1260
tgttctgcgc cgttacagat ccaagctgtg accggcgcct ac 1302
```

<210> 36
<211> 1168
<212> DNA
<213> Artificial Sequence

<220>
<223> séquence artificielle

<400> 36

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct cccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccacta gttaacgctg gtgggtaggg atgagggagg gaggggcatt     720
gtgatgtaca gggctgctct gtgagatcaa gggtctctta agggtgggag ctggggcagg     780
gactacgaga gcagccagat gggctgaaag tggaactcaa ggggtttctg gcacctacct     840
acctgcttcc cgctgggggg tggggagttg gcccagagtc ttaagattgg ggcagggtgg     900
agaggtgggc tcttcctgct tcccactcat cttatagctt tctttcccca gatccgaatt     960
cgagatccaa accaaggagg aaaggatatc acagaggaga gctagtgcag aagttggtcg    1020
tgaggcactg ggcaggtaag tatcaaggtt acaagacagg tttaaggaga ccaatagaaa    1080
ctgggcttgt cgagacagag aagactcttg cgtttctgat aggcacctat tggtcttact    1140
gacatccact ttgcctttct ctccacag                                       1168
```

<210> 37
<211> 2584
<212> DNA
<213> Artificial Sequence

<220>
<223> séquence artificielle

<400> 37

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
```

caagtacgcc ccctattgac gtcaatgacg gtaaatggcc cgcctggcat tatgcccagt 240

acatgacctt atgggacttt cctacttggc agtacatcta cgtattagtc atcgctatta 300

ccatggcccg ggtcgcgaca tgcagcggga cgcgccaccc cgagccccag ctccggcgcc 360

ccggctcccc gcgcccccga tcggggccgc cgctagtagt ggcggcggcg gaggcggggg 420

cagcggcggc ggcggcggag gcgcctctgc agctccggct ccccctggcc tctcgggaac 480

tacaagtccc agggggcctg gcggtgggcg gcgggcggaa gaggcggggt cggcgccgcg 540

aggccggaag tggccgtgga ggcggaagtg gcgcggccgc ggagggggcct ggagtgcggc 600

ggcggcggga cccggagcag gagcggcggc agcagcgact gggggcggcg gcggcgcgtt 660

ggaggcggcc ggatccgttt aaacggctcg catctctcct tcacgcgccc gccgccctac 720

ctgaggccgc catccacgcc ggttgagtcg cgttctgccg cctcccgcct gtggtgcctc 780

ctgaactgcg tccgccgtct aggtaagttt aaagctcagg tcgagaccgg gcctttgtcc 840

ggcgctccct tggagcctac ctagactcag ccggctctcc acgctttgcc tgaccctgct 900

tgctcaactc tacgtctttg tttcgttttc tgttctgcgc cgttacagat cactagtgtt 960

taaacagagt aatgacatgg ttccttccat cctccaaagg tgaccaataa tagtttgtaa 1020

gtatcattat gaactaatga attttcaaca tatttgatat atttcaatcc attgccatca 1080

ttgttcttat cgatatttga gttggctcac tttgccagta agagtctatt caaattggct 1140

tctgagtcca tttgacacaa cacctttgat ctttgacagt ttccttggtt ttaggtgcta 1200

gatgatttct caggctcacc ttagacattt cctgccacag acttagaatc agccatttct 1260

ctaaggaccc tgattccatt tcatgagaaa tgatagagac cacaatcaaa acaagtcatg 1320

aatttatact gatattttca attcaaatta aagatgaggt tttgctaaa ttttttttgag 1380

tttatatttg tatgtcttat gctgaaaaat cttgtttcct aattagtaac ataattattc 1440

atttgatggg taaatatttt agggccgatt ctttggtttt atagccaaga taccctgttg 1500

ataaagtctt gtgggagcaa ttataagact ggcttatttt gaagcttttt aaaaaagaca 1560

tccttacctg ttttaactgt agattatatt aacttaaata ggtacagccc acgcttgact 1620

agtcgggttt gccgccagaa cacaggtaag tgccgtgtgt ggttcccgcg ggcctggcct 1680

ctttacgggt tatggccctt gcgtgccttg aattacttcc acctggctgc agtacgtgat 1740

tcttgatccc gagcttcggg ttggaagtgg gtgggagagt tcgaggcctt gcgcttaagg 1800

agccccttcg cctcgtgctt gagttgaggc ctggcctggg cgctggggcc gccgcgtgcg 1860

aatctggtgg caccttcgcg cctgtctcgc tgctttcgat aagtctctag ccatttaaaa 1920

tttttgatga cctgctgcga cgcttttttt ctggcaagat agtcttgtaa atgcgggcca 1980

agatctgcac actggtattt cggtttttgg ggccgcgggc ggcgacgggg cccgtgcgtc 2040

ccagcgcaca tgttcggcga ggcggggcct gcgagcgcgg ccaccgagaa tcggacgggg 2100

gtagtctcaa gctggccggc ctgctctggt gcctggcctc gcgccgccgt gtatcgcccc 2160
gccctgggcg gcaaggctgg cccggtcggc accagttgcg tgagcggaaa gatggccgct 2220
tcccggccct gctgcaggga gctcaaaatg gaggacgcgg cgctcgggag agcgggcggg 2280
tgagtcaccc acacaaagga aaagggcctt tccgtcctca gccgtcgctt catgtgactc 2340
cacggagtac cgggcgccgt ccaggcacct cgattagttc tcgagctttt ggagtacgtc 2400
gtctttaggt tggggggagg ggttttatgc gatggagttt ccccacactg agtgggtgga 2460
gactgaagtt aggccagctt ggcacttgat gtaattctcc ttggaatttg ccctttttga 2520
gtttggatct tggttcattc tcaagcctca gacagtggtt caaagttttt ttcttccatt 2580
tcag 2584

<210> 38
<211> 1992
<212> DNA
<213> Artificial Sequence

<220>
<223> séquence artificielle

<400> 38

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aaacggctcg catctctcct tcacgcgccc gccgccctac     720
ctgaggccgc catccacgcc ggttgagtcg cgttctgccg cctcccgcct gtggtgcctc     780
ctgaactgcg tccgccgtct aggtaagttt aaagctcagg tcgagaccgg gcctttgtcc     840
ggcgctccct tggagcctac ctagactcag ccggctctcc acgctttgcc tgaccctgct     900
tgctcaactc tacgtctttg tttcgttttc tgttctgcgc cgttacagat cactagtgtt     960
taaacagagt aatgacatgg ttccttccat cctccaaagg tgaccaataa tagtttgtaa    1020
```

```
gtatcattat gaactaatga attttcaaca tatttgatat atttcaatcc attgccatca     1080
ttgttcttat cgatatttga gttggctcac tttgccagta agagtctatt caaattggct     1140
tctgagtcca tttgacacaa cacctttgat ctttgacagt ttccttggtt ttaggtgcta     1200
gatgatttct caggctcacc ttagacattt cctgccacag acttagaatc agccatttct     1260
ctaaggaccc tgattccatt tcatgagaaa tgatagagac cacaatcaaa acaagtcatg     1320
aatttatact gatattttca attcaaatta aagatgaggt ttttgctaaa tttttttgag     1380
tttatatttg tatgtcttat gctgaaaaat cttgtttcct aattagtaac ataattattc     1440
atttgatggg taaatatttt agggccgatt ctttggtttt atagccaaga taccctgttg     1500
ataaagtctt gtgggagcaa ttataagact ggcttatttt gaagcttttt aaaaaagaca     1560
tccttacctg ttttaactgt agattatatt aacttaaata ggtacagccc acgcttgact     1620
agttcagaga gcctcggcta ggtaggggat cgggactctg gcgggagggc ggcttggtgc     1680
gtttgcgggg atgggcggcc gcggcaggcc ctccgagcgt ggtggagccg ttctgtgaga     1740
cagccgggta cgagtcgtga cgctggaagg ggcaagcggg tggtgggcag gaatgcggtc     1800
cgccctgcag caaccggagg gggagggaga agggagcgga aaagtctcca ccggacgcgg     1860
ccatggctcg gggggggggg ggcagcggag gagcgcttcc ggccgacgtc tcgtcgctga     1920
ttggcttctt ttcctcccgc cgtgtgtgaa aacacaattg tactaacctt cttctctttc     1980
ctctcctgac ag                                                         1992
```

<210> 39
<211> 1929
<212> DNA
<213> Artificial Sequence

<220>
<223> séquence artificielle

<400> 39

```
gcgttacata acttacggta aatggcccgc ctggctgacc gcccaacgac ccccgcccat        60
tgacgtcaat aatgacgtat gttcccatag taacgccaat agggactttc cattgacgtc       120
aatgggtgga gtatttacgg taaactgccc acttggcagt acatcaagtg tatcatatgc       180
caagtacgcc ccctattgac gtcaatgacg gtaaatggcc cgcctggcat tatgcccagt       240
acatgacctt atgggacttt cctacttggc agtacatcta cgtattagtc atcgctatta       300
ccatggcccg ggtcgcgaca tgcagcggga cgcgccaccc cgagccccag ctccggcgcc       360
ccggctcccc gcgcccccga tcggggccgc cgctagtagt ggcggcggcg gaggcggggg       420
cagcggcggc ggcggcggag gcgcctctgc agctccggct ccccctggcc tctcgggaac       480
tacaagtccc agggggcctg gcggtgggcg gcgggcggaa gaggcggggt cggcgccgcg       540
aggccggaag tggccgtgga ggcggaagtg gcgcggccgc ggaggggcct ggagtgcggc       600
```

ggcggcggga cccggagcag gagcggcggc agcagcgact gggggcggcg gcggcgcgtt 660

ggaggcggcc ggatccacta ggatccgttt aaacggctcg catctctcct tcacgcgccc 720

gccgccctac ctgaggccgc catccacgcc ggttgagtcg cgttctgccg cctcccgcct 780

gtggtgcctc ctgaactgcg tccgccgtct aggtaagttt aaagctcagg tcgagaccgg 840

gcctttgtcc ggcgctccct tggagcctac ctagactcag ccggctctcc acgctttgcc 900

tgaccctgct tgctcaactc tacgtctttg tttcgttttc tgttctgcgc cgttacagat 960

cactagtgtt taaacagagt aatgacatgg ttccttccat cctccaaagg tgaccaataa 1020

tagtttgtaa gtatcattat gaactaatga attttcaaca tatttgatat atttcaatcc 1080

attgccatca ttgttcttat cgatatttga gttggctcac tttgccagta agagtctatt 1140

caaattggct tctgagtcca tttgacacaa cacctttgat ctttgacagt ttccttggtt 1200

ttaggtgcta gatgatttct caggctcacc ttagacattt cctgccacag acttagaatc 1260

agccatttct ctaaggaccc tgattccatt tcatgagaaa tgatagagac cacaatcaaa 1320

acaagtcatg aatttatact gatattttca attcaaatta aagatgaggt ttttgctaaa 1380

ttttttttgag tttatatttg tatgtcttat gctgaaaaat cttgtttcct aattagtaac 1440

ataattattc atttgatggg taaatatttt agggccgatt ctttggtttt atagccaaga 1500

taccctgttg ataaagtctt gtgggagcaa ttataagact ggcttatttt gaagcttttt 1560

aaaaaagaca tccttacctg ttttaactgt agattatatt aacttaaata ggtacagccc 1620

acgcttgact agtcttcgag ggctcgcat ctctccttca cgcgcccgcc gccctacctg 1680

aggccgccat ccacgccggt tgagtcgcgt tctgccgcct cccgcctgtg gtgcctcctg 1740

aactgcgtcc gccgtctagg taagtttaaa gctcaggtcg agaccgggcc tttgtccggc 1800

gctcccttgg agcctaccta gactcagccg gctctccacg ctttgcctga ccctgcttgc 1860

tcaactctac gtctttgttt cgttttctgt tctgcgccgt tacagatcca agctgtgacc 1920

ggcgcctac 1929

<210> 40
<211> 1785
<212> DNA
<213> Artificial Sequence

<220>
<223> séquence artificielle

<400> 40

gcgttacata acttacggta aatggcccgc ctggctgacc gcccaacgac ccccgcccat 60

tgacgtcaat aatgacgtat gttcccatag taacgccaat agggactttc cattgacgtc 120

aatgggtgga gtatttacgg taaactgccc acttggcagt acatcaagtg tatcatatgc 180

caagtacgcc ccctattgac gtcaatgacg gtaaatggcc cgcctggcat tatgcccagt 240

acatgacctt atgggacttt cctacttggc agtacatcta cgtattagtc atcgctatta 300

ccatggcccg ggtcgcgaca tgcagcggga cgcgccaccc cgagccccag ctccggcgcc 360

ccggctcccc gcgcccccga tcggggccgc cgctagtagt ggcggcggcg gaggcggggg 420

cagcggcggc ggcggcggag gcgcctctgc agctccggct ccccctggcc tctcgggaac 480

tacaagtccc agggggcctg gcggtgggcg gcgggcggaa gaggcggggt cggcgccgcg 540

aggccggaag tggccgtgga ggcggaagtg gcgcggccgc ggaggggcct ggagtgcggc 600

ggcggcggga cccggagcag gagcggcggc agcagcgact gggggcggcg gcggcgcgtt 660

ggaggcggcc ggatccgttt aaacggctcg catctctcct tcacgcgccc gccgccctac 720

ctgaggccgc catccacgcc ggttgagtcg cgttctgccg cctcccgcct gtggtgcctc 780

ctgaactgcg tccgccgtct aggtaagttt aaagctcagg tcgagaccgg gcctttgtcc 840

ggcgctccct tggagcctac ctagactcag ccggctctcc acgctttgcc tgaccctgct 900

tgctcaactc tacgtctttg tttcgttttc tgttctgcgc cgttacagat cactagtgtt 960

taaacagagt aatgacatgg ttccttccat cctccaaagg tgaccaataa tagtttgtaa 1020

gtatcattat gaactaatga attttcaaca tatttgatat atttcaatcc attgccatca 1080

ttgttcttat cgatatttga gttggctcac tttgccagta agagtctatt caaattggct 1140

tctgagtcca tttgacacaa cacctttgat ctttgacagt ttccttggtt ttaggtgcta 1200

gatgatttct caggctcacc ttagacattt cctgccacag acttagaatc agccatttct 1260

ctaaggaccc tgattccatt tcatgagaaa tgatagagac cacaatcaaa acaagtcatg 1320

aatttatact gatattttca attcaaatta aagatgaggt tttgctaaa ttttttttgag 1380

tttatatttg tatgtcttat gctgaaaaat cttgtttcct aattagtaac ataattattc 1440

atttgatggg taaatatttt agggccgatt ctttggtttt atagccaaga taccctgttg 1500

ataaagtctt gtgggagcaa ttataagact ggcttatttt gaagcttttt aaaaaagaca 1560

tccttacctg ttttaactgt agattatatt aacttaaata ggtacagccc acgcttgact 1620

agtgcagaag ttggtcgtga ggcactgggc aggtaagtat caaggttaca agacaggttt 1680

aaggagacca atagaaactg ggcttgtcga gacagagaag actcttgcgt ttctgatagg 1740

cacctattgg tcttactgac atccactttg cctttctctc cacag 1785

<210> 41

<211> 2242
<212> DNA
<213> Artificial Sequence

<220>
<223> séquence artificielle

<400> 41

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagttaa 960
cgctggtggg tagggatgag ggagggaggg gcattgtgat gtacagggct gctctgtgag 1020
atcaagggtc tcttaagggt gggagctggg gcagggacta cgagagcagc cagatgggct 1080
gaaagtggaa ctcaaggggt ttctggcacc tacctacctg cttcccgctg gggggtgggg 1140
agttggccca gagtcttaag attggggcag ggtggagagg tgggctcttc ctgcttccca 1200
ctcatcttat agctttcttt ccccagatcc gaattcgaga tccaaaccaa ggaggaaagg 1260
atatcacaga ggagagctag tcgggtttgc cgccagaaca caggtaagtg ccgtgtgtgg 1320
ttcccgcggg cctggcctct ttacgggtta tggcccttgc gtgccttgaa ttacttccac 1380
ctggctgcag tacgtgattc ttgatcccga gcttcgggtt ggaagtgggt gggagagttc 1440
gaggccttgc gcttaaggag ccccttcgcc tcgtgcttga gttgaggcct ggcctgggcg 1500
ctggggccgc cgcgtgcgaa tctggtggca ccttcgcgcc tgtctcgctg ctttcgataa 1560
gtctctagcc atttaaaatt tttgatgacc tgctgcgacg ctttttttct ggcaagatag 1620
tcttgtaaat gcgggccaag atctgcacac tggtatttcg gtttttgggg ccgcgggcgg 1680
cgacggggcc cgtgcgtccc agcgcacatg ttcggcgagg cggggcctgc gagcgcggcc 1740
accgagaatc ggacgggggt agtctcaagc tggccggcct gctctggtgc ctggcctcgc 1800
gccgccgtgt atcgccccgc cctgggcggc aaggctggcc cggtcggcac cagttgcgtg 1860

```
agcggaaaga tggccgcttc ccggccctgc tgcagggagc tcaaaatgga ggacgcggcg     1920
ctcgggagag cgggcgggtg agtcacccac acaaaggaaa agggcctttc cgtcctcagc     1980
cgtcgcttca tgtgactcca cggagtaccg ggcgccgtcc aggcacctcg attagttctc     2040
gagcttttgg agtacgtcgt ctttaggttg gggggagggg ttttatgcga tggagtttcc     2100
ccacactgag tgggtggaga ctgaagttag gccagcttgg cacttgatgt aattctcctt     2160
ggaatttgcc ctttttgagt ttggatcttg gttcattctc aagcctcaga cagtggttca     2220
aagttttttt cttccatttc ag                                              2242
```

<210> 42
<211> 1650
<212> DNA
<213> Artificial Sequence

<220>
<223> séquence artificielle

<400> 42

```
gcgttacata acttacggta aatggcccgc ctggctgacc gcccaacgac ccccgcccat        60
tgacgtcaat aatgacgtat gttcccatag taacgccaat agggactttc cattgacgtc       120
aatgggtgga gtatttacgg taaactgccc acttggcagt acatcaagtg tatcatatgc       180
caagtacgcc ccctattgac gtcaatgacg gtaaatggcc cgcctggcat tatgcccagt       240
acatgacctt atgggacttt cctacttggc agtacatcta cgtattagtc atcgctatta       300
ccatggcccg ggtcgcgaca tgcagcggga cgcgccaccc cgagccccag ctccggcgcc       360
ccggctcccc gcgcccccga tcggggccgc cgctagtagt ggcggcggcg gaggcggggg       420
cagcggcggc ggcggcggag gcgcctctgc agctccggct ccccctggcc tctcgggaac       480
tacaagtccc aggggggcctg gcggtgggcg gcgggcggaa gaggcggggt cggcgccgcg      540
aggccggaag tggccgtgga ggcggaagtg gcgcggccgc ggaggggcct ggagtgcggc       600
ggcggcggga cccggagcag gagcggcggc agcagcgact gggggcggcg gcggcgcgtt       660
ggaggcggcc ggatccgttt aaacggctcg catctctcct tcacgcgccc gccgccctac       720
ctgaggccgc catccacgcc ggttgagtcg cgttctgccg cctcccgcct gtggtgcctc       780
ctgaactgcg tccgccgtct aggtaagttt aaagctcagg tcgagaccgg gcctttgtcc       840
ggcgctccct tggagcctac ctagactcag ccggctctcc acgctttgcc tgaccctgct       900
tgctcaactc tacgtctttg tttcgttttc tgttctgcgc cgttacagat cactagttaa       960
cgctggtggg tagggatgag ggagggaggg gcattgtgat gtacagggct gctctgtgag      1020
atcaagggtc tcttaagggt gggagctggg gcagggacta cgagagcagc cagatgggct      1080
gaaagtggaa ctcaaggggt ttctggcacc tacctacctg cttcccgctg gggggtgggg      1140
agttggccca gagtcttaag attggggcag ggtggagagg tgggctcttc ctgcttccca      1200

ctcatcttat agctttcttt ccccagatcc gaattcgaga tccaaaccaa ggaggaaagg      1260
atatcacaga ggagagctag ttcagagagc ctcggctagg taggggatcg ggactctggc      1320
gggagggcgg cttggtgcgt ttgcggggat gggcggccgc ggcaggccct ccgagcgtgg      1380
tggagccgtt ctgtgagaca gccgggtacg agtcgtgacg ctggaagggg caagcgggtg      1440
gtgggcagga atgcggtccg ccctgcagca accggagggg gagggagaag ggagcggaaa      1500
agtctccacc ggacgcggcc atggctcggg gggggggggg cagcggagga gcgcttccgg      1560
ccgacgtctc gtcgctgatt ggcttctttt cctcccgccg tgtgtgaaaa cacaattgta      1620
ctaaccttct tctctttcct ctcctgacag                                       1650
```

<210> 43
<211> 1587
<212> DNA
<213> Artificial Sequence

<220>
<223> séquence artificielle

<400> 43

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc aggggggctg gcggtgggcg gcgggcggaa gaggcggggt cggcgccgcg     540
aggccggaag tggccgtgga ggcggaagtg gcgcggccgc ggagggggcct ggagtgcggc     600
ggcggcggga cccggagcag gagcggcggc agcagcgact gggggcggcg gcggcgcgtt     660
ggaggcggcc ggatccacta ggatccgttt aaacggctcg catctctcct tcacgcgccc     720
gccgccctac ctgaggccgc catccacgcc ggttgagtcg cgttctgccg cctcccgcct     780
gtggtgcctc ctgaactgcg tccgccgtct aggtaagttt aaagctcagg tcgagaccgg     840
gcctttgtcc ggcgctccct tggagcctac ctagactcag ccggctctcc acgctttgcc     900
tgaccctgct tgctcaactc tacgtctttg tttcgttttc tgttctgcgc cgttacagat     960
cactagttaa cgctggtggg tagggatgag ggagggaggg gcattgtgat gtacagggct    1020
gctctgtgag atcaagggtc tcttaagggt gggagctggg gcagggacta cgagagcagc    1080
cagatgggct gaaagtggaa ctcaaggggt ttctggcacc tacctacctg cttcccgctg    1140
gggggtgggg agttggccca gagtcttaag attggggcag ggtggagagg tgggctcttc    1200
ctgcttccca ctcatcttat agctttcttt ccccagatcc gaattcgaga tccaaaccaa    1260
ggaggaaagg atatcacaga ggagagctag tcttcgaggg gctcgcatct ctccttcacg    1320
cgcccgccgc cctacctgag gccgccatcc acgccggttg agtcgcgttc tgccgcctcc    1380
cgcctgtggt gcctcctgaa ctgcgtccgc cgtctaggta agtttaaagc tcaggtcgag    1440
accgggcctt tgtccggcgc tcccttggag cctacctaga ctcagccggc tctccacgct    1500
ttgcctgacc ctgcttgctc aactctacgt ctttgtttcg ttttctgttc tgcgccgtta    1560
cagatccaag ctgtgaccgg cgcctac                                        1587
```

<210> 44

<211> 1443
<212> DNA
<213> Artificial Sequence

<220>
<223> séquence artificielle

<400> 44

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aaacggctcg catctctcct tcacgcgccc gccgccctac     720
ctgaggccgc catccacgcc ggttgagtcg cgttctgccg cctcccgcct gtggtgcctc     780
ctgaactgcg tccgccgtct aggtaagttt aaagctcagg tcgagaccgg gcctttgtcc     840
ggcgctccct tggagcctac ctagactcag ccggctctcc acgctttgcc tgaccctgct     900
tgctcaactc tacgtctttg tttcgttttc tgttctgcgc cgttacagat cactagttaa     960
cgctggtggg tagggatgag ggagggaggg gcattgtgat gtacagggct gctctgtgag    1020
atcaagggtc tcttaagggt gggagctggg gcagggacta cgagagcagc cagatgggct    1080
gaaagtggaa ctcaaggggt ttctggcacc tacctacctg cttcccgctg gggggtgggg    1140
agttggccca gagtcttaag attggggcag ggtggagagg tgggctcttc ctgcttccca    1200
ctcatcttat agctttcttt ccccagatcc gaattcgaga tccaaaccaa ggaggaaagg    1260
atatcacaga ggagagctag tgcagaagtt ggtcgtgagg cactgggcag gtaagtatca    1320
aggttacaag acaggtttaa ggagaccaat agaaactggg cttgtcgaga cagagaagac    1380
tcttgcgttt ctgataggca cctattggtc ttactgacat ccactttgcc tttctctcca    1440
cag                                                                  1443
```

<210> 45

<211> 2627
<212> DNA
<213> Artificial Sequence

<220>
<223> séquence artificielle

<400> 45

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggagggggcct ggagtgcggc    600
ggcggcggga cccggagcag gagcggcggc agcagcgact gggggcggcg gcggcgcgtt     660
ggaggcggcc ggatccgttt aaacagagta atgacatggt tccttccatc ctccaaaggt     720
gaccaataat agtttgtaag tatcattatg aactaatgaa ttttcaacat atttgatata     780
tttcaatcca ttgccatcat tgttcttatc gatatttgag ttggctcact ttgccagtaa     840
gagtctattc aaattggctt ctgagtccat ttgacacaac acctttgatc tttgacagtt     900
tccttggttt taggtgctag atgatttctc aggctcacct tagacatttc ctgccacaga     960
cttagaatca gccatttctc taaggaccct gattccattt catgagaaat gatagagacc    1020
acaatcaaaa caagtcatga atttatactg atattttcaa ttcaaattaa agatgaggtt    1080
tttgctaaat ttttttgagt ttatatttgt atgtcttatg ctgaaaaatc ttgtttccta    1140
```

attagtaaca taattattca tttgatgggt aaatatttta gggccgattc tttggtttta 1200

tagccaagat accctgttga taaagtcttg tgggagcaat tataagactg gcttattttg 1260

aagcttttta aaaaagacat ccttacctgt tttaactgta gattatatta acttaaatag 1320

gtacagccca cgcttgacta gttaacgctg gtgggtaggg atgagggagg gaggggcatt 1380

gtgatgtaca gggctgctct gtgagatcaa gggtctctta agggtgggag ctggggcagg 1440

gactacgaga gcagccagat gggctgaaag tggaactcaa ggggtttctg gcacctacct 1500

acctgcttcc cgctgggggg tggggagttg gcccagagtc ttaagattgg ggcagggtgg 1560

agaggtgggc tcttcctgct tcccactcat cttatagctt tctttcccca gatccgaatt 1620

cgagatccaa accaaggagg aaaggatatc acagaggaga gctagtcggg tttgccgcca 1680

gaacacaggt aagtgccgtg tgtggttccc gcgggcctgg cctctttacg ggttatggcc 1740

cttgcgtgcc ttgaattact tccacctggc tgcagtacgt gattcttgat cccgagcttc 1800

gggttggaag tgggtgggag agttcgaggc cttgcgctta aggagcccct tcgcctcgtg 1860

cttgagttga ggcctggcct gggcgctggg gccgccgcgt gcgaatctgg tggcaccttc 1920

gcgcctgtct cgctgctttc gataagtctc tagccattta aaatttttga tgacctgctg 1980

cgacgctttt tttctggcaa gatagtcttg taaatgcggg ccaagatctg cacactggta 2040

tttcggtttt tggggccgcg ggcggcgacg gggcccgtgc gtcccagcgc acatgttcgg 2100

cgaggcgggg cctgcgagcg cggccaccga gaatcggacg gggtagtct caagctggcc 2160

ggcctgctct ggtgcctggc ctcgcgccgc cgtgtatcgc cccgccctgg gcggcaaggc 2220

tggcccggtc ggcaccagtt gcgtgagcgg aaagatggcc gcttcccggc cctgctgcag 2280

ggagctcaaa atggaggacg cggcgctcgg gagagcgggc gggtgagtca cccacacaaa 2340

ggaaaagggc ctttccgtcc tcagccgtcg cttcatgtga ctccacggag taccgggcgc 2400

cgtccaggca cctcgattag ttctcgagct tttggagtac gtcgtcttta ggttgggggg 2460

aggggtttta tgcgatggag tttccccaca ctgagtgggt ggagactgaa gttaggccag 2520

cttggcactt gatgtaattc tccttggaat ttgccctttt tgagtttgga tcttggttca 2580

ttctcaagcc tcagacagtg gttcaaagtt tttttcttcc atttcag 2627

<210> 46
<211> 1946
<212> DNA
<213> Artificial Sequence

<220>
<223> séquence artificielle

<400> 46

gtaacgccaa tagggacttt ccattgacgt caatgggtgg agtatttacg gtaaactgcc 60

cacttggcag tacatcaagt gtatcatatg ccaagtacgc cccctattga cgtcaatgac 120

```
ggtaaatggc ccgcctggca ttatgcccag tacatgacct tatgggactt tcctacttgg      180
cagtacatct acgtattagt catcgctatt accatggccc gggtcgcgac atgcagcggg      240
acgcgccacc ccgagcccca gctccggcgc cccggctccc cgcgcccccg atcggggccg      300
ccgctagtag tggcggcggc ggaggcgggg gcagcggcgg cggcggcgga ggcgcctctg      360
cagctccggc tccccctggc ctctcgggaa ctacaagtcc caggggggcct ggcggtgggc      420
ggcgggcgga agaggcgggg tcggcgccgc gaggccggaa gtggccgtgg aggcggaagt      480
ggcgcggccg cggaggggcc tggagtgcgg cggcggcggg acccggagca ggagcggcgg      540
cagcagcgac tgggggcggc ggcggcgcgt tggaggcggc cggatccgtt taaacagagt      600
aatgacatgg ttccttccat cctccaaagg tgaccaataa tagtttgtaa gtatcattat      660
gaactaatga attttcaaca tatttgatat atttcaatcc attgccatca ttgttcttat      720
cgatatttga gttggctcac tttgccagta agagtctatt caaattggct tctgagtcca      780
tttgacacaa cacctttgat ctttgacagt ttccttggtt ttaggtgcta gatgatttct      840
caggctcacc ttagacattt cctgccacag acttagaatc agccatttct ctaaggaccc      900
tgattccatt tcatgagaaa tgatagagac cacaatcaaa acaagtcatg aatttatact      960
gatattttca attcaaatta aagatgaggt ttttgctaaa tttttttgag tttatatttg     1020
tatgtcttat gctgaaaaat cttgtttcct aattagtaac ataattattc atttgatggg     1080
taaatatttt agggccgatt ctttggtttt atagccaaga taccctgttg ataaagtctt     1140
gtgggagcaa ttataagact ggcttatttt gaagcttttt aaaaaagaca tccttacctg     1200
ttttaactgt agattatatt aacttaaata ggtacagccc acgcttgact agttaacgct     1260
ggtgggtagg gatgagggag ggaggggcat tgtgatgtac agggctgctc tgtgagatca     1320
agggtctctt aagggtggga gctggggcag ggactacgag agcagccaga tgggctgaaa     1380
gtggaactca aggggtttct ggcacctacc tacctgcttc ccgctggggg gtggggagtt     1440
ggcccagagt cttaagattg gggcagggtg gagaggtggg ctcttcctgc ttcccactca     1500
tcttatagct ttctttcccc agatccgaat tcgagatcca aaccaaggag gaaaggatat     1560
cacagaggag agctagttca gagagcctcg gctaggtagg ggatcgggac tctggcggga     1620
gggcggcttg gtgcgtttgc ggggatgggc ggccgcggca ggccctccga gcgtggtgga     1680
gccgttctgt gagacagccg ggtacgagtc gtgacgctgg aaggggcaag cgggtggtgg     1740
gcaggaatgc ggtccgccct gcagcaaccg gagggggagg gagaagggag cggaaaagtc     1800
tccaccggac gcggccatgg ctcggggggg gggggcagc ggaggagcgc ttccggccga      1860
cgtctcgtcg ctgattggct tcttttcctc ccgccgtgtg tgaaaacaca attgtactaa     1920
ccttcttctc tttcctctcc tgacag                                          1946
```

<210> 47
<211> 1968
<212> DNA
<213> Artificial Sequence

<220>
<223> séquence artificielle

<400> 47

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccacta gtgtttaaac agagtaatga catggttcct tccatcctcc 720
aaaggtgacc aataatagtt tgtaagtatc attatgaact aatgaatttt caacatattt 780
gatatatttc aatccattgc catcattgtt cttatcgata tttgagttgg ctcactttgc 840
cagtaagagt ctattcaaat tggcttctga gtccatttga cacaacacct ttgatctttg 900
acagtttcct tggttttagg tgctagatga tttctcaggc tcaccttaga catttcctgc 960
cacagactta gaatcagcca tttctctaag gaccctgatt ccatttcatg agaaatgata 1020
gagaccacaa tcaaaacaag tcatgaattt atactgatat tttcaattca aattaaagat 1080
gaggtttttg ctaaattttt ttgagtttat atttgtatgt cttatgctga aaaatcttgt 1140
ttcctaatta gtaacataat tattcatttg atgggtaaat attttagggc cgattctttg 1200
gttttatagc caagataccc tgttgataaa gtcttgtggg agcaattata agactggctt 1260
attttgaagc tttttaaaaa agacatcctt acctgtttta actgtagatt atattaactt 1320
aaataggtac agcccacgct tgactagtta acgctggtgg gtagggatga gggagggagg 1380
ggcattgtga tgtacagggc tgctctgtga gatcaagggt ctcttaaggg tgggagctgg 1440
ggcagggact acgagagcag ccagatgggc tgaaagtgga actcaagggg tttctggcac 1500
ctacctacct gcttcccgct gggggtggg gagttggccc agagtcttaa gattggggca 1560
gggtggagag gtgggctctt cctgcttccc actcatctta tagctttctt tccccagatc 1620

cgaattcgag atccaaacca aggaggaaag gatatcacag aggagagcta gtcttcgagg 1680

ggctcgcatc tctccttcac gcgcccgccg ccctacctga ggccgccatc cacgccggtt 1740

gagtcgcgtt ctgccgcctc ccgcctgtgg tgcctcctga actgcgtccg ccgtctaggt 1800

aagtttaaag ctcaggtcga gaccgggcct ttgtccggcg ctcccttgga gcctacctag 1860

actcagccgg ctctccacgc tttgcctgac cctgcttgct caactctacg tctttgtttc 1920

gttttctgtt ctgcgccgtt acagatccaa gctgtgaccg gcgcctac 1968

<210> 48
<211> 1834
<212> DNA
<213> Artificial Sequence

<220>
<223> séquence artificielle

<400> 48

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccacta gtgtttaaac agagtaatga catggttcct tccatcctcc     720
aaaggtgacc aataatagtt tgtaagtatc attatgaact aatgaatttt caacatattt     780
gatatatttc aatccattgc catcattgtt cttatcgata tttgagttgg ctcactttgc     840
cagtaagagt ctattcaaat tggcttctga gtccatttga cacaacacct ttgatctttg     900
acagtttcct tggttttagg tgctagatga tttctcaggc tcaccttaga catttcctgc     960
cacagactta gaatcagcca tttctctaag gaccctgatt ccatttcatg agaaatgata    1020
gagaccacaa tcaaaacaag tcatgaattt atactgatat tttcaattca aattaaagat    1080
gaggtttttg ctaaattttt ttgagtttat atttgtatgt cttatgctga aaaatcttgt    1140
ttcctaatta gtaacataat tattcatttg atgggtaaat attttagggc cgattctttg    1200
gttttatagc caagataccc tgttgataaa gtcttgtggg agcaattata agactggctt    1260
attttgaagc tttttaaaaa agacatcctt acctgtttta actgtagatt atattaactt    1320
aaataggtac agcccacgct tgactagtta acgctggtgg gtagggatga gggagggagg    1380
ggcattgtga tgtacagggc tgctctgtga gatcaagggt ctcttaaggg tgggagctgg    1440
ggcagggact acgagagcag ccagatgggc tgaaagtgga actcaagggg tttctggcac    1500
ctacctacct gcttcccgct ggggggtggg gagttggccc agagtcttaa gattggggca    1560
gggtggagag gtgggctctt cctgcttccc actcatctta tagctttctt tccccagatc    1620
cgaattcgag atccaaacca aggaggaaag gatatcacag aggagagcta gtgcagaagt    1680
tggtcgtgag gcactgggca ggtaagtatc aaggttacaa gacaggttta aggagaccaa    1740
tagaaactgg gcttgtcgag acagagaaga ctcttgcgtt tctgataggc acctattggt    1800
cttactgaca tccactttgc ctttctctcc acag                                1834
```

<210> 49
<211> 2908
<212> DNA
<213> Artificial Sequence

<220>
<223> séquence artificielle

<400> 49

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aaacggctcg catctctcct tcacgcgccc gccgccctac     720
ctgaggccgc catccacgcc ggttgagtcg cgttctgccg cctcccgcct gtggtgcctc     780
ctgaactgcg tccgccgtct aggtaagttt aaagctcagg tcgagaccgg gcctttgtcc     840
ggcgctccct tggagcctac ctagactcag ccggctctcc acgctttgcc tgaccctgct     900
tgctcaactc tacgtctttg tttcgttttc tgttctgcgc cgttacagat cactagtgtt     960
```

taaacagagt aatgacatgg ttccttccat cctccaaagg tgaccaataa tagtttgtaa 1020
gtatcattat gaactaatga attttcaaca tatttgatat atttcaatcc attgccatca 1080
ttgttcttat cgatatttga gttggctcac tttgccagta agagtctatt caaattggct 1140
tctgagtcca tttgacacaa cacctttgat ctttgacagt ttccttggtt ttaggtgcta 1200
gatgatttct caggctcacc ttagacattt cctgccacag acttagaatc agccatttct 1260
ctaaggaccc tgattccatt tcatgagaaa tgatagagac cacaatcaaa acaagtcatg 1320
aatttatact gatattttca attcaaatta aagatgaggt ttttgctaaa ttttttttgag 1380
tttatatttg tatgtcttat gctgaaaaat cttgtttcct aattagtaac ataattattc 1440
atttgatggg taaatatttt agggccgatt ctttggtttt atagccaaga taccctgttg 1500
ataaagtctt gtgggagcaa ttataagact ggcttatttt gaagcttttt aaaaaagaca 1560
tccttacctg ttttaactgt agattatatt aacttaaata ggtacagccc acgcttgact 1620
agttaacgct ggtgggtagg gatgagggag ggaggggcat tgtgatgtac agggctgctc 1680
tgtgagatca agggtctctt aagggtggga gctggggcag ggactacgag agcagccaga 1740
tgggctgaaa gtggaactca aggggtttct ggcacctacc tacctgcttc ccgctggggg 1800
gtggggagtt ggcccagagt cttaagattg gggcagggtg gagaggtggg ctcttcctgc 1860
ttcccactca tcttatagct ttctttcccc agatccgaat tcgagatcca aaccaaggag 1920
gaaaggatat cacagaggag agctagtcgg gtttgccgcc agaacacagg taagtgccgt 1980
gtgtggttcc cgcgggcctg gcctctttac gggttatggc ccttgcgtgc cttgaattac 2040
ttccacctgg ctgcagtacg tgattcttga tcccgagctt cgggttggaa gtgggtggga 2100
gagttcgagg ccttgcgctt aaggagcccc ttcgcctcgt gcttgagttg aggcctggcc 2160
tgggcgctgg ggccgccgcg tgcgaatctg gtggcacctt cgcgcctgtc tcgctgcttt 2220
cgataagtct ctagccattt aaaatttttg atgacctgct gcgacgcttt ttttctggca 2280
agatagtctt gtaaatgcgg gccaagatct gcacactggt atttcggttt ttggggccgc 2340
gggcggcgac ggggcccgtg cgtcccagcg cacatgttcg gcgaggcggg gcctgcgagc 2400
gcggccaccg agaatcggac gggggtagtc tcaagctggc cggcctgctc tggtgcctgg 2460
cctcgcgccg ccgtgtatcg ccccgccctg ggcggcaagg ctggcccggt cggcaccagt 2520
tgcgtgagcg gaaagatggc cgcttcccgg ccctgctgca gggagctcaa aatggaggac 2580
gcggcgctcg ggagagcggg cgggtgagtc acccacacaa aggaaaaggg cctttccgtc 2640
ctcagccgtc gcttcatgtg actccacgga gtaccgggcg ccgtccaggc acctcgatta 2700
gttctcgagc ttttggagta cgtcgtcttt aggttggggg gaggggtttt atgcgatgga 2760
gtttccccac actgagtggg tggagactga agttaggcca gcttggcact tgatgtaatt 2820

```
ctccttggaa tttgcccttt ttgagtttgg atcttggttc attctcaagc ctcagacagt     2880

ggttcaaagt tttttcttc catttcag                                         2908
```

<210> 50
<211> 2316
<212> DNA
<213> Artificial Sequence

<220>
<223> séquence artificielle

<400> 50

gcgttacata acttacggta aatggcccgc ctggctgacc gcccaacgac ccccgcccat 60

tgacgtcaat aatgacgtat gttcccatag taacgccaat agggactttc cattgacgtc 120

aatgggtgga gtatttacgg taaactgccc acttggcagt acatcaagtg tatcatatgc 180

caagtacgcc ccctattgac gtcaatgacg gtaaatggcc cgcctggcat tatgcccagt 240

acatgacctt atgggacttt cctacttggc agtacatcta cgtattagtc atcgctatta 300

ccatggcccg ggtcgcgaca tgcagcggga cgcgccaccc cgagccccag ctccggcgcc 360

ccggctcccc gcgcccccga tcggggccgc cgctagtagt ggcggcggcg gaggcggggg 420

cagcggcggc ggcggcggag gcgcctctgc agctccggct ccccctggcc tctcgggaac 480

tacaagtccc agggggcctg gcggtgggcg gcgggcggaa gaggcggggt cggcgccgcg 540

aggccggaag tggccgtgga ggcggaagtg gcgcggccgc ggaggggcct ggagtgcggc 600

ggcggcggga cccggagcag gagcggcggc agcagcgact gggggcggcg gcggcgcgtt 660

ggaggcggcc ggatccgttt aaacggctcg catctctcct tcacgcgccc gccgccctac 720

ctgaggccgc catccacgcc ggttgagtcg cgttctgccg cctcccgcct gtggtgcctc 780

ctgaactgcg tccgccgtct aggtaagttt aaagctcagg tcgagaccgg gcctttgtcc 840

ggcgctccct tggagcctac ctagactcag ccggctctcc acgctttgcc tgaccctgct 900

tgctcaactc tacgtctttg tttcgttttc tgttctgcgc cgttacagat cactagtgtt 960

taaacagagt aatgacatgg ttccttccat cctccaaagg tgaccaataa tagtttgtaa 1020

gtatcattat gaactaatga attttcaaca tatttgatat atttcaatcc attgccatca 1080

ttgttcttat cgatatttga gttggctcac tttgccagta agagtctatt caaattggct 1140

tctgagtcca tttgacacaa cacctttgat ctttgacagt ttccttggtt ttaggtgcta 1200

gatgatttct caggctcacc ttagacattt cctgccacag acttagaatc agccatttct 1260

ctaaggaccc tgattccatt tcatgagaaa tgatagagac cacaatcaaa acaagtcatg 1320

aatttatact gatattttca attcaaatta aagatgaggt tttgctaaa ttttttgag 1380

tttatatttg tatgtcttat gctgaaaaat cttgtttcct aattagtaac ataattattc 1440

atttgatggg taaatatttt agggccgatt ctttggtttt atagccaaga taccctgttg 1500

```
ataaagtctt gtgggagcaa ttataagact ggcttatttt gaagcttttt aaaaaagaca     1560
tccttacctg ttttaactgt agattatatt aacttaaata ggtacagccc acgcttgact     1620
agttaacgct ggtgggtagg gatgagggag ggaggggcat tgtgatgtac agggctgctc     1680
tgtgagatca agggtctctt aagggtggga gctggggcag ggactacgag agcagccaga     1740
tgggctgaaa gtggaactca aggggtttct ggcacctacc tacctgcttc ccgctggggg     1800
gtggggagtt ggcccagagt cttaagattg gggcagggtg gagaggtggg ctcttcctgc     1860
ttcccactca tcttatagct ttctttcccc agatccgaat tcgagatcca aaccaaggag     1920
gaaaggatat cacagaggag agctagttca gagagcctcg gctaggtagg ggatcgggac     1980
tctggcggga gggcggcttg gtgcgtttgc ggggatgggc ggccgcggca ggccctccga     2040
gcgtggtgga gccgttctgt gagacagccg ggtacgagtc gtgacgctgg aaggggcaag     2100
cgggtggtgg gcaggaatgc ggtccgccct gcagcaaccg gagggggagg gagaagggag     2160
cggaaaagtc tccaccggac gcggccatgg ctcggggggg ggggggcagc ggaggagcgc     2220
ttccggccga cgtctcgtcg ctgattggct tcttttcctc ccgccgtgtg tgaaaacaca     2280
attgtactaa ccttcttctc tttcctctcc tgacag                               2316
```

<210> 51
<211> 2253
<212> DNA
<213> Artificial Sequence

<220>
<223> séquence artificielle

<400> 51

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccacta ggatccgttt aaacggctcg catctctcct tcacgcgccc     720
```

gccgccctac ctgaggccgc catccacgcc ggttgagtcg cgttctgccg cctcccgcct 780
gtggtgcctc ctgaactgcg tccgccgtct aggtaagttt aaagctcagg tcgagaccgg 840
gcctttgtcc ggcgctccct tggagcctac ctagactcag ccggctctcc acgctttgcc 900
tgaccctgct tgctcaactc tacgtctttg tttcgttttc tgttctgcgc cgttacagat 960
cactagtgtt taaacagagt aatgacatgg ttccttccat cctccaaagg tgaccaataa 1020
tagtttgtaa gtatcattat gaactaatga attttcaaca tatttgatat atttcaatcc 1080
attgccatca ttgttcttat cgatatttga gttggctcac tttgccagta agagtctatt 1140
caaattggct tctgagtcca tttgacacaa cacctttgat ctttgacagt ttccttggtt 1200
ttaggtgcta gatgatttct caggctcacc ttagacattt cctgccacag acttagaatc 1260
agccatttct ctaaggaccc tgattccatt tcatgagaaa tgatagagac cacaatcaaa 1320
acaagtcatg aatttatact gatattttca attcaaatta aagatgaggt ttttgctaaa 1380
ttttttgag tttatatttg tatgtcttat gctgaaaaat cttgtttcct aattagtaac 1440
ataattattc atttgatggg taaatatttt agggccgatt ctttggtttt atagccaaga 1500
taccctgttg ataaagtctt gtgggagcaa ttataagact ggcttatttt gaagcttttt 1560
aaaaaagaca tccttacctg ttttaactgt agattatatt aacttaaata ggtacagccc 1620
acgcttgact agttaacgct ggtgggtagg gatgagggag ggaggggcat tgtgatgtac 1680
agggctgctc tgtgagatca agggtctctt aagggtggga gctggggcag ggactacgag 1740
agcagccaga tgggctgaaa gtggaactca aggggtttct ggcacctacc tacctgcttc 1800
ccgctggggg gtggggagtt ggcccagagt cttaagattg ggcagggtg gagaggtggg 1860
ctcttcctgc ttcccactca tcttatagct ttctttcccc agatccgaat tcgagatcca 1920
aaccaaggag gaaaggatat cacagaggag agctagtctt cgaggggctc gcatctctcc 1980
ttcacgcgcc cgccgcccta cctgaggccg ccatccacgc cggttgagtc gcgttctgcc 2040
gcctcccgcc tgtggtgcct cctgaactgc gtccgccgtc taggtaagtt taaagctcag 2100
gtcgagaccg ggcctttgtc cggcgctccc ttggagccta cctagactca gccggctctc 2160
cacgctttgc ctgaccctgc ttgctcaact ctacgtcttt gtttcgtttt ctgttctgcg 2220
ccgttacaga tccaagctgt gaccggcgcc tac 2253

<210> 52
<211> 2109
<212> DNA
<213> Artificial Sequence

<220>
<223> UBC

<400> 52

gcgttacata acttacggta aatggcccgc ctggctgacc gcccaacgac ccccgcccat 60

```
tgacgtcaat aatgacgtat gttcccatag taacgccaat agggactttc cattgacgtc      120
aatgggtgga gtatttacgg taaactgccc acttggcagt acatcaagtg tatcatatgc      180
caagtacgcc ccctattgac gtcaatgacg gtaaatggcc cgcctggcat tatgcccagt      240
acatgacctt atgggacttt cctacttggc agtacatcta cgtattagtc atcgctatta      300
ccatggcccg ggtcgcgaca tgcagcggga cgcgccaccc cgagccccag ctccggcgcc      360
ccggctcccc gcgcccccga tcggggccgc cgctagtagt ggcggcggcg gaggcggggg      420
cagcggcggc ggcggcggag gcgcctctgc agctccggct ccccctggcc tctcgggaac      480
tacaagtccc aggggcctg gcggtgggcg gcgggcggaa gaggcggggt cggcgccgcg       540
aggccggaag tggccgtgga ggcggaagtg gcgcggccgc ggaggggcct ggagtgcggc      600
ggcggcggga cccggagcag gagcggcggc agcagcgact gggggcggcg gcggcgcgtt      660
ggaggcggcc ggatccgttt aaacggctcg catctctcct tcacgcgccc gccgccctac      720
ctgaggccgc catccacgcc ggttgagtcg cgttctgccg cctcccgcct gtggtgcctc      780
ctgaactgcg tccgccgtct aggtaagttt aaagctcagg tcgagaccgg gcctttgtcc      840
ggcgctccct tggagcctac ctagactcag ccggctctcc acgctttgcc tgaccctgct      900
tgctcaactc tacgtctttg tttcgttttc tgttctgcgc cgttacagat cactagtgtt      960
taaacagagt aatgacatgg ttccttccat cctccaaagg tgaccaataa tagtttgtaa     1020
gtatcattat gaactaatga attttcaaca tatttgatat atttcaatcc attgccatca     1080
ttgttcttat cgatatttga gttggctcac tttgccagta agagtctatt caaattggct     1140
tctgagtcca tttgacacaa cacctttgat ctttgacagt ttccttggtt ttaggtgcta     1200
gatgatttct caggctcacc ttagacattt cctgccacag acttagaatc agccatttct     1260
ctaaggaccc tgattccatt tcatgagaaa tgatagagac cacaatcaaa acaagtcatg     1320
aatttatact gatattttca attcaaatta aagatgaggt ttttgctaaa tttttttgag     1380
tttatatttg tatgtcttat gctgaaaaat cttgtttcct aattagtaac ataattattc     1440
atttgatggg taaatatttt agggccgatt ctttggtttt atagccaaga taccctgttg     1500
ataaagtctt gtgggagcaa ttataagact ggcttatttt gaagcttttt aaaaaagaca     1560
tccttacctg ttttaactgt agattatatt aacttaaata ggtacagccc acgcttgact     1620
agttaacgct ggtgggtagg gatgagggag ggaggggcat tgtgatgtac agggctgctc     1680
tgtgagatca agggtctctt aagggtggga gctggggcag ggactacgag agcagccaga     1740
tgggctgaaa gtggaactca aggggtttct ggcacctacc tacctgcttc ccgctggggg     1800
gtggggagtt ggcccagagt cttaagattg ggcagggtg gagaggtggg ctcttcctgc      1860
ttcccactca tcttatagct ttctttcccc agatccgaat tcgagatcca aaccaaggag     1920
```

gaaaggatat cacagaggag agctagtgca gaagttggtc gtgaggcact gggcaggtaa 1980

gtatcaaggt tacaagacag gtttaaggag accaatagaa actgggcttg tcgagacaga 2040

gaagactctt gcgtttctga taggcaccta ttggtcttac tgacatccac tttgcctttc 2100

tctccacag 2109

<210> 53
<211> 812
<212> DNA
<213> Artificial Sequence

<220>
<223> séquence artificielle

<400> 53

gtgagtagcg ggctgctggg ctggccgggg ctttcgtggc cgccgggccg ctcggtggga 60

cggaagcgtg tggagagacc gccaagggct gtagtctggg tccgcgagca aggttgccct 120

gaactggggg ttgggggag cgcagcaaaa tggcggctgt tcccgagtct tgaatggaag 180

acgcttgtga ggcgggctgt gaggtcgttg aaacaaggtg gggggcatgg tgggcggcaa 240

gaacccaagg tcttgaggcc ttcgctaatg cgggaaagct cttattcggg tgagatgggc 300

tggggcacca tctggggacc ctgacgtgaa gtttgtcact gactggagaa ctcggtttgt 360

cgtctgttgc gggggcggca gttatggcgg tgccgttggg cagtgcaccc gtacctttgg 420

gagcgcgcgc cctcgtcgtg tcgtgacgtc acccgttctg ttggcttata atgcagggtg 480

gggccacctg ccggtaggtg tgcggtaggc ttttctccgt cgcaggacgc agggttcggg 540

cctagggtag gctctcctga atcgacaggc gccggacctc tggtgagggg agggataagt 600

gaggcgtcag tttctttggt cggttttatg tacctatctt cttaagtagc tgaagctccg 660

gttttgaact atgcgctcgg ggttggcgag tgtgttttgt gaagtttttt aggcaccttt 720

tgaaatgtaa tcatttgggt caatatgtaa ttttcagtgt tagactagta aattgtccgc 780

taaattctgg ccgtttttgg cttttttgtt ag 812

<210> 54
<211> 1217
<212> DNA
<213> Artificial Sequence

<220>
<223> séquence artificielle

<400> 54

```
gtaggggagc ggaactctgg tgggagggga ggtgcggtgc actgggggga tgggtggcta        60
ggggggccgt ctggtggctt gcgggggttg cctttcccgt gggaagtcgg gaacataatg       120
tttgttacgt tgggagggaa aggggtggct ggatgcaggc gggagggagg cccgccctgc       180
ggcaaccgga gggggaggga gaagggagcg gaaaatgctc gaaaccggac ggagccattg       240

ctctcgcaga gggaggagcg cttccggcta gcctcttgtc gccgattggc cgtttctcct       300
cccgccgtgt gtgaaaacac aaatggcgta ttctggttgg agtaaagctc ctgtcagtta       360
caccgtcggg agtacgcagc cgcttagcga ctctcgcgtt gccccctggg tggggcgggt       420
aggtaggtgg ggtgtagaga tgctgggtgt gcgggcgcgg ccggcctcct gcggcgggag       480
gggagggtca gtgaaattgg ctctggcgcg ggcgtcctcc caccctcccc ttccttcggg       540
ggagtcggtt tacccgccgc ctgcttgtct tcgacacctg attggctgtc gaagctgtgg       600
gaccgggccc ttgctactgg ctcgagtctc acatgagcga aaccactgcg cggggcgcgg       660
gggtggcggg gaggcgggcg ttggtacggt cctccccgag gccgagcgcc gcagtgtctg       720
gccccgcgcc cctgcgcaac gtggcaggaa gcgcgcgctg gaggcggggg cgggctgccg       780
gccgagactt ctggatggcg gcggccgcgg ctccgccccg ggttcccacc gcctgaaggg       840
cgagacaagc ccgacctgct acaggcactc gtgggggtgg gggaggagcg ggggtcggtc       900
cggctggttt gtgggtggga ggcgcttgtt ctccaaaaac cggcgcgagc tgcaatcctg       960
agggagctgc ggtggaggag gtggagagaa ggccgcaccc ttctgggcag ggggagggga      1020
gtgccgcaat acctttatgg gagttctttg ctgcctcccg tcttgtaagg accgccctgg      1080
gcctggaaga agccctccct cctttcctcc tcgcgtgatc tcgtcatcgc ctccatgtcg      1140
agtcgcttct cgattatggg cgggattctt ttgcctagac aattgtacta accttcttct      1200
ctttcctctc ctgacag                                                     1217
```

<210> 55
<211> 1494
<212> DNA
<213> Artificial Sequence

<220>
<223> séquence artificielle

<400> 55

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc aggggggcctg gcggtgggcg gcgggcggaa gaggcggggt cggcgccgcg 540

aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccacta gcgtgagtag cgggctgctg ggctggccgg ggctttcgtg 720
gccgccgggc cgctcggtgg gacggaagcg tgtggagaga ccgccaaggg ctgtagtctg 780
ggtccgcgag caaggttgcc ctgaactggg ggttgggggg agcgcagcaa aatggcggct 840
gttcccgagt cttgaatgga agacgcttgt gaggcgggct gtgaggtcgt tgaaacaagg 900
tgggggggcat ggtgggcggc aagaacccaa ggtcttgagg ccttcgctaa tgcgggaaag 960
ctcttattcg ggtgagatgg gctggggcac catctgggga ccctgacgtg aagtttgtca 1020
ctgactggag aactcggttt gtcgtctgtt gcgggggcgg cagttatggc ggtgccgttg 1080
ggcagtgcac ccgtaccttt gggagcgcgc gccctcgtcg tgtcgtgacg tcacccgttc 1140
tgttggctta taatgcaggg tggggccacc tgccggtagg tgtgcggtag gcttttctcc 1200
gtcgcaggac gcagggttcg ggcctagggt aggctctcct gaatcgacag gcgccggacc 1260
tctggtgagg ggagggataa gtgaggcgtc agtttctttg gtcggtttta tgtacctatc 1320
ttcttaagta gctgaagctc cggttttgaa ctatgcgctc ggggttggcg agtgtgtttt 1380
gtgaagtttt ttaggcacct tttgaaatgt aatcatttgg gtcaatatgt aattttcagt 1440
gttagactag taaattgtcc gctaaattct ggccgttttt ggcttttttg ttag 1494

<210> 56
<211> 1918
<212> DNA
<213> Artificial Sequence

<220>
<223> séquence artificielle

<400> 56

gcgttacata acttacggta aatggcccgc ctggctgacc gcccaacgac ccccgcccat 60

tgacgtcaat aatgacgtat gttcccatag taacgccaat agggactttc cattgacgtc 120

aatgggtgga gtatttacgg taaactgccc acttggcagt acatcaagtg tatcatatgc 180

caagtacgcc ccctattgac gtcaatgacg gtaaatggcc cgcctggcat tatgcccagt 240

acatgacctt atgggacttt cctacttggc agtacatcta cgtattagtc atcgctatta 300

ccatggcccg ggtcgcgaca tgcagcggga cgcgccaccc cgagccccag ctccggcgcc 360

ccggctcccc gcgcccccga tcggggccgc cgctagtagt ggcggcggcg gaggcggggg 420

cagcggcggc ggcggcggag gcgcctctgc agctccggct ccccctggcc tctcgggaac 480

tacaagtccc aggggggcctg gcggtgggcg gcgggcggaa gaggcggggt cggcgccgcg 540

aggccggaag tggccgtgga ggcggaagtg gcgcggccgc ggaggggcct ggagtgcggc 600

ggcggcggga cccggagcag gagcggcggc agcagcgact gggggcggcg gcggcgcgtt 660

```
ggaggcggcc ggatccacta gtctcagaga gcctcggcta ggtaggggag cggaactctg      720
gtgggagggg aggtgcggtg cactgggggg atgggtggct agggggggccg tctggtggct      780
tgcgggggtt gcctttcccg tgggaagtcg ggaacataat gtttgttacg ttgggaggga      840
aaggggtggc tggatgcagg cgggagggag gcccgccctg cggcaaccgg agggggaggg      900
agaagggagc ggaaaatgct cgaaaccgga cggagccatt gctctcgcag agggaggagc      960
gcttccggct agcctcttgt cgccgattgg ccgtttctcc tcccgccgtg tgtgaaaaca     1020
caaatggcgt attctggttg gagtaaagct cctgtcagtt acaccgtcgg gagtacgcag     1080
ccgcttagcg actctcgcgt tgccccctgg gtggggcggg taggtaggtg ggtgtagag      1140
atgctgggtg tgcgggcgcg gccggcctcc tgcggcggga ggggagggtc agtgaaattg     1200
gctctggcgc gggcgtcctc ccaccctccc cttccttcgg gggagtcggt ttacccgccg     1260
cctgcttgtc ttcgacacct gattggctgt cgaagctgtg ggaccgggcc cttgctactg     1320
gctcgagtct cacatgagcg aaaccactgc gcggggcgcg ggggtggcgg ggaggcgggc     1380
gttggtacgg tcctccccga ggccgagcgc cgcagtgtct ggccccgcgc ccctgcgcaa     1440
cgtggcagga agcgcgcgct ggaggcgggg gcgggctgcc ggccgagact tctggatggc     1500
ggcggccgcg gctccgcccc gggttcccac cgcctgaagg gcgagacaag cccgacctgc     1560
tacaggcact cgtgggggtg ggggaggagc gggggtcggt ccggctggtt tgtgggtggg     1620
aggcgcttgt tctccaaaaa ccggcgcgag ctgcaatcct gagggagctg cggtggagga     1680
ggtggagaga aggccgcacc cttctgggca gggggagggg agtgccgcaa tacctttatg     1740
ggagttcttt gctgcctccc gtcttgtaag gaccgccctg ggcctggaag aagccctccc     1800
tcctttcctc ctcgcgtgat ctcgtcatcg cctccatgtc gagtcgcttc tcgattatgg     1860
gcgggattct tttgcctaga caattgtact aaccttcttc tctttcctct cctgacag       1918
```

<210> 57
<211> 1779
<212> DNA
<213> Artificial Sequence

<220>
<223> séquence artificielle

<400> 57

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct cccccctggcc tctcgggaac    480
tacaagtccc agggggcctg gcggtgggcg gcgggcggaa gaggcggggt cggcgccgcg     540
aggccggaag tggccgtgga ggcggaagtg gcgcggccgc ggagggccct ggagtgcggc     600
ggcggcggga cccggagcag gagcggcggc agcagcgact gggggcggcg gcggcgcgtt     660
ggaggcggcc ggatccgttt aaacggctcg catctctcct tcacgcgccc gccgccctac     720
ctgaggccgc catccacgcc ggttgagtcg cgttctgccg cctcccgcct gtggtgcctc     780
ctgaactgcg tccgccgtct aggtaagttt aaagctcagg tcgagaccgg gcctttgtcc     840
ggcgctccct tggagcctac ctagactcag ccggctctcc acgctttgcc tgaccctgct     900
tgctcaactc tacgtctttg tttcgttttc tgttctgcgc cgttacagat cactaggatc     960
cactagcgtg agtagcgggc tgctgggctg gccggggctt tcgtggccgc cgggccgctc    1020
ggtgggacgg aagcgtgtgg agagaccgcc aagggctgta gtctgggtcc gcgagcaagg    1080
ttgccctgaa ctgggggttg gggggagcgc agcaaaatgg cggctgttcc cgagtcttga    1140
atggaagacg cttgtgaggc gggctgtgag gtcgttgaaa caaggtgggg ggcatggtgg    1200
gcggcaagaa cccaaggtct tgaggccttc gctaatgcgg gaaagctctt attcgggtga    1260
gatgggctgg ggcaccatct ggggaccctg acgtgaagtt tgtcactgac tggagaactc    1320
ggtttgtcgt ctgttgcggg ggcggcagtt atggcggtgc cgttgggcag tgcacccgta    1380
cctttgggag cgcgcgccct cgtcgtgtcg tgacgtcacc cgttctgttg gcttataatg    1440
cagggtgggg ccacctgccg gtaggtgtgc ggtaggcttt tctccgtcgc aggacgcagg    1500
gttcgggcct agggtaggct ctcctgaatc gacaggcgcc ggacctctgg tgaggggagg    1560
gataagtgag gcgtcagttt ctttggtcgg ttttatgtac ctatcttctt aagtagctga    1620
agctccggtt ttgaactatg cgctcggggt tggcgagtgt gttttgtgaa gtttttttagg    1680
caccttttga aatgtaatca tttgggtcaa tatgtaattt tcagtgttag actagtaaat    1740
tgtccgctaa attctggccg tttttggctt ttttgttag                           1779
```

<210> 58

<211> 2168
<212> DNA
<213> Artificial Sequence

<220>
<223> séquence artificielle

<400> 58

```
gcgttacata acttacggta aatggcccgc ctggctgacc gcccaacgac ccccgcccat      60
tgacgtcaat aatgacgtat gttcccatag taacgccaat agggactttc cattgacgtc     120
```

aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc aggggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatcctagt gtttaaacag agtaatgaca tggttccttc catcctccaa 720
aggtgaccaa taatagtttg taagtatcat tatgaactaa tgaattttca acatatttga 780
tatatttcaa tccattgcca tcattgttct tatcgatatt tgagttggct cactttgcca 840
gtaagagtct attcaaattg gcttctgagt ccatttgaca caacaccttt gatctttgac 900
agtttccttg gttttaggtg ctagatgatt tctcaggctc accttagaca tttcctgcca 960
cagacttaga atcagccatt tctctaagga ccctgattcc atttcatgag aaatgataga 1020
gaccacaatc aaaacaagtc atgaatttat actgatattt tcaattcaaa ttaaagatga 1080
ggtttttgct aaattttttt gagtttatat ttgtatgtct tatgctgaaa aatcttgttt 1140
cctaattagt aacataatta ttcatttgat gggtaaatat tttagggccg attctttggt 1200
tttatagcca agataccctg ttgataaagt cttgtgggag caattataag actggcttat 1260
tttgaagctt tttaaaaaag acatccttac ctgttttaac tgtagattat attaacttaa 1320
ataggtacag cccacgcttg actaggatcc actagcgtga gtagcgggct gctgggctgg 1380
ccggggcttt cgtggccgcc gggccgctcg gtgggacgga agcgtgtgga gagaccgcca 1440
agggctgtag tctgggtccg cgagcaaggt tgccctgaac tgggggttgg ggggagcgca 1500
gcaaaatggc ggctgttccc gagtcttgaa tggaagacgc ttgtgaggcg ggctgtgagg 1560
tcgttgaaac aaggtggggg gcatggtggg cggcaagaac ccaaggtctt gaggccttcg 1620
ctaatgcggg aaagctctta ttcgggtgag atgggctggg gcaccatctg gggaccctga 1680
cgtgaagttt gtcactgact ggagaactcg gtttgtcgtc tgttgcgggg gcggcagtta 1740
tggcggtgcc gttgggcagt gcacccgtac ctttgggagc gcgcgccctc gtcgtgtcgt 1800
gacgtcaccc gttctgttgg cttataatgc agggtggggc cacctgccgg taggtgtgcg 1860
gtaggctttt ctccgtcgca ggacgcaggg ttcgggccta gggtaggctc tcctgaatcg 1920
acaggcgccg gacctctggt gaggggaggg ataagtgagg cgtcagtttc tttggtcggt 1980

**tttatgtacc tatcttctta agtagctgaa gctccggttt tgaactatgc gctcggggtt 2040**

**ggcgagtgtg ttttgtgaag ttttttaggc accttttgaa atgtaatcat ttgggtcaat 2100**

**atgtaatttt cagtgttaga ctagtaaatt gtccgctaaa ttctggccgt ttttggcttt 2160**

**tttgttag 2168**

<210> 59
<211> 1825
<212> DNA
<213> Artificial Sequence

<220>
<223> séquence artificielle

<400> 59

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aacgctggtg ggtagggatg agggagggag gggcattgtg     720
atgtacaggg ctgctctgtg agatcaaggg tctcttaagg gtgggagctg gggcagggac     780
tacgagagca gccagatggg ctgaaagtgg aactcaaggg gtttctggca cctacctacc     840
tgcttcccgc tgggggtgg ggagttggcc cagagtctta agattggggc agggtggaga     900
ggtgggctct tcctgcttcc cactcatctt atagctttct ttccccagat ccgaattcga     960
gatccaaacc aaggaggaaa ggatatcaca gaggagagct aggatccact agcgtgagta    1020
gcgggctgct gggctggccg ggctttcgt ggccgccggg ccgctcggtg ggacggaagc    1080
gtgtggagag accgccaagg gctgtagtct gggtccgcga gcaaggttgc cctgaactgg    1140
gggttggggg gagcgcagca aaatggcggc tgttcccgag tcttgaatgg aagacgcttg    1200
tgaggcgggc tgtgaggtcg ttgaaacaag gtgggggca tggtgggcgg caagaaccca    1260
aggtcttgag gccttcgcta atgcgggaaa gctcttattc gggtgagatg ggctggggca    1320
ccatctgggg accctgacgt gaagtttgtc actgactgga gaactcggtt tgtcgtctgt    1380
```

```
tgcgggggcg gcagttatgg cggtgccgtt gggcagtgca cccgtacctt tgggagcgcg     1440
cgccctcgtc gtgtcgtgac gtcacccgtt ctgttggctt ataatgcagg gtggggccac     1500
ctgccggtag gtgtgcggta ggcttttctc cgtcgcagga cgcagggttc gggcctaggg     1560
taggctctcc tgaatcgaca ggcgccggac ctctggtgag gggagggata agtgaggcgt     1620
cagtttcttt ggtcggtttt atgtacctat cttcttaagt agctgaagct ccggttttga     1680
actatgcgct cggggttggc gagtgtgttt tgtgaagttt tttaggcacc ttttgaaatg     1740
taatcatttg ggtcaatatg taattttcag tgttagacta gtaaattgtc cgctaaattc     1800
tggccgtttt tggctttttt gttag                                           1825
```

<210> 60
<211> 2447
<212> DNA
<213> Artificial Sequence

<220>
<223> séquence artificielle

<400> 60

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aaacggctcg catctctcct tcacgcgccc gccgccctac     720
ctgaggccgc catccacgcc ggttgagtcg cgttctgccg cctcccgcct gtggtgcctc     780
ctgaactgcg tccgccgtct aggtaagttt aaagctcagg tcgagaccgg gcctttgtcc     840
ggcgctccct tggagcctac ctagactcag ccggctctcc acgctttgcc tgaccctgct     900
tgctcaactc tacgtctttg tttcgttttc tgttctgcgc cgttacagat cactagtgtt     960
taaacagagt aatgacatgg ttccttccat cctccaaagg tgaccaataa tagtttgtaa    1020
gtatcattat gaactaatga attttcaaca tatttgatat atttcaatcc attgccatca    1080
```

```
ttgttcttat cgatatttga gttggctcac tttgccagta agagtctatt caaattggct     1140
tctgagtcca tttgacacaa cacctttgat ctttgacagt ttccttggtt ttaggtgcta     1200
gatgatttct caggctcacc ttagacattt cctgccacag acttagaatc agccatttct     1260
ctaaggaccc tgattccatt tcatgagaaa tgatagagac cacaatcaaa acaagtcatg     1320
aatttatact gatattttca attcaaatta aagatgaggt ttttgctaaa tttttttgag     1380
tttatatttg tatgtcttat gctgaaaaat cttgtttcct aattagtaac ataattattc     1440
atttgatggg taaatatttt agggccgatt ctttggtttt atagccaaga taccctgttg     1500
ataaagtctt gtgggagcaa ttataagact ggcttatttt gaagcttttt aaaaaagaca     1560
tccttacctg ttttaactgt agattatatt aacttaaata ggtacagccc acgcttgact     1620
agttgatcca ctagcgtgag tagcgggctg ctgggctggc cggggctttc gtggccgccg     1680
ggccgctcgg tgggacggaa gcgtgtggag agaccgccaa gggctgtagt ctgggtccgc     1740
gagcaaggtt gccctgaact gggggttggg gggagcgcag caaaatggcg gctgttcccg     1800
agtcttgaat ggaagacgct tgtgaggcgg gctgtgaggt cgttgaaaca aggtgggggg     1860
catggtgggc ggcaagaacc caaggtcttg aggccttcgc taatgcggga aagctcttat     1920
tcgggtgaga tgggctgggg caccatctgg ggaccctgac gtgaagtttg tcactgactg     1980
gagaactcgg tttgtcgtct gttgcggggg cggcagttat ggcggtgccg ttgggcagtg     2040
cacccgtacc tttgggagcg cgcgccctcg tcgtgtcgtg acgtcacccg ttctgttggc     2100
ttataatgca gggtggggcc acctgccggt aggtgtgcgg taggcttttc tccgtcgcag     2160
gacgcagggt tcgggcctag ggtaggctct cctgaatcga caggcgccgg acctctggtg     2220
aggggaggga taagtgaggc gtcagtttct ttggtcggtt ttatgtacct atcttcttaa     2280
gtagctgaag ctccggtttt gaactatgcg ctcggggttg gcgagtgtgt tttgtgaagt     2340
tttttaggca ccttttgaaa tgtaatcatt tgggtcaata tgtaattttc agtgttagac     2400
tagtaaattg tccgctaaat tctggccgtt tttggctttt ttgttag                   2447
```

<210> 61
<211> 2447
<212> DNA
<213> Artificial Sequence

<220>
<223> séquence artificielle

<400> 61

```
gcgttacata acttacggta aatggcccgc ctggctgacc gcccaacgac ccccgcccat        60
tgacgtcaat aatgacgtat gttcccatag taacgccaat agggactttc cattgacgtc       120
aatgggtgga gtatttacgg taaactgccc acttggcagt acatcaagtg tatcatatgc       180
caagtacgcc ccctattgac gtcaatgacg gtaaatggcc cgcctggcat tatgcccagt       240
```

acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagtgtt 960
taaacagagt aatgacatgg ttccttccat cctccaaagg tgaccaataa tagtttgtaa 1020
gtatcattat gaactaatga attttcaaca tatttgatat atttcaatcc attgccatca 1080
ttgttcttat cgatatttga gttggctcac tttgccagta agagtctatt caaattggct 1140
tctgagtcca tttgacacaa cacctttgat ctttgacagt ttccttggtt ttaggtgcta 1200
gatgatttct caggctcacc ttagacattt cctgccacag acttagaatc agccatttct 1260
ctaaggaccc tgattccatt tcatgagaaa tgatagagac cacaatcaaa acaagtcatg 1320
aatttatact gatattttca attcaaatta aagatgaggt tttgctaaa ttttttttgag 1380
tttatatttg tatgtcttat gctgaaaaat cttgtttcct aattagtaac ataattattc 1440
atttgatggg taaatatttt agggccgatt ctttggtttt atagccaaga taccctgttg 1500
ataaagtctt gtgggagcaa ttataagact ggcttatttt gaagcttttt aaaaaagaca 1560
tccttacctg ttttaactgt agattatatt aacttaaata ggtacagccc acgcttgact 1620
agttgatcca ctagcgtgag tagcgggctg ctgggctggc cggggctttc gtggccgccg 1680
ggccgctcgg tgggacggaa gcgtgtggag agaccgccaa gggctgtagt ctgggtccgc 1740
gagcaaggtt gccctgaact gggggttggg gggagcgcag caaaatggcg gctgttcccg 1800
agtcttgaat ggaagacgct tgtgaggcgg gctgtgaggt cgttgaaaca aggtgggggg 1860
catggtgggc ggcaagaacc caaggtcttg aggccttcgc taatgcggga aagctcttat 1920
tcgggtgaga tgggctgggg caccatctgg ggaccctgac gtgaagtttg tcactgactg 1980
gagaactcgg tttgtcgtct gttgcggggg cggcagttat ggcggtgccg ttgggcagtg 2040
cacccgtacc tttgggagcg cgcgccctcg tcgtgtcgtg acgtcacccg ttctgttggc 2100

```
ttataatgca gggtggggcc acctgccggt aggtgtgcgg taggcttttc tccgtcgcag     2160
gacgcagggt tcgggcctag ggtaggctct cctgaatcga caggcgccgg acctctggtg     2220
aggggaggga taagtgaggc gtcagtttct ttggtcggtt ttatgtacct atcttcttaa     2280
gtagctgaag ctccggtttt gaactatgcg ctcggggttg gcgagtgtgt tttgtgaagt     2340
tttttaggca ccttttgaaa tgtaatcatt tgggtcaata tgtaattttc agtgttagac     2400
tagtaaattg tccgctaaat tctggccgtt tttggctttt ttgttag                   2447
```

<210> 62
<211> 2483
<212> DNA
<213> Artificial Sequence

<220>
<223> séquence artificielle

<400> 62

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatcaaaca gagtaatgac atggttcctt ccatcctcca aaggtgacca     720
ataatagttt gtaagtatca ttatgaacta atgaattttc aacatatttg atatatttca     780
atccattgcc atcattgttc ttatcgatat ttgagttggc tcactttgcc agtaagagtc     840
tattcaaatt ggcttctgag tccatttgac acaacacctt tgatctttga cagtttcctt     900
ggttttaggt gctagatgat ttctcaggct caccttagac atttcctgcc acagacttag     960
aatcagccat ttctctaagg accctgattc catttcatga gaaatgatag agaccacaat    1020
caaaacaagt catgaattta tactgatatt ttcaattcaa attaaagatg aggtttttgc    1080
taaatttttt tgagtttata tttgtatgtc ttatgctgaa aaatcttgtt tcctaattag    1140
taacataatt attcatttga tgggtaaata ttttagggcc gattctttgg ttttatagcc    1200
aagataccct gttgataaag tcttgtggga gcaattataa gactggctta ttttgaagct    1260
```

```
ttttaaaaaa gacatcctta cctgttttaa ctgtagatta tattaactta aataggtaca      1320
gcccacgctt gactagttaa cgctggtggg tagggatgag ggagggaggg gcattgtgat      1380
gtacagggct gctctgtgag atcaagggtc tcttaagggt gggagctggg gcagggacta      1440
cgagagcagc cagatgggct gaaagtggaa ctcaaggggt ttctggcacc tacctacctg      1500
cttcccgctg gggggtgggg agttggccca gagtcttaag attggggcag ggtggagagg      1560
tgggctcttc ctgcttccca ctcatcttat agctttcttt ccccagatcc gaattcgaga      1620
tccaaaccaa ggaggaaagg atatcacaga ggagagctag gatccactag cgtgagtagc      1680
gggctgctgg gctggccggg gctttcgtgg ccgccgggcc gctcggtggg acggaagcgt      1740
gtggagagac cgccaagggc tgtagtctgg gtccgcgagc aaggttgccc tgaactgggg      1800
gttgggggga gcgcagcaaa atggcggctg ttcccgagtc ttgaatggaa gacgcttgtg      1860
aggcgggctg tgaggtcgtt gaaacaaggt gggggcatg gtgggcggca agaacccaag       1920
gtcttgaggc cttcgctaat gcgggaaagc tcttattcgg gtgagatggg ctggggcacc      1980
atctggggac cctgacgtga agtttgtcac tgactggaga actcggtttg tcgtctgttg      2040
cgggggcggc agttatggcg gtgccgttgg gcagtgcacc cgtacctttg ggagcgcgcg      2100
ccctcgtcgt gtcgtgacgt cacccgttct gttggcttat aatgcagggt ggggccacct      2160
gccggtaggt gtgcggtagg cttttctccg tcgcaggacg cagggttcgg gcctagggta      2220
ggctctcctg aatcgacagg cgccggacct ctggtgaggg gagggataag tgaggcgtca      2280
gtttctttgg tcggttttat gtacctatct tcttaagtag ctgaagctcc ggttttgaac      2340
tatgcgctcg ggttggcga gtgtgttttg tgaagttttt taggcacctt ttgaaatgta       2400
atcatttggg tcaatatgta attttcagtg ttagactagt aaattgtccg ctaaattctg      2460
gccgtttttg gcttttttgt tag                                              2483
```

<210> 63
<211> 2769
<212> DNA
<213> Artificial Sequence

<220>
<223> séquence artificielle

<400> 63

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
```

ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct cccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagtgtt 960
taaacagagt aatgacatgg ttccttccat cctccaaagg tgaccaataa tagtttgtaa 1020
gtatcattat gaactaatga attttcaaca tatttgatat atttcaatcc attgccatca 1080
ttgttcttat cgatatttga gttggctcac tttgccagta agagtctatt caaattggct 1140
tctgagtcca tttgacacaa cacctttgat ctttgacagt ttccttggtt ttaggtgcta 1200
gatgatttct caggctcacc ttagacattt cctgccacag acttagaatc agccatttct 1260
ctaaggaccc tgattccatt tcatgagaaa tgatagagac cacaatcaaa acaagtcatg 1320
aatttatact gatattttca attcaaatta aagatgaggt ttttgctaaa ttttttgag 1380
tttatatttg tatgtcttat gctgaaaaat cttgtttcct aattagtaac ataattattc 1440
atttgatggg taaatatttt agggccgatt ctttggtttt atagccaaga taccctgttg 1500
ataaagtctt gtgggagcaa ttataagact ggcttatttt gaagcttttt aaaaaagaca 1560
tccttacctg ttttaactgt agattatatt aacttaaata ggtacagccc acgcttgact 1620
agttaacgct ggtgggtagg gatgagggag ggaggggcat tgtgatgtac agggctgctc 1680
tgtgagatca agggtctctt aagggtggga gctggggcag ggactacgag agcagccaga 1740
tgggctgaaa gtggaactca aggggtttct ggcacctacc tacctgcttc ccgctggggg 1800
gtggggagtt ggcccagagt cttaagattg ggcagggtg gagaggtggg ctcttcctgc 1860
ttcccactca tcttatagct ttctttcccc agatccgaat tcgagatcca aaccaaggag 1920
gaaaggatat cacagaggag agctaggatc cactagcgtg agtagcgggc tgctgggctg 1980
gccggggctt tcgtggccgc cgggccgctc ggtgggacgg aagcgtgtgg agagaccgcc 2040
aagggctgta gtctgggtcc gcgagcaagg ttgccctgaa ctgggggttg gggggagcgc 2100
agcaaaatgg cggctgttcc cgagtcttga atggaagacg cttgtgaggc gggctgtgag 2160
gtcgttgaaa caaggtgggg ggcatggtgg gcggcaagaa cccaaggtct tgaggccttc 2220

```
gctaatgcgg gaaagctctt attcgggtga gatgggctgg ggcaccatct ggggaccctg     2280
acgtgaagtt tgtcactgac tggagaactc ggtttgtcgt ctgttgcggg ggcggcagtt     2340
atggcggtgc cgttgggcag tgcacccgta cctttgggag cgcgcgccct cgtcgtgtcg     2400
tgacgtcacc cgttctgttg gcttataatg cagggtgggg ccacctgccg gtaggtgtgc     2460
ggtaggcttt tctccgtcgc aggacgcagg gttcgggcct agggtaggct ctcctgaatc     2520
gacaggcgcc ggacctctgg tgaggggagg gataagtgag gcgtcagttt ctttggtcgg     2580
ttttatgtac ctatcttctt aagtagctga agctccggtt ttgaactatg cgctcggggt     2640
tggcgagtgt gttttgtgaa gtttttttagg caccttttga aatgtaatca tttgggtcaa     2700
tatgtaattt tcagtgttag actagtaaat tgtccgctaa attctggccg tttttggctt     2760
ttttgttag                                                             2769
```

<210> 64
<211> 2193
<212> DNA
<213> Artificial Sequence

<220>
<223> séquence artificielle

<400> 64

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc agggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aaacggctcg catctctcct tcacgcgccc gccgccctac     720
ctgaggccgc catccacgcc ggttgagtcg cgttctgccg cctcccgcct gtggtgcctc     780
ctgaactgcg tccgccgtct aggtaagttt aaagctcagg tcgagaccgg gcctttgtcc     840
ggcgctccct tggagcctac ctagactcag ccggctctcc acgctttgcc tgaccctgct     900
tgctcaactc tacgtctttg tttcgttttc tgttctgcgc cgttacagat cactagtctc     960
```

```
agagagcctc ggctaggtag gggagcggaa ctctggtggg aggggaggtg cggtgcactg    1020
gggggatggg tggctagggg ggccgtctgg tggcttgcgg gggttgcctt tcccgtggga    1080
agtcgggaac ataatgtttg ttacgttggg agggaaaggg gtggctggat gcaggcggga    1140
gggaggcccg ccctgcggca accggagggg gagggagaag ggagcggaaa atgctcgaaa    1200
ccggacggag ccattgctct cgcagaggga ggagcgcttc cggctagcct cttgtcgccg    1260
attggccgtt tctcctcccg ccgtgtgtga aaacacaaat ggcgtattct ggttggagta    1320
aagctcctgt cagttacacc gtcgggagta cgcagccgct tagcgactct cgcgttgccc    1380
cctgggtggg gcgggtaggt aggtggggtg tagagatgct gggtgtgcgg gcgcggccgg    1440
cctcctgcgg cgggagggga gggtcagtga aattggctct ggcgcgggcg tcctcccacc    1500
ctccccttcc ttcgggggag tcggtttacc cgccgcctgc ttgtcttcga cacctgattg    1560
gctgtcgaag ctgtgggacc gggcccttgc tactggctcg agtctcacat gagcgaaacc    1620
actgcgcggg gcgcgggggt ggcggggagg cgggcgttgg tacggtcctc cccgaggccg    1680
agcgccgcag tgtctggccc cgcgcccctg cgcaacgtgg caggaagcgc gcgctggagg    1740
cgggggcggg ctgccggccg agacttctgg atggcggcgg ccgcggctcc gccccgggtt    1800
cccaccgcct gaagggcgag acaagcccga cctgctacag gcactcgtgg gggtggggga    1860
ggagcggggg tcggtccggc tggtttgtgg gtgggaggcg cttgttctcc aaaaaccggc    1920
gcgagctgca atcctgaggg agctgcggtg gaggaggtgg agagaaggcc gcacccttct    1980
gggcaggggg aggggagtgc cgcaatacct ttatgggagt tctttgctgc ctcccgtctt    2040
gtaaggaccg ccctgggcct ggaagaagcc ctccctcctt tcctcctcgc gtgatctcgt    2100
catcgcctcc atgtcgagtc gcttctcgat tatgggcggg attcttttgc ctagacaatt    2160
gtactaacct tcttctcttt cctctcctga cag                                  2193
```

<210> 65
<211> 2584
<212> DNA
<213> Artificial Sequence

<220>
<223> séquence artificielle

<400> 65

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
```

ccggctcccc gcgcccccga tcggggccgc cgctagtagt ggcggcggcg gaggcggggg 420

cagcggcggc ggcggcggag gcgcctctgc agctccggct cccccctggcc tctcgggaac 480

tacaagtccc agggggcctg gcggtgggcg gcgggcggaa gaggcggggt cggcgccgcg 540

aggccggaag tggccgtgga ggcggaagtg gcgcggccgc ggaggggcct ggagtgcggc 600

ggcggcggga cccggagcag gagcggcggc agcagcgact gggggcggcg gcggcgcgtt 660

ggaggcggcc ggatccacta gtgtttaaac agagtaatga catggttcct tccatcctcc 720

aaaggtgacc aataatagtt tgtaagtatc attatgaact aatgaatttt caacatattt 780

gatatatttc aatccattgc catcattgtt cttatcgata tttgagttgg ctcactttgc 840

cagtaagagt ctattcaaat tggcttctga gtccatttga cacaacacct ttgatctttg 900

acagtttcct tggttttagg tgctagatga tttctcaggc tcaccttaga catttcctgc 960

cacagactta gaatcagcca tttctctaag gaccctgatt ccatttcatg agaaatgata 1020

gagaccacaa tcaaaacaag tcatgaattt atactgatat tttcaattca aattaaagat 1080

gaggtttttg ctaaattttt ttgagtttat atttgtatgt cttatgctga aaatcttgt 1140

ttcctaatta gtaacataat tattcatttg atgggtaaat attttagggc cgattctttg 1200

gttttatagc caagataccc tgttgataaa gtcttgtggg agcaattata agactggctt 1260

attttgaagc tttttaaaaa agacatcctt acctgtttta actgtagatt atattaactt 1320

aaataggtac agcccacgct tgactagtct cagagagcct cggctaggta ggggagcgga 1380

actctggtgg gaggggaggt gcggtgcact gggggggatgg gtggctaggg gggccgtctg 1440

gtggcttgcg gggttgcct ttcccgtggg aagtcgggaa cataatgttt gttacgttgg 1500

gagggaaagg ggtggctgga tgcaggcggg agggaggccc gccctgcggc aaccggaggg 1560

ggagggagaa gggagcggaa aatgctcgaa accggacgga gccattgctc tcgcagaggg 1620

aggagcgctt ccggctagcc tcttgtcgcc gattggccgt ttctcctccc gccgtgtgtg 1680

aaaacacaaa tggcgtattc tggttggagt aaagctcctg tcagttacac cgtcgggagt 1740

acgcagccgc ttagcgactc tcgcgttgcc ccctgggtgg ggcgggtagg taggtggggt 1800

gtagagatgc tgggtgtgcg ggcgcggccg gcctcctgcg gcgggagggg agggtcagtg 1860

aaattggctc tggcgcgggc gtcctcccac cctccccttc cttcgggga gtcggtttac 1920

ccgccgcctg cttgtcttcg acacctgatt ggctgtcgaa gctgtgggac cgggcccttg 1980

ctactggctc gagtctcaca tgagcgaaac cactgcgcgg ggcgcggggg tggcggggag 2040

gcgggcgttg gtacggtcct ccccgaggcc gagcgccgca gtgtctggcc ccgcgcccct 2100

gcgcaacgtg gcaggaagcg cgcgctggag gcgggggcgg gctgccggcc gagacttctg 2160

gatggcggcg gccgcggctc cgccccgggt tcccaccgcc tgaagggcga gacaagcccg 2220

acctgctaca ggcactcgtg ggggtggggg aggagcgggg gtcggtccgg ctggtttgtg 2280

ggtgggaggc gcttgttctc caaaaaccgg cgcgagctgc aatcctgagg gagctgcggt 2340

ggaggaggtg gagagaaggc cgcacccttc tgggcagggg gaggggagtg ccgcaatacc 2400

tttatgggag ttctttgctg cctcccgtct tgtaaggacc gccctgggcc tggaagaagc 2460

cctccctcct ttcctcctcg cgtgatctcg tcatcgcctc catgtcgagt cgcttctcga 2520

ttatgggcgg gattcttttg cctagacaat tgtactaacc ttcttctctt tcctctcctg 2580

acag 2584

<210> 66
<211> 2239
<212> DNA
<213> Artificial Sequence

<220>
<223> séquence artificielle

<400> 66

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc aggggggcctg gcggtgggcg gcgggcggaa gaggcggggt cggcgccgcg     540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aacgctggtg ggtagggatg agggagggag gggcattgtg     720
atgtacaggg ctgctctgtg agatcaaggg tctcttaagg gtgggagctg gggcagggac     780
tacgagagca gccagatggg ctgaaagtgg aactcaaggg gtttctggca cctacctacc     840
tgcttcccgc tgggggggtgg ggagttggcc cagagtctta agattggggc agggtggaga     900
ggtgggctct tcctgcttcc cactcatctt atagctttct ttccccagat ccgaattcga     960
gatccaaacc aaggaggaaa ggatatcaca gaggagagct agtctcagag agcctcggct    1020
aggtagggga gcggaactct ggtgggaggg gaggtgcggt gcactggggg gatgggtggc    1080
tagggggggcc gtctggtggc ttgcgggggt tgcctttccc gtgggaagtc gggaacataa    1140
tgtttgttac gttgggaggg aaaggggtgg ctggatgcag gcgggaggga ggcccgccct    1200
```

```
gcggcaaccg gagggggagg gagaagggag cggaaaatgc tcgaaaccgg acggagccat      1260
tgctctcgca gagggaggag cgcttccggc tagcctcttg tcgccgattg gccgtttctc      1320
ctcccgccgt gtgtgaaaac acaaatggcg tattctggtt ggagtaaagc tcctgtcagt      1380
tacaccgtcg ggagtacgca gccgcttagc gactctcgcg ttgcccccctg ggtggggcgg      1440
gtaggtaggt ggggtgtaga gatgctgggt gtgcgggcgc ggccggcctc ctgcggcggg      1500
aggggagggt cagtgaaatt ggctctggcg cgggcgtcct cccaccctcc ccttccttcg      1560
ggggagtcgg tttacccgcc gcctgcttgt cttcgacacc tgattggctg tcgaagctgt      1620
gggaccgggc ccttgctact ggctcgagtc tcacatgagc gaaaccactg cgcggggcgc      1680
gggggtggcg gggaggcggg cgttggtacg gtcctccccg aggccgagcg ccgcagtgtc      1740
tggccccgcg cccctgcgca acgtggcagg aagcgcgcgc tggaggcggg ggcgggctgc      1800
cggccgagac ttctggatgg cggcggccgc ggctccgccc cgggttccca ccgcctgaag      1860
ggcgagacaa gcccgacctg ctacaggcac tcgtgggggt gggggaggag cgggggtcgg      1920
tccggctggt ttgtgggtgg gaggcgcttg ttctccaaaa accggcgcga gctgcaatcc      1980
tgagggagct gcggtggagg aggtggagag aaggccgcac ccttctgggc agggggaggg      2040
gagtgccgca ataccttttat gggagttctt tgctgcctcc cgtcttgtaa ggaccgccct      2100
gggcctggaa gaagccctcc ctcctttcct cctcgcgtga tctcgtcatc gcctccatgt      2160
cgagtcgctt ctcgattatg ggcgggattc ttttgcctag acaattgtac taaccttctt      2220
ctctttcctc tcctgacag                                                   2239
```

<210> 67
<211> 2859
<212> DNA
<213> Artificial Sequence

<220>
<223> séquence artificielle

<400> 67

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480

tacaagtccc aggggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagtgtt 960
taaacagagt aatgacatgg ttccttccat cctccaaagg tgaccaataa tagtttgtaa 1020
gtatcattat gaactaatga attttcaaca tatttgatat atttcaatcc attgccatca 1080
ttgttcttat cgatatttga gttggctcac tttgccagta agagtctatt caaattggct 1140
tctgagtcca tttgacacaa cacctttgat ctttgacagt ttccttggtt ttaggtgcta 1200
gatgatttct caggctcacc ttagacattt cctgccacag acttagaatc agccatttct 1260
ctaaggaccc tgattccatt tcatgagaaa tgatagagac cacaatcaaa acaagtcatg 1320
aatttatact gatattttca attcaaatta aagatgaggt ttttgctaaa tttttttgag 1380
tttatatttg tatgtcttat gctgaaaaat cttgtttcct aattagtaac ataattattc 1440
atttgatggg taaatatttt agggccgatt ctttggtttt atagccaaga taccctgttg 1500
ataaagtctt gtgggagcaa ttataagact ggcttatttt gaagcttttt aaaaaagaca 1560
tccttacctg ttttaactgt agattatatt aacttaaata ggtacagccc acgcttgact 1620
agtctcagag agcctcggct aggtagggga gcggaactct ggtgggaggg gaggtgcggt 1680
gcactggggg gatgggtggc taggggggcc gtctggtggc ttgcgggggt tgcctttccc 1740
gtgggaagtc gggaacataa tgtttgttac gttgggaggg aaaggggtgg ctggatgcag 1800
gcgggaggga ggcccgccct gcggcaaccg gagggggagg gagaagggag cggaaaatgc 1860
tcgaaaccgg acggagccat tgctctcgca gagggaggag cgcttccggc tagcctcttg 1920
tcgccgattg gccgtttctc ctcccgccgt gtgtgaaaac acaaatggcg tattctggtt 1980
ggagtaaagc tcctgtcagt tacaccgtcg ggagtacgca gccgcttagc gactctcgcg 2040
ttgcccccctg ggtggggcgg gtaggtaggt ggggtgtaga gatgctgggt gtgcgggcgc 2100
ggccggcctc ctgcggcggg aggggagggt cagtgaaatt ggctctggcg cgggcgtcct 2160
cccaccctcc ccttccttcg ggggagtcgg tttacccgcc gcctgcttgt cttcgacacc 2220
tgattggctg tcgaagctgt gggaccgggc ccttgctact ggctcgagtc tcacatgagc 2280
gaaaccactg cgcggggcgc gggggtggcg gggaggcggg cgttggtacg gtcctccccg 2340
aggccgagcg ccgcagtgtc tggccccgcg cccctgcgca acgtggcagg aagcgcgcgc 2400

tggaggcggg ggcgggctgc cggccgagac ttctggatgg cggcggccgc ggctccgccc 2460

cgggttccca ccgcctgaag ggcgagacaa gcccgacctg ctacaggcac tcgtgggggt 2520

gggggaggag cgggggtcgg tccggctggt ttgtgggtgg gaggcgcttg ttctccaaaa 2580

accggcgcga gctgcaatcc tgagggagct gcggtggagg aggtggagag aaggccgcac 2640

ccttctgggc agggggaggg gagtgccgca ataccttat gggagttctt tgctgcctcc 2700

cgtcttgtaa ggaccgccct gggcctggaa gaagccctcc ctcctttcct cctcgcgtga 2760

tctcgtcatc gcctccatgt cgagtcgctt ctcgattatg ggcgggattc ttttgcctag 2820

acaattgtac taaccttctt ctctttcctc tcctgacag 2859

<210> 68
<211> 2517
<212> DNA
<213> Artificial Sequence

<220>
<223> séquence artificielle

<400> 68

gcgttacata acttacggta aatggcccgc ctggctgacc gcccaacgac ccccgcccat 60
tgacgtcaat aatgacgtat gttcccatag taacgccaat agggactttc cattgacgtc 120
aatgggtgga gtatttacgg taaactgccc acttggcagt acatcaagtg tatcatatgc 180
caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc aggggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacggctcg catctctcct tcacgcgccc gccgccctac 720
ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagttaa 960
cgctggtggg tagggatgag ggagggaggg gcattgtgat gtacagggct gctctgtgag 1020
atcaagggtc tcttaagggt gggagctggg gcagggacta cgagagcagc cagatgggct 1080

gaaagtggaa ctcaaggggt ttctggcacc tacctacctg cttcccgctg gggggtgggg 1140

agttggccca gagtcttaag attggggcag ggtggagagg tgggctcttc ctgcttccca 1200

ctcatcttat agctttcttt ccccagatcc gaattcgaga tccaaaccaa ggaggaaagg 1260

atatcacaga ggagagctag tctcagagag cctcggctag gtaggggagc ggaactctgg 1320

tgggaggggga ggtgcggtgc actgggggga tgggtggcta ggggggccgt ctggtggctt 1380

gcgggggttg cctttcccgt gggaagtcgg gaacataatg tttgttacgt tgggagggaa 1440

aggggtggct ggatgcaggc gggagggagg cccgccctgc ggcaaccgga gggggaggga 1500

gaagggagcg gaaaatgctc gaaaccggac ggagccattg ctctcgcaga gggaggagcg 1560

cttccggcta gcctcttgtc gccgattggc cgtttctcct cccgccgtgt gtgaaaacac 1620

aaatggcgta ttctggttgg agtaaagctc ctgtcagtta caccgtcggg agtacgcagc 1680

cgcttagcga ctctcgcgtt gccccctggg tggggcgggt aggtaggtgg ggtgtagaga 1740

tgctgggtgt gcgggcgcgg ccggcctcct gcggcgggag gggagggtca gtgaaattgg 1800

ctctggcgcg ggcgtcctcc caccctcccc ttccttcggg ggagtcggtt tacccgccgc 1860

ctgcttgtct tcgacacctg attggctgtc gaagctgtgg gaccgggccc ttgctactgg 1920

ctcgagtctc acatgagcga aaccactgcg cggggcgcgg gggtggcggg gaggcgggcg 1980

ttggtacggt cctccccgag gccgagcgcc gcagtgtctg gccccgcgcc cctgcgcaac 2040

gtggcaggaa gcgcgcgctg gaggcggggg cgggctgccg gccgagactt ctggatggcg 2100

gcggccgcgg ctccgccccg ggttcccacc gcctgaaggg cgagacaagc ccgacctgct 2160

acaggcactc gtgggggtgg gggaggagcg gggtcggtc cggctggttt gtgggtggga 2220

ggcgcttgtt ctccaaaaac cggcgcgagc tgcaatcctg agggagctgc ggtggaggag 2280

gtggagagaa ggccgcaccc ttctgggcag gggagggga gtgccgcaat acctttatgg 2340

gagttctttg ctgcctcccg tcttgtaagg accgccctgg gcctggaaga agccctccct 2400

cctttcctcc tcgcgtgatc tcgtcatcgc ctccatgtcg agtcgcttct cgattatggg 2460

cgggattctt ttgcctagac aattgtacta accttcttct ctttcctctc ctgacag 2517

<210> 69
<211> 2902
<212> DNA
<213> Artificial Sequence

<220>
<223> séquence artificielle

<400> 69

gcgttacata acttacggta aatggcccgc ctggctgacc gcccaacgac ccccgcccat 60

tgacgtcaat aatgacgtat gttcccatag taacgccaat agggactttc cattgacgtc 120

aatgggtgga gtatttacgg taaactgccc acttggcagt acatcaagtg tatcatatgc 180

caagtacgcc ccctattgac gtcaatgacg gtaaatggcc cgcctggcat tatgcccagt 240
acatgacctt atgggacttt cctacttggc agtacatcta cgtattagtc atcgctatta 300
ccatggcccg ggtcgcgaca tgcagcggga cgcgccaccc cgagccccag ctccggcgcc 360
ccggctcccc gcgcccccga tcggggccgc cgctagtagt ggcggcggcg gaggcggggg 420
cagcggcggc ggcggcggag gcgcctctgc agctccggct ccccctggcc tctcgggaac 480
tacaagtccc agggggcctg gcggtgggcg gcgggcggaa gaggcggggt cggcgccgcg 540
aggccggaag tggccgtgga ggcggaagtg gcgcggccgc ggaggggcct ggagtgcggc 600
ggcggcggga cccggagcag gagcggcggc agcagcgact gggggcggcg gcggcgcgtt 660
ggaggcggcc ggatccgttt aaacagagta atgacatggt tccttccatc ctccaaaggt 720
gaccaataat agtttgtaag tatcattatg aactaatgaa ttttcaacat atttgatata 780
tttcaatcca ttgccatcat tgttcttatc gatatttgag ttggctcact ttgccagtaa 840
gagtctattc aaattggctt ctgagtccat ttgacacaac acctttgatc tttgacagtt 900
tccttggttt taggtgctag atgatttctc aggctcacct tagacatttc ctgccacaga 960
cttagaatca gccatttctc taaggaccct gattccattt catgagaaat gatagagacc 1020
acaatcaaaa caagtcatga atttatactg atattttcaa ttcaaattaa agatgaggtt 1080
tttgctaaat ttttttgagt ttatatttgt atgtcttatg ctgaaaaatc ttgtttccta 1140
attagtaaca taattattca tttgatgggt aaatatttta gggccgattc tttggtttta 1200
tagccaagat accctgttga taaagtcttg tgggagcaat tataagactg gcttattttg 1260
aagcttttta aaaaagacat ccttacctgt tttaactgta gattatatta acttaaatag 1320
gtacagccca cgcttgacta gttaacgctg gtgggtaggg atgagggagg gaggggcatt 1380
gtgatgtaca gggctgctct gtgagatcaa gggtctctta agggtgggag ctggggcagg 1440
gactacgaga gcagccagat gggctgaaag tggaactcaa ggggtttctg gcacctacct 1500
acctgcttcc cgctgggggg tggggagttg gcccagagtc ttaagattgg ggcagggtgg 1560
agaggtgggc tcttcctgct tcccactcat cttatagctt tctttcccca gatccgaatt 1620
cgagatccaa accaaggagg aaaggatatc acagaggaga gctagtctca gagagcctcg 1680
gctaggtagg ggagcggaac tctggtggga ggggaggtgc ggtgcactgg ggggatgggt 1740
ggctaggggg gccgtctggt ggcttgcggg ggttgccttt cccgtgggaa gtcgggaaca 1800
taatgtttgt tacgttggga gggaaagggg tggctggatg caggcgggag ggaggcccgc 1860
cctgcggcaa ccggaggggg agggagaagg gagcggaaaa tgctcgaaac cggacggagc 1920
cattgctctc gcagagggag gagcgcttcc ggctagcctc ttgtcgccga ttggccgttt 1980
ctcctcccgc cgtgtgtgaa aacacaaatg gcgtattctg gttggagtaa agctcctgtc 2040

```
agttacaccg tcgggagtac gcagccgctt agcgactctc gcgttgcccc ctgggtgggg    2100
cgggtaggta ggtggggtgt agagatgctg ggtgtgcggg cgcggccggc ctcctgcggc    2160
gggaggggag ggtcagtgaa attggctctg gcgcgggcgt cctcccaccc tccccttcct    2220
tcgggggagt cggtttaccc gccgcctgct tgtcttcgac acctgattgg ctgtcgaagc    2280
tgtgggaccg ggcccttgct actggctcga gtctcacatg agcgaaacca ctgcgcgggg    2340
cgcgggggtg gcggggaggc gggcgttggt acggtcctcc ccgaggccga gcgccgcagt    2400
gtctggcccc gcgcccctgc gcaacgtggc aggaagcgcg cgctggaggc gggggcgggc    2460
tgccggccga gacttctgga tggcggcggc cgcggctccg ccccgggttc ccaccgcctg    2520
aagggcgaga caagcccgac ctgctacagg cactcgtggg ggtgggggag gagcgggggt    2580
cggtccggct ggtttgtggg tgggaggcgc ttgttctcca aaaaccggcg cgagctgcaa    2640
tcctgaggga gctgcggtgg aggaggtgga gagaaggccg caccccttctg ggcaggggga    2700
ggggagtgcc gcaatacctt tatgggagtt ctttgctgcc tcccgtcttg taaggaccgc    2760
cctgggcctg gaagaagccc tccctccttt cctcctcgcg tgatctcgtc atcgcctcca    2820
tgtcgagtcg cttctcgatt atgggcggga ttcttttgcc tagacaattg tactaacctt    2880
cttctctttc ctctcctgac ag                                              2902
```

<210> 70
<211> 3183
<212> DNA
<213> Artificial Sequence

<220>
<223> séquence artificielle

<400> 70

```
gcgttacata acttacggta aatggcccgc ctggctgacc gcccaacgac ccccgcccat      60
tgacgtcaat aatgacgtat gttcccatag taacgccaat agggactttc cattgacgtc     120
aatgggtgga gtatttacgg taaactgccc acttggcagt acatcaagtg tatcatatgc     180
caagtacgcc ccctattgac gtcaatgacg gtaaatggcc cgcctggcat tatgcccagt     240
acatgacctt atgggacttt cctacttggc agtacatcta cgtattagtc atcgctatta     300
ccatggcccg ggtcgcgaca tgcagcggga cgcgccaccc cgagccccag ctccggcgcc     360
ccggctcccc gcgcccccga tcggggccgc cgctagtagt ggcggcggcg gaggcggggg     420
cagcggcggc ggcggcggag gcgcctctgc agctccggct ccccctggcc tctcgggaac     480
tacaagtccc aggggggcctg gcggtgggcg gcgggcggaa gaggcggggt cggcgccgcg    540
aggccggaag tggccgtgga ggcggaagtg gcgcggccgc ggaggggcct ggagtgcggc     600
ggcggcggga cccggagcag gagcggcggc agcagcgact gggggcggcg gcggcgcgtt     660
ggaggcggcc ggatccgttt aaacggctcg catctctcct tcacgcgccc gccgccctac     720
```

ctgaggccgc catccacgcc ggttgagtcg cgttctgccg cctcccgcct gtggtgcctc 780
ctgaactgcg tccgccgtct aggtaagttt aaagctcagg tcgagaccgg gcctttgtcc 840
ggcgctccct tggagcctac ctagactcag ccggctctcc acgctttgcc tgaccctgct 900
tgctcaactc tacgtctttg tttcgttttc tgttctgcgc cgttacagat cactagtgtt 960
taaacagagt aatgacatgg ttccttccat cctccaaagg tgaccaataa tagtttgtaa 1020
gtatcattat gaactaatga attttcaaca tatttgatat atttcaatcc attgccatca 1080
ttgttcttat cgatatttga gttggctcac tttgccagta agagtctatt caaattggct 1140
tctgagtcca tttgacacaa cacctttgat ctttgacagt ttccttggtt ttaggtgcta 1200
gatgatttct caggctcacc ttagacattt cctgccacag acttagaatc agccatttct 1260
ctaaggaccc tgattccatt tcatgagaaa tgatagagac cacaatcaaa acaagtcatg 1320
aatttatact gatattttca attcaaatta aagatgaggt ttttgctaaa tttttttgag 1380
tttatatttg tatgtcttat gctgaaaaat cttgtttcct aattagtaac ataattattc 1440
atttgatggg taaatatttt agggccgatt ctttggtttt atagccaaga taccctgttg 1500
ataaagtctt gtgggagcaa ttataagact ggcttatttt gaagcttttt aaaaaagaca 1560
tccttacctg ttttaactgt agattatatt aacttaaata ggtacagccc acgcttgact 1620
agttaacgct ggtgggtagg gatgagggag ggagggcat tgtgatgtac agggctgctc 1680
tgtgagatca agggtctctt aagggtggga gctggggcag ggactacgag agcagccaga 1740
tgggctgaaa gtggaactca aggggtttct ggcacctacc tacctgcttc ccgctggggg 1800
gtggggagtt ggcccagagt cttaagattg gggcagggtg gagaggtggg ctcttcctgc 1860
ttcccactca tcttatagct ttctttcccc agatccgaat tcgagatcca aaccaaggag 1920
gaaaggatat cacagaggag agctagtctc agagagcctc ggctaggtag gggagcggaa 1980
ctctggtggg aggggaggtg cggtgcactg gggggatggg tggctagggg ggccgtctgg 2040
tggcttgcgg gggttgcctt tcccgtggga agtcgggaac ataatgtttg ttacgttggg 2100
agggaaaggg gtggctggat gcaggcggga gggaggcccg ccctgcggca accggagggg 2160
gagggagaag ggagcggaaa atgctcgaaa ccggacggag ccattgctct cgcagaggga 2220
ggagcgcttc cggctagcct cttgtcgccg attggccgtt tctcctcccg ccgtgtgtga 2280
aaacacaaat ggcgtattct ggttggagta aagctcctgt cagttacacc gtcgggagta 2340
cgcagccgct tagcgactct cgcgttgccc cctgggtggg gcgggtaggt aggtggggtg 2400
tagagatgct gggtgtgcgg gcgcggccgg cctcctgcgg cgggagggga gggtcagtga 2460
aattggctct ggcgcgggcg tcctcccacc ctccccttcc ttcgggggag tcggtttacc 2520
cgccgcctgc ttgtcttcga cacctgattg gctgtcgaag ctgtgggacc gggcccttgc 2580

tactggctcg agtctcacat gagcgaaacc actgcgcggg gcgcgggggt ggcggggagg 2640

cgggcgttgg tacggtcctc cccgaggccg agcgccgcag tgtctggccc cgcgcccctg 2700

cgcaacgtgg caggaagcgc gcgctggagg cgggggcggg ctgccggccg agacttctgg 2760

atggcggcgg ccgcggctcc gccccgggtt cccaccgcct gaagggcgag acaagcccga 2820

cctgctacag gcactcgtgg gggtggggga ggagcggggg tcggtccggc tggtttgtgg 2880

gtgggaggcg cttgttctcc aaaaaccggc gcgagctgca atcctgaggg agctgcggtg 2940

gaggaggtgg agagaaggcc gcacccttct gggcaggggg aggggagtgc cgcaatacct 3000

ttatgggagt tctttgctgc ctcccgtctt gtaaggaccg ccctgggcct ggaagaagcc 3060

ctccctcctt tcctcctcgc gtgatctcgt catcgcctcc atgtcgagtc gcttctcgat 3120

tatgggcggg attcttttgc ctagacaatt gtactaacct tcttctcttt cctctcctga 3180

cag 3183

<210> 71
<211> 961
<212> DNA
<213> Artificial Sequence

<220>
<223> Intron EF+exon

<400> 71

```
cgggtttgcc gccagaacac aggtaagtgc cgtgtgtggt tcccgcgggc ctggcctctt      60
tacgggttat ggcccttgcg tgccttgaat tacttccacc tggctgcagt acgtgattct     120
tgatcccgag cttcgggttg gaagtgggtg ggagagttcg aggccttgcg cttaaggagc     180
cccttcgcct cgtgcttgag ttgaggcctg gcctgggcgc tggggccgcc gcgtgcgaat     240
ctggtggcac cttcgcgcct gtctcgctgc tttcgataag tctctagcca tttaaaattt     300
ttgatgacct gctgcgacgc tttttttctg gcaagatagt cttgtaaatg cgggccaaga     360
tctgcacact ggtatttcgg tttttggggc cgcgggcggc gacggggccc gtgcgtccca     420
gcgcacatgt tcggcgaggc ggggcctgcg agcgcggcca ccgagaatcg gacgggggta     480
gtctcaagct ggccggcctg ctctggtgcc tggcctcgcg ccgccgtgta tcgccccgcc     540
ctgggcggca aggctggccc ggtcggcacc agttgcgtga gcggaaagat ggccgcttcc     600
cggccctgct gcagggagct caaaatggag gacgcggcgc tcgggagagc gggcgggtga     660
gtcacccaca caaaggaaaa gggcctttcc gtcctcagcc gtcgcttcat gtgactccac     720
ggagtaccgg gcgccgtcca ggcacctcga ttagttctcg agcttttgga gtacgtcgtc     780
tttaggttgg ggggaggggt tttatgcgat ggagtttccc cacactgagt gggtggagac     840
tgaagttagg ccagcttggc acttgatgta attctccttg gaatttgccc tttttgagtt     900
tggatcttgg ttcattctca agcctcagac agtggttcaa agtttttttc ttccatttca     960

g                                                                     961
```

## Description

La présente invention concerne de nouvelles unités de transcription susceptibles d'être utilisées dans des vecteurs d'expression.

A ce jour, l'expression de protéines recombinantes est toujours une des méthodes majeures pour la production des protéines thérapeutiques, telles que des anticorps pharmacologiques.

Les acides nucléiques codant pour les protéines recombinantes sont généralement introduits dans un vecteur d'expression contenant des éléments géniques permettant la transcription et la traduction de ces molécules d'intérêt.

L'un des buts de l'invention est de fournir une unité de transcription permettant de produire une protéine recombinante dont le gain de productivité n'est pas lié à un anticorps de cible antigénique particulière et donc à une protéine recombinante donnée, ni lié au milieu de culture.

L'un des buts de l'invention est de mettre à disposition une unité de transcription universelle permettant de fournir une meilleure capacité de transcription et de traduction d'une protéine d'intérêt par rapport aux vecteurs d'expression traditionnels pour les cellules de mammifères telles que la lignée cellulaire de rat YB2/0 et apparentées, ou la lignée cellulaire CHO et apparentées.

L'un des autres buts de l'invention est de fournir une unité de transcription permettant de limiter la taille de vecteur d'expression, afin de limiter les problèmes de clonage, d'efficacité de transfection dans les lignées d'expression ou encore d'interférence entre le vecteur d'expression et le génome de la lignée receveuse pouvant conduire à une instabilité génétique et à l'extinction du gène d'intérêt.

Enfin, l'un des autres buts est de fournir une unité de transcription dépourvue des promoteurs viraux, afin de limiter les risques sanitaires potentiels.

La présente invention concerne des unités de transcription pour construire les vecteurs d'expression.

L'invention est illustrée par une unité de transcription constituée d'un polynucléotide comprenant les éléments régulateurs suivants :

(i) - l'enhancer du virus hCMVie (E2), ledit enhancer ayant la séquence nucléotidique SEQ ID NO : 1, ou

- un acide nucléotidique ayant au moins 70% d'identité de séquence avec la séquence SEQ ID NO : 1 et possédant essentiellement des propriétés d'activation de la transcription, et

(ii) - la région promotrice de la Cyclin-Dependant Kinase 9 (CDK9), ladite région promotrice ayant la séquence nucléotidique SEQ ID NO : 2, ou

- un acide nucléotidique ayant au moins 70% d'identité de séquence avec la séquence SEQ ID NO: 2 et possédant essentiellement une activité promotrice de la transcription.

Selon un aspect général, l'invention concerne une unité de transcription constituée d'un polynucléotide comprenant les éléments régulateurs suivants :

(i) - l'enhancer du virus hCMVie (E2), ledit enhancer ayant la séquence nucléotidique SEQ ID NO : 1, et
(ii) - la région promotrice de la Cyclin-Dependant Kinase 9 (CDK9), ladite région promotrice ayant la séquence nucléotidique SEQ ID NO : 2.

On entend par « **éléments régulateurs »** dans le sens de la présente invention, des éléments géniques non codants permettant de contrôler la transcription et/ou la traduction d'un acide nucléique codant pour une protéine d'intérêt.

On entend par « **unité de transcription** » un polynucléotide contenant les éléments régulateurs nécessaires à la transcription d'un acide nucléique d'intérêt en ARN. Une ARN polymérase, qui permet de synthétiser un ARNm à partir d'un gène d'intérêt lié à ladite unité de transcription, ainsi que des facteurs de transcription activateurs ou inhibiteurs qui vont moduler en + ou en - la transcription en ARNm, peuvent se fixer sur une telle unité de transcription.

On entend par « **région promotrice** », une région d'ADN qui contient une séquence d'ADN particulière permettant d'initier la transcription d'un gène d'intérêt particulier.

Au sens de la présente invention, les termes « région promotrice » et « promoteur » peuvent être remplacés l'un par l'autre.

La région promotrice contient la zone de l'ADN sur laquelle se fixe initialement l'ARN polymérase, avant de déclencher la synthèse de l'ARN.

Un promoteur est en général proche (d'une vingtaine à une centaine de nucléotides) de l'acide nucléique d'intérêt à contrôler et est situé en amont du site de démarrage de la transcription d'un gène. La présence d'un promoteur est essentielle pour la transcription d'un gène particulier.

Le promoteur du gène CDK9 représenté par la séquence SEQ ID NO : 2 est un promoteur riche en GC et dépourvu de TATA box.

« Un acide nucléotidique ayant au moins 70% d'identité de séquence avec la séquence SEQ ID : NO 2 et possédant essentiellement une activité promotrice » contenu dans une unité de transcription selon la présente divulgation est un acide nucléotidique possédant essentiellement la même capacité pour initier la transcription de gène que celle de la région promotrice du gène CDK9, représentée par la séquence SEQ ID NO : 2.

La capacité de la région promotrice du gène CDK9 pour initier la transcription d'un gène peut être déterminée selon la méthode décrite par Liu et al. (Gene 252, 51-59 (2000)).

On entend par « **enhancer** », un segment d'ADN qui peut fixer des protéines comme les facteurs de transcription pour stimuler la transcription d'un gène. Un enhancer n'est pas nécessairement proche du gène d'intérêt à contrôler, et peut être situé en 5' ou en 3', ou même au milieu du gène à contrôler ou dans un intron.

La présence d'un enhancer dans un vecteur d'expression permet d'augmenter le niveau de transcription d'un gène.

«Un acide nucléotidique ayant au moins 70% d'identité de séquence avec la séquence SEQ ID : NO 1 et possédant essentiellement des propriétés d'activation de la transcription » est un acide nucléotidique possédant essentiellement la même capacité pour stimuler la transcription de gène que celui de l'enhancer du virus hCMVie représenté par la séquence SEQ ID NO : 1, désigné ci-après également E2.

Les propriétés d'activation de la transcription d'un gène peuvent être déterminées par l'utilisation des gènes rapporteurs comme la luciférase.

Plusieurs enhancers peuvent coexister dans une unité de transcription selon la présente invention ; cela permet de stimuler davantage la transcription de gène.

Une unité de transcription selon la présente divulgation peut comprendre :

- l'enhancer du virus hCMVie, ledit enhancer ayant la séquence nucléotidique SEQ ID NO : 1 (E2), ou un acide nucléotidique ayant au moins 70% d'identité de séquence avec la séquence SEQ ID NO : 1 et possédant essentiellement des propriétés d'activation de la transcription, et
- au moins un autre enhancer choisi parmi un SV40 enhancer et un Eμ enhancer.

Une unité de transcription selon la présente invention peut comprendre :

- l'enhancer du virus hCMVie, ledit enhancer ayant la séquence nucléotidique SEQ ID NO : 1 (E2), et
- au moins un autre enhancer choisi parmi un SV40 enhancer et un Eμ enhancer.

Dans ce qui précède et dans ce qui suit, le pourcentage d'identité entre deux séquences d'acides nucléiques peut être calculé selon la formule suivante :

$$\frac{\text{le nombre des résidus identiques x 100}}{\text{le nombre des résidus de la séquence la plus courte}}$$

Dans un mode de réalisation particulier de l'invention, l'enhancer se situe en amont de la région promotrice. Autrement dit, l'enhancer est situé à l'extrémité 5' de l'ADN de la région promotrice, pour faciliter le clonage des séquences codantes dans le vecteur d'expression. L'enhancer est un élément génique non positionnel.

Une unité de transcription selon la présente divulgation est constituée d'un polynucléotide comprenant les éléments régulateurs suivants :

(i) - l'enhancer du virus hCMVie (E2), ledit enhancer ayant la séquence nucléotidique SEQ ID NO : 1, ou

- un acide nucléotidique ayant au moins 70% d'identité de séquence avec la séquence SEQ ID NO : 1 et possédant essentiellement des propriétés d'activation de la transcription, et

(ii) - la région promotrice de la Cyclin-Dependant Kinase 9 (CDK9), ladite région promotrice ayant la séquence nucléotidique SEQ ID NO : 2, ou

- un acide nucléotidique ayant au moins 70% d'identité de séquence avec la séquence SEQ ID : NO 2 et possédant essentiellement une activité promotrice, l'enhancer étant situé en amont de la région promotrice.

Dans un mode de réalisation plus particulier de l'invention, une unité de transcription selon la présente invention est constituée d'un polynucléotide comprenant les éléments régulateurs suivants :

(i) - l'enhancer du virus hCMVie (E2), ledit enhancer ayant la séquence nucléotidique SEQ ID NO : 1, et
(ii) - la région promotrice de la Cyclin-Dependant Kinase 9 (CDK9), ladite région promotrice ayant la séquence nucléotidique SEQ ID NO : 2, l'enhancer étant situé en amont de la région promotrice.

Une unité de transcription selon la présente invention peut comprendre également un acide nucléotidique situé en aval de la région promotrice et en amont du site d'initiation de la traduction, ledit acide nucléotidique comprenant au moins l'une des régions 5' non traduite (5' UTR) choisie parmi les suivantes :

(i) - la région régulatrice R du 5' Long Terminal Repeat (LTR) (RU-5') du virus HTLV-1 ayant la séquence nucléotidique SEQ ID NO : 3 (U1), ou

- un acide nucléotidique ayant au moins 70% d'identité de séquence avec la séquence SEQ ID NO : 3,

(ii) - la région 5' UTR du gène NF-κB Repressing Factor (NRF) ayant la séquence nucléotidique SEQ ID NO : 4 (U2), ou

- un acide nucléotidique ayant au moins 70% d'identité de séquence avec la séquence SEQ ID NO : 4,

(iii) - la région 5' UTR du gène eukaryotic Initiation Factor 4GI (eIF4GI) ayant la séquence nucléotidique SEQ ID NO : 5 (U3), ou

- un acide nucléotidique ayant au moins 70% d'identité de séquence avec la séquence SEQ ID NO : 5,

les susdits acides nucléotidiques ayant au moins 70% d'identité de séquence avec l'une des séquences représentées par les séquences SEQ ID NO : 3, SEQ ID NO : 4 ou SEQ ID NO : 5 et ayant essentiellement des propriétés de stabilisation des ARNm et de facilitateur de la traduction.

Les propriétés de stabilisation des ARNm et de facilitateur de la traduction peuvent être mesurées par Fritz et al (Sci. STKE, 5 December 2000Vol. 2000, Issue 61, p. p11) et Ross et al (Microbiol Rev. 1995 Sep; 59(3):423-50).

La facilitation de la traduction peut se faire en comparant la quantité d'ARNm qui reste constante analysée par q-RT-PCR tout en montrant une augmentation du taux de protéine.

La région 5' non traduite dans un gène correspond à la portion de l'ARN messager (ARNm) placée en amont du site d'initiation de la traduction. Cette région permet la fixation de ribosome et peut être impliquée dans la régulation de l'expression du gène concerné.

Le site d'initiation de la traduction est un triplet de nucléotides qui dirige l'initiation de la traduction protéique. Ce triplet est souvent le triplet ATG.

« Les acides nucléotidiques ayant au moins 70% d'identité de séquence avec l'une des séquences représentées par les séquences SEQ ID NO : 3, SEQ ID NO : 4 ou SEQ ID NO : 5 » contenus dans les unités de transcription selon la présente invention permettent la fixation de ribosome et la stabilisation des ARNm.

Le susdit acide nucléotidique situé en aval de la région promotrice et en amont du site d'initiation de la traduction peut comprendre une seule région 5'UTR choisie parmi :

(i) - la région R du Long Terminal Repeat (LTR) du virus HTLV-1 ayant la séquence nucléotidique SEQ ID NO : 3 (U1), ou

- un acide nucléotidique ayant au moins 70% d'identité de séquence avec la séquence SEQ ID NO : 3,

(ii) - la région 5' UTR du gène NF-κB Repressing Factor (NRF) ayant la séquence nucléotidique SEQ ID NO : 4 (U2), ou

- un acide nucléotidique ayant au moins 70% d'identité de séquence avec la séquence SEQ ID NO : 4,

(iii) - la région 5' UTR du gène eukaryotic Initiation Factor 4GI (eIF4GI) ayant la séquence nucléotidique SEQ ID NO : 5 (U3), ou

- un acide nucléotidique ayant au moins 70% d'identité de séquence avec la séquence SEQ ID NO : 5.

On entend par une région 5'UTR « situé en aval de la région promotrice et en amont du site d'initiation de la traduction» une région 5'UTR situé après l'extrémité 3' de l'ADN de la région promotrice et avant l'extrémité 5'de l'ADN du site d'initiation de la traduction.

Le susdit acide nucléotidique situé en aval de la région promotrice et en amont du site d'initiation de la traduction peut comprendre deux régions 5'UTR.

La présence de deux ou plusieurs régions 5'UTR dans une unité de transcription selon l'invention permet d'accumuler ou de synergiser les effets positifs sur la stabilité des ARNm et l'efficacité de traduction.

Un susdit acide nucléotidique utilisé dans une unité de transcription selon la présente invention peut comprendre la région R du Long Terminal Repeat (LTR) du virus HTLV-1 et la région 5' UTR du gène NF-κB Repressing Factor (NRF), ledit acide nucléotidique étant représenté par la séquence SEQ ID NO : 6, ou étant un acide nucléotidique ayant au moins 70% d'identité de séquence avec la séquence SEQ ID NO : 6.

Un susdit acide nucléotidique utilisé dans une unité de transcription selon la présente invention peut également comprendre la région R du Long Terminal Repeat (LTR) du virus HTLV-1 et la région 5' UTR du gène eukaryotic Initiation Factor 4GI (eIF4GI), ledit acide nucléotidique étant représenté par la séquence SEQ ID NO: 7, ou étant un acide nucléotidique ayant au moins 70% d'identité de séquence avec la séquence SEQ ID NO : 7.

Un susdit acide nucléotidique utilisé dans une unité de transcription selon la présente invention peut également comprendre la région 5' UTR du gène NF-κB Repressing Factor (NRF) et la région 5' UTR du gène eukaryotic Initiation Factor 4GI (eIF4GI), ledit acide nucléotidique étant représenté par la séquence SEQ ID NO : 8 ou étant un acide nucléotidique ayant au moins 70% d'identité de séquence avec la séquence SEQ ID NO : 8.

Le susdit acide nucléotidique situé en aval de la région promotrice et en amont du site d'initiation de la traduction peut aussi comprendre trois régions 5'UTR, à savoir la région R du Long Terminal Repeat (LTR) du virus HTLV-1, la région 5' UTR du gène NF-κB Repressing Factor (NRF) et la région 5' UTR du gène eukaryotic Initiation Factor 4GI (eIF4GI), ledit acide nucléotidique étant représenté par la séquence SEQ ID NO : 9 ou étant un acide nucléotidique ayant au moins 70% d'identité de séquence avec la séquence SEQ ID NO: 9.

Une unité de transcription selon la présente divulgation est constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) la région R du Long Terminal Repeat (LTR) du virus HTLV-1 représentée par la séquence nucléotidique SEQ ID NO : 3, ou un acide nucléotidique ayant au moins 70% d'identité de séquence avec la séquence SEQ ID NO : 3,

ladite région 5' UTR étant située en aval de la région promotrice et en amont du site d'initiation de la traduction.

Dans un mode de réalisation particulier de l'invention, une unité de transcription selon la présente invention est constituée d'un polynucléotide comprenant les éléments régulateurs suivants :

(i) l'enhancer du virus hCMVie représenté par la séquence nucléotidique SEQ ID NO:1,
(ii) la région promotrice de la Cyclin-Dependent Kinase 9 (CDK9) représentée par la séquence nucléotidique SEQ ID NO : 2, et
(iii) la région R du Long Terminal Repeat (LTR) du virus HTLV-1 représentée par la séquence nucléotidique SEQ ID NO : 3, ou un acide nucléotidique ayant au moins 70% d'identité de séquence avec la séquence SEQ ID NO : 3,

ladite région 5' UTR étant située en aval de la région promotrice et en amont du site d'initiation de la traduction.

Les avantages des éléments combinés sont apportés avec une potentielle synergie entre la région 5'UTR et les autres éléments dans une unité de transcription.

Dans un mode de réalisation plus particulier, une unité de transcription selon l'invention est constituée d'un polynucléotide comprenant un acide nucléotidique représenté par la séquence SEQ ID NO : 14 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5'UTR du LTR du virus HTLV-1, représentée par la séquence SEQ ID NO:3,

ou d'un acide nucléotidique ayant au moins 70% d'identité de séquence avec la séquence SEQ ID NO : 14.

Une unité de transcription selon la présente divulgation est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin Dependent Kinase 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) la région 5' UTR du gène NF-κB Repressing Factor (NRF) représentée par la séquence nucléotidique SEQ ID NO : 4, ou un acide nucléotidique ayant au moins 70% d'identité de séquence avec la séquence SEQ ID NO : 4,

ladite région 5' UTR étant située en aval de la région promotrice et en amont du site d'initiation de la traduction.
Dans un autre mode de réalisation particulier de l'invention, une unité de transcription selon la présente invention est constituée d'un polynucléotide comprenant les éléments régulateurs suivants :

(i) l'enhancer du virus hCMVie représentée par la séquence nucléotidique SEQ ID NO : 1,
(ii) la région promotrice de la Cyclin Dependent Kinase 9 (CDK9) représentée par la séquence nucléotidique SEQ ID NO : 2, et
(iii) la région 5' UTR du gène NF-κB Repressing Factor (NRF) représentée par la séquence nucléotidique SEQ ID NO : 4, ou un acide nucléotidique ayant au moins 70% d'identité de séquence avec la séquence SEQ ID NO : 4,

ladite région 5' UTR étant située en aval de la région promotrice et en amont du site d'initiation de la traduction.
Dans un mode de réalisation plus particulier, une unité de transcription selon l'invention est constituée d'un polynucléotide comprenant un acide nucléotidique représenté par la séquence SEQ ID NO : 15 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5' UTR du gène NRF, représentée par la séquence SEQ ID NO : 4,

ou un acide nucléotidique ayant au moins 70% d'identité de séquence avec la séquence SEQ ID NO : 15.
Une unité de transcription selon la présente divulgation est constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) la région 5' UTR du gène eukaryotic Initiation Factor 4GI (eIF4GI) représentée par la séquence nucléotidique SEQ ID NO : 5, ou un acide nucléotidique ayant au moins 70% d'identité de séquence avec la séquence SEQ ID NO : 5,

ladite région 5' UTR étant située en aval de la région promotrice et en amont du site d'initiation de la traduction.
Dans un autre mode de réalisation particulier de l'invention, une unité de transcription selon la présente invention est constituée d'un polynucléotide comprenant les éléments régulateurs suivants :

(i) l'enhancer du virus hCMVie représenté par la séquence nucléotidique SEQ ID NO:1,
(ii) la région promotrice de la Cyclin-Dependent Kinase 9 (CDK9) représentée par la séquence nucléotidique SEQ ID NO : 2, et
(iii) la région 5' UTR du gène eukaryotic Initiation Factor 4GI (eIF4GI) représentée par la séquence nucléotidique SEQ ID NO : 5, ou un acide nucléotidique ayant au moins 70% d'identité de séquence avec la séquence SEQ ID NO : 5,

ladite région 5' UTR étant située en aval de la région promotrice et en amont du site d'initiation de la traduction.
Dans un mode de réalisation plus particulier, une unité de transcription selon l'invention est constituée d'un polynucléotide comprenant un acide nucléotidique représenté par la séquence SEQ ID NO : 16 et constitué de :

(i) l'enhancer du virus hCMVie représenté par la séquence SEQ ID NO : 1,
(ii) la région promotrice du gène CDK9 représentée par la séquence SEQ ID NO : 2, et

(iii) la région 5' UTR du gène eIF4GI représentée par la séquence SEQ ID NO : 5,

ou d'un acide nucléotidique ayant au moins 70% d'identité de séquence avec la séquence SEQ ID NO : 16.

Une unité de transcription selon la présente divulgation peut comprendre deux régions 5'UTR. Une telle unité de transcription est constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région R du Long Terminal Repeat (LTR) du virus HTLV-1 représentée par la séquence nucléotidique SEQ ID NO : 3, ou un acide nucléotidique ayant au moins 70% d'identité de séquence avec la séquence SEQ ID NO : 3, et
(iv) la région 5' UTR du gène NF-κB Repressing Factor (NRF) représentée par la séquence nucléotidique SEQ ID NO : 4, ou un acide nucléotidique ayant au moins 70% d'identité de séquence avec la séquence SEQ ID NO : 4,

les régions 5' UTR étant situées en aval de la région promotrice et en amont du site d'initiation de la traduction.

Dans un autre mode de réalisation particulier de l'invention, une unité de transcription selon la présente invention peut comprendre deux régions 5'UTR. Une telle unité de transcription est constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région R du Long Terminal Repeat (LTR) du virus HTLV-1 représentée par la séquence nucléotidique SEQ ID NO : 3, ou un acide nucléotidique ayant au moins 70% d'identité de séquence avec la séquence SEQ ID NO : 3, et
(iv) la région 5' UTR du gène NF-κB Repressing Factor (NRF) représentée par la séquence nucléotidique SEQ ID NO : 4, ou un acide nucléotidique ayant au moins 70% d'identité de séquence avec la séquence SEQ ID NO : 4,

les régions 5' UTR étant situées en aval de la région promotrice et en amont du site d'initiation de la traduction.

Dans un mode de réalisation plus particulier, une unité de transcription selon l'invention est constituée d'un polynucléotide comprenant un acide nucléotidique représenté par la séquence SEQ ID NO: 17 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5' UTR représentée par la séquence SEQ ID NO : 6,

ou d'un acide nucléotidique ayant au moins 70% d'identité de séquence avec la séquence SEQ ID NO : 17.

Une unité de transcription selon la présente divulgation peut comprendre deux régions 5'UTR. Une telle unité de transcription est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3, et
(iv) la région 5' UTR du gène eukaryotic Initiation Factor 4GI (eIF4GI) ayant la séquence nucléotidique SEQ ID NO : 5, ou un acide nucléotidique ayant au moins 70% d'identité de séquence avec la séquence SEQ ID NO : 5,

les régions 5' UTR étant situées en aval de la région promotrice et en amont du site d'initiation de la traduction.

Dans un autre mode de réalisation particulier de l'invention, une unité de transcription selon la présente invention peut comprendre deux régions 5'UTR. Une telle unité de transcription est constituée d'un polynucléotide comprenant les éléments régulateurs suivants :

(i) l'enhancer du virus hCMVie représentée par la séquence nucléotidique SEQ ID NO:1,
(ii) la région promotrice de la Cyclin-Dependent Kinase 9 (CDK9) représentée par la séquence nucléotidique SEQ ID NO : 2,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3, et
(iv) la région 5' UTR du gène eukaryotic Initiation Factor 4GI (eIF4GI) ayant la séquence nucléotidique SEQ ID NO : 5, ou un acide nucléotidique ayant au moins 70% d'identité de séquence avec la séquence SEQ ID NO : 5,

les régions 5' UTR étant situées en aval de la région promotrice et en amont du site d'initiation de la traduction.

Dans un mode de réalisation plus particulier, une unité de transcription selon l'invention est constituée d'un polynucléotide comprenant un acide nucléotidique représenté par la séquence SEQ ID NO : 18 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5' UTR représentée par la séquence SEQ ID NO : 7,

ou d'un acide nucléotidique ayant au moins 70% d'identité de séquence avec la séquence SEQ ID NO : 18.

Une unité de transcription selon la présente divulgation peut comprendre deux régions 5'UTR. Une telle unité de transcription est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(iv) la région 5' UTR du gène eukaryotic Initiation Factor 4GI (eIF4GI) ayant la séquence nucléotidique SEQ ID NO : 5, ou un acide nucléotidique ayant au moins 70% d'identité de séquence avec la séquence SEQ ID NO : 5,

les régions 5' UTR étant situées en aval de la région promotrice et en amont du site d'initiation de la traduction.

Dans un autre mode de réalisation particulier de l'invention, une unité de transcription selon la présente invention peut comprendre deux régions 5'UTR. Une telle unité de transcription est constituée d'un polynucléotide comprenant les éléments régulateurs suivants :

(i) l'enhancer du virus hCMVie représentée par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(iv) la région 5' UTR du gène eukaryotic Initiation Factor 4GI (eIF4GI) ayant la séquence nucléotidique SEQ ID NO : 5, ou un acide nucléotidique ayant au moins 70% d'identité de séquence avec la séquence SEQ ID NO : 5,

les régions 5' UTR étant situées en aval de la région promotrice et en amont du site d'initiation de la traduction.

Dans un mode de réalisation plus particulier, une unité de transcription selon l'invention est constituée d'un polynucléotide comprenant un acide nucléotidique représenté par la séquence SEQ ID NO : 19 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5' UTR représentée par la séquence SEQ ID NO : 8,

ou d'un acide nucléotidique ayant au moins 70% d'identité de séquence avec la séquence SEQ ID NO : 19.

Une unité de transcription selon la présente divulgation peut comprendre trois régions 5'UTR. Une telle unité de transcription est constituée d'un polynucléotide comprenant les éléments régulateurs suivants :

(i) l'enhancer du virus hCMVie représentée par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3,
(iv) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(v) la région 5' UTR du gène eukaryotic Initiation Factor 4GI (eIF4GI) ayant la séquence nucléotidique SEQ ID NO : 5, ou un acide nucléotidique ayant au moins 70% d'identité de séquence avec la séquence SEQ ID NO : 5,

les régions 5' UTR étant situées en aval de la région promotrice et en amont du site d'initiation de la traduction.

Dans un autre mode de réalisation particulier de l'invention, une unité de transcription selon la présente invention peut comprendre trois régions 5'UTR. Une telle unité de transcription est constituée d'un polynucléotide comprenant les éléments régulateurs suivants :

(i) l'enhancer du virus hCMVie représentée par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3,
(iv) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(v) la région 5' UTR du gène eukaryotic Initiation Factor 4GI (eIF4GI) ayant la séquence nucléotidique SEQ ID NO : 5, ou un acide nucléotidique ayant au moins 70% d'identité de séquence avec la séquence SEQ ID NO : *5,*

les régions 5' UTR étant situées en aval de la région promotrice et en amont du site d'initiation de la traduction.

Dans un mode de réalisation plus particulier, une unité de transcription selon l'invention est constituée d'un polynucléotide comprenant un acide nucléotidique représenté par la séquence SEQ ID NO : 20 et constitué de :

(i) l'enhancer du virus hCMVie représenté par la séquence SEQ ID NO : 1,
(ii) la région promotrice du gène CDK9 représentée par la séquence SEQ ID NO : 2, et
(iii) la région 5' UTR représentée par la séquence SEQ ID NO : 9,

ou d'un acide nucléotidique ayant au moins 70% d'identité de séquence avec la séquence SEQ ID NO : 20.

Une unité de transcription selon la présente invention peut comprendre également un ou plusieurs introns situés en aval de ladite région promotrice.

On entend par « intron », une partie non codante d'un gène. Un intron est situé souvent entre deux exons. Après la transcription, cette partie est excisée de l'ARN pré-messager (épissage des introns) pour donner l'ARN messager. La présence d'un intron hétérologue permet d'optimiser l'expression des gènes exogènes dans une construction d'ADN. En effet ce dernier peut contenir des éléments de régulation qui peuvent stabiliser l'ARNm ou favoriser sa transcription.

Dans la construction d'une unité de transcription selon la présente invention, un ou plusieurs introns peuvent être situés :

(i) en aval de la région 5' UTR et en amont du site d'initiation de la traduction, et/ou
(ii) en aval du promoteur et en amont de la région 5'UTR, et/ou
(iii) après le site d'initiation de la traduction et à l'intérieur d'une séquence codante, et/ou
(iv) entre le codon stop de la séquence codante et le signal de polyadénylation.

Lorsqu'un intron est situé après le site d'initiation de la traduction et à l'intérieur d'une séquence codante, il est important de ne pas changer le cadre de lecture de l'ARNm lors de la traduction et de conserver les sites donneurs et accepteurs ainsi que la boite de branchement de séquence (UAUAAC) permettant l'épissage par le splicéosome.

On entend par « un intron situé en aval de ladite région promotrice » un intron situé en 3' de l'ADN de la région promotrice.

Ledit intron peut être choisi parmi les suivants :

- l'intron du gène Elongation Factor 1α (EF1α) ayant la séquence nucléotidique SEQ ID NO : 10, ou un acide nucléotidique présentant au moins 70% d'identité de séquence avec la séquence SEQ ID NO : 10, telle que la séquence SEQ ID NO : 71.

- l'intron ROSA murin ayant la séquence nucléotidique SEQ ID NO : 11, ou un acide nucléotidique ayant au moins 70% d'identité de séquence avec la séquence SEQ ID NO : 11,
- intron 5'-Long Terminal Repeat (5'-LTR) du virus HTLV-1 ayant la séquence nucléotidique SEQ ID NO : 12, ou un acide nucléotidique présentant au moins 70% d'identité de séquence avec la séquence SEQ ID NO: 12,
- intron chimérique pCI-neo ayant la séquence nucléotidique SEQ ID NO : 13, ou un acide nucléotidique présentant au moins 70% d'identité de séquence avec la séquence SEQ ID NO : 13,
- intron du gène de l'ubiquitine ayant la séquence nucléotidique SEQ ID NO : 53, ou un acide nucléotidique présentant au moins 70% d'identité de séquence avec la séquence SEQ ID NO : 53,
- intron du gène humain ROSA ayant la séquence nucléotidique SEQ ID NO : 54, ou un acide nucléotidique présentant au moins 70% d'identité de séquence avec la séquence SEQ ID NO : 54.

L'acide nucléotidique représenté par la séquence SEQ ID NO : 10 est désigné dans la présente demande par « EF1$\alpha$ » ou « EFss ».

L'acide nucléotidique représenté par la séquence SEQ ID NO : 71 est désigné dans la présente demande par « EF1$\alpha$ avec exon» ou «EF». Cet acide nucléotidique contient l'intron EF1$\alpha$ de la séquence SEQ ID NO : 10 et une séquence exonique en région 5'.

Une unité de transcription selon la présente divulgation peut comprendre :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) une région promotrice de la Cyclin-Dependent Kinase 9 (CDK9), ladite région promotrice ayant la séquence nucléotidique SEQ ID NO : 2, ou un acide nucléotidique ayant au moins 70% d'identité de séquence avec la séquence SEQ ID NO : 2 et possédant essentiellement une activité promotrice, et
(iii) un intron choisi parmi :

- l'intron du gène Elongation Factor 1$\alpha$ (EF1$\alpha$) ayant la séquence nucléotidique SEQ ID NO : 10, ou un acide nucléotidique présentant au moins 70% d'identité de séquence avec la séquence SEQ ID NO : 10,
- l'intron murin ROSA ayant la séquence nucléotidique SEQ ID NO : 11, ou un acide nucléotidique ayant au moins 70% d'identité de séquence avec la séquence SEQ ID NO : 11,
- l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12,
- l'intron chimérique pCI-neo ayant la séquence nucléotidique SEQ ID NO : 13, ou un acide nucléotidique présentant au moins 70% d'identité de séquence avec la séquence SEQ ID NO : 13,
- intron du gène l'ubiquitine ayant la séquence nucléotidique SEQ ID NO : 53, ou un acide nucléotidique présentant au moins 70% d'identité de séquence avec la séquence SEQ ID NO : 53,
- intron du gène humain ROSA ayant la séquence nucléotidique SEQ ID NO : 54, ou un acide nucléotidique présentant au moins 70% d'identité de séquence avec la séquence SEQ ID NO : 54.

ledit enhancer étant situé en 5' ou en 3' de l'unité de transcription, ou à l'intérieur de la séquence codante au niveau d'un intron ;
ledit intron étant situé :

(i) en aval de la région 5' UTR et en amont du site d'initiation de la traduction, ou
(ii) en aval du promoteur et en amont de la région 5'UTR, ou
(iii) après le site d'initiation de la traduction et à l'intérieur de la séquence codante, ou
(iv) entre le codon stop de la séquence codante et le signal de polyadénylation.

Une unité de transcription selon la présente invention peut comprendre :

(i) l'enhancer du virus hCMVie représenté par la séquence nucléotidique SEQ ID NO : 1,
(ii) une région promotrice de la Cyclin-Dependent Kinase 9 (CDK9), ladite région promotrice ayant la séquence nucléotidique SEQ ID NO : 2, et
(iii) un intron choisi parmi :

- l'intron du gène Elongation Factor 1$\alpha$ (EF1$\alpha$) ayant la séquence nucléotidique SEQ ID NO : 10, ou un acide nucléotidique présentant au moins 70% d'identité de séquence avec la séquence SEQ ID NO : 10,

- l'intron murin ROSA ayant la séquence nucléotidique SEQ ID NO : 11, ou un acide nucléotidique ayant au moins 70% d'identité de séquence avec la séquence SEQ ID NO : 11,
- l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12,
- l'intron chimérique pCI-neo ayant la séquence nucléotidique SEQ ID NO : 13, ou un acide nucléotidique présentant au moins 70% d'identité de séquence avec la séquence SEQ ID NO : 13,
- intron du gène l'ubiquitine ayant la séquence nucléotidique SEQ ID NO : 53, ou un acide nucléotidique présentant au moins 70% d'identité de séquence avec la séquence SEQ ID NO : 53,
- intron du gène humain ROSA ayant la séquence nucléotidique SEQ ID NO : 54, ou un acide nucléotidique présentant au moins 70% d'identité de séquence avec la séquence SEQ ID NO : 54.

ledit enhancer étant situé en 5' ou en 3' de l'unité de transcription, ou à l'intérieur de la séquence codante au niveau d'un intron ;
ledit intron étant situé :

(i) en aval de la région 5' UTR et en amont du site d'initiation de la traduction, ou
(ii) en aval du promoteur et en amont de la région 5'UTR, ou
(iii) après le site d'initiation de la traduction et à l'intérieur de la séquence codante, ou
(iv) entre le codon stop de la séquence codante et le signal de polyadénylation.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) l'intron du gène Elongation Factor 1$\alpha$ (EF1$\alpha$) ayant la séquence nucléotidique SEQ ID NO : 10, ou un acide nucléotidique présentant au moins 70% d'identité de séquence avec la séquence SEQ ID NO : 10.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO: 1, et
(ii) la région promotrice de la Cyclin-Dependent Kinase 9 (CDK9) représentée par la séquence nucléotidique SEQ ID NO : 2, et
(iii) l'intron du gène Elongation Factor 1$\alpha$ (EF1$\alpha$) ayant la séquence nucléotidique SEQ ID NO : 10, ou un acide nucléotidique présentant au moins 70% d'identité de séquence avec la séquence SEQ ID NO : 10.

Dans un mode de réalisation plus particulier de l'invention, une unité de transcription selon l'invention est constituée d'un polynucléotide comprenant un acide nucléotidique représenté par la séquence SEQ ID NO : 21 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) l'intron du gène EF1$\alpha$ représenté par la séquence nucléotidique SEQ ID NO : 10, ou d'un acide nucléotidique ayant au moins 70% d'identité de séquence avec la séquence SEQ ID NO: 21.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) l'intron murin ROSA ayant la séquence nucléotidique SEQ ID NO : 11, ou un acide nucléotidique ayant au moins 70% d'identité de séquence avec la séquence SEQ ID NO : 11.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO:1, et
(ii) la région promotrice de la Cyclin-Dependent Kinase 9 (CDK9) représentée par la séquence nucléotidique SEQ ID NO : 2, et
(iii) l'intron murin ROSA ayant la séquence nucléotidique SEQ ID NO : 11, ou un acide nucléotidique ayant au moins 70% d'identité de séquence avec la séquence SEQ ID NO : 11.

Dans un mode de réalisation plus particulier de l'invention, une unité de transcription selon l'invention est constituée d'un polynucléotide comprenant un acide nucléotidique représenté par la séquence SEQ ID NO : 22 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) l'intron murin ROSA ayant la séquence nucléotidique SEQ ID NO : 11,

ou d'un acide nucléotidique ayant au moins 70% d'identité de séquence avec la séquence SEQ ID NO : 22.
La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice, et
(iii) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2, et
(iii) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Dans un mode de réalisation plus particulier de l'invention, une unité de transcription selon l'invention est constituée d'un polynucléotide comprenant un acide nucléotidique représenté par la séquence SEQ ID NO : 23 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) l'intron 5'LTR du virus HTLV-1 ayant la séquence nucléotidique SEQ ID NO : 12,

ou un acide nucléotidique présentant au moins 70% d'identité de séquence avec la séquence SEQ ID NO : 23.
La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) l'intron pCI-neo ayant la séquence nucléotidique SEQ ID NO : 13, ou un acide nucléotidique présentant au moins 70% d'identité de séquence avec la séquence SEQ ID NO : 13.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2, et
(iii) l'intron pCI-neo ayant la séquence nucléotidique SEQ ID NO : 13, ou un acide nucléotidique présentant au moins 70% d'identité de séquence avec la séquence SEQ ID NO : 13.

Dans un mode de réalisation plus particulier de l'invention, une unité de transcription selon l'invention est constituée d'un polynucléotide comprenant un acide nucléotidique représenté par la séquence SEQ ID NO : 24 et constitué de :

(i) l'enhancer du virus hCMVie représenté par la séquence nucléotidique SEQ ID NO:1,
(ii) la région promotrice du gène CDK9 représentée par la séquence nucléotidique SEQ ID NO : 2, et
(iii) l'intron chimérique pCI-neo représenté par la séquence nucléotidique SEQ ID NO : 13,

ou d'un acide nucléotidique ayant au moins 70% d'identité de séquence avec la séquence SEQ ID NO : 24.
La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) l'intron du gène ubiquitine ayant la séquence nucléotidique SEQ ID NO : 53, ou un acide nucléotidique présentant au moins 70% d'identité de séquence avec la séquence SEQ ID NO : 53.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2, et
(iii) l'intron du gène ubiquitine ayant la séquence nucléotidique SEQ ID NO : 53, ou un acide nucléotidique présentant au moins 70% d'identité de séquence avec la séquence SEQ ID NO : 53.

Dans un mode de réalisation plus particulier de l'invention, une unité de transcription selon l'invention est constituée d'un polynucléotide comprenant un acide nucléotidique représenté par la séquence SEQ ID NO : 55 et constitué de :

(i) l'enhancer du virus hCMVie représenté par la séquence nucléotidique SEQ ID NO: 1,
(ii) la région promotrice du gène CDK9 représentée par la séquence nucléotidique SEQ ID NO : 2, et
(iii) l'intron du gène ubiquitine représenté par la séquence nucléotidique SEQ ID NO : 53,

ou d'un acide nucléotidique ayant au moins 70% d'identité de séquence avec la séquence SEQ ID NO : 55.
La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID : NO 2 et possédant essentiellement une activité promotrice, et
(iii) l'intron humain ROSA ayant la séquence nucléotidique SEQ ID NO : 54, ou un acide nucléotidique présentant au moins 70% d'identité de séquence avec la séquence SEQ ID NO : 54.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2, et
(iii) l'intron humain ROSA ayant la séquence nucléotidique SEQ ID NO : 54, ou un acide nucléotidique présentant au moins 70% d'identité de séquence avec la séquence SEQ ID NO : 54.

Dans un mode de réalisation plus particulier de l'invention, une unité de transcription selon l'invention est constituée d'un polynucléotide comprenant un acide nucléotidique représenté par la séquence SEQ ID NO : 56 et constitué de :

(i) l'enhancer du virus hCMVie représenté par la séquence nucléotidique SEQ ID NO : 1,
(ii) la région promotrice du gène CDK9 représentée par la séquence nucléotidique SEQ ID NO : 2, et
(iii) l'intron humain ROSA représenté par la séquence nucléotidique SEQ ID NO : 54,

ou d'un acide nucléotidique ayant au moins 70% d'identité de séquence avec la séquence SEQ ID NO : 56.
Une unité de transcription selon la présente divulgation peut comprendre :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) une région promotrice de la Cyclin-Dependent Kinases 9 (CDK9), ladite région promotrice ayant la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) au moins l'une des régions 5' non traduite (5' UTR) choisie parmi :

- la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3,
- la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4,
- la région 5' UTR du gène eukaryotic Initiation Factor 4GI (eIF4GI) ayant la séquence nucléotidique SEQ ID NO : 5, ou un acide nucléotidique présentant au moins 70% d'identité de séquence avec la séquence SEQ ID NO : 5, et

(iv) au moins un intron choisi parmi :

- l'intron du gène Elongation Factor 1α (EF1α) ayant la séquence nucléotidique SEQ ID NO : 10, ou un acide nucléotidique présentant au moins 70% d'identité de séquence avec la séquence SEQ ID NO : 10,
- l'intron murin ROSA ayant la séquence nucléotidique SEQ ID NO : 11, ou un acide nucléotidique ayant au moins 70% d'identité de séquence avec la séquence SEQ ID NO : 11,
- intron 5'LTR du virus HTLV-1 ayant la séquence nucléotidique SEQ ID NO : 12, ou un acide nucléotidique présentant au moins 70% d'identité de séquence avec la séquence SEQ ID NO : 12,
- l'intron chimérique pCI-neo ayant la séquence nucléotidique SEQ ID NO : 13, ou un acide nucléotidique présentant au moins 70% d'identité de séquence avec la séquence SEQ ID NO : 13,
- intron du gène l'ubiquitine ayant la séquence nucléotidique SEQ ID NO : 53, ou un acide nucléotidique présentant au moins 70% d'identité de séquence avec la séquence SEQ ID NO : 53,
- intron du gène humain ROSA ayant la séquence nucléotidique SEQ ID NO : 54, ou un acide nucléotidique présentant au moins 70% d'identité de séquence avec la séquence SEQ ID NO : 54

ledit enhancer étant situé en 5' ou en 3' de l'unité de transcription, entre le promoteur et la région 5'UTR ou dans un intron ;
ladite région promotrice étant situé en amont de la région 5'UTR ;
lesdits introns étant situés :

(i) en aval de la région 5' UTR et en amont du site d'initiation de la traduction, et/ou
(ii) en aval du promoteur et en amont de la région 5'UTR, et/ou
(iii) après le site d'initiation de la traduction et à l'intérieur de la séquence codante, et/ou
(iv) entre le codon stop de la séquence codante et le signal de polyadénylation.

Une unité de transcription selon la présente invention peut comprendre :

(i) l'enhancer du virus hCMVie représenté par la séquence nucléotidique SEQ ID NO : 1,
(ii) une région promotrice de la Cyclin-Dependent Kinases 9 (CDK9), ladite région promotrice ayant la séquence nucléotidique SEQ ID NO : 2,
(iii) au moins l'une des régions 5' non traduite (5' UTR) choisie parmi :

- la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3,
- la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4,
- la région 5' UTR du gène eukaryotic Initiation Factor 4GI (eIF4GI) ayant la séquence nucléotidique SEQ ID NO : 5, ou un acide nucléotidique présentant au moins 70% d'identité de séquence avec la séquence SEQ ID NO : 5, et

(iv) au moins un intron choisi parmi :

- l'intron du gène Elongation Factor 1α (EF1α) ayant la séquence nucléotidique SEQ ID NO : 10, ou un acide nucléotidique présentant au moins 70% d'identité de séquence avec la séquence SEQ ID NO: 10,
- l'intron murin ROSA ayant la séquence nucléotidique SEQ ID NO : 11, ou un acide nucléotidique ayant au moins 70% d'identité de séquence avec la séquence SEQ ID NO : 11,
- intron 5'LTR du virus HTLV-1 ayant la séquence nucléotidique SEQ ID NO : 12, ou un acide nucléotidique présentant au moins 70% d'identité de séquence avec la séquence SEQ ID NO : 12,
- l'intron chimérique pCI-neo ayant la séquence nucléotidique SEQ ID NO : 13, ou un acide nucléotidique présentant au moins 70% d'identité de séquence avec la séquence SEQ ID NO : 13,
- intron du gène l'ubiquitine ayant la séquence nucléotidique SEQ ID NO : 53, ou un acide nucléotidique présentant au moins 70% d'identité de séquence avec la séquence SEQ ID NO : 53,
- intron du gène humain ROSA ayant la séquence nucléotidique SEQ ID NO : 54, ou un acide nucléotidique présentant au moins 70% d'identité de séquence avec la séquence SEQ ID NO : 54

ledit enhancer étant situé en 5' ou en 3' de l'unité de transcription, entre le promoteur et la région 5'UTR ou dans un intron ;
ladite région promotrice étant situé en amont de la région 5'UTR ;
lesdits introns étant situés :

(i) en aval de la région 5' UTR et en amont du site d'initiation de la traduction, et/ou
(ii) en aval du promoteur et en amont de la région 5'UTR, et/ou
(iii) après le site d'initiation de la traduction et à l'intérieur de la séquence codante, et/ou
(iv) entre le codon stop de la séquence codante et le signal de polyadénylation.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3, et
(iv) l'intron du gène Elongation Factor 1α (EF1α) ayant la séquence nucléotidique SEQ ID NO : 10, ou un acide nucléotidique présentant au moins 70% d'identité de séquence avec la séquence SEQ ID NO : 10.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3, et
(iv) l'intron du gène Elongation Factor 1α (EF1α) ayant la séquence nucléotidique SEQ ID NO : 10, ou un acide nucléotidique présentant au moins 70% d'identité de séquence avec la séquence SEQ ID NO : 10.

Dans un mode de réalisation plus particulier de l'invention, une unité de transcription selon l'invention est constituée d'un polynucléotide comprenant un acide nucléotidique représenté par la séquence SEQ ID NO : 25 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région R du LTR du virus HTLV-1 ayant la séquence nucléotidique SEQ ID NO : 3, et
(iv) l'intron du gène Elongation Factor 1α (EF1α) ayant la séquence nucléotidique SEQ ID NO : 10,

ou d'un acide nucléotidique ayant au moins 70% d'identité de séquence avec la séquence SEQ ID NO : 25.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3, et
(iv) l'intron murin ROSA ayant la séquence nucléotidique SEQ ID NO : 11, ou un acide nucléotidique ayant au moins 70% d'identité de séquence avec la séquence SEQ ID NO : 11.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3, et
(iv) l'intron murin ROSA ayant la séquence nucléotidique SEQ ID NO : 11, ou un acide nucléotidique ayant au moins 70% d'identité de séquence avec la séquence SEQ ID NO : 11.

Dans un mode de réalisation plus particulier de l'invention, une unité de transcription selon l'invention est constituée d'un polynucléotide comprenant un acide nucléotidique représenté par la séquence SEQ ID NO : 26 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région R du LTR du virus HTLV-1 ayant la séquence nucléotidique SEQ ID NO : 3, et
(iv) l'intron murin ROSA ayant la séquence nucléotidique SEQ ID NO : 11,

ou d'un acide nucléotidique ayant au moins 70% d'identité de séquence avec la séquence SEQ ID NO : 26.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3, et
(iv) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3, et
(iv) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Dans un mode de réalisation plus particulier de l'invention, une unité de transcription selon l'invention est constituée d'un polynucléotide comprenant un acide nucléotidique représenté par la séquence SEQ ID NO : 27 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région R du LTR du virus HTLV-1 ayant la séquence nucléotidique SEQ ID NO : 3, et
(iv) l'intron 5'LTR du virus HTLV-1 ayant la séquence nucléotidique SEQ ID NO : 12,

ou d'un acide nucléotidique ayant au moins 70% d'identité de séquence avec la séquence SEQ ID NO : 27.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3, et
(iv) intron chimérique du pCI-neo ayant la séquence nucléotidique SEQ ID NO : 13, ou un acide nucléotidique présentant au moins 70% d'identité de séquence avec la séquence SEQ ID NO : 13.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3, et
(iv) intron chimérique du pCI-neo ayant la séquence nucléotidique SEQ ID NO : 13, ou un acide nucléotidique présentant au moins 70% d'identité de séquence avec la séquence SEQ ID NO : 13.

Dans un mode de réalisation plus particulier de l'invention, une unité de transcription selon l'invention est constituée d'un polynucléotide comprenant un acide nucléotidique représenté par la séquence SEQ ID NO : 28 et constitué de :

(i) l'enhancer du virus hCMVie représenté par la séquence nucléotidique SEQ ID NO: 1,
(ii) la région promotrice de la Cyclin-Dependent Kinase 9 (CDK9) représentée par la séquence nucléotidique SEQ ID NO : 2,
(iii) la région R du LTR du virus HTLV-1 ayant la séquence nucléotidique SEQ ID NO : 3, et
(iv) intron chimérique du pCI-neo ayant la séquence nucléotidique SEQ ID NO : 13, ou d'un acide nucléotidique ayant au moins 70% d'identité de séquence avec la séquence SEQ ID NO : 28.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3, et
(iv) intron du gène l'ubiquitine ayant la séquence nucléotidique SEQ ID NO : 53, ou un acide nucléotidique présentant au moins 70% d'identité de séquence avec la séquence SEQ ID NO : 53.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3, et
(iv) intron du gène l'ubiquitine ayant la séquence nucléotidique SEQ ID NO : 53, ou un acide nucléotidique présentant au moins 70% d'identité de séquence avec la séquence SEQ ID NO : 53.

Dans un mode de réalisation plus particulier de l'invention, une unité de transcription selon l'invention est constituée d'un polynucléotide comprenant un acide nucléotidique représenté par la séquence SEQ ID NO : 57 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région R du LTR du virus HTLV-1 ayant la séquence nucléotidique SEQ ID NO : 3, et
(iv) intron du gène l'ubiquitine ayant la séquence nucléotidique SEQ ID NO : 53, ou d'un acide nucléotidique ayant au moins 70% d'identité de séquence avec la séquence SEQ ID NO : 57.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3, et
(iv) intron du gène humain ROSA représenté par la séquence nucléotidique SEQ ID NO : 54, ou un acide nucléotidique présentant au moins 70% d'identité de séquence avec la séquence SEQ ID NO : 54.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2,
(iii) la région R du Long Terminal Repeat (LTR) du virus HTLV-1 ayant la séquence nucléotidique SEQ ID NO : 3, ou un acide nucléotidique ayant au moins 70% d'identité de séquence avec la séquence SEQ ID NO : 3, et
(iv) intron du gène humain ROSA représenté par la séquence nucléotidique SEQ ID NO : 54, ou un acide nucléotidique présentant au moins 70% d'identité de séquence avec la séquence SEQ ID NO : 54.

Dans un mode de réalisation plus particulier de l'invention, une unité de transcription selon l'invention est constituée d'un polynucléotide comprenant un acide nucléotidique représenté par la séquence SEQ ID NO : 64 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région R du LTR du virus HTLV-1 ayant la séquence nucléotidique SEQ ID NO : 3, et
(iv) intron du gène humain ROSA représenté par la séquence nucléotidique SEQ ID NO : 54,

ou d'un acide nucléotidique ayant au moins 70% d'identité de séquence avec la séquence SEQ ID NO : 64.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la cyclin-dependant kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(iv) l'intron du gène Elongation Factor 1α (EF1α) ayant la séquence nucléotidique SEQ ID NO : 10, ou un acide nucléotidique présentant au moins 70% d'identité de séquence avec la séquence SEQ ID NO : 10.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO:1,
(ii) la région promotrice de la cyclin-dependant kinase 9 (CDK9) représentée par la séquence nucléotidique SEQ ID NO : 2,
(iii) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(iv) l'intron du gène Elongation Factor 1α (EF1α) ayant la séquence nucléotidique SEQ ID NO : 10, ou un acide nucléotidique présentant au moins 70% d'identité de séquence avec la séquence SEQ ID NO : 10.

Dans un mode de réalisation plus particulier de l'invention, une unité de transcription selon l'invention est constituée d'un polynucléotide comprenant un acide nucléotidique représenté par la séquence SEQ ID NO : 29 et constitué de :

(i) l'enhancer du virus hCMVie représenté par la séquence nucléotidique SEQ ID NO: 1,
(ii) la région promotrice de la Cyclin-Dependant Kinase 9 (CDK9) représentée par la séquence nucléotidique SEQ ID NO : 2,
(iii) la région 5' UTR du gène NF-κB Repressing Factor (NRF) ayant la séquence nucléotidique SEQ ID NO : 4, et
(iv) l'intron du gène Elongation Factor 1α (EF1α) ayant la séquence nucléotidique SEQ ID NO : 10,

ou d'un acide nucléotidique ayant au moins 70% d'identité de séquence avec la séquence SEQ ID NO : 29.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(iv) l'intron murin ROSA ayant la séquence nucléotidique SEQ ID NO : 11, ou un acide nucléotidique ayant au moins 70% d'identité de séquence avec la séquence SEQ ID NO : 11.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(iv) l'intron murin ROSA ayant la séquence nucléotidique SEQ ID NO : 11, ou un acide nucléotidique ayant au moins 70% d'identité de séquence avec la séquence SEQ ID NO : 11.

Dans un mode de réalisation plus particulier de l'invention, une unité de transcription selon l'invention est constituée d'un polynucléotide comprenant un acide nucléotidique représenté par la séquence SEQ ID NO : 30 et constitué de :

(i) l'enhancer du virus hCMVie représenté par la séquence nucléotidique SEQ ID NO:1,
(ii) la région promotrice de la Cyclin-Dependent Kinase 9 (CDK9) représentée par la séquence nucléotidique SEQ ID NO : 2,
(iii) la région 5' UTR du gène NF-κB Repressing Factor (NRF) ayant la séquence nucléotidique SEQ ID NO : 4, et
(iv) l'intron murin ROSA ayant la séquence nucléotidique SEQ ID NO : 11,

ou d'un acide nucléotidique ayant au moins 70% d'identité de séquence avec la séquence SEQ ID NO : 30.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,

(iii) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(iv) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2,
(iii) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(iv) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Dans un mode de réalisation plus particulier de l'invention, une unité de transcription selon l'invention est constituée d'un polynucléotide comprenant un acide nucléotidique représenté par la séquence SEQ ID NO : 31 et constitué de :

(i) l'enhancer du virus hCMVie représenté par la séquence nucléotidique SEQ ID NO:1,
(ii) la région promotrice de la Cyclin-Dependent Kinase 9 (CDK9) représentée par la séquence nucléotidique SEQ ID NO : 2,
(iii) la région 5' UTR du gène NF-κB Repressing Factor (NRF) ayant la séquence nucléotidique SEQ ID NO : 4, et
(iv) l'intron du gène 5'LTR du virus HTLV-1 ayant la séquence nucléotidique SEQ ID NO : 12,

ou d'un acide nucléotidique ayant au moins 70% d'identité de séquence avec la séquence SEQ ID NO: 31.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(iv) intron chimérique du pCI-neo ayant la séquence nucléotidique SEQ ID NO : 13, ou un acide nucléotidique présentant au moins 70% d'identité de séquence avec la séquence SEQ ID NO : 13.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2,
(iii) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(iv) intron chimérique du pCI-neo ayant la séquence nucléotidique SEQ ID NO : 13, ou un acide nucléotidique présentant au moins 70% d'identité de séquence avec la séquence SEQ ID NO : 13.

Dans un mode de réalisation plus particulier de l'invention, une unité de transcription selon l'invention est constituée d'un polynucléotide comprenant un acide nucléotidique représenté par la séquence SEQ ID NO : 32 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR du gène NF-κB Repressing Factor (NRF) ayant la séquence nucléotidique SEQ ID NO : 4, et
(iv) intron chimérique du pCI-neo ayant la séquence nucléotidique SEQ ID NO : 13, ou d'un acide nucléotidique ayant au moins 70% d'identité de séquence avec la séquence SEQ ID NO : 32.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(iv) intron du gène l'ubiquitine ayant la séquence nucléotidique SEQ ID NO : 53, ou un acide nucléotidique présentant au moins 70% d'identité de séquence avec la séquence SEQ ID NO : 53.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2,
(iii) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO: 4, et
(iv) intron du gène l'ubiquitine ayant la séquence nucléotidique SEQ ID NO : 53, ou un acide nucléotidique présentant au moins 70% d'identité de séquence avec la séquence SEQ ID NO : 53.

Dans un mode de réalisation plus particulier de l'invention, une unité de transcription selon l'invention est constituée d'un polynucléotide comprenant un acide nucléotidique représenté par la séquence SEQ ID NO : 58 et constitué de :

(i) l'enhancer du virus hCMVie représenté par la séquence nucléotidique SEQ ID NO:1,
(ii) la région promotrice de la Cyclin-Dependent Kinase 9 (CDK9) représentée par la séquence nucléotidique SEQ ID NO : 2,
(iii) la région 5' UTR du gène NF-κB Repressing Factor (NRF) ayant la séquence nucléotidique SEQ ID NO : 4, et
(iv) intron du gène l'ubiquitine ayant la séquence nucléotidique SEQ ID NO : 53, ou d'un acide nucléotidique ayant au moins 70% d'identité de séquence avec la séquence SEQ ID NO : 58.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(iv) intron du gène humain ROSA représenté par la séquence nucléotidique SEQ ID NO : 54, ou un acide nucléotidique présentant au moins 70% d'identité de séquence avec la séquence SEQ ID NO : 54.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2,
(iii) la région 5' UTR du gène NF-κB Repressing Factor (NRF) ayant la séquence nucléotidique SEQ ID NO : 4, ou un acide nucléotidique ayant au moins 70% d'identité de séquence avec la séquence SEQ ID NO : 4, et
(iv) intron du gène humain ROSA représenté par la séquence nucléotidique SEQ ID NO : 54, ou un acide nucléotidique présentant au moins 70% d'identité de séquence avec la séquence SEQ ID NO : 54.

Dans un mode de réalisation plus particulier de l'invention, une unité de transcription selon l'invention est constituée d'un polynucléotide comprenant un acide nucléotidique représenté par la séquence SEQ ID NO : 65 et constitué de :

(i) l'enhancer du virus hCMVie représenté par la séquence nucléotidique SEQ ID NO:1,
(ii) la région promotrice de la Cyclin-Dependent Kinase 9 (CDK9) représentée par la séquence nucléotidique SEQ ID NO : 2,
(iii) la région 5' UTR du gène NF-κB Repressing Factor (NRF) ayant la séquence nucléotidique SEQ ID NO : 4, et
(iv) intron du gène humain ROSA représenté par la séquence nucléotidique SEQ ID NO : 54,

ou d'un acide nucléotidique ayant au moins 70% d'identité de séquence avec la séquence SEQ ID NO : 65.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR du gène eukaryotic Initiation Factor 4GI (eIF4GI) ayant la séquence nucléotidique SEQ ID NO : 5, ou un acide nucléotidique présentant au moins 70% d'identité de séquence avec la séquence SEQ ID NO : 5, et
(iv) l'intron du gène Elongation Factor 1α (EF1α) ayant la séquence nucléotidique SEQ ID NO : 10, ou un acide nucléotidique présentant au moins 70% d'identité de séquence avec la séquence SEQ ID NO : 10.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO:1,
(ii) la région promotrice de la Cyclin-Dependent Kinase 9 (CDK9) représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 5, ou un acide nucléotidique présentant au moins 70% d'identité de séquence avec la séquence SEQ ID NO : 5, et
(iv) l'intron du gène Elongation Factor 1α (EF1α) ayant la séquence nucléotidique SEQ ID NO : 10, ou un acide nucléotidique présentant au moins 70% d'identité de séquence avec la séquence SEQ ID NO : 10.

Dans un mode de réalisation plus particulier de l'invention, une unité de transcription selon l'invention est constituée d'un polynucléotide comprenant un acide nucléotidique représenté par la séquence SEQ ID NO : 33 et constitué de :

(i) l'enhancer du virus hCMVie représenté par la séquence nucléotidique SEQ ID NO:1,
(ii) la région promotrice de la Cyclin-Dependent Kinase 9 (CDK9) représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 5, et
(iv) l'intron du gène Elongation Factor 1α (EF1α) ayant la séquence nucléotidique SEQ ID NO: 10,

ou d'un acide nucléotidique ayant au moins 70% d'identité de séquence avec la séquence SEQ ID NO : 33.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR du gène eukaryotic Initiation Factor 4GI (eIF4GI) ayant la séquence nucléotidique SEQ ID NO : 5, ou un acide nucléotidique présentant au moins 70% d'identité de séquence avec la séquence SEQ ID NO : 5, et
(iv) l'intron murin ROSA ayant la séquence nucléotidique SEQ ID NO : 11, ou un acide nucléotidique ayant au moins 70% d'identité de séquence avec la séquence SEQ ID NO : 11.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 5, ou un acide nucléotidique présentant au moins 70% d'identité de séquence avec la séquence SEQ ID NO : 5, et
(iv) l'intron murin ROSA ayant la séquence nucléotidique SEQ ID NO : 11, ou un acide nucléotidique ayant au moins 70% d'identité de séquence avec la séquence SEQ ID NO : 11.

Dans un mode de réalisation plus particulier de l'invention, une unité de transcription selon l'invention est constituée d'un polynucléotide comprenant un acide nucléotidique représenté par la séquence SEQ ID NO : 34 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 5, et
(iv) l'intron murin ROSA ayant la séquence nucléotidique SEQ ID NO : 11,

ou d'un acide nucléotidique ayant au moins 70% d'identité de séquence avec la séquence SEQ ID NO : 34.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR du gène eukaryotic Initiation Factor 4GI (eIF4GI) ayant la séquence nucléotidique SEQ ID NO : 5, ou un acide nucléotidique présentant au moins 70% d'identité de séquence avec la séquence SEQ ID NO : 5, et
(iv) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-dependent Kinase 9 (CDK9) représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 5, ou un acide nucléotidique présentant au moins 70% d'identité de séquence avec la séquence SEQ ID NO : 5, et
(iv) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Dans un mode de réalisation plus particulier de l'invention, une unité de transcription selon l'invention est constituée d'un polynucléotide comprenant un acide nucléotidique représenté par la séquence SEQ ID NO : 35 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 5, et
(iv) l'intron 5'LTR du virus HTLV-1 ayant la séquence nucléotidique SEQ ID NO : 12,

ou d'un acide nucléotidique ayant au moins 70% d'identité de séquence avec la séquence SEQ ID NO : 35.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR du gène eukaryotic Initiation Factor 4GI (eIF4GI) ayant la séquence nucléotidique SEQ ID NO : 5, ou un acide nucléotidique présentant au moins 70% d'identité de séquence avec la séquence SEQ ID NO : 5, et
(iv) intron chimérique du pCI-neo ayant la séquence nucléotidique SEQ ID NO : 13, ou un acide nucléotidique présentant au moins 70% d'identité de séquence avec la séquence SEQ ID NO : 13.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 5, ou un acide nucléotidique présentant au moins 70% d'identité de séquence avec la séquence SEQ ID NO : 5, et
(iv) intron chimérique du pCI-neo ayant la séquence nucléotidique SEQ ID NO : 13, ou un acide nucléotidique présentant au moins 70% d'identité de séquence avec la séquence SEQ ID NO : 13.

Dans un mode de réalisation plus particulier de l'invention, une unité de transcription selon l'invention est constituée d'un polynucléotide comprenant un acide nucléotidique représenté par la séquence SEQ ID NO : 36 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 5, et
(iv) intron chimérique du pCI-neo ayant la séquence nucléotidique SEQ ID NO : 13, ou d'un acide nucléotidique ayant au moins 70% d'identité de séquence avec la séquence SEQ ID NO : 36.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,

(iii) la région 5' UTR du gène eukaryotic Initiation Factor 4GI (eIF4GI) ayant la séquence nucléotidique SEQ ID NO : 5, ou un acide nucléotidique présentant au moins 70% d'identité de séquence avec la séquence SEQ ID NO : 5, et
(iv) intron du gène l'ubiquitine ayant la séquence nucléotidique SEQ ID NO : 53, ou un acide nucléotidique présentant au moins 70% d'identité de séquence avec la séquence SEQ ID NO : 53.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 5, ou un acide nucléotidique présentant au moins 70% d'identité de séquence avec la séquence SEQ ID NO : 5, et
(iv) intron du gène l'ubiquitine ayant la séquence nucléotidique SEQ ID NO : 53, ou un acide nucléotidique présentant au moins 70% d'identité de séquence avec la séquence SEQ ID NO : 53.

Dans un mode de réalisation plus particulier de l'invention, une unité de transcription selon l'invention est constituée d'un polynucléotide comprenant un acide nucléotidique représenté par la séquence SEQ ID NO : 59 et constitué de :

(i) l'enhancer du virus hCMVie représenté par la séquence nucléotidique SEQ ID NO: 1,
(ii) la région promotrice de la Cyclin-Dependent Kinase 9 (CDK9) représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 5, et
(iv) intron du gène l'ubiquitine ayant la séquence nucléotidique SEQ ID NO : 53,

ou d'un acide nucléotidique ayant au moins 70% d'identité de séquence avec la séquence SEQ ID NO : 59.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR du gène eukaryotic Initiation Factor 4GI (eIF4GI) ayant la séquence nucléotidique SEQ ID NO : 5, ou un acide nucléotidique présentant au moins 70% d'identité de séquence avec la séquence SEQ ID NO : 5, et
(iv) intron du gène humain ROSA représenté par la séquence nucléotidique SEQ ID NO : 54, ou un acide nucléotidique présentant au moins 70% d'identité de séquence avec la séquence SEQ ID NO : 54.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1, et
(ii) la région promotrice de la Cyclin-Dependent Kinase 9 (CDK9) représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 5, ou un acide nucléotidique présentant au moins 70% d'identité de séquence avec la séquence SEQ ID NO : 5, et
(iv) intron du gène humain ROSA représenté par la séquence nucléotidique SEQ ID NO : 54, ou un acide nucléotidique présentant au moins 70% d'identité de séquence avec la séquence SEQ ID NO : 54.

Dans un mode de réalisation plus particulier de l'invention, une unité de transcription selon l'invention est constituée d'un polynucléotide comprenant un acide nucléotidique représenté par la séquence SEQ ID NO : 66 et constitué de :

(i) l'enhancer du virus hCMVie représenté par la séquence nucléotidique SEQ ID NO : 1,

(ii) la région promotrice de la Cyclin-Dependent Kinase 9 (CDK9) représentée par la séquence nucléotidique SEQ ID NO : 2,
(iii) la région 5' UTR du gène eukaryotic Initiation Factor 4GI (eIF4GI) ayant la séquence nucléotidique SEQ ID NO : 5, et
(iv) intron du gène humain ROSA représenté par la séquence nucléotidique SEQ ID NO : 54,

ou d'un acide nucléotidique ayant au moins 70% d'identité de séquence avec la séquence SEQ ID NO : 66.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent;Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 6, ou un acide nucléotidique présentant au moins 70% d'identité de séquence avec la séquence SEQ ID NO : 6,
(iv) l'intron du gène Elongation Factor 1$\alpha$ (EF1$\alpha$) ayant la séquence nucléotidique SEQ ID NO : 10, ou un acide nucléotidique présentant au moins 70% d'identité de séquence avec la séquence SEQ ID NO : 10.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent;Kinase 9 (CDK9) représentée par la séquence nucléotidique SEQ ID NO : 2,
(iii) la région 5' UTR représentée par la séquence SEQ ID NO : 6, ou un acide nucléotidique présentant au moins 70% d'identité de séquence avec la séquence SEQ ID NO : 6,
(iv) l'intron du gène Elongation Factor 1$\alpha$ (EF1$\alpha$) ayant la séquence nucléotidique SEQ ID NO : 10, ou un acide nucléotidique présentant au moins 70% d'identité de séquence avec la séquence SEQ ID NO : 10.

Dans un mode de réalisation plus particulier de l'invention, une unité de transcription selon l'invention est constituée d'un polynucléotide comprenant un acide nucléotidique représenté par la séquence SEQ ID NO : 37 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 6, et
(iv) l'intron du gène Elongation Factor 1$\alpha$ (EF1$\alpha$) ayant la séquence nucléotidique SEQ ID NO : 11,

ou d'un acide nucléotidique ayant au moins 70% d'identité de séquence avec la séquence SEQ ID NO : 37.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 6, ou un acide nucléotidique présentant au moins 70% d'identité de séquence avec la séquence SEQ ID NO : 6, et
(iv) l'intron murin ROSA ayant la séquence nucléotidique SEQ ID NO : 11, ou un acide nucléotidique ayant au moins 70% d'identité de séquence avec la séquence SEQ ID NO : 11.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 6, ou un acide nucléotidique présentant au moins 70% d'identité de séquence avec la séquence SEQ ID NO : 6, et
(iv) l'intron murin ROSA ayant la séquence nucléotidique SEQ ID NO : 11, ou un acide nucléotidique ayant au moins 70% d'identité de séquence avec la séquence SEQ ID NO : 11.

Dans un mode de réalisation plus particulier de l'invention, une unité de transcription selon l'invention est constituée d'un polynucléotide comprenant un acide nucléotidique représenté par la séquence SEQ ID NO : 38 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 6, et
(iv) l'intron murin ROSA ayant la séquence nucléotidique SEQ ID NO : 11,

ou d'un acide nucléotidique ayant au moins 70% d'identité de séquence avec la séquence SEQ ID NO : 38.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 6, ou un acide nucléotidique présentant au moins 70% d'identité de séquence avec la séquence SEQ ID NO : 6, et
(iv) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 6, ou un acide nucléotidique présentant au moins 70% d'identité de séquence avec la séquence SEQ ID NO : 6, et
(iv) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Dans un mode de réalisation plus particulier de l'invention, une unité de transcription selon l'invention est constituée d'un polynucléotide comprenant un acide nucléotidique représenté par la séquence SEQ ID NO : 39 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 6, et
(iv) l'intron 5'LTR du virus HTLV-1 ayant la séquence nucléotidique SEQ ID NO : 12,

ou d'un acide nucléotidique ayant au moins 70% d'identité de séquence avec la séquence SEQ ID NO : 39.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 6, ou un acide nucléotidique présentant au moins 70% d'identité de séquence avec la séquence SEQ ID NO : 6, et
(iv) l'intron chimérique du pCI-neo ayant la séquence nucléotidique SEQ ID NO : 13, ou un acide nucléotidique présentant au moins 70% d'identité de séquence avec la séquence SEQ ID NO : 13.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 6, ou un acide nucléotidique présentant au moins 70% d'identité de séquence avec la séquence SEQ ID NO : 6, et
(iv) l'intron chimérique du pCI-neo ayant la séquence nucléotidique SEQ ID NO : 13, ou un acide nucléotidique présentant au moins 70% d'identité de séquence avec la séquence SEQ ID NO : 13.

Dans un mode de réalisation plus particulier de l'invention, une unité de transcription selon l'invention est constituée d'un polynucléotide comprenant un acide nucléotidique représenté par la séquence SEQ ID NO : 40 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 6, et
(iv) l'intron chimérique du pCI-neo ayant la séquence nucléotidique SEQ ID NO : 13, ou d'un acide nucléotidique ayant au moins 70% d'identité de séquence avec la séquence SEQ ID NO : 40.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 6, ou un acide nucléotidique présentant au moins 70% d'identité de séquence avec la séquence SEQ ID NO : 6, et
(iv) intron du gène l'ubiquitine ayant la séquence nucléotidique SEQ ID NO : 53, ou un acide nucléotidique présentant au moins 70% d'identité de séquence avec la séquence SEQ ID NO : 53.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO: 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 6, ou un acide nucléotidique présentant au moins 70% d'identité de séquence avec la séquence SEQ ID NO : 6, et
(iv) intron du gène l'ubiquitine ayant la séquence nucléotidique SEQ ID NO : 53, ou un acide nucléotidique présentant au moins 70% d'identité de séquence avec la séquence SEQ ID NO : 53.

Dans un mode de réalisation plus particulier de l'invention, une unité de transcription selon l'invention est constituée d'un polynucléotide comprenant un acide nucléotidique représenté par la séquence SEQ ID NO : 60 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 6, et
(iv) intron du gène l'ubiquitine ayant la séquence nucléotidique SEQ ID NO : 53,

ou d'un acide nucléotidique ayant au moins 70% d'identité de séquence avec la séquence SEQ ID NO : 60.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 6, ou un acide nucléotidique présentant au moins 70% d'identité de séquence avec la séquence SEQ ID NO : 6, et
(iv) intron du gène humain ROSA ayant la séquence nucléotidique SEQ ID NO : 54, ou un acide nucléotidique présentant au moins 70% d'identité de séquence avec la séquence SEQ ID NO : 54.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 6, ou un acide nucléotidique présentant au moins 70% d'identité de séquence avec la séquence SEQ ID NO : 6, et
(iv) intron du gène humain ROSA ayant la séquence nucléotidique SEQ ID NO : 54, ou un acide nucléotidique présentant au moins 70% d'identité de séquence avec la séquence SEQ ID NO : 54.

Dans un mode de réalisation plus particulier de l'invention, une unité de transcription selon l'invention est constituée d'un polynucléotide comprenant un acide nucléotidique représenté par la séquence SEQ ID NO : 67 et constitué de :

(i) l'enhancer du virus hCMVie représenté par la séquence nucléotidique SEQ ID NO: 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 6, et
(iv) intron du gène humain ROSA ayant la séquence nucléotidique SEQ ID NO : 54, ou d'un acide nucléotidique ayant au moins 70% d'identité de séquence avec la séquence SEQ ID NO : 67.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,

(iii) la région 5' UTR représentée par la séquence SEQ ID NO : 7, ou un acide nucléotidique présentant au moins 70% d'identité de séquence avec la séquence SEQ ID NO : 7, et
(iv) l'intron du gène Elongation Factor 1α (EF1α) ayant la séquence nucléotidique SEQ ID NO : 10, ou un acide nucléotidique présentant au moins 70% d'identité de séquence avec la séquence SEQ ID NO : 10.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron du gène Elongation Factor 1α (EF1α) ayant la séquence nucléotidique SEQ ID NO : 10, ou un acide nucléotidique présentant au moins 70% d'identité de séquence avec la séquence SEQ ID NO : 10.

Dans un mode de réalisation plus particulier de l'invention, une unité de transcription selon l'invention est constituée d'un polynucléotide comprenant un acide nucléotidique représenté par la séquence SEQ ID NO : 41 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 7, et
(iv) l'intron du gène Elongation Factor 1α (EF1α) ayant la séquence nucléotidique SEQ ID NO : 10,

ou d'un acide nucléotidique ayant au moins 70% d'identité de séquence avec la séquence SEQ ID NO: 41.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron murin ROSA ayant la séquence nucléotidique SEQ ID NO : 11, ou un acide nucléotidique ayant au moins 70% d'identité de séquence avec la séquence SEQ ID NO : 11.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron murin ROSA ayant la séquence nucléotidique SEQ ID NO : 11, ou un acide nucléotidique ayant au moins 70% d'identité de séquence avec la séquence SEQ ID NO : 11.

Dans un mode de réalisation plus particulier de l'invention, une unité de transcription selon l'invention est constituée d'un polynucléotide comprenant un acide nucléotidique représenté par la séquence SEQ ID NO : 42 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 7, et
(iv) l'intron murin ROSA ayant la séquence nucléotidique SEQ ID NO : 11,

ou d'un acide nucléotidique ayant au moins 70% d'identité de séquence avec la séquence SEQ ID NO : 42.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Dans un mode de réalisation plus particulier de l'invention, une unité de transcription selon l'invention est constituée d'un polynucléotide comprenant un acide nucléotidique représenté par la séquence SEQ ID NO : 43 et constitué de :

(i) l'enhancer du virus hCMVie représenté par la séquence nucléotidique SEQ ID NO : 1,
(ii) la région promotrice de la Cyclin-Dependant Kinase 9 (CDK9) représentée par la séquence nucléotidique SEQ ID NO : 2,
(iii) la région 5' UTR représentée par la séquence nucléotidique SEQ ID NO : 7, et
(iv) l'intron 5'LTR du virus HTLV-1 ayant la séquence nucléotidique SEQ ID NO : 12,

ou d'un acide nucléotidique ayant au moins 70% d'identité de séquence avec la séquence SEQ ID NO : 43.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron chimérique du pCI-neo ayant la séquence nucléotidique SEQ ID NO : 13, ou un acide nucléotidique présentant au moins 70% d'identité de séquence avec la séquence SEQ ID NO : 13.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron chimérique du pCI-neo ayant la séquence nucléotidique SEQ ID NO : 13, ou un acide nucléotidique présentant au moins 70% d'identité de séquence avec la séquence SEQ ID NO : 13.

Dans un mode de réalisation plus particulier de l'invention, une unité de transcription selon l'invention est constituée d'un polynucléotide comprenant un acide nucléotidique représenté par la séquence SEQ ID NO : 44 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 7, et
(iv) l'intron chimérique du pCI-neo ayant la séquence nucléotidique SEQ ID NO : 13, ou d'un acide nucléotidique ayant au moins 70% d'identité de séquence avec la séquence SEQ ID NO : 44.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron du gène l'ubiquitine ayant la séquence nucléotidique SEQ ID NO : 53, ou un acide nucléotidique présentant au moins 70% d'identité de séquence avec la séquence SEQ ID NO : 53.

Un mode de réalisation particulier de l'invention concerne une unité de transcription constituée d'unpolynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 7, ou un acide nucléotidique présentant au moins 70% d'identité de séquence avec la séquence SEQ ID NO : 7, et
(iv) l'intron du gène l'ubiquitine ayant la séquence nucléotidique SEQ ID NO : 53, ou un acide nucléotidique présentant au moins 70% d'identité de séquence avec la séquence SEQ ID NO : 53.

Dans un mode de réalisation plus particulier de l'invention, une unité de transcription selon l'invention est constituée d'un polynucléotide comprenant un acide nucléotidique représenté par la séquence SEQ ID NO : 61 et constitué de :

(i) l'enhancer du virus hCMVie représenté par la séquence nucléotidique SEQ ID NO: 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 7, et
(iv) l'intron du gène l'ubiquitine ayant la séquence nucléotidique SEQ ID NO : 53, ou d'un acide nucléotidique ayant au moins 70% d'identité de séquence avec la séquence SEQ ID NO: 61.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 7, ou un acide nucléotidique présentant au moins 70% d'identité de séquence avec la séquence SEQ ID NO : 7, et
(iv) intron du gène humain ROSA ayant la séquence nucléotidique SEQ ID NO : 54, ou un acide nucléotidique présentant au moins 70% d'identité de séquence avec la séquence SEQ ID NO : 54.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 7, ou un acide nucléotidique présentant au moins 70% d'identité de séquence avec la séquence SEQ ID NO : 7, et
(iv) intron du gène humain ROSA ayant la séquence nucléotidique SEQ ID NO : 54, ou un acide nucléotidique présentant au moins 70% d'identité de séquence avec la séquence SEQ ID NO : 54.

Dans un mode de réalisation plus particulier de l'invention, une unité de transcription selon l'invention est constituée d'un polynucléotide comprenant un acide nucléotidique représenté par la séquence SEQ ID NO : 68 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 7, et
(iv) intron du gène humain ROSA ayant la séquence nucléotidique SEQ ID NO : 54, ou d'un acide nucléotidique ayant au moins 70% d'identité de séquence avec la séquence SEQ ID NO : 68.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription, et
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 8, ou un acide nucléotidique présentant au moins 70% d'identité de séquence avec la séquence SEQ ID NO : 8, et
(iv) l'intron du gène Elongation Factor 1$\alpha$ (EF1$\alpha$) ayant la séquence nucléotidique SEQ ID NO : 10, ou un acide nucléotidique présentant au moins 70% d'identité de séquence avec la séquence SEQ ID NO : 10.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO: 1, et
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 8, ou un acide nucléotidique présentant au moins 70% d'identité de séquence avec la séquence SEQ ID NO : 8, et
(iv) l'intron du gène Elongation Factor 1$\alpha$ (EF1$\alpha$) ayant la séquence nucléotidique SEQ ID NO : 10, ou un acide nucléotidique présentant au moins 70% d'identité de séquence avec la séquence SEQ ID NO : 10.

Dans un mode de réalisation plus particulier de l'invention, une unité de transcription selon l'invention est constituée d'un polynucléotide comprenant un acide nucléotidique représenté par la séquence SEQ ID NO : 45 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 8, et
(iv) l'intron du gène Elongation Factor 1$\alpha$ (EF1$\alpha$) ayant la séquence nucléotidique SEQ ID NO : 10,

ou d'un acide nucléotidique ayant au moins 70% d'identité de séquence avec la séquence SEQ ID NO : 45.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 8, ou un acide nucléotidique présentant au moins 70% d'identité de séquence avec la séquence SEQ ID NO : 8,
(iv) l'intron murin ROSA ayant la séquence nucléotidique SEQ ID NO : 11, ou un acide nucléotidique ayant au moins 70% d'identité de séquence avec la séquence SEQ ID NO : 11.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 8, ou un acide nucléotidique présentant au moins 70% d'identité de séquence avec la séquence SEQ ID NO : 8,
(iv) l'intron murin ROSA ayant la séquence nucléotidique SEQ ID NO : 11, ou un acide nucléotidique ayant au moins 70% d'identité de séquence avec la séquence SEQ ID NO : 11.

Dans un mode de réalisation plus particulier de l'invention, une unité de transcription selon l'invention est constituée d'un polynucléotide comprenant un acide nucléotidique représenté par la séquence SEQ ID NO : 46 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 8, et
(iv) l'intron murin ROSA ayant la séquence nucléotidique SEQ ID NO : 11,

ou d'un acide nucléotidique ayant au moins 70% d'identité de séquence avec la séquence SEQ ID NO : 46.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 8, ou un acide nucléotidique présentant au moins 70% d'identité de séquence avec la séquence SEQ ID NO : 8, et

(iv) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 8, ou un acide nucléotidique présentant au moins 70% d'identité de séquence avec la séquence SEQ ID NO : 8, et
(iv) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Dans un mode de réalisation plus particulier de l'invention, une unité de transcription selon l'invention est constituée d'un polynucléotide comprenant un acide nucléotidique représenté par la séquence SEQ ID NO : 47 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 8, et
(iv) l'intron 5'LTR du virus HTLV-1 ayant la séquence nucléotidique SEQ ID NO : 12,

ou d'un acide nucléotidique ayant au moins 70% d'identité de séquence avec la séquence SEQ ID NO : 47.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 8, ou un acide nucléotidique présentant au moins 70% d'identité de séquence avec la séquence SEQ ID NO : 8, et
(iv) l'intron chimérique du pCI-neo ayant la séquence nucléotidique SEQ ID NO : 13, ou un acide nucléotidique présentant au moins 70% d'identité de séquence avec la séquence SEQ ID NO : 13.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO: 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 8, ou un acide nucléotidique présentant au moins 70% d'identité de séquence avec la séquence SEQ ID NO : 8, et
(iv) l'intron chimérique du pCI-neo ayant la séquence nucléotidique SEQ ID NO : 13, ou un acide nucléotidique présentant au moins 70% d'identité de séquence avec la séquence SEQ ID NO : 13.

Dans un mode de réalisation plus particulier de l'invention, une unité de transcription selon l'invention est constituée d'un polynucléotide comprenant un acide nucléotidique représenté par la séquence SEQ ID NO : 48 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 8, et

(iv) l'intron chimérique du pCI-neo ayant la séquence nucléotidique SEQ ID NO : 13, ou d'un acide nucléotidique ayant au moins 70% d'identité de séquence avec la séquence SEQ ID NO : 48.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 8, ou un acide nucléotidique présentant au moins 70% d'identité de séquence avec la séquence SEQ ID NO : 8, et
(iv) intron du gène l'ubiquitine ayant la séquence nucléotidique SEQ ID NO : 53, ou un acide nucléotidique présentant au moins 70% d'identité de séquence avec la séquence SEQ ID NO : 53.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 8, ou un acide nucléotidique présentant au moins 70% d'identité de séquence avec la séquence SEQ ID NO : 8, et
(iv) intron du gène l'ubiquitine ayant la séquence nucléotidique SEQ ID NO : 53, ou un acide nucléotidique présentant au moins 70% d'identité de séquence avec la séquence SEQ ID NO : 53.

Dans un mode de réalisation plus particulier de l'invention, une unité de transcription selon l'invention est constituée d'un polynucléotide comprenant un acide nucléotidique représenté par la séquence SEQ ID NO : 62 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 8, et
(iv) intron du gène l'ubiquitine ayant la séquence nucléotidique SEQ ID NO : 53,

ou d'un acide nucléotidique ayant au moins 70% d'identité de séquence avec la séquence SEQ ID NO : 62.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 8, ou un acide nucléotidique présentant au moins 70% d'identité de séquence avec la séquence SEQ ID NO : 8, et
(iv) intron du gène humain ROSA ayant la séquence nucléotidique SEQ ID NO : 54, ou un acide nucléotidique présentant au moins 70% d'identité de séquence avec la séquence SEQ ID NO : 54.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 8, ou un acide nucléotidique présentant au moins 70% d'identité de séquence avec la séquence SEQ ID NO : 8, et
(iv) intron du gène humain ROSA ayant la séquence nucléotidique SEQ ID NO : 54, ou un acide nucléotidique présentant au moins 70% d'identité de séquence avec la séquence SEQ ID NO : 54.

Dans un mode de réalisation plus particulier de l'invention, une unité de transcription selon l'invention est constituée d'un polynucléotide comprenant un acide nucléotidique représenté par la séquence SEQ ID NO : 69 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 8, et
(iv) intron du gène humain ROSA ayant la séquence nucléotidique SEQ ID NO : 54, ou d'un acide nucléotidique ayant au moins 70% d'identité de séquence avec la séquence SEQ ID NO : 69.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron du gène Elongation Factor 1α (EF1α) ayant la séquence nucléotidique SEQ ID NO : 10, ou un acide nucléotidique présentant au moins 70% d'identité de séquence avec la séquence SEQ ID NO : 10.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO: 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron du gène Elongation Factor 1α (EF1α) ayant la séquence nucléotidique SEQ ID NO : 10, ou un acide nucléotidique présentant au moins 70% d'identité de séquence avec la séquence SEQ ID NO : 10.

Dans un mode de réalisation plus particulier de l'invention, une unité de transcription selon l'invention est constituée d'un polynucléotide comprenant un acide nucléotidique représenté par la séquence SEQ ID NO : 49 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 9, et
(iv) l'intron du gène Elongation Factor 1α (EF1α) ayant la séquence nucléotidique SEQ ID NO : 10,

ou d'un acide nucléotidique ayant au moins 70% d'identité de séquence avec la séquence SEQ ID NO : 49.
La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron murin ROSA ayant la séquence nucléotidique SEQ ID NO : 11, ou un acide nucléotidique ayant au moins 70% d'identité de séquence avec la séquence SEQ ID NO : 11.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron murin ROSA ayant la séquence nucléotidique SEQ ID NO : 11, ou un acide nucléotidique ayant au moins 70% d'identité de séquence avec la séquence SEQ ID NO : 11.

Dans un mode de réalisation plus particulier de l'invention, une unité de transcription selon l'invention est constituée d'un polynucléotide comprenant un acide nucléotidique représenté par la séquence SEQ ID NO : 50 et constitué de :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence nucléotidique SEQ ID NO : 9, et
(iv) l'intron murin ROSA ayant la séquence nucléotidique SEQ ID NO : 11,

ou d'un acide nucléotidique ayant au moins 70% d'identité de séquence avec la séquence SEQ ID NO : 50.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO: 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron 5'LTR du virus HTLV-1 ayant la séquence nucléotidique SEQ ID NO : 12, ou un acide nucléotidique présentant au moins 70% d'identité de séquence avec la séquence SEQ ID NO : 12.

Dans un mode de réalisation plus particulier de l'invention, une unité de transcription selon l'invention est constituée d'un polynucléotide comprenant un acide nucléotidique représenté par la séquence SEQ ID NO : 51 et constitué de :

(i) l'enhancer du virus hCMVie représenté par la séquence nucléotidique SEQ ID NO : 1,
(ii) la région promotrice de la cyclin-Dependent Kinase 9 (CDK9) représentée par la séquence nucléotidique SEQ ID NO : 2,
(iii) la région 5' UTR représentée par la séquence nucléotidique SEQ ID NO : 9, et
(iv) l'intron 5'LTR du virus HTLV-1 ayant la séquence nucléotidique SEQ ID NO : 12,

ou d'un acide nucléotidique ayant au moins 70% d'identité de séquence avec la séquence SEQ ID NO : 51.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron chimérique du pCI-neo ayant la séquence nucléotidique SEQ ID NO : 13, ou un acide nucléotidique présentant au moins 70% d'identité de séquence avec la séquence SEQ ID NO : 13.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 9, ou un acide nucléotidique présentant au moins 70% d'identité de séquence avec la séquence SEQ ID NO : 9, et
(iv) l'intron chimérique du pCI-neo ayant la séquence nucléotidique SEQ ID NO : 13, ou un acide nucléotidique présentant au moins 70% d'identité de séquence avec la séquence SEQ ID NO : 13.

Dans un mode de réalisation plus particulier de l'invention, une unité de transcription selon l'invention est constituée d'un polynucléotide comprenant un acide nucléotidique représenté par la séquence SEQ ID NO : 52 et constitué de :

(i) l'enhancer du virus hCMVie représenté par la séquence nucléotidique SEQ ID NO : 1,
(ii) la région promotrice de la cyclin-Dependent Kinase 9 (CDK9) représentée par la séquence nucléotidique SEQ ID NO : 2,
(iii) la région 5' UTR représentée par la séquence nucléotidique SEQ ID NO : 9, et
(iv) l'intron chimérique du pCI-neo ayant la séquence nucléotidique SEQ ID NO : 13, ou d'un acide nucléotidique ayant au moins 70% d'identité de séquence avec la séquence SEQ ID NO : 52.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et
(iv) intron du gène l'ubiquitine ayant la séquence nucléotidique SEQ ID NO : 53, ou un acide nucléotidique présentant au moins 70% d'identité de séquence avec la séquence SEQ ID NO : 53.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 9, ou un acide nucléotidique présentant au moins 70% d'identité de séquence avec la séquence SEQ ID NO : 9, et
(iv) intron du gène l'ubiquitine ayant la séquence nucléotidique SEQ ID NO : 53, ou un acide nucléotidique présentant au moins 70% d'identité de séquence avec la séquence SEQ ID NO : 53.

Dans un mode de réalisation plus particulier de l'invention, une unité de transcription selon l'invention est constituée d'un polynucléotide comprenant un acide nucléotidique représenté par la séquence SEQ ID NO : 63 et constitué de :

(i) l'enhancer du virus hCMVie représenté par la séquence nucléotidique SEQ ID NO : 1,
(ii) la région promotrice de la cyclin-Dependent Kinase 9 (CDK9) représentée par la séquence nucléotidique SEQ ID NO : 2,
(iii) la région 5' UTR représentée par la séquence nucléotidique SEQ ID NO : 9, et
(iv) intron du gène l'ubiquitine ayant la séquence nucléotidique SEQ ID NO : 53, ou d'un acide nucléotidique ayant au moins 70% d'identité de séquence avec la séquence SEQ ID NO : 63.

La présente divulgation décrit une unité de transcription constituée d'un polynucléotide comprenant les éléments régulateurs suivants :

(i) l'enhancer du virus hCMVie représenté par la séquence nucléotidique SEQ ID NO : 1, ou un acide nucléotidique ayant au moins 70% d'identité de séquence avec la séquence SEQ ID NO : 1 et possédant essentiellement des propriétés d'activation de la transcription,
(ii) la région promotrice de la Cyclin-Dependent Kinase 9 (CDK9) représentée par la séquence nucléotidique SEQ ID NO : 2, ou un acide nucléotidique ayant au moins 70% d'identité de séquence avec la séquence SEQ ID NO : 2 et possédant essentiellement une activité promotrice,
(iii) la région 5' UTR représentée par la séquence SEQ ID NO : 9, ou un acide nucléotidique présentant au moins 70% d'identité de séquence avec la séquence SEQ ID NO : 9, et
(iv) intron du gène humain ROSA ayant la séquence nucléotidique SEQ ID NO : 54, ou un acide nucléotidique présentant au moins 70% d'identité de séquence avec la séquence SEQ ID NO : 54.

Un mode de réalisation particulier de l'invention concerne une unité de transcription constituée d'un polynucléotide comprenant les éléments régulateurs suivants :

(i) l'enhancer du virus hCMVie représenté par la séquence nucléotidique SEQ ID NO : 1,
(ii) la région promotrice de la Cyclin-Dependent Kinase 9 (CDK9) représentée par la séquence nucléotidique SEQ ID NO : 2,
(iii) la région 5' UTR représentée par la séquence SEQ ID NO : 9, ou un acide nucléotidique présentant au moins 70% d'identité de séquence avec la séquence SEQ ID NO : 9, et
(iv) intron du gène humain ROSA ayant la séquence nucléotidique SEQ ID NO : 54, ou un acide nucléotidique présentant au moins 70% d'identité de séquence avec la séquence SEQ ID NO : 54.

Dans un mode de réalisation plus particulier de l'invention, une unité de transcription selon l'invention est constituée d'un polynucléotide comprenant un acide nucléotidique représenté par la séquence SEQ ID NO : 70 et constitué de :

(i) l'enhancer du virus hCMVie représenté par la séquence nucléotidique SEQ ID NO: 1,
(ii) la région promotrice de la cyclin-Dependent Kinase 9 (CDK9) représentée par la séquence nucléotidique SEQ ID NO : 2,
(iii) la région 5' UTR représentée par la séquence nucléotidique SEQ ID NO : 9, et
(iv) intron du gène humain ROSA ayant la séquence nucléotidique SEQ ID NO : 54, ou d'un acide nucléotidique ayant au moins 70% d'identité de séquence avec la séquence SEQ ID NO : 70.

Dans un mode de réalisation avantageux, la présente invention concerne une unité de transcription, dans laquelle la région promotrice est celle de CDK9, la région 5' UTR est celle du gène eIF4GI (U3) et l'intron est celui du gène EF1$\alpha$ ladite unité de transcription ayant la séquence nucléotidique SEQ ID NO : 33, ou une séquence nucléotidique présentant au moins 70% d'identité avec la séquence SEQ ID NO : 33 et permettant une production volumique d'une protéine d'intérêt supérieure à celle obtenue avec la combinaison de l'enhancer CMV associé à la région promotrice de CDK9.

On entend par une « production volumique », une quantité de protéine exprimée en masse par unité de volume (g/L) aussi appelée titre en protéine ou concentration de la protéine d'intérêt.

La présente invention concerne également un vecteur d'expression comprenant au moins une unité de transcription telle que définie ci-dessus et au moins un site de clonage permettant l'intégration d'un acide nucléique codant pour une protéine d'intérêt.

Ledit acide nucléique peut être un ADN génomique, un ADN complémentaire (ADNc), un acide nucléique synthétique ou un acide nucléique chimérique.

Par « site de clonage », on entend un segment court d'ADN qui comporte un ou plusieurs sites de restriction, reconnus respectivement par une ou plusieurs enzymes de restriction et permettant l'insertion d'une séquence nucléotidique d'intérêt.

La présente invention concerne également un vecteur d'expression comprenant au moins une unité de transcription telle que définie ci-dessus et au moins un site pour la recombinaison site spécifique permettant l'intégration d'un acide nucléotidique codant pour une protéine d'intérêt.

Ledit acide nucléotidique peut être un ADN génomique ou un ADN complémentaire (ADNc).

Par « site pour la recombinaison site spécifique », on entend un segment court d'ADN qui est reconnu par une recombinase, tel que le site *loxP* qui est reconnu par la recombinase Cre, le site *xis* qui est reconnu par l'intégrase Int, le site FRT qui est reconnu par la recombinase FLP.

Un vecteur d'expression selon la présente invention peut comprendre en outre un gène de résistance eucaryote, un gène de résistance bactérien, une origine de réplication bactérienne et une unité dédiée à l'amplification génique.

Un gène de résistance eucaryote peut être un gène de résistance à la Généticine (G418), Blasticidine, zéocine,

Un gène de résistance bactérien peut être un gène de résistance à l'ampicilline, Kanamycine, Puromycine, Blasticidine, Zéocine

Une origine de réplication (Ori) bactérienne est une séquence d'ADN particulier d'origine bactérienne permettant l'initiation de la réplication du matériel génétique comme un vecteur d'expression et de conditionner chez la bactérie le nombre de copies de vecteur par bactérie. Une telle origine de réplication peut être choisie parmi Ori-P, Ori-C, Ori-f1, ColE1, pSC101 Ori, p15A Ori, pACYC Ori, SV40 Ori, pMB1 Ori, pUC ori.

Par « une unité dédiée à l'amplification génique », on entend toute unité permettant de réaliser une amplification génique et/ou un enrichissement en forts en cellules fortement productrices. Le plus souvent, cette unité permet l'expression d'un gène de résistance à un inhibiteur agissant de manière dose dépendante ; par augmentation de la dose d'inhibiteur, il y a sélection de variants cellulaires exprimant plus fortement le gène de résistance, en particulier suite à amplification génique ou intégration dans un site de forte expression. Le plus souvent les gènes proches de cette unité sont également amplifiés génétiquement et/ou ont une expression augmentée. Une telle unité peut être le gène dhfr (dihydrofolate réductase) dont l'inhibiteur est le méthotrexate ou le gène glutamine synthétase dont l'inhibiteur est le méthionyl sulfoximine un système d'amplification de fragments géniques qui repose sur la sélection de transformants résistant au méthotrexate (MTX). Il nécessite l'introduction préalable d'une unité de transcription comprenant l'acide nucléique codant pour l'enzyme DHFR (dihydrofolate réductase) dans le vecteur d'expression pour la production de la molécule recombinante d'intérêt (SHITARI et al, 1994)

Une protéine recombinante d'intérêt susceptible d'être produit par un vecteur selon l'invention est une protéine naturelle ou modifiée dans sa séquence primaire et choisie parmi le groupe constitué des protéines impliquées dans la cascade de la coagulation ou une immunoglobuline, des enzymes métaboliques, des cytokines, chimiokines, des hormones, des facteurs de croissance ou facteurs du complément et de toute protéine de fusion.

La présente invention a également pour objectif de fournir les cellules hôtes comprenant un vecteur d'expression tel que décrit dans la présente invention.

Lesdites cellules hôtes peuvent être une lignée cellulaire de mammifère telle qu'une lignée cellulaire YB2/0 (N°**ATCC** : **CRL-1662**), ou une lignée cellulaire CHO.

La présente invention concerne également l'utilisation d'un vecteur d'expression décrit ci-dessus pour transfecter une cellule hôte.

Un autre objectif de la présente invention est de mettre à disposition d'un système d'expression comprenant un vecteur d'expression selon la présente invention et une cellule hôte telle que décrite ci-dessus, permettant l'expression d'une protéine d'intérêt codée par un acide nucléotidique.

La présente invention concerne également l'utilisation d'un vecteur d'expression comprenant au moins une unité de transcription selon la présente invention dans une cellule hôte telle que décrite ci-dessus pour produire une protéine codée par un acide nucléotidique, ladite protéine étant produite avec un titre plus élevé que dans le vecteur d'expression de référence comprenant au moins un promoteur RSV, un intron chimérique provenant du vecteur pCI-neo, une séquence de polyadénylation, un gène de résistance eucaryote, un gène de résistance bactérien, une origine de réplication bactérienne et une unité dédiée à l'amplification génique, ledit vecteur de référence comportant la même séquence nucléotidique.

La présente invention a également pour objet un procédé de production *in vitro* d'une protéine recombinante d'intérêt comprenant les étapes de :

- introduction du vecteur d'expression comprenant au moins une unité de transcription selon la présente invention et une séquence nucléotidique sous forme génomique ou d'ADNc codant pour une protéine d'intérêt dans une cellule hôte,
- sélection et identification des cellules hôtes obtenues à l'étape précédente exprimant de manière stable ladite protéine d'intérêt,
- extraction et purification de ladite protéine d'intérêt.

Dans un autre mode de réalisation particulier, le procédé de production selon la présente invention comprend les étapes de :

- introduction du vecteur d'expression comprenant au moins une unité de transcription selon la présente invention et une séquence nucléotidique sous forme génomique ou d'ADNc codant pour une protéine d'intérêt dans une cellule hôte par transfection transitoire,
- extraction et purification de ladite protéine d'intérêt.

Une telle protéine recombinante peut être une protéine impliquée dans la cascade de la coagulation ou une immunoglobuline, des enzymes métaboliques, des cytokines, chimiokines, des hormones, des facteurs de croissance ou facteurs du complément et de toute protéine de fusion.

Un procédé selon la présente invention peut comprendre en outre une étape de sélection et identification des cellules hôtes obtenues exprimant de manière stable ladite protéine d'intérêt.

La présente invention est illustrée par les figures et les exemples ci-après. Cependant, la présente invention n'est limitée en aucun cas aux figures et exemples ci-après.

**Figures**

Figure 1 illustre le vecteur E2-CDK9-U1U2U3 comportant une unité de transcription comprenant l'enhancer de hCMVie (E2), la région promotrice du gène CDK9, la région R du LTR du virus HTLV-1 (U1), la région 5'UTR du gène NRF (U2) et la région 5'UTR du gène eIF4G1 (U3).

Figure 2 illustre le vecteur E2- CDK9-U2U3 comportant une unité de transcription comprenant l'enhancer de hCMVie, la région promotrice du gène CDK9, la région 5'UTR du gène NRF et la région 5'UTR du gène eIF4G1.

Figure 3 illustre le vecteur E2-CDK9-U2 comportant une unité de transcription comprenant l'enhancer de hCMVie, la région promotrice du gène CDK9 et la région 5'UTR du gène NRF.

Figure 4 illustre le vecteur E2-CDK9-U1 comportant une unité de transcription comprenant l'enhancer de hCMVie, la région promotrice du gène CDK9 et la région R du LTR du virus HTLV-1.

Figure 5 illustre le vecteur E2- CDK9-U3 comportant une unité de transcription comprenant l'enhancer de hCMVie, la région promotrice du gène CDK9, et la région 5'UTR du gène eIF4G1.

Figure 6 illustre le vecteur E2-CDK9-U1U3 comportant une unité de transcription comprenant l'enhancer de hCMVie, la région promotrice du gène CDK9, la région R du LTR du virus HTLV-1 et la région 5'UTR du gène eIF4G1.

Figure 7 illustre le vecteur E2-CDK9-EF1$\alpha$ comportant une unité de transcription comprenant l'enhancer de hCMVie, la région promotrice du gène CDK9 et le premier intron du gène EF1$\alpha$.

Figure 8 illustre le vecteur E2-CDK9-UIU2U3-EF1$\alpha$ comportant une unité de transcription comprenant l'enhancer de hCMVie, la région promotrice du gène CDK9, la région R du LTR du virus HTLV-1, la région 5'UTR du gène NRF, la région 5'UTR du gène eIF4GI et le premier intron du gène EF1$\alpha$,.

Figure 9 illustre le vecteur E2-CDK9-U1U3-EF1$\alpha$ comportant une unité de transcription comprenant l'enhancer de hCMVie, la région promotrice du gène CDK9, la région R du LTR du virus HTLV-1, la région 5'UTR du gène eIF4G1 et le premier intron du gène EF1$\alpha$.

Figure 10 illustre le vecteur E2-CDK9-U2U3-EF1$\alpha$ comportant une unité de transcription comprenant l'enhancer de hCMVie, la région promotrice du gène CDK9, la région 5'UTR du gène NRF, la région 5'UTR du gène eIF4G1 et le premier intron du gène EF1$\alpha$.

Figure 11 illustre le vecteur E2-CDK9-U2-EF1$\alpha$ comportant une unité de transcription comprenant l'enhancer de hCMVie, la région promotrice du gène CDK9, la région 5'UTR du gène NRF et le premier intron du gène EF1$\alpha$.

Figure 12A illustre le vecteur E2-CDK9-U1-EF1$\alpha$ comportant une unité de transcription comprenant l'enhancer de hCMVie, la région promotrice du gène CDK9, la région R du LTR du virus HTLV-1 et le premier intron du gène EF1$\alpha$.

Figure 12B illustre le vecteur E2-CDK9-U3-EF1$\alpha$, comportant une unité de transcription comprenant l'enhancer de hCMVie, la région promotrice du gène CDK9, la région 5'UTR du gène eIF4G1 et le premier intron du gène EF1$\alpha$.

Figure 13 illustre le vecteur E2-CDK9-U1U2-EF1$\alpha$ comportant une unité de transcription comprenant l'enhancer de hCMVie, la région promotrice du gène CDK9, le premier intron du gène EF1$\alpha$, la région R du LTR du virus HTLV-1 et la région 5'UTR du gène NRF.

Figure 14 illustre le vecteur bicistronique CHK622-21 pour exprimer une IgG1/K. Les unités de transcription d'intérêt sont sous la dépendance du promoteur LTR RSV en association avec l'intron chimérique pCI-neo.

Figure 15 illustre le vecteur HK622-21_138H11B comportant la chaîne légère et la chaîne lourde de l'anticorps anti-GGT 138H11B. Les unités de transcription d'intérêt sont sous la dépendance du promoteur LTR RSV en association avec l'intron chimérique pCI-neo.

Figure 16 illustre le vecteur HK622-21_138H11B_MB7 comportant la chaîne légère avec le peptide signal MB7 et la chaîne lourde le peptide signal MB7 de l'anticorps anti-GGT 138H11B. Les unités de transcription d'intérêt sont sous la dépendance du promoteur LTR RSV en association avec l'intron chimérique pCIneo.

Figure 17 illustre le vecteur bicistronique E2-CDK9-U3-Gen pour exprimer une IgG1/K. Les unités de transcription d'intérêt sont sous la dépendance de l'enhancer E2 (hCMVie) du promoteur CDK9 et la région 5'UTR du gène eIF4GI (U3).

Figure 18 illustre le vecteur E2-CDK9-U3-HK138H11B_MB7 comportant comportant la chaîne légère avec le peptide signal MB7 et la chaîne lourde le peptide signal MB7 de l'anticorps anti-GGT 138H11B la chaîne légère avec le peptide signal MB7 et la chaîne lourde le peptide signal MB7 de l'anticorps anti-GGT 138H11B. Les unités de transcription d'intérêt sont sous la dépendance de l'enhancer E2 (hCMVie) du promoteur CDK9 et la région 5'UTR du gène eIF4G1 (U3)

Figure 19 illustre le vecteur HK1358-4 comportant la chaîne légère avec le peptide signal MB7 et la chaîne lourde le peptide signal MB7 de l'anticorps anti-GGT 138H11B. Les unités de transcription d'intérêt sont sous la dépendance de l'enhancer E2 (hCMVie) du promoteur CDK9, la région 5'UTR du gène eIF4G1 (U3) et l'intron chimérique pCI-néo.

Figure 20 illustre le vecteur HK1358-5 comportant la chaîne légère avec le peptide signal MB7 et la chaîne lourde le peptide signal MB7 de l'anticorps anti-GGT 138H11B. Les unités de transcription d'intérêt sont sous la dépendance de l'enhancer E2 (hCMVie) du promoteur CDK9, la région 5'UTR du gène eIF4GI (U3) et l'intron EF1$\alpha$.

Figure 21 illustre le vecteur HK1358-8 comportant la chaîne légère avec le peptide signal MB7 et la chaîne lourde le peptide signal MB7 de l'anticorps anti-GGT 138H11B. Les unités de transcription d'intérêt sont sous la dépendance de l'enhancer E2 (hCMVie) du promoteur CDK9, la région 5'UTR du gène eIF4G1 (U3) et l'intron mROSA.

Figure 22 illustre le vecteur HK1358-11 comportant la chaîne légère avec le peptide signal MB7 et la chaîne lourde le peptide signal MB7 de l'anticorps anti-GGT 138H11B. Les unités de transcription d'intérêt sont sous la dépendance de l'enhancer E2 (hCMVie) du promoteur CDK9, la région 5'UTR du gène eIF4G1 (U3) et l'intron 5'LTR HTLV1.

Figure 23 illustre le vecteur HK1358-10 comportant la chaîne légère avec le peptide signal MB7 et la chaîne lourde le peptide signal MB7 de l'anticorps anti-GGT 138H11B. Les unités de transcription d'intérêt sont sous la dépendance de l'enhancer E2 (hCMVie) du promoteur CDK9, la région 5'UTR du gène eIF4G1 (U3) et l'intron pEF avec exon.

Figure 24 illustre le vecteur HK1358-6 comportant la chaîne légère avec le peptide signal MB7 et la chaîne lourde le peptide signal MB7 de l'anticorps anti-GGT 138H11B. Les unités de transcription d'intérêt sont sous la dépendance de l'enhancer E2 (hCMVie) du promoteur CDK9, la région 5'UTR du gène eIF4G1 (U3) et l'intron humain ROSA.

Figure 25 illustre le vecteur HK1358-9 comportant la chaîne légère avec le peptide signal MB7 et la chaîne lourde le peptide signal MB7 de l'anticorps anti-GGT 138H11B. Les unités de transcription d'intérêt sont sous la dépendance de l'enhancer E2 (hCMVie) du promoteur CDK9, la région 5'UTR du gène eIF4G1 (U3) et l'intron du gène ubiquitine.

Figure 26 illustre la productivité de l'anticorps anti-GGT (138H11B) dans le contexte E2CDK9U3 avec différents introns, en pools stables en milieu avec sérum, en comparaison avec la référence LTR RSV + intron pCI neo.. « EF » correspond à l'intron représenté par la séquence SEQ ID NO : 71. « EFss » correspond à l'intron représenté par la séquence SEQ ID NO : 10.

Figure 27 illustre la productivité de l'anticorps anti-AMHRII (3C23K) dans le contexte E2CDK9U3 avec différents introns, en pool en milieu sans sérum, en comparaison avec la référence LTR RSV + intron pCI neo. « EF » correspond à l'intron représenté par la séquence SEQ ID NO : 71. « EFss » correspond à l'intron représenté par la séquence SEQ ID NO : 10.

Figure 28 illustre la productivité des 3 anticorps anti-GGT (138H11B), anti-AMHRII (3C23K) et anti-CD20 (R603) dans le contexte E2CDK9U3 et intron EFss, en comparaison avec la référence LTR RSV + intron pCI neo.

Figure 29 illustre la comparaison de l'effet de différents introns en association avec le LTR RSV sur l'expression en transfection transitoire de la chaîne kappa libre de l'anticorps anti-Rh(D) T125 dans la lignée CHO-S évaluée en transfection transitoire. Les colonnes de points, de gauche à droite, représentent respectivement le taux d'expression de la chaîne kappa libre sous le contrôle des introns : β-actine (Bact), EF1α, mROSA, hROSA, 5'-LTR HTLV1, ubiquitine (ubc), pCI neo. Le vecteur de référence est RSV_T125_K2. L'axe des ordonnées représente la concentration en chaînes kappa libres dans le milieu de culture.

Figure 30 illustre la comparaison de l'effet de différents introns en association avec l'unité de transcription E2-CDK9-U3 ou le LTR RSV sur l'expression de la chaîne kappa libre de l'anticorps anti-Rh(D) T125 dans la lignée CHO-S évaluée en transfection transitoire. Les colonnes de points, de gauche à droite, représentent respectivement le taux d'expression de la chaîne kappa libre sous le contrôle des associations : E2-CDK9-U3 sans intron, E2-CDK9-U3 avec intron hROSA, E2-CDK9-U3 avec intron mROSA, LTR RSV avec intron EF1α, LTR RSV avec intron mROSA, E2-CDK9-U3 avec intron EF1α, LTR RSV avec intron hROSA. Les vecteurs de référence sont RSV_T125_K2 et pRep4KT125. L'axe des ordonnées représente la concentration en chaînes kappa libres dans le milieu de culture.E2 représente l'enhanceur hCMVie. U3 correspond à la région 5'UTR du gène eIF4G1.

Figure 31 illustre la comparaison de l'expression en pools stables de transfectants exprimant l'IgG anti-Rh(D) dans la lignée CHO-S en fonction du vecteur (E2CDK9U3 / LTR RSV intron pCIneo) et plus précisément la productivité en pools stables de l'anticorps entier anti-Rh(D) T125 avec le vecteur contenant l'unité de transcription E2-CDK9-U3 (HK E2 CDK9 U3) en comparaison avec la référence avec LTR RSV intron pCIneo (HK463-18). E2 représente l'enhanceur hCMVie. U3 correspond à la région 5'UTR du gène eIF4G1.

Figure 32 est un diagramme de répartition des transfectants exprimant l'IgG anti-Rh(D) dans la lignée CHO-S en fonction du vecteur (E2CDK9U3 / LTR RSV intron pCI neo). Ce diagramme illustre la productivité de clones produisant l'anticorps entier anti-Rh(D) T125 avec le vecteur contenant l'unité de transcription E2-CDK9-U3 (HK E2 CDK9 U3) en comparaison avec la référence avec LTR RSV intron pCIneo (HK463-18).

Figure 33 illustre la comparaison des titres moyens de chaînes kappa T125 obtenus dans la lignée YB2/0 à partir des vecteurs contenant différentes unités de transcription selon l'invention, à savoir E2-CDK9-U1, E2-CDK9-U2, E2-CDK9-U3, E2-CDK9-U2U3, E2-CDK9-U1U2U3. Les 6 moyennes obtenues sont comparées afin de déterminer lesquelles sont significativement différentes les unes des autres (tests des étendus multiples).

Figure 34 illustre la comparaison des titres moyens en immunoglobuline entière anti-Rh(D) obtenus dans la lignée YB2/0 à partir du vecteur E2-CDK9-U3 et du vecteur de référence HK463-18 contenant RSV + intron pCIneo. Les moyennes obtenues sont comparées afin de déterminer si elles sont significativement différentes l'une de l'autre (test des étendus multiples).

Figure 35 illustre la comparaison des titres moyens de l'immunoglobuline anti-CD71 (H7) obtenue dans la lignée YB2/0 à partir du vecteur E2-CDK9-U3 contenant l'intron EF1α avec celle obtenu à partir du vecteur de référence RSV_pCLneo contenant également l'intron EF. Les moyennes obtenues sont comparées afin de déterminer lesquelles sont significativement différentes les unes des autres (tests des étendus multiples).

## Exemples :

### 1. Matériels et Méthodes

#### 1.1. Transfection transitoire

Dans YB2/0, les cellules parentales sont ensemencées la veille de la transfection (J-1) à $2^E5$ cv/ml en EMS (Invitrogen, milieu à façon) + 5 % SVF (Invitrogen) en Flask. Le jour de l'électroporation (J0), centrifugation de $4^E6$ cellules par cuvette (Biorad) de 4 mm repris dans 100 μl de tampon V (Cell line nucleofector kitV, Lonza) qui sont nucléofectées par AMAXA avec 4 μg d'ADN plasmidique en utilisant le programme T020 de l'appareil. Les cellules sont cultivées en puits plaque P6 à 37°C, 7 % de $CO_2$ dans 3 ml de milieu EMS + 5 % de SVF. Les surnageants sont récoltés pour dosage ELISA à J+5.

Dans CHO-S, les séquences à exprimer sont évaluées en transfection transitoire selon le protocole du kit FreeStyle (Invitrogen). Les cellules parentales sont ensemencées 24 h avant la transfection (J-1) en Erlenmeyer (VWR) à $6^E5$ cv/ml en FreeStyle CHO EM (Fisher Bioblock scientific) et incubées sous agitation à 120 tr/min, 37°C, 8 % $CO_2$. Le jour de la transfection un complexe FreeStyle MAX Reagent (Fisher Bioblock Scientific) /ADN, au ratio 1:1, est formé en Opti Pro SFM (Invitrogen). Le complexe est ensuite déposé sur les cellules en suspension préalablement centrifugées et reprises à $1^E6$ cv/ml en FreeStyle CHO EM dans un cultiflask (Sartorius) (5ml) et incubé à 200 tr/min à 37°C, 8 % de $CO_2$. Les surnageants sont récoltés à J+5 pour évaluation du taux de molécules sécrétées dans le milieu.

### 1.2. Transfection stable

#### 1.2.1 Transfection stable de la lignée YB2/0 en milieu avec sérum

Les cellules doivent avoir une croissance stabilisée et être décongelées depuis au moins 4 semaines en milieu EMS (LFB) + 5 % SVF dans des flask F150 (80 ml). Les cellules sont repiquées la veille à 2E5 cv/ml en milieu EMS + 5 % SVF.

Le jour de l'électroporation, les cellules sont électroporées par Gene Pulser Xcell (BioRad) avec un voltage de 230 V et une capacitance de 960 µF dans des cuvettes (Biorad) de 4 mm avec 5E6 cv (qsp 500 µl de tampon d'électroporation du kit electrobuffer (Ozyme) contenant l'ADN plasmidique linéarisé). Après électroporation l'étalement est réalisé en plaques de 24 puits (P24) (25000 cellules/puits) en milieu EMS + 5 % SVF.

A J+3 : Mise en milieu sélectif pour obtenir les concentrations finales suivantes : EMS + 5%SVF + G418 1 mg/ml + rouge de phénol 1 %.

A J+7 : Renouvellement des plaques avec le milieu correspondant.

A J+10 : Lorsque les cellules sont proches de la confluence, faire 3 pools de 8 puits P24, repiquer les cellules à 2E5 cv/ml en F25 et réaliser une production maximale (Prod max à J+7), le surnageant étant récolté et dosé avec le kit Fast ELYSA (RD-biotech).

#### 1.2.2 Transfection stable de la lignée YB2/0 en milieu sans sérum

Les cellules doivent avoir une croissance stabilisée, et être décongelées depuis au moins 3 semaines, en milieu EMABPRO1 (LFB) en cultiflask sous une agitation de 250 tr/min. Les cellules sont repiquées la veille à 3E5 cv/ml en milieu EMABPRO1.

Le jour de l'électroporation, les cellules sont électroporées par Gene Pulser Xcell (BioRad) avec un voltage de 230 V et une capacitance de 950 µF dans des cuvettes (Biorad) de 4 mm avec 5E6 cv (qsp 500 µl de tampon d'électroporation du kit electrobuffer (Ozyme) contenant l'ADN plasmidique linéarisé). Après électroporation les cellules sont reprises à 3E5 cv/ml en milieu EMABPRO1 en flacon de culture F75.

A J+3 : Mise en milieu sélectif pour obtenir les concentrations finales suivantes : EMABPRO1+additif LFB pour clonage cellulaire de faible densité LDCC + G418 1 mg/ml.

A J+10 : si la densité cellulaire est supérieure à 6E5 cv/ml, repiquer les cellules à 3E5 cv/ml EMABPRO1+G418 1 mg/ml en F25, sinon les diluer de moitié en EMABPRO1 +additif LFB for LDCC + G418 1 mg/ml.

A partir de J+12 et 3 fois par semaine : si la densité cellulaire est supérieure à 6E5 cv/ml, repiquer les cellules à 3E5 cv/ml en F25.

A partir de J+17 et si la viabilité est supérieure à 80%, réaliser une production en mode fed batch simplifié : inoculer des cultiflasks à $3^{E}5$ cv/ml, cultiver sous agitation à 250tr/min, ajouter un feed de glucose et glutamine à J+3, J+5 et J+7.

Le surnageant est récolté à J+10 et dosé avec le kit Fast ELYSA (RD-biotech).

#### 1.2.3 Transfection stable de la lignée CHO-S

Les évaluations sont réalisées sur des pools de transfectants (« transfection en pool stable ») afin de comparer les différentes constructions sur la base d'un niveau d'expression moyenné sur un grand nombre de transfectants (plusieurs milliers) ainsi que sur les meilleurs clones sélectionnés par ClonePixFL sur ces pools.

1.2.3.1. Obtention des pools et évaluations en pools

La lignée CHO-S est cultivée en milieu Freestyle CHO EM + 8 mM de glutamine, en flask à 37°C, 8% CO2, sous agitation à 135 tr/min.

Les cellules sont repiquées la veille à $6x10^5$ cell/ ml.

Le jour de l'électroporation, les cellules sont électroporées par Gene Pulser Xcell (BioRad) avec un voltage de 300 V et une capacitance de 500 µF dans des cuvettes (Biorad) de 4 mm avec 5E6 cv (qsp 500 µl de tampon d'électroporation du kit electrobuffer (Ozyme) contenant l'ADN plasmidique linéarisé). Après électroporation les cellules sont reprises à 3E5 cv/ml en flacon de culture F75.

A J+3 : Mise en milieu sélectif pour obtenir les concentrations finales suivantes : Freestyle CHO EM + additifs LFB pour clonage cellulaire de faible densité LDCC + G418 1 mg/ml.

A J+10 : Dilution de moitié en Freestyle CHO EM + additifs LFB pour clonage cellulaire de faible densité LDCC + G418 1 mg/ml.

A partir de J+12 et 3 fois par semaine : si la densité cellulaire est supérieure à 6E5 cv/ml, repiquer les cellules à 3E5 cv/ml en F25.

A partir de J+17 repiquage en flacon F25 ou F75 en Freestyle CHO EM + G418 1 mg/ml.

A partir de J+25, réaliser une production en mode batch : inoculer les F25 à $3^{E}5$ cv/ml en Freestyle CHO EM + G418 1 mg/ml (production en pool).

Le surnageant est récolté à J+12 et dosé avec le kit Fast ELYSA (RD-biotech).

1.2.3.2. Obtention de clones et évaluations des clones

Les pools de cellules obtenus précédemment sont étalés en milieu semi-solide (CloneMedia CHO - Molecular Devices) en présence d'anticorps fluorescent de détection.

Les clones les plus forts producteurs de chaque pool sont sélectionnés tout d'abord en fonction de leur intensité de fluorescence (screening et picking par ClonePix$^{FL}$) puis en fonction de leur titre à saturation en P24.

Les meilleurs clones sont alors évalués en production en mode batch par inoculation de cultiflasks à $3^{E}5$ cv/ml en Freestyle CHO EM + G418 1 mg/ml et culture sous agitation à 250tr/min.

Le surnageant est récolté lorsque la viabilité est inférieure à 50% et dosé avec le kit Fast ELYSA (RD-biotech).

**1.3. Evaluation du taux de protéine recombinante sécrétée**

L'évaluation du taux de chaîne kappa libre de l'anticorps anti-Rh(D) T125 ainsi que la production d'IgG1 d'anti-CD20, anti-AMHRII ou d'anti-GGT sont déterminés par la technique Enzyme-linked immunosorbent assay (ELISA).

La chaine kappa libre présente dans le surnageant de culture est capturée pendant 2 h par un anticorps de chèvre anti-kappa humain (Caltag Lab) qui est adsorbé sur des plaques de 96 puits. L'anticorps capturé est ensuite révélé par un anti-kappa humain de chèvre biotinylé (Pierce) puis ajout de streptavidine couplée à la peroxydase (Pierce). Entre chaque étape 4 lavages sont effectués pour éliminer les protéines et réactifs n'entrant pas dans la formation du complexe. La révélation est faite par ajout du substrat de l'enzyme, l'OPD (Sigma) et arrêt de la réaction par du HCl 1N. La lecture se fait au spectrophotomètre à 492nm. La concentration d'anticorps est déterminée en comparaison d'une gamme étalon.

Les IgG1 produites en transfections transitoires et stables sont évaluées par le kit Fast ELYSA (RD-biotech) selon les instructions du fournisseur. La lecture de la densité optique se fait au spectrophotomètre à 450 nm. La concentration d'anticorps est déterminée en comparaison d'une gamme étalon contenue dans le kit.

**1.4. Analyses statistiques**

Les résultats de production de chaine Kappa libre ou des immunoglobulines entières sont comparés sur des valeurs normalisées par les médianes d'une expérimentation à une autre. Les analyses statistiques sont faites à l'aide du logiciel STATGRAPHICS Centurion XV. Des tests étendus multiples sont appliqués aux données avec la méthode 95.0% LSD. Les paires de données ont des différences statistiquement significatives au niveau de confiance de 95,0%.

**Exemple 1 : Construction du Vecteur E2-CDK9-U1U2U3 (Figure 1)**

- Digestion du vecteur E2-CDK9 par BamHI et NheI
- Récupération du fragment de 5630 bases, élimination du fragment de 204 bases
- Digestion de l'insert synthétique par BamHI et NheI
- Récupération sur gel de l'insert de 1271 bases
- Ligation et obtention de E2-CDK9-U1U2U3
- Criblage des clones bactérien par une technique adaptée comme la PCR, à l'aide d'amorces appropriées

**Exemple 2 : Construction du vecteur E2-CDK9-U2U3 (Figure 2)**

- Digestion PmeI sur E2- CDK9-U1U2U3
- Récupération du fragment 6620 bases élimination du fragment de 281 bases
- Ligation et obtention de E2-CDK9-U2U3
- Criblage des clones bactérien par une technique adaptée comme la PCR, à l'aide d'amorces appropriées

**Exemple 3 : Construction du vecteur E2-CDK9-U2 (Figure 3)**

- Digestion SpeI+NheI de E2- CDK9-U2U3
- Récupération sur gel du fragment à 6296 bases, élimination du fragment à 324 bases
- Ligation et obtention de E2-CDK9-U2
- Criblage des clones bactérien par une technique adaptée comme la PCR, à l'aide d'amorces appropriées

**Exemple 4 : Construction du vecteur E2-CDK9-U1 (Figure 4)**

- Digestion SpeI+NheI E2-CDK9-U1U2U3
- Récupération sur gel du fragment à 5911 bases, élimination du fragment à 990 bases
- Ligation et obtention de E2- CDK9-U1
- Criblage des clones bactérien par une technique adaptée comme la PCR, à l'aide d'amorces appropriées

**Exemple 5 : Construction du vecteur E2-CDK9-U3 (Figure 5)**

- Digestion HpaI+PmeI sur E2- CDK9-U1U2U3
- Récupération sur gel du fragment à 5957 bases, élimination du fragment à 944 bases
- Ligation et obtention de E2- CDK9-U3
- Criblage des clones bactérien par une technique adaptée comme la PCR, à l'aide d'amorces appropriées

**Exemple 6 : Construction du vecteur E2-CDK9-U1U3 (Figure 6)**

- Digestion SpeI sur E2-CDK9-U1U2U3 pour libérer la région 5'UTR U2
- Récupération sur gel du fragment à 6235 bases, élimination du fragment à 666 bases
- Ligation et obtention de E2-CDK9-U1U3
- Criblage des clones bactérien par une technique adaptée comme la PCR, à l'aide d'amorces appropriées

**Exemple 7 : Construction du vecteur E2-CDK9-EF1$\alpha$ (figure 7)**

- Digestion SpeI+NheI de E2- CDK9
- Récupération sur gel du fragment à 5636 bases, élimination du fragment à 198 bases
- Digestion de l'insert synthétique par SpeI et NheI
- Récupération sur gel de l'insert de 1001 bases
- Ligation et obtention de E2-CDK9- EF1$\alpha$
- Criblage des clones bactérien par une technique adaptée comme la PCR, à l'aide d'amorces appropriées

**Exemple 8 : Construction du vecteur E2-CDK9-EF1$\alpha$-U1U2U3 (figure 8)**

- Digestion SpeI+BamHI de E2- CDK9-EF1$\alpha$
- Récupération sur gel du fragment à ??? bases, élimination du fragment à ??? bases

- Digestion de l'insert synthétique par BamHI et NheI
- Récupération sur gel de l'insert de 1271 bases
- Ligation et obtention de E2-CDK9- EF1α-U1U2U3
- Criblage des clones bactérien par une technique adaptée comme la PCR, à l'aide d'amorces appropriées

**Exemple 9 : Construction du vecteur E2-CDK9-EF1α-U1U3 (figure 9)**

- Digestion SpeI sur E2- CDK9-EF1α-U1U2U3
- Récupération du fragment 7236 bases et élimination du fragment de 666 bases
- Ligation et obtention de E2- CDK9-EF1α-U1U3
- Criblage des clones bactérien par une technique adaptée comme la PCR, à l'aide d'amorces appropriées

**Exemple 10 : Construction du vecteur E2-CDK9-EF1α-U2U3 (figure 10)**

- Digestion HpaI/PmeI sur E2- CDK9-EF1α-U1U2U3
- Récupération du fragment 7230 bases élimination du fragment de 672 bases
- Ligation et obtention de E2- CDK9-EF1α-U2U3
- Criblage des clones bactérien par une technique adaptée comme la PCR, à l'aide d'amorces appropriées

**Exemple 11 : Construction du vecteur E2-CDK9-EF1α-U2 (figure 11)**

- Digestion SpeI de E2- CDK9-EF1α
- Récupération sur gel du fragment à 6637 bases,
- Digestion de l'insert synthétique par SpeI
- Récupération sur gel de l'insert de 666 bases
- Ligation et obtention de E2-CDK9- EF1α-U2
- Criblage des clones bactérien par une technique adaptée comme la PCR, à l'aide d'amorces appropriées

**Exemple 12 : Construction du vecteur E2-CDK9-EF1α-U1 (figure 12A)**

- Digestion BamI+SpeI de E2- CDK9-EF1α
- Récupération sur gel
- Digestion de l'insert synthétique par BamI+SpeI
- Récupération sur gel de l'insert de 947 bases
- Ligation et obtention de E2-CDK9- EF1α-U1
- Criblage des clones bactérien par une technique adaptée comme la PCR, à l'aide d'amorces appropriées

**Exemple 13 : Construction du vecteur E2-CDK9-EF1α-U1U2 (figure 13)**

- Digestion SpeI de E2-CDK9-EF1α-U1
- Récupération sur gel du fragment à 9612 bases
- Digestion de l'insert synthétique par SpeI
- Récupération sur gel de l'insert de 947 bases
- Ligation et obtention de E2-CDK9- EF1α-U1U2
- Criblage des clones bactérien par une technique adaptée comme la PCR, à l'aide d'amorces appropriées

**Exemple 14: Construction du vecteur E2-CDK9-U3-HK138H11B pour l'expression de l'anticorps anti-GGT dans YB2/0**

Le vecteur E2-CDK9-U3-HK138H11B MB7 est construit pour l'expression en pools stable de l'anticorps chimérique anti-GGT 138H11_B dans la lignée YB2/0 en tenant compte des résultats de séquençage par 5' RACE de l'hybridome source.

L'acide nucléotidique de la chaîne lourde de l'anticorps 138H11 et l'acide nucléotidique de la chaîne légère dudit anticorps sont clonées dans le vecteur CHK622-21.

**Clonage des chaînes légères de l'anticorps 138H11 sans peptide signal**

- ➢ Digestion du vecteur CHK622-21 (figure 14) par DraIII et SpeI
- ➢ Récupération d'un fragment de 9917 pb par nucléospin extract.
- ➢ 1er PCR de 15 cycles en dimers avec TAQ Proof Reading en utilisant les amorces GGT-KP1 (acagctcttactagtgccgccaccatggacatgagggtgccagctcagctgctgggac) et GGT-KP2 (ctggatgtcgcatctagcgcctggcagccacagcagcagcagtcccagcagctgag) pour obtenir un fragment de 99 pb
- ➢ 2ème PCR de 15 cycles en utilisant les amorces GGT-KP3 (gcgctagatgcgacatccagatgacacaatctagctcctctttcagtgtgag) et GGT-KD3 (CAAAAGTCCAGGGTGTGGACAGATAC) pour obtenir un fragment de 306 pb
- ➢ 3ème PCR de 15 cycles en dimers en utilisant les amorces GGT-KD1 (CACCCTGGACTTTTGGCGGAGGGACCAAGCTGGAAATCAAAAG) et GGT-KD2 (GAAAGATGAAGACACTTGGTGCAGCCACGGTTCTTTTGATTTCC) pour obtenir un fragment de 75 pb
- ➢ Purification sur gel et nucléospin extract du produit obtenu par la 2ème PCR
- ➢ Purification et nucléospin extract des produits obtenus par la 1er PCR1 et la 3ème PCR3
- ➢ Assemblage des 3 fragments par PCR avec les amorces GGT-KP1 et GGT-KD2 pour obtenir un fragment de 445 pb.
- ➢ Digestion du fragment de 445 pb par DraIII+SpeI et récupération d'un fragment de 420 pb par purification et nucléospin extract
- ➢ Ligation dudit fragment digéré dans le vecteur CHK622-21 digéré pour otenir le vecteur CHK622-21_138H11B de 10337 pb
- ➢ Screening par PCR avec les amorces 5'1PLC et GGT-KP2 qui donne un amplicon de 143pb.

**Clonage des chaînes légères de l'anticorps 138H11 avec peptide signalMB7**

- ➢ Digestion du vecteur CHK622-21 par DraIII et SpeI
- ➢ Récupération d'un fragment de 9917 pb par nucléospin extract.
- ➢ 1er PCR de 15 cycles en dimers avec TAQ Proof Reading en utilisant les amorces GGT-KP1MB7 (tacagctcttactagtgccgccaccatgcgatggagctggatcttcctg) et GGT-KP2MB7 (atctggatgtcggcgttggcgctggtgatgctcagcagcagcaggaagatc) pour obtenir un fragment de 90 pb
- ➢ 2ème PCR de 15 cycles en utilisant les amorces GGT-KP3MB7 (gccaacgccgacatccagatgacacaatctagctcctctttcagtgtgag) et GGT-KD3 pour obtenir un fragment de 304 pb
- ➢ 3ème PCR de 15 cycles en dimers en utilisant les amorces GGT-KD1 et GGT-KD2 pour obtenir un fragment de 75 pb
- ➢ Purification sur gel et nucléospin extract du produit obtenu par la 2ème PCR
- ➢ Purification et nucléospin extract des produits obtenus par la 1er PCR1 et la 3ème PCR3
- ➢ Assemblage des 3 fragments par PCR avec les amorces GGT-KP1MB7 et GGT-KD2 pour obtenir un fragment de 434 pb.
- ➢ Digestion du fragment de 434 pb par DraIII+SpeI et récupération d'un fragment de 408 pb par purification et nucléospin extract
- ➢ Ligation dudit fragment digéré dans le vecteur CHK622-21 digéré pour obtenir le vecteur CHK622-21_138H11B_MB7 de 10325 pb
- ➢ Screening par PCR avec les amorces 5'1PLC et GGT-KP2 qui donne un amplicon de 133pb.

**Clonage des chaînes lourdes de l'anticorps 138H11 sans peptide signal**

- ➢ Digestion du vecteur CHK622-21_138H11B par NheI et ApaI
- ➢ Récupération d'un fragment de 10316 pb par nucléospin extract.
- ➢ 1er PCR de 15 cycles en utilisant les amorces GGT-GP1 (tacagctcttgctagcgccgccaccatg) et GGT-GP2 (caccagctgcacttggcactgcaccccctccaggatg) pour obtenir un fragment de 97 pb
- ➢ 2ème PCR de 15 cycles en utilisant les amorces GGT-GP3 (caagtgcagctggtggagagcggcggaaccctggtgaag) et GGT-GApaI (gggggaacacggatgggcccttagtg) pour obtenir un fragment de 400 pb
- ➢ Purification et nucléospin extract des produits obtenus par les deux PCR
- ➢ Assemblage des 3 fragments par PCR avec les amorces GGT-GP1 et GGT-GApaI pour obtenir un fragment de 482 pb.
- ➢ Digestion du fragment de 482 pb par NheI et ApaI et récupération d'un fragment de 456 pb par purification et nucléospin extract
- ➢ Ligation dudit fragment digéré dans le vecteur CHK622-21 digéré pour obtenir le vecteur HK622-21_138H11B de 10772 pb (figure 15)
- ➢ Screening par PCR avec les amorces appropriées qui donne un amplicon de 604 pb

**Clonage des chaînes lourdes de l'anticorps 138H11 avec peptide signal MB7**

- ➢ Digestion du vecteur CHK622-21_138H11B_MB7 par NheI et ApaI
- ➢ Récupération d'un fragment de 10304 pb par nucléospin extract

1er PCR de 15 cycles en utilisant les amorces GGT-GP1MB7 (tacagctcttgctagcgccgccaccatgcgatggagctggatcttcctgctgctgctgag) et GGT-GP2MB7 (caccagctgcacttgggcgttggcgctggtgatgctcagcagcagcaggaagatc) pour obtenir un fragment de 94 pb

- ➢ 2ème PCR de 15 cycles en utilisant les amorces GGT-GP3 et GGT-GApaI pour obtenir un fragment de 400 pb
- ➢ Purification et nucléospin extract des produits obtenus par les deux PCR
- ➢ Assemblage des 3 fragments par PCR avec les amorces GGT-GP1 et GGT-GApaI pour obtenir un fragment de 479 pb.
- ➢ Digestion du fragment de 479 pb par NheI et ApaI et récupération d'un fragment de 453 pb par purification et nucléospin extract
- ➢ Ligation dudit fragment digéré dans le vecteur CHK622-21 digéré pour obtenir le vecteur HK622-21_138H11B_MB7 de 10757 pb (figure 16)
- ➢ Screening par PCR avec les amorces appropriées qui donne un amplicon de 601 pb.

**Clonage des chaînes lourdes de l'anticorps 138H11 avec peptide signal MB7 dans le vecteur générique E2-CDK9-U3-Gen**

- ➢ Digestion du vecteur E2-CDK9-U3-Gen (figure 17) par NheI et AseI
- ➢ Récupération d'un fragment de 8928 pb par nucléospin extract
- ➢ Digestion du vecteur HK622-21_138H11B_MB7 par NheI et AseI
- ➢ Récupération d'un fragment de 1435 pb par nucléospin extract
- ➢ Ligation dudit fragment digéré dans le vecteur E2-CDK9-U3-Gen digéré pour obtenir le vecteur E2-CDK9-U3-H138H11B_MB7
- ➢ Screening par PCR avec les amorces appropriées qui donne un amplicon de 512 pb

**Clonage des chaînes légères de l'anticorps 138H11 avec peptide signal MB7 dans le vecteur générique E2-CDK9-U3-Gen**

- ➢ Digestion du vecteur E2-CDK9-U3-H138H11B_MB7 par SpeI et XbaI
- ➢ Déphosphorylation du vecteur digéré et récupération d'un fragment de 10347 pb par nucléospin extract
- ➢ Digestion du vecteur HK622-21_138H11B_MB7 par SpeI et XbaI
- ➢ Récupération d'un fragment de 709 pb par nucléospin extract
- ➢ Ligation dudit fragment digéré dans le vecteur E2-CDK9-U3-Gen digéré pour obtenir le vecteur E2-CDK9-U3-HK138H11B_MB7 (figure 18)

➢ Screening par PCR avec les amorces appropriées qui donne un amplicon de 407 pb

**Exemple 15: Construction du vecteur E2-CDK9-U3-pCI-neo-HK138H11B**

Le vecteur HK1358-4 (figure 19), dans lequel l'intron chimérique du pCI-neo est inséré dans le vecteur E2-CDK9-U3-HK138H11B MB7, est construit pour l'expression en pools stable de l'anticorps chimérique anti-GGT 138H11_B dans la lignée YB2/0.

**Clonage de l'intron chimérique du pCI-neo dans le vecteur E2-CDK9-U3-HK138H11B MB7**

Le vecteur E2-CDK9-U3-HK138H11B MB7 est digéré par NheI et SpeI. 2 fragments de 7978 pb et 3088 pb sont obtenus par nucléospin extract. L'acide nucléotidique de l'intron chimérique pCI-neo est amplifié à partir du vecteur CHK622-21 en utilisant les amorces P1pCiNeo-NheI (acagaggagagctaggtaagtatcaaggttacaagac) et P2p-pCI-neo-NheI (tacgcattgagctagctgtggagagaaaggcaaagtg ) qui donne un amplicon de 163 pb et les amorces P1pCiNeo-SpeI (acagaggagaactaggtaagtatcaaggttacaagac) et P2p-pCI-neo-SpeI (cagccacagtactagctgtggagagaaaggcaaagtg) qui donne un amplicon de 164 pb.

Les PCR sont réalisées avec l'enzyme KAPAHIFI. Chaque primer est composé de 15 bases complémentaires à la séquence du vecteur E2-CDK9-U3-HK138H11B_MB7 au niveau du site d'insertion et d'une vingtaine de bases appartenant à la séquence de l'intron à réinsérer.

Une base supplémentaire a été ajoutée afin de recréer le site d'insertion.

L'intron chimérique pCI-neo est inséré dans le vecteur E2-CDK9-U3-HK138H11B MB7 digéré par la méthode IN-FUSION. La méthode IN-FUSION est une méthode décrite dans le kit commercial d'Ozyme (ref 639690).

Les deux fragments de 163 pb et 164 pb obtenus par PCR, ainsi que le vecteur E2-CDK9-U3-HK138H11B MB7 digéré sont assemblés en une seule étape pour obtenir le vecteur HK1358-4. L'insertion de l'intron dans le vecteur est vérifiée par les amorces 5'1PLC/CHoptiREV qui donne un amplicon de 570 pb et les amorces 5'PLC/GGT KD3 qui donne un amplicom de 387 pb.

**Exemple 16: Construction du vecteur E2-CDK9-U3-pEF**

Le vecteur HK1358-5 (figure 20), dans lequel l'intron EF1α est inséré dans le vecteur E2-CDK9-U3-HK138H11B MB7, est construit pour l'expression en pools stable de l'anticorps chimérique anti-GGT 138H11_B dans la lignée YB2/0.

Le vecteur E2-CDK9-U3-HK138H11B MB7 est digéré par NheI et SpeI. Deux fragments de 7978 pb et 3088 pb sont obtenus par nucléospin extract. L'acide nucléotidique de l'intron EF1α est amplifié à partir du vecteur K622-37EF en utilisant les amorces P1EF-NheI (ACAGAGGAGAGCTAGGTAAGTGCCGTGTGTGGTTCC) et P22-pEF-NheI (tggtggcggcgctagctgaaatggaagaaaaaaactttgaac) qui donne un amplicon de 969 pb et les amorces P1pEF-SpeI (ACAGAGGAGAACTAGGTAAGTGCCGTGTGTGGTTCC) et P22-pEF-SpeI (tggtggcggcactagtctgaaatggaagaaaaaaactttgaac ) qui donne un amplicon de 970 pb.

L'intron EF1α est inséré dans le vecteur E2-CDK9-U3-HK138H11B MB7 digéré par la méthode IN-FUSION.

Les deux fragments de 969 pb et 970 pb obtenus par PCR, ainsi que le vecteur E2-CDK9-U3-HK138H11B MB7 digéré sont assemblés en une seule étape pour obtenir le vecteur HK1358-5. L'insertion de l'intron dans le vecteur est vérifiée par les amorces eIF4g1-1/CHoptiREV qui donne un amplicon de 1534 pb et les amorces eIF4gl-11GGT KD3 qui donne un amplicom de 1351 pb.

**Exemple 17: Construction du vecteur E2-CDK9-U3-mROSA**

Le vecteur HK1358-8 (figure 21), dans lequel l'intron mROSA est inséré dans le vecteur E2-CDK9-U3-HK138H11B MB7, est construit pour l'expression en pools stable de l'anticorps chimérique anti-GGT 138H11_B dans la lignée YB2/0.

Le vecteur E2-CDK9-U3-HK138H11B MB7 est digéré par NheI et SpeI. Deux fragments de 7978 pb et 3088 pb sont obtenus par nucléospin extract. L'acide nucléotidique de l'intron mROSA est amplifié à partir du vecteur K622-37 mrosa en utilisant les amorces P1p-mROSA-NheI (acagaggagagctaggtaggggatcgggactctgg) et P22-hROSA-NheI (tggtggcggcgctagctgtcaggagaggaaagagaag) qui donne un amplicon de 381 pb et les amorces P1pmROSA-SpeI (acagaggagaactaggtaggggatcgggactctgg) et P22-hROSA-SpeI (tggtggcggcactagtctgtcaggagaggaaagagaag) qui donne un amplicon de 382 pb.

L'intron mROSA est inséré dans le vecteur E2-CDK9-U3-HK138H11B MB7 digéré par la méthode IN-FUSION.

Les deux fragments de 381 pb et 382 pb obtenus par PCR, ainsi que le vecteur E2-CDK9-U3-HK138H11B MB7 digéré sont assemblés en une seule étape pour obtenir le vecteur HK1358-8. L'insertion de l'intron dans le vecteur est vérifiée par les amorces eIF4g1-1/CHoptiREV qui donne un amplicon de 949 pb et les amorces eIF4g1-1/GGT KD3

qui donne un amplicon de 765 pb.

**Exemple 18: Construction du vecteur E2-CDK9-U3-HTLV1**

Le vecteur HK1358-11 (figure 22), dans lequel l'intron 5'-LTR HTLV1 est inséré dans le vecteur E2-CDK9-U3-HK138H11B MB7, est construit pour l'expression en pools stable de l'anticorps chimérique anti-GGT 138H11_B dans la lignée YB2/0.

Le vecteur E2-CDK9-U3-HK138H11B MB7 est digéré par NheI et SpeI. Deux fragments de 7978 pb et 3088 pb sont obtenus par nucléospin extract. L'acide nucléotidique de l'intron HCLV-1 est amplifié à partir du vecteur K622-37 HTLV en utilisant les amorces P1htlv-NheI (acagaggagagctagggctcgcatctctccttcac) et P22-htlv-NheI (tggtggcggcgctagGTAGGCGCCGGTCACAGC) qui donne un amplicon de 318 pb et les amorces P1htlv-SpeI (acagaggagaactaggctcgcatctctccttcac) et P22-htlv-SpeI (tggtggcggcactagtGTAGGCGCCGGTCACAGC ) qui donne un amplicon de 318 pb.

L'intron 5'-LTR HTLV1 est inséré dans le vecteur E2-CDK9-U3-HK138H11B MB7 digéré par la méthode IN-FUSION.

Les deux fragments de 318 pb obtenus par PCR, ainsi que le vecteur E2-CDK9-U3-HK138H11B MB7 digéré sont assemblés en une seule étape pour obtenir le vecteur HK1358-11. L'insertion de l'intron dans le vecteur est vérifiée par les amorces 5'HTLV/CHoptiREV qui donne un amplicon de 519 pb et les amorces 5'HTLV/GGT KD3 qui donne un amplicom de 702 pb.

**Exemple 19: Construction du vecteur E2-CDK9-U3-pEF-exon**

Le vecteur HK1358-10 (figure 23), dans lequel l'intron EF1α avec des bases exoniques est inséré dans le vecteur E2-CDK9-U3-HK138H11B MB7, est construit pour l'expression en pools stable de l'anticorps chimérique anti-GGT 138H11_B dans la lignée YB2/0.

Le vecteur E2-CDK9-U3-HK138H11B MB7 est digéré par NheI et SpeI. Deux fragments de 7978 pb et 3088 pb sont obtenus par nucléospin extract. L'acide nucléotidique de l'intron EF1α-exon est amplifié à partir du vecteur K622-37 EF en utilisant les amorces P12EF-NheI (ACAGAGGAGAGCTAGCGGGTTTGCCGCCAGAACACAG) et P22-pEF-NheI (TGGTGGCGGCGCTAGCTGAAATGGAAGAAAAAAACTTTGAAC) qui donne un amplicon de 991 pb et les amorces P12EF-SpeI (ACAGAGGAGAACTAGCGGGTTTGCCGCCAGAACACAG) et P22-pEF-SpeI (TGGTGGCGGCACTAGTCTGAAATGGAAGAAAAAAACTTTGAAC) qui donne un amplicon de 992 pb.

L'intron EF1α-exon est inséré dans le vecteur E2-CDK9-U3-HK138H11B MB7 digéré par la méthode IN-FUSION.

Les deux fragments de 991 pb et 992 pb obtenus par PCR, ainsi que le vecteur E2-CDK9-U3-HK138H11B MB7 digéré sont assemblés en une seule étape pour obtenir le vecteur HK1358-10. L'insertion de l'intron dans le vecteur est vérifiée par les amorces 5'EF/CHoptiREV qui donne un amplicon de 843 pb et les amorces 5'EF1/GGT KD3 qui donne un amplicon de 1023 pb.

**Exemple 20 : Construction du vecteur E2-CDK9-U3-hROSA**

Le vecteur HK1358-6 (figure 24), dans lequel l'intron hROSA est inséré dans le vecteur E2-CDK9-U3-HK138H11B MB7, est construit pour l'expression en pools stable de l'anticorps chimérique anti-GGT 138H11_B dans la lignée YB2/0.

Le vecteur E2-CDK9-U3-HK138H11B MB7 est digéré par NheI et SpeI. Deux fragments de 7978 pb et 3088 pb sont obtenus par nucléospin extract. L'acide nucléotidique de l'intron hROSA est amplifié à partir du vecteur K622-37hROSA en utilisant P1hROSA-NheI (acagaggagagctaggtaggggagcggaactctggtg) et P22-hROSA-NheI (tggtggcggcgctagctgtcaggagaggaaagagaag) qui donne un amplicon de 1247 pb et les amorces P1hROSA-SpeI (acagaggagaactaggtaggggagcggaactctggtg) et P22-hROSA-SpeI (tggtggcggcactagtctgtcaggagaggaaagagaag) qui donne un amplicon de 1248 pb.

L'intron hROSA est inséré dans le vecteur E2-CDK9-U3-HK138H11B MB7 digéré par la méthode IN-FUSION.

Les deux fragments de 1247 pb et 1248 pb obtenus par PCR, ainsi que le vecteur E2-CDK9-U3-HK138H11B MB7 digéré sont assemblés en une seule étape pour obtenir le vecteur HK1358-6. L'insertion de l'intron dans le vecteur est vérifiée par les amorces appropriées qui donne un amplicon de 1812 pb et les amorces eIF4g1-1/GGT KD3 qui donne un amplicom de 1629 pb.

**Exemple 21 : Construction du vecteur E2-CDK9-U3-UBC**

Le vecteur HK1358-9 (figure 25), dans lequel l'intron du gène ubiquitine est inséré dans le vecteur E2-CDK9-U3-HK138H11B MB7, est construit pour l'expression en pools stable de l'anticorps chimérique anti-GGT 138H11_B dans la lignée YB2/0.

Le vecteur E2-CDK9-U3-HK138H11B MB7 est digéré par NheI et SpeI. Deux fragments de 7978 pb et 3088 pb sont obtenus par nucléospin extract. L'acide nucléotidique de l'intron UBC est amplifié à partir du vecteur K622-37UBC en utilisant les amorces P12UBC-NheI (ACAGAGGAGAGCTAGAGTTCCGTCGCAGCCGGGATTTG) et P22-UBC-NheI (tggtggcggcgctagCTAACAAAAAAGCCAAAAACGGC) qui donne un amplicon de 906 pb et les amorces P1UBC-SpeI (acagaggagaactaGTGAGTAGCGGGCTGCTGG) et P22-UBC-SpeI (tggtggcggcactagtCTAACAAAAAAGCCAAAAACGGC) qui donne un amplicon de 906 pb.

L'intron ubiquitine est inséré dans le vecteur E2-CDK9-U3-HK138H11B MB7 digéré par la méthode IN-FUSION.

Les deux fragments de 906 pb et 906 pb obtenus par PCR, ainsi que le vecteur E2-CDK9-U3-HK138H11B MB7 digéré sont assemblés en une seule étape pour obtenir le vecteur HK1358-6. L'insertion de l'intron dans le vecteur est vérifiée par les amorces appropriées qui donne un amplicon de 830 pb et les amorces 5'UBC/GGT KD3 qui donne un amplicom de 1629 pb.

**Exemple 22 : Production de 2 anticorps entiers, anti-GGT et anti-AMHRII, par les vecteurs contenant l'unité de transcription E2CDK9U3 avec différents introns**

Les anticorps entiers anti-GGT (138H11B MB7) et anti-AMHRII (3C23K) ont été produits à partir de pool stables dans YB2/0, respectivement en milieu avec sérum et sans sérum, par les vecteurs dans le contexte E2CDK9U3 respectivement avec l'intron EF1$\alpha$ avec exon (EF), l'intron EF1$\alpha$ sans exon (EFss), l'intron ubiquitine, l'intron hROSA, l'intron mROSA, l'intron 5'LTR HTLV1, l'intron chimérique pCI-neo, l'intron $\beta$-actine ou sans intron. Les titres en anticorps obtenus avec ces vecteurs sont montrés dans les figures 26 et 27.

Le gain apporté par la structure E2CDK9U3+intron est estimé par comparaison avec un vecteur de référence codant pour la même IgG mais avec une structure d'UT comportant le LTR RSV + intron pCIneo à la place de la structure E2CDK9U3+intron.

La figure 26 illustre la productivité de l'anticorps anti-GGT (138H11B) dans le contexte E2CDK9U3 avec différents introns, en pool en milieu avec sérum, en comparaison avec la référence LTR RSV + intron pCI neo. Elle montre notamment que :

> ➢ la combinaison E2CDK9U3 sans intron additionnel apporte déjà un gain substantiel (x2,2) par rapport à LTR RSV + intron pCI neo.
> ➢ tous les introns testés apportent un gain supplémentaire à la combinaison E2CDK9U3 : plutôt modeste pour les introns beta-actine, pCIneo et HTLV, assez important pour les introns murine et human ROSA, très importants pour les introns ubiquitine et EF (avec ou sans le petit exon en 5') permettant des gains maximaux d'environ 6x par rapport à la référence LTR RSV + intron pCI neo.

La figure 27 montre notamment que :

> ➢ La hiérarchie globale des introns en combinaison avec E2CDK9U3 est maintenue par rapport à l'essai avec l'anticorps anti-GGT. En particulier, les introns EF (avec et sans exon) et ubiquitine sont les plus forts (environ x2 par rapport à la référence LTR RSV + intron pCI neo), l'intron mROSA garde un effet important (x1,6). L'intron hROSA n'a pas été testé dans cet essai.
> ➢ Les gains par rapport à la référence LTR RSV + intron pCI neo sont moins importants dans cet essai sans cause identifiée. La hiérarchie des introns n'est cependant pas remise en cause et des essais ultérieurs, avec le même anticorps à exprimer et le même procédé en milieu sans sérum, ont montré des gains plus élevés et similaires à ceux obtenus en milieu avec sérum (x5 pour l'intron EFss ; cf figure 28).

**Exemple 23 : Production de 3 anticorps différents dans YB2/0, avec et sans sérum, par un vecteur contenant l'unité de transcription E2CDK9U3+EFss (ou EF).**

Les séquences codantes pour 3 anticorps : anti-CD20 (R603), anti-GGT (138H11B MB7) et anti-AMHRII (3C23K) ont été intégrées dans un vecteur contenant l'unité de transcription E2CDK9U3+EFss. Ces vecteurs, ainsi que leurs vecteurs homologues excepté que l'unité de transcription est sous le contrôle du LTR RSV+intron pCI neo (témoin de référence) à la place de E2CDK9U3+EFss, ont été exprimés en pool, avec et sans sérum pour anti-CD20 et anti-AMHRII, avec sérum pour anti-GGT, dans des transfections indépendantes.

Le gain apporté par la structure E2CDK9U3+intron EF est estimé par comparaison avec le vecteur de référence codant pour la même IgG mais avec une structure d'UT comportant le LTR RSV + intron pCIneo à la place de la structure E2CDK9U3+intron EF.

La figure 28 illustre la productivité des 3 anticorps anti-GGT (138H11B), anti-AMHRII (3C23K) et anti-CD20 (R603) dans le contexte E2CDK9U3 + intron EFss, en comparaison avec la référence LTR RSV + intron pCI neo.

Elle montre notamment que la combinaison E2CDK9U3+intron EFss apporte toujours un gain significatif par rapport à LTR RSV + intron pCI neo : de 4,6 à 6,1x pour les 3 anticorps en milieu avec sérum. En milieu sans sérum, pour les 2 anticorps testés, les résultats sont plus variables mais montrent également un effet important de la combinaison E2CDK9U3+intron EFss (le gain le plus faible de 2x est celui déjà montré dans la figure 27).

**Exemple 24 : Comparaison des introns en association avec le LTR RSV**

Les introns à tester (Bact (β-actine), EF1α, mROSA, hROSA, 5'-LTR HTLV1, ubc (ubiquitine) sont insérés dans le vecteur d'expression K622_37, comprenant le LTR RSV, pour produire la chaîne légère kappa de l'anticorps T125. Le gain de productivité des vecteurs ainsi construits est comparé avec celui des vecteurs de référence RSV_int_KT125_2STP et RSV_T125_K2.

Les résultats obtenus à partir de 3 transfections réalisées sur 3 semaines différentes sont illustrés sur la figure 29 et permettent d'observer des différences significatives entre les introns.

Une comparaison multiple est réalisée pour les moyennes (ng/mL) de production de chaîne légère d'Ig obtenues avec les différents introns dans la lignée CHO-S (Tableau 1). La méthode actuellement utilisée pour discriminer entre les moyennes est la procédure des différences significatives minimales de Fisher (LSD). Des tests des étendues multiples sont effectués avec la méthode 95.0% LSD. Ces paires ont des différences statistiquement significatives au niveau de confiance de 95,0%.

Tableau 1

| | *Effectif* | *Moyenne* | *Groupe homogène* |
|---|---|---|---|
| RSV_int_KT125_2STP | 18 | 25506,3 | X |
| K622_37_HTLV | 18 | 26511,3 | X |
| K622_37_Ubc | 18 | 28790,0 | XX |
| K622_37_Bact | 17 | 31992,3 | XX |
| K622_37_hROSA | 18 | 33561,0 | X |
| RSV_T125_K2 | 16 | 34362,8 | X |
| K622_37_mROSA | 15 | 38874,8 | X |
| K622_37_EF | 14 | 44104,4 | X |

Cinq groupes homogènes sont identifiés en utilisant des colonnes de X. L'intron EF1α est significativement le plus efficace. En deuxième position est situé l'intron mROSA. Les autres introns n'ont pas d'effet positif en association avec le LTR RSV.

**Exemple 25 : Comparaison des unités de transcription dans les contextes E2-CDK9-U3 et LTR RSV**

Les différentes unités de transcription à tester sont testées pour la production de la chaîne légère kappa de l'anticorps T125. Le gain de productivité des vecteurs ainsi construits est comparé avec celui des vecteurs de référence pRep4KT125 et RSV_T125_K2.

Les résultats obtenus à partir de 3 transfections réalisées sur 3 semaines différentes sont illustrés sur la figure 30 et permettent d'observer des différences significatives entre les associations testées.

Une comparaison multiple est réalisée pour les moyennes (ng/mL) de production de chaîne légère d'Ig obtenues avec les différentes associations dans la lignée CHO-S (Tableau 2). La méthode actuellement utilisée pour discriminer entre les moyennes est la procédure des différences significatives minimales de Fisher (LSD). Des tests des étendues multiples sont effectués avec la méthode 95.0% LSD. Ces paires ont des différences statistiquement significatives au niveau de confiance de 95,0%.

Tableau 2

| | *Effectif* | *Moyenne* | *Groupe homogène* |
|---|---|---|---|
| RSVT125K2 | 12 | 10940,2 | X |
| E2CDK9U3 hRosa | 12 | 15847,6 | X |

(suite)

| | *Effectif* | *Moyenne* | *Groupe homogène* | | | | |
|---|---|---|---|---|---|---|---|
| K622_37 hRosa | 12 | 23340,0 | X | | | | |
| pRep4KT125 | 12 | 23843,2 | X | | | | |
| E2CDK9U3 | 12 | 31903,9 | | X | | | |
| K622_37 mRosa | 12 | 35041,1 | | X | | | |
| E2CDK9U3 mRosa | 12 | 40688,4 | | | X | | |
| K622_37 EF | 12 | 41708,2 | | | X | | |
| E2CDK9U3 EF | 12 | 51907,2 | | | | X | |

Cinq groupes homogènes sont identifiés en utilisant des colonnes de X.

L'association de E2-CDK9-U3 avec l'intron EF1$\alpha$ est significativement la plus efficace. Dans le contexte E2-CDK9-U3, l'intron EF1$\alpha$ apporte ainsi un gain de 63%.

Les associations LTR RSV avec intron EF et E2-CDK9-U3 avec intron mROSA sont également significativement très efficaces.

Dans une moindre mesure, les autres associations testées sont plus efficaces que la référence RSV T125 K2.

**Exemple 26 : Production de l'anticorps anti-Rh(D) entier (HK) par des vecteurs contenant E2CDK9U3 dans les cellules CHO-S**

Les anticorps anti-Rh(D) entiers (HK) sont produits respectivement dans les cellules CHO-S transfectées par les vecteurs contenant une unité de transcription de structure E2-CDK9-U3 et dans les cellules CHO-S transfectées par les vecteur contenant une unité de transcription de structure RSV-intron pCineo (vecteur de référence).

Le tableau 3 ci-dessous montre les résultats de dosage des anticorps anti-Rh(D) entiers produits par des pools de cellules transfectées par le vecteur HK463-18 ou par le vecteur HK E2-CDK9-U3. La figure 31 illustre ces résultats.

Tableau 3: F6-2= pool issu de transfection avec HK463-18, F11-2 = pool issu de transfection avec HK E2-CDK9-U3

| Pool | Milieu | Type de production batch | Dosage IgG ELISA en ng/ml | Gain E2CDK9U3 / RSV+intronpCI |
|---|---|---|---|---|
| F6-2 | Freestyle + G418 | J+12 F25 | 2324 | |
| F11-2 | Freestyle + G418 | J+12 F25 | 14 193 | 6,1 |

L'unité de transcription E2CDK9U3 permet d'obtenir un gain de productivité de l'ordre de 6 fois plus élevé que celui obtenu avec le vecteur de référence.

Le tableau 4 ci-dessous montre les résultats de dosage des anticorps anti-Rh(D) entiers produits par les meilleurs clones (issus du procédé de criblage décrit en matériels et méthodes, sur un nombre limité de colonies) issus des pools précédemment décrits, transfectés par le vecteur HK463-18 ou par le vecteur HK E2-CDK9-U3. La figure 32 illustre ces résultats.

Tableau 4

| | **cultiflask** | |
|---|---|---|
| **nom du vecteur** | **Prod Max J -1 ELISA IgG en ng/ml** | **prod max ELISA IgG en ng/ml** |
| | | |
| HK 463-18 | NA | <min |
| HK 463-18 | NA | 2071 |
| H K 463-18 | NA | 2732 |

(suite)

| | cultiflask | |
|---|---|---|
| nom du vecteur | Prod Max J -1 ELISA IgG en ng/ml | prod max ELISA IgG en ng/ml |
| HK 463-18 | NA | 4110 |
| HK 463-18 | NA | 16 937 |
| | | |
| | | |
| HK-E2-CDK9-U3 | NA | 4061 |
| HK-E2-CDK9-U3 | NA | 10 585 |
| HK-E2-CDK9-U3 | 6863 | 13 235 |
| HK-E2-CDK9-U3 | 13389 | 14221 |
| HK-E2-CDK9-U3 | 21 318 | 20 203 |
| HK-E2-CDK9-U3 | 29 860 | 33 069 |
| HK-E2-CDK9-U3 | 37611 | 33 402 |
| HK-E2-CDK9-U3 | NA | 36 830 |
| HK-E2-CDK9-U3 | NA | 43 851 |
| HK-E2-CDK9-U3 | NA | 47 315 |
| HK-E2-CDK9-U3 | 58 007 | 58 007 |
| HK-E2-CDK9-U3 | 47 056 | 60 304 |
| HK-E2-CDK9-U3 | 61 902 | 74233 |

**Exemple 27 : Production de la chaîne kappa T125 dans les cellules YB2/0**

La chaîne kappa T125 a été exprimée dans la lignée YB2/0 transfectée transitoirement par différents vecteurs contenant différentes constructions d'unité de transcription selon la présente invention. Les constructions d'unité de transcription testées, ainsi que les résultats d'expression obtenus sont montrées dans la figure 33.

**Exemple 28 : Production de l'anticorps anti-Rh(D) entier (HK) par des vecteurs contenant E2CDK9U3 dans les cellules YB2/0**

Les anticorps anti-Rh(D) entiers (HK) sont produits respectivement dans les cellules YB2/0 en transfection stable par les vecteurs contenant une unité de transcription de structure E2-CDK9-U3 ou par les vecteur contenant une unité de transcription de structure RSV-intron pCineo (vecteur de référence). Le résultat d'expression de l'anticorps anti-Rh(D) en μg/mL est montré dans la figure 34.

**Exemple 29 : Production de l'anticorps anti-CD71 entier (H7) dans les cellules YB2/0 par des vecteurs contenant E2CDK9U3**

Les anticorps anti-CD71 sont produits respectivement dans les cellules YB2/0 transfectées par un vecteur contenant l'unité de transcription E2-CDK9-U3 et l'intron EF ou par le vecteur de référence contenant RSV-intron pCineo. Le résultat d'expression de l'anticorps anti-CD71 en μg/mL est montré dans la figure 35.

**Revendications**

1. Unité de transcription constituée d'un polynucléotide comprenant les éléments régulateurs suivant :

   - l'enhancer du virus hCMVe, ledit enhancer ayant la séquence nucléotidique SEQ ID NO : 1, et
   - la région promotrice de la cycline dépendante des kinases 9 (CDK9), ladite région promotrice ayant la séquence nucléotidique SEQ ID NO : 2.

2. Unité de transcription selon la revendication 1, ledit polynucléotide comprenant également une séquence nucléotidique située en aval de ladite région promotrice et en amont du site d'initiation de la traduction, ladite séquence nucléotidique comprenant au moins l'une des régions 5' non traduite (5' UTR) choisie parmi les suivantes :

   - région R du Long Terminal Repeat (LTR) du virus HTLV-1 ayant la séquence nucléotidique SEQ ID NO : 3, ou un acide nucléotidique présentant au moins 70% d'identité de séquence avec la séquence SEQ ID NO : 3,
   - région 5' UTR du gène NF-κB Repressing Factor (NRF) ayant la séquence nucléotidique SEQ ID NO : 4, ou un acide nucléotidique présentant au moins 70% d'identité de séquence avec la séquence SEQ ID NO : 4,
   - région 5' UTR du gène eukaryotic Initiation Factor 4GI (eIF4GI) ayant la séquence nucléotidique SEQ IDNO : 5, ou un acide nucléotidique présentant au moins 70% d'identité de séquence avec la séquence SEQ ID NO : 5,

   les susdits acides nucléotidiques possédant au moins 70% d'identité de séquence avec les susdites séquences SEQ ID NO: 3, 4, 5 ayant essentiellement des propriétés de stabilisation des ARNm et de facilitateur de la traduction.

3. Unité de transcription selon la revendication 1, ledit polynucléotide comprenant également un intron situé en aval de ladite région promotrice et en amont du site d'initiation de la traduction, ledit intron étant choisi parmi les suivants :

- intron du gène Elongation Factor 1$\alpha$ (EF1$\alpha$) ayant la séquence nucléotidique SEQ ID NO : 10, ou un acide nucléotidique présentant au moins 70% d'identité de séquence avec la séquence SEQ ID NO : 10,
- intron murin ROSA ayant la séquence nucléotidique SEQ IDNO : 11, ou un acide nucléotidique présentant au moins 70% d'identité de séquence avec la séquence SEQ ID NO: 11,
- intron 5'LTR du virus HTLV-1 ayant la séquence nucléotidique SEQ ID NO : 12, ou un acide nucléotidique présentant au moins 70% d'identité de séquence avec la séquence SEQ ID NO : 12,
- intron pcinéo ayant la séquence nucléotidique SEQ ID NO : 13, ou un acide nucléotidique présentant au moins 70% d'identité de séquence avec la séquence SEQ ID NO: 13,
- intron du gène ubiquitine ayant la séquence nucléotidique SEQ ID NO : 53, ou un acide nucléotidique présentant au moins 70% d'identité de séquence avec la séquence SEQ ID NO : 53,
- intron humain ROSA ayant la séquence nucléotidique SEQ ID NO : 54, ou un acide nucléotidique présentant au moins 70% d'identité de séquence avec la séquence SEQ ID NO : 54.

4. Unité de transcription selon la revendication 2, ledit polynucléotide comprenant également un intron, ledit intron étant choisi parmi les suivants :

- intron du gène Elongation Factor 1$\alpha$ (EF1$\alpha$) ayant la séquence nucléotidique SEQ ID NO : 10, ou un acide nucléotidique présentant au moins 70% d'identité de séquence avec la séquence SEQ ID NO : 10,
- intron murin ROSA ayant la séquence nucléotidique SEQ IDNO : 11, ou un acide nucléotidique présentant au moins 70% d'identité de séquence avec la séquence SEQ ID NO : 11,
- intron 5'LTR du virus HTLV-1 ayant la séquence nucléotidique SEQ ID NO : 12, ou un acide nucléotidique présentant au moins 70% d'identité de séquence avec la séquence SEQ ID NO : 12,
- intron pcinéo ayant la séquence nucléotidique SEQ ID NO : 13, ou un acide nucléotidique présentant au moins 70% d'identité de séquence avec la séquence SEQ ID NO : 13,
- intron ubiquitine ayant la séquence nucléotidique SEQ ID NO : 53, ou un acide nucléotidique présentant au moins 70% d'identité de séquence avec la séquence SEQ ID NO : 53,
- intron humain ROSA ayant la séquence nucléotidique SEQ ID NO : 54, ou un acide nucléotidique présentant au moins 70% d'identité de séquence avec la séquence SEQ ID NO : 54.

ledit intron étant situé en aval de ladite région 5' UTR et en amont du site d'initiation de la transcription.

5. Unité de transcription selon la revendication 4, dans laquelle la région promotrice est

- celle de CDK9, la région 5' UTR est celle du gène eIF4GI et l'intron est celui du gène EF1$\alpha$, ladite unité de transcription ayant la séquence nucléotidique SEQ ID NO : 33, ou
- un acide nucléotidique présentant au moins 70% d'identité de séquence avec la séquence SEQ ID NO : 33 et permettant une production volumique d'une protéine d'intérêt supérieure à celle obtenue avec la combinaison de l'enhancer CMV associé à la région promotrice de CDK9.

6. Vecteur d'expression comprenant au moins une unité de transcription telle que définie selon l'une quelconque des revendications 1 à 5 et au moins un site de clonage permettant l'intégration d'un acide nucléotidique codant pour une protéine d'intérêt.

7. Vecteur d'expression comprenant au moins une unité de transcription telle que définie selon l'une quelconque des revendications 1 à 5 et au moins un site pour la recombinaison site spécifique permettant l'intégration d'un acide nucléotidique codant pour une protéine d'intérêt.

8. Vecteur d'expression selon la revendication 6 ou 7, comprenant en outre un gène de résistance eucaryote, un gène de résistance bactérien, une origine de réplication bactérienne et une unité dédiée à l'amplification génique.

9. Vecteur d'expression selon la revendication 6 ou 7, dans lequel ladite protéine d'intérêt est choisie parmi le groupe constitué des protéines participant de la coagulation ou une immunoglobuline, des cytokines, des hormones, des facteurs de croissance ou facteurs du complément et de toute protéine de fusion.

10. Cellule hôte comprenant un vecteur d'expression tel que défini dans l'une quelconque des revendications 6 à 9, ladite cellule hôte étant notamment la lignée cellulaire YB2/0.

**11.** Utilisation d'un vecteur d'expression selon la revendication 6 pour transfecter une cellule hôte.

**12.** Système d'expression comprenant un vecteur d'expression tel que défini selon l'une quelconque des revendications 6 à 9 et une cellule hôte telle que définie selon la revendication 10 permettant l'expression d'une protéine d'intérêt codée par un acide nucléotidique.

**13.** Utilisation d'un vecteur d'expression comprenant au moins une unité de transcription selon l'une quelconque des revendications 1 à 5 dans une cellule hôte selon la revendication 10 pour produire une protéine d'intérêt codée par un acide nucléotidique, ladite protéine d'intérêt étant produite avec un titre plus élevé que dans le vecteur d'expression de référence comprenant au moins un promoteur RSV, un intron pCInéo, une séquence de polyadénylation, un gène de résistance eucaryote, un gène de résistance bactérien, une origine de réplication bactérienne et une unité dédiée à l'amplification génique, ledit vecteur de référence comportant la même séquence nucléotidique.

**14.** Procédé de production *in vitro* d'une protéine recombinante comprenant les étapes de :

- introduction du vecteur d'expression comprenant au moins une unité de transcription selon l'une quelconque des revendications 1 à 5 et un ADNc codant pour une protéine d'intérêt dans une cellule hôte,
- sélection et identification des cellules hôtes obtenues à l'étape précédente exprimant de manière stable ladite protéine d'intérêt,
- extraction et purification de ladite protéine d'intérêt.

**15.** Procédé selon la revendication 14 comprenant en outre une étape de sélection et identification des cellules hôtes obtenues exprimant de manière stable ladite protéine d'intérêt.

**Patentansprüche**

**1.** Transkriptionseinheit, die aus einem Polynukleotid besteht, welches die folgenden regulierenden Elemente umfasst:

- den Enhancer des Virus hCMVe, wobei der Enhancer die Nukleotidsequenz SEQ-ID Nr.: 1 hat, und
- die Promotorregion des kinasenabhängigen Cyclins 9 (CDK 9), wobei die Promotorregion die Nukleotidsequenz SEQ-ID Nr.: 2 hat.

**2.** Transkriptionseinheit nach Anspruch 1, wobei das Polynukleotid weiterhin eine Nukleotidsequenz umfasst, die strangabwärts der Promotorregion und strangaufwärts der Initiationsstelle der Translation gelegen ist, wobei die Nukleotidsequenz mindestens eine nicht-translatierte 5'-Region (5' UTR) umfasst, welche aus den folgenden ausgewählt ist:

- dem Abschnitt R der langen endständigen Wiederholeinheit (LTR) des Virus HTLV-1 mit der Nukleotidsequenz SEQ-ID Nr.: 3, oder einer Nukleotidsäure, die eine mindestens 70 %ige Sequenzübereinstimmung mit der Sequenz SEQ-ID Nr.: 3 aufweist,
- der 5'-UTR-Region des Gens für den NF-κB Repressing Factor (NRF) mit der Nukleotidsequenz SEQ-ID Nr.: 4, oder einer Nukleotidsäure, die eine mindestens 70 %ige Sequenzübereinstimmung mit der Sequenz SEQ-ID Nr.: 4 aufweist,
- der 5'UTR-Region des Gens für den eukaryotischen Initiationsfaktor 4G1 (eIF4GI) mit der Nukleotidsequenz SEQ-ID Nr. 5, oder einer Nukleotidsäure, die eine mindestens 70 %ige Sequenzübereinstimmung mit der Sequenz SEQ-ID Nr.: 5 aufweist,

wobei die oben genannten Nukleotidsäuren, welche eine mindestens 70 %ige Sequenzübereinstimmung mit den oben genannten Sequenzen SEQ-ID Nr. 3, 4, 5 besitzen, im Wesentlichen Eigenschaften der Stabilisierung der mRNA und der Erleichterung der Translation haben.

**3.** Transkriptionseinheit nach Anspruch 1, wobei das Polynukleotid weiterhin ein Intron umfasst, welches strangabwärts der Promotorregion und strangaufwärts der Initiationsstelle der Translation gelegen ist, wobei das Intron aus den folgenden ausgewählt ist:

- dem Intron des Gens für den Elongationsfaktor 1α (EF1α) mit der Nukleotidsequenz SEQ-ID Nr.: 10, oder einer Nukleotidsäure, die eine mindestens 70 %ige Sequenzübereinstimmung mit der Sequenz SEQ-ID Nr.: 10 aufweist,
- dem ROSA-Intron der Maus mit der Nukleotidsequenz SEQ-ID Nr.: 11, oder einer Nukleotidsäure, die eine mindestens 70 %ige Sequenzübereinstimmung mit der Sequenz SEQ-ID Nr.: 11 aufweist,
- dem 5'-LTR-Intron des Virus HTLV-1 mit der Nukleotidsequenz SEQ-ID Nr.: 12, oder einer Nukleotidsäure, die eine mindestens 70 %ige Sequenzübereinstimmung mit der Sequenz SEQ-ID Nr.: 12 aufweist,
- dem pcineo-Intron mit der Nukleotidsequenz SEQ-ID Nr.: 13, oder einer Nukleotidsäure, die eine mindestens 70 %ige Sequenzübereinstimmung mit der Sequenz SEQ-ID Nr.: 13 aufweist,
- dem Intron des Ubiquitin-Gens mit der Nukleotidsequenz SEQ-ID Nr.: 53 oder einer Nukleotidsäure, die eine mindestens 70 %ige Sequenzübereinstimmung mit der Sequenz SEQ-ID Nr.: 53 aufweist,
- dem ROSA-Intron des Menschen mit der Nukleotidsequenz SEQ-ID Nr.: 54 oder einer Nukleotidsäure, die eine mindestens 70 %ige Sequenzübereinstimmung mit der Sequenz SEQ-ID Nr.: 54 aufweist.

**4.** Transkriptionseinheit nach Anspruch 2, wobei das Polynukleotid weiterhin ein Intron umfasst, wobei das Intron aus den folgenden ausgewählt ist:

- dem Intron des Gens für den Elongationsfaktor 1α (EF1α) : mit der Nukleotidsequenz SEQ-ID Nr.: 10 oder einer Nukleotidsäure, die eine mindestens 70 %ige Sequenzübereinstimmung mit der Sequenz SEQ-ID Nr.: 10 aufweist,
- dem;ROSA-Intron der Maus mit der Nukleotidsequenz SEQ-ID Nr.: 11 oder einer Nukleotidsäure, die eine mindestens 70 %ige Sequenzübereinstimmung mit der Sequenz SEQ-ID Nr.: 11 aufweist,
- dem 5'-LTR-Intron des Virus HTLV-1 mit der Nukleotidsequenz SEQ-ID Nr.: 12 oder einer Nukleotidsäure, die eine mindestens 70 %ige Sequenzübereinstimmung mit der Sequenz SEQ-ID Nr.: 12 aufweist,
- dem pcineo-Intron mit der Nukleotidsequenz SEQ-ID Nr.: 13 oder einer Nukleotidsäure, die eine mindestens 70 %ige Sequenzübereinstimmung mit der Sequenz SEQ-ID Nr.: 13 aufweist,
- dem Ubiquitin-Intron mit der Nukleotidsequenz SEQ-ID Nr.: 53 oder einer Nukleotidsäure, die eine mindestens 70 %ige Sequenzübereinstimmung mit der Sequenz SEQ-ID Nr.: 53 aufweist,
- dem ROSA-Intron des Menschen mit der Nukleotidsequenz SEQ-ID Nr.: 54 oder einer Nukleotidsäure, die eine mindestens 70 %ige Sequenzübereinstimmung mit der Sequenz SEQ-ID Nr.: 54 aufweist,

wobei das Intron strangabwärts der 5'-UTR-Region und strangaufwärts der Initiationsstelle der Transkription gelegen ist.

**5.** Transkriptionseinheit nach Anspruch 4, wobei es sich bei der Promotorregion um

- diejenige von CDK9 handelt, es sich bei der 5'-UTR-Region um diejenige des Gens eIF4GI handelt, und es sich bei dem Intron um dasjenige des Gens EF1α handelt, wobei die Transkriptionseinheit die Nukleotidsequenz SEQ-ID Nr.: 33 aufweist, oder
- oder um eine Nukleotidsäure, die eine mindestens 70 %ige Sequenzübereinstimmung mit der Sequenz SEQ-ID Nr.: 33 aufweist und eine Produktion eines Proteins von Interesse ermöglicht, die nach Volumen größer als diejenige ist, welche mit der Kombination des Enhancers CMV in Verbindung mit der Promotorregion von CDK9 erzielt wird.

**6.** Expressionsvektor, der mindestens eine Transkriptionseinheit gemäß der Begriffsbestimmung in einem beliebigen der Ansprüche 1 bis 5 und mindestens eine Klonierungsstelle umfasst, wobei letztere das Einfügen einer Nukleotidsäure ermöglicht, die für ein Protein von Interesse codiert.

**7.** Expressionsvektor, der mindestens eine Transkriptionseinheit gemäß der Begriffsbestimmung in einem beliebigen der Ansprüche 1 bis 5 und mindestens eine Stelle für die Rekombination umfasst, wobei es sich um eine spezifische Stelle handelt, die das Einfügen einer Nukleotidsäure ermöglicht, welche für ein Protein von Interesse codiert.

**8.** Expressionsvektor nach Anspruch 6 oder 7, der darüber hinaus ein eukaryotisches Resistenzgen, eine bakterielles Resistenzgen, einen bakteriellen Replikationsursprung sowie eine Einheit umfasst, die zur Genamplifikation bestimmt ist.

**9.** Expressionsvektor nach Anspruch 6 oder 7, wobei das Protein von Interesse aus der Gruppe ausgewählt ist, die aus den Proteinen, welche an der Blutgerinnung beteiligt sind, oder einem Immunglobulin, den Zytokinen, den Hormonen, den Wachstumsfaktoren oder Komplementfaktoren sowie beliebigen Fusionsproteinen besteht.

**10.** Wirtszelle, die einen Expressionsvektor gemäß der Begriffsbestimmung in einem beliebigen der Ansprüche 6 bis 9 umfasst, wobei es sich bei der Wirtszelle insbesondere um die Zelllinie YB2/0 handelt.

**11.** Verwendung eines Expressionsvektors nach Anspruch 6 zur Transfektion einer Wirtszelle.

**12.** Expressionssystem, das einen Expressionsvektor gemäß der Begriffsbestimmung in einem beliebigen der Ansprüche 6 bis 9 und eine Wirtszelle gemäß der Begriffsbestimmung in Anspruch 10 umfasst, wobei es die Expression eines Proteins von Interesse ermöglicht, welches von einer Nukleotidsäure codiert wird.

**13.** Verwendung eines Expressionsvektors, der mindestens eine Transkriptionseinheit nach einem beliebigen der Ansprüche 1 bis 5 in einer Wirtszelle nach Anspruch 10 umfasst, um ein Protein von Interesse zu produzieren, welches von einer Nukleotidsäure codiert wird,
wobei das Protein von Interesse in einem stärkeren Ausmaß produziert wird als in dem Bezugs-Expressionsvektor, welcher mindestens einen RSV-Promotor, ein pCI-neo-Intron, eine Polyadenylierungssequenz, ein eukaryotisches Resistenzgen, ein bakterielles Resistenzgen, einen bakteriellen Replikationsursprung sowie eine Einheit umfasst, die zur Genamplifikation bestimmt ist, wobei der Bezugsvektor dieselbe Nukleotidsequenz aufweist.

**14.** Verfahren zur in-vitro-Produktion eines rekombinanten Proteins, wobei es die folgenden Schritte umfasst:

- Einfügen des Expressionsvektors, der mindestens eine Transkriptionseinheit nach einem beliebigen der Ansprüche 1 bis 5 umfasst, und einer cDNA, die für ein Protein von Interesse codiert, in eine Wirtszelle,
- Selektion und Identifizierung derjenigen Wirtszellen, die im vorhergehenden Schritt erhalten wurden und auf stabile Weise das Protein von Interesse exprimieren,
- Extraktion und Aufreinigung des Proteins von Interesse.

**15.** Verfahren nach Anspruch 14, wobei es darüber hinaus einen Schritt der Selektion und Identifizierung derjenigen Wirtszellen umfasst, die erhalten werden und auf stabile Weise das Protein von Interesse exprimieren.

## Claims

**1.** Transcription unit constituted by a polynucleotide comprising the following regulatory elements:

- the hCMVie virus enhancer, said enhancer having the nucleotide sequence SEQ ID NO: 1, and
- the promoter region of Cyclin-Dependent Kinase 9 (CDK9), said promoter region having the nucleotide sequence SEQ ID NO: 2.

**2.** Transcription unit according to claim 1, said polynucleotide also comprising a nucleotide sequence situated downstream of said promoter region and upstream of the translation initiation site, said nucleotide sequence comprising at least one of the 5' untranslated regions (5' UTR) chosen from the following:

- R region of the Long Terminal Repeat (LTR) of the HTLV-1 virus having the nucleotide sequence SEQ ID NO: 3, or a nucleotide acid having at least 70% sequence identity with the sequence SEQ ID NO: 3,
- 5' UTR region of the NF-κB Repressing Factor (NRF) gene having the nucleotide sequence SEQ ID NO: 4, or a nucleotide acid having at least 70% sequence identity with the sequence SEQ ID NO: 4,
- 5' UTR region of the eukaryotic Initiation Factor 4GI (eIF4GI) gene having the nucleotide sequence SEQ ID NO: 5, or a nucleotide acid having at least 70% sequence identity with the sequence SEQ ID NO: 5,

the abovementioned nucleotide acids having at least 70% sequence identity with the abovementioned sequences SEQ ID NO: 3, 4, 5 essentially having mRNA stabilization and translation facilitator properties.

**3.** Transcription unit according to claim 1, said polynucleotide also comprising an intron situated downstream of said promoter region and upstream of the translation initiation site, said intron being chosen from the following:

- intron of the Elongation Factor 1α (EF1α) gene having the nucleotide sequence SEQ ID NO: 10, or a nucleotide acid having at least 70% sequence identity with the sequence SEQ ID NO: 10,

- murine ROSA intron having the nucleotide sequence SEQ ID NO: 11, or a nucleotide acid having at least 70% sequence identity with the sequence SEQ ID NO: 11,
- 5'LTR intron of the HTLV-1 virus having the nucleotide sequence SEQ ID NO: 12, or a nucleotide acid having at least 70% sequence identity with the sequence SEQ ID NO: 12,
- pCI-neo intron having the nucleotide sequence SEQ ID NO: 13, or a nucleotide acid having at least 70% sequence identity with the sequence SEQ ID NO: 13,
- ubiquitin gene intron having the nucleotide sequence SEQ ID NO: 53, or a nucleotide acid having at least 70% sequence identity with the sequence SEQ ID NO: 53,
- human ROSA intron having the nucleotide sequence SEQ ID NO: 54, or a nucleotide acid having at least 70% sequence identity with the sequence SEQ ID NO: 54.

**4.** Transcription unit according to claim 2, said polynucleotide also comprising an intron, said intron being chosen from the following:

- intron of the Elongation Factor 1$\alpha$ (EF1$\alpha$) gene having the nucleotide sequence SEQ ID NO: 10, or a nucleotide acid having at least 70% sequence identity with the sequence SEQ ID NO: 10,
- murine ROSA intron having the nucleotide sequence SEQ ID NO: 11, or a nucleotide acid having at least 70% sequence identity with the sequence SEQ ID NO: 11,
- 5'LTR intron of the HTLV-1 virus having the nucleotide sequence SEQ ID NO: 12, or a nucleotide acid having at least 70% sequence identity with the sequence SEQ ID NO: 12,
- pCI-neo intron having the nucleotide sequence SEQ ID NO: 13, or a nucleotide acid having at least 70% sequence identity with the sequence SEQ ID NO: 13,
- ubiquitin intron having the nucleotide sequence SEQ ID NO: 53, or a nucleotide acid having at least 70% sequence identity with the sequence SEQ ID NO: 53,
- human ROSA intron having the nucleotide sequence SEQ ID NO: 54, or a nucleotide acid having at least 70% sequence identity with the sequence SEQ ID NO: 54.

said intron being situated downstream of said 5' UTR region and upstream of the transcription initiation site.

**5.** Transcription unit according to claim 4, in which the promoter region is

- that of CDK9, the 5' UTR region is that of the eIF4GI gene and the intron is that of the EF1$\alpha$ gene, said transcription unit having the nucleotide sequence SEQ ID NO: 33, or
- a nucleotide acid having at least 70% sequence identity with the sequence SEQ ID NO: 33 and allowing a volume production of a protein of interest greater than that obtained with the combination of the CMV enhancer combined with the promoter region of CDK9.

**6.** Expression vector comprising at least one transcription unit as defined according to any one of claims 1 to 5 and at least one cloning site allowing the integration of a nucleotide acid coding for a protein of interest.

**7.** Expression vector comprising at least one transcription unit as defined according to any one of claims 1 to 5 and at least one site for the site-specific recombination allowing the integration of a nucleotide acid coding for a protein of interest.

**8.** Expression vector according to claim 6 or 7, also comprising a eukaryotic resistance gene, a bacterial resistance gene, a bacterial origin of replication and a dedicated gene amplification unit.

**9.** Expression vector according to claim 6 or 7, in which said protein of interest is chosen from the group constituted by the proteins participating in coagulation or an immunoglobulin, cytokines, hormones, growth factors or complement factors and any fusion protein.

**10.** Host cell comprising an expression vector as defined in any one of claims 6 to 9, said host cell being particularly the YB2/0 cell line.

**11.** Use of an expression vector according to claim 6 for transfecting a host cell.

**12.** Expression system comprising an expression vector as defined according to any one of claims 6 to 9 and a host cell as defined according to claim 10 allowing the expression of a protein of interest encoded by a nucleotide acid.

**13.** Use of an expression vector comprising at least one transcription unit according to any one of claims 1 to 5 in a host cell according to claim 10 for producing a protein of interest encoded by a nucleotide acid, said protein of interest being produced with a higher titre than in the reference expression vector comprising at least one RSV promoter, a pCIneo intron, a polyadenylation sequence, a eukaryotic resistance gene, a bacterial resistance gene, a bacterial origin of replication and a dedicated gene amplification unit, said reference vector comprising the same nucleotide sequence.

**14.** Method for *the in vitro* production of a recombinant protein comprising the stages of:

- introduction of the expression vector comprising at least one transcription unit according to any one of claims 1 to 5 and a cDNA coding for a protein of interest into a host cell,
- selection and identification of the host cells obtained in the previous stage expressing said protein of interest in a stable manner,
- extraction and purification of said protein of interest.

**15.** Method according to claim 14 also comprising a stage of selection and identification of the host cells obtained expressing said protein of interest in a stable manner.

Figure 1

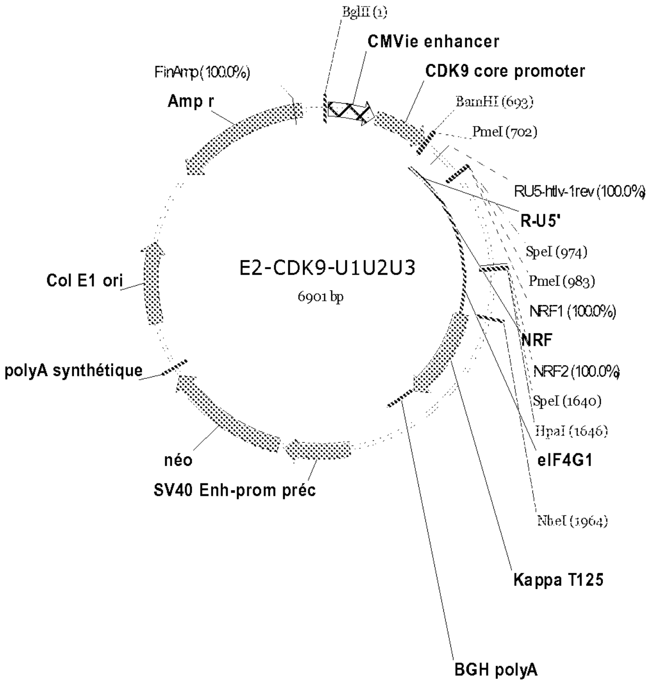


Figure2

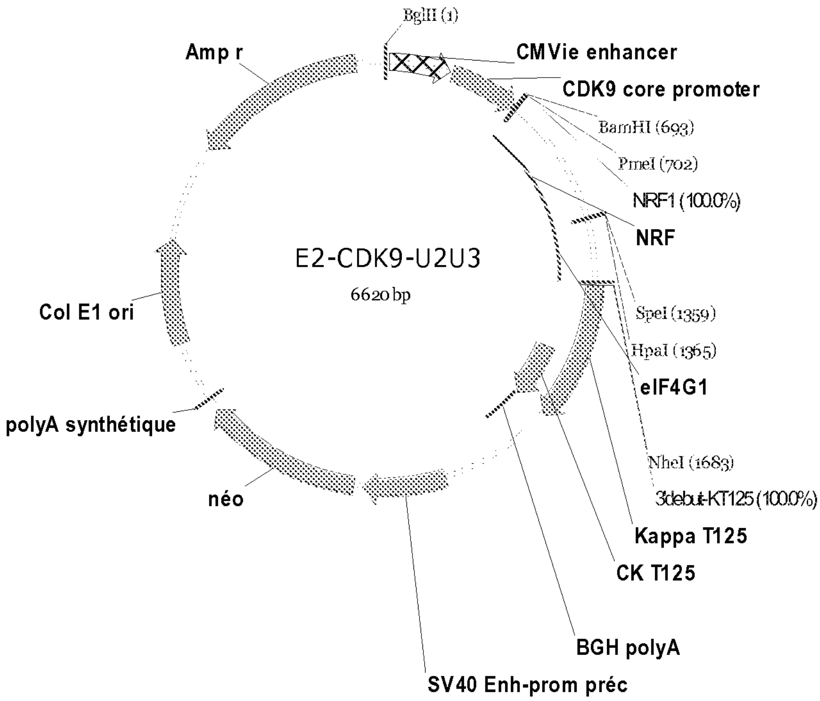

Figure 3

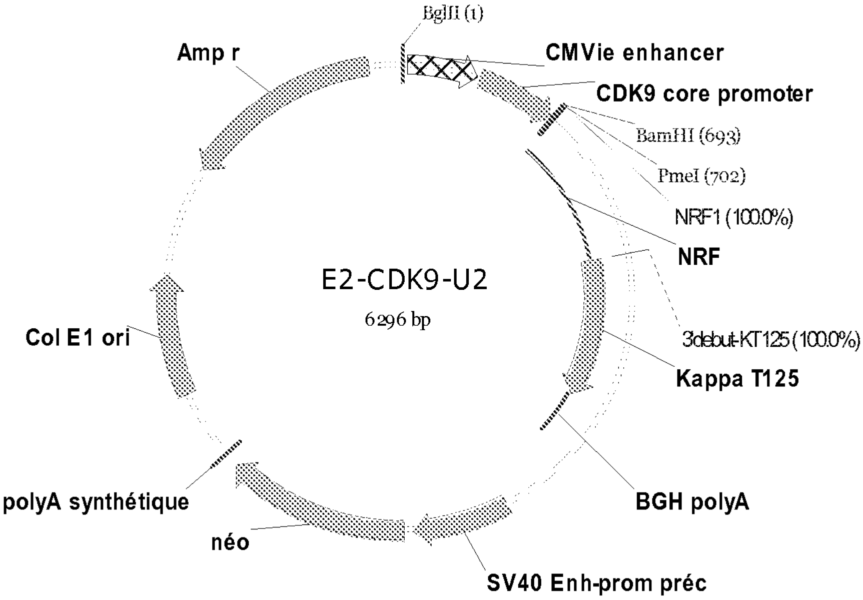


Figure 4

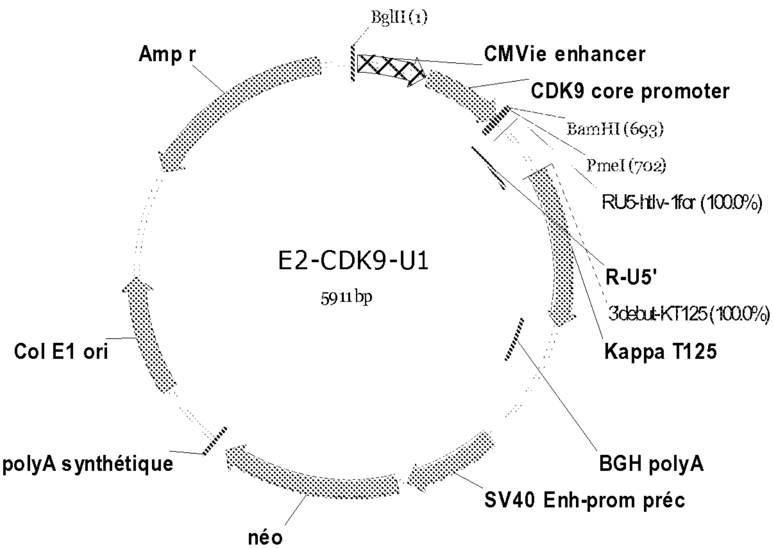

Figure 5

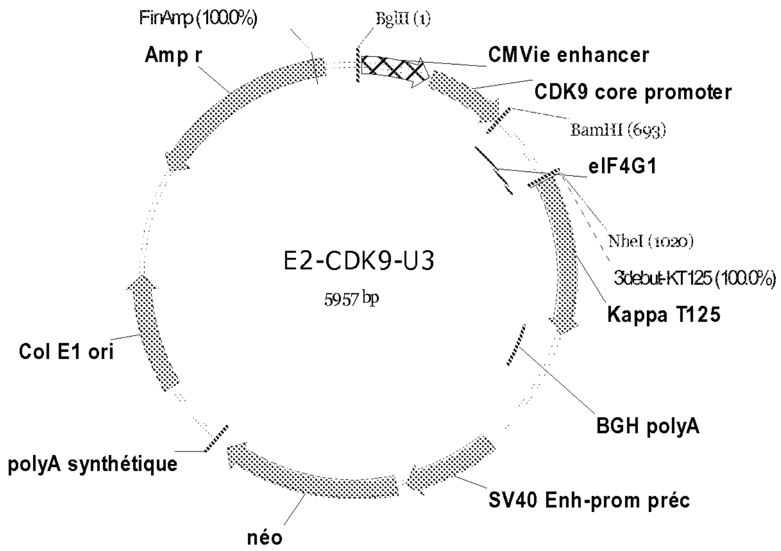


Figure 6

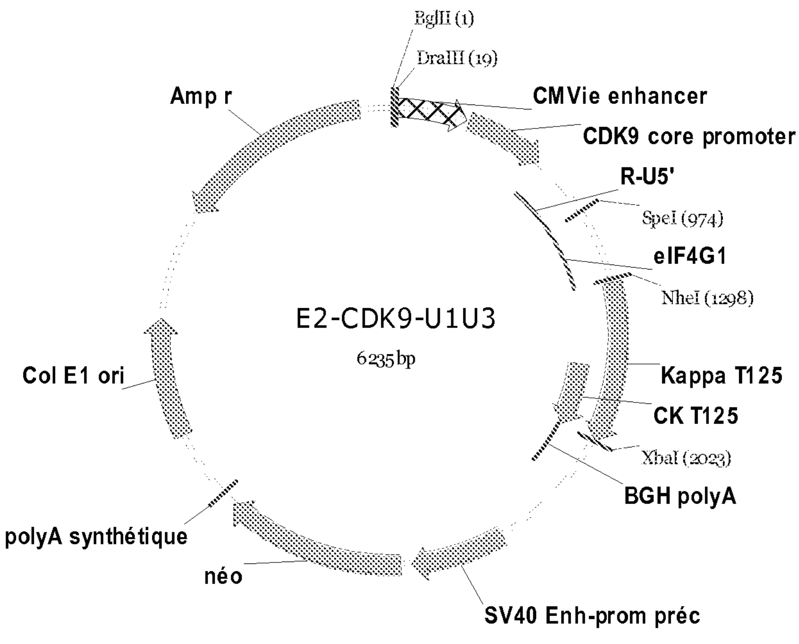

Figure 7

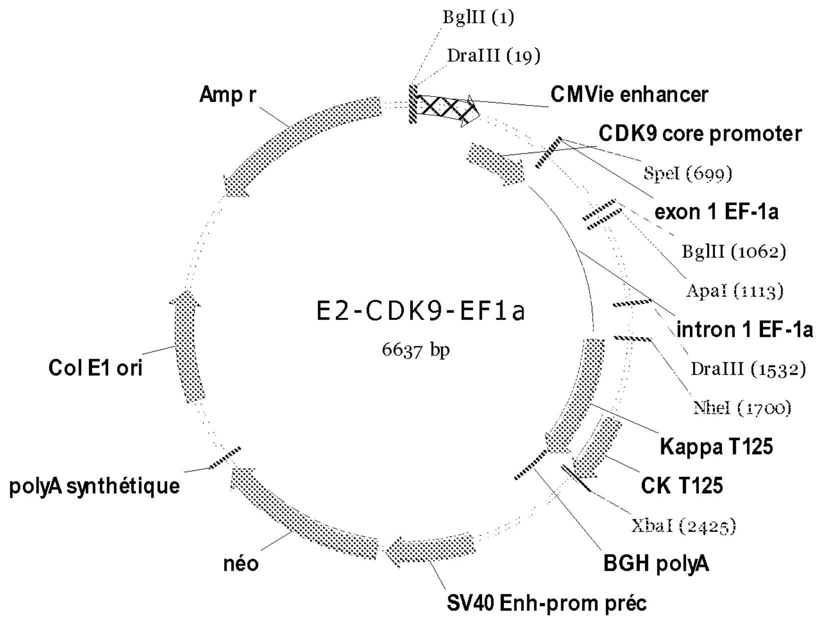


Figure 8

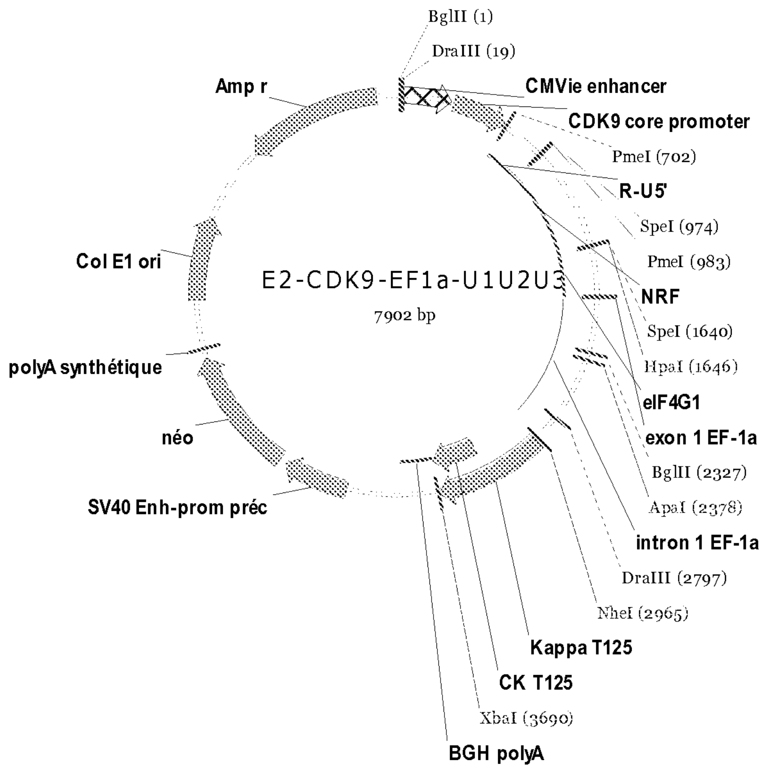

Figure 9

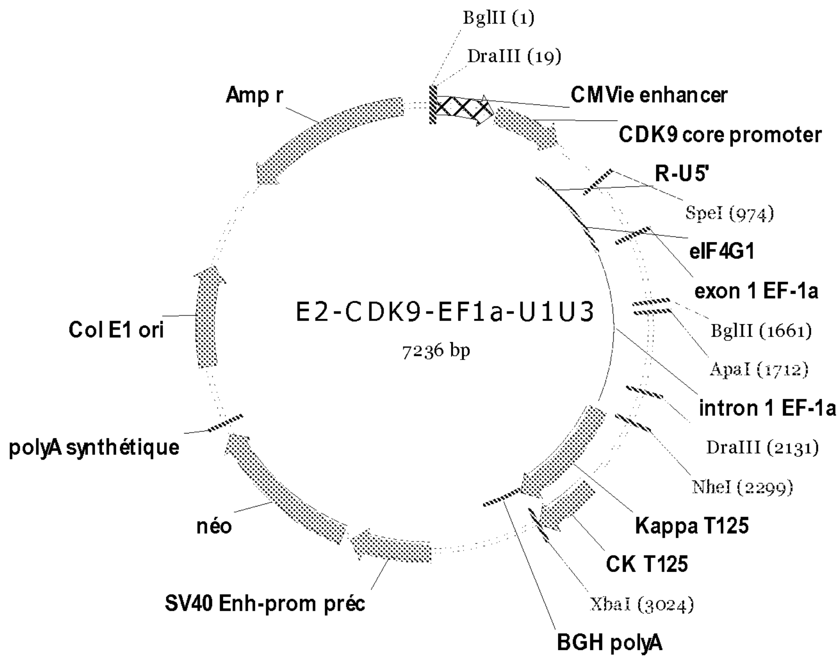


Figure 10

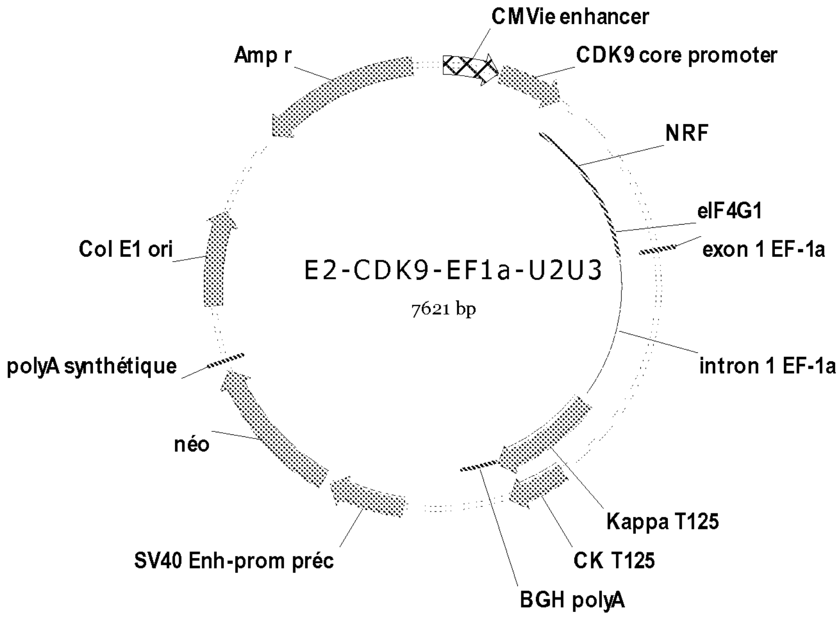

Figure 11

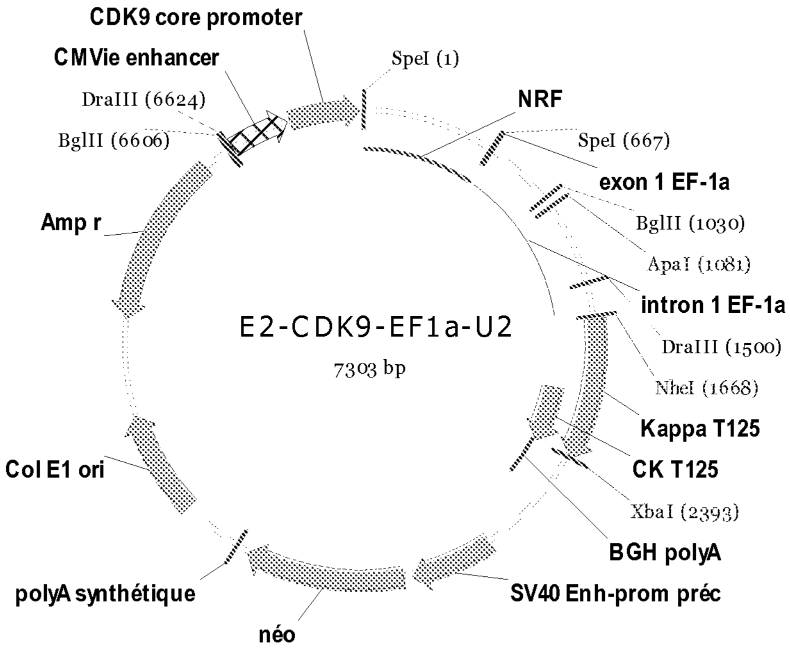


Figure 12A

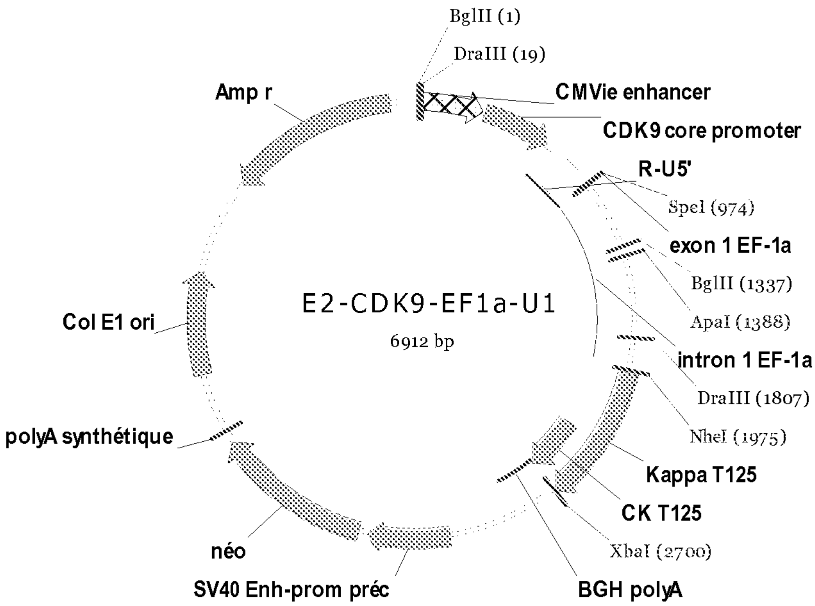

Figure 12B

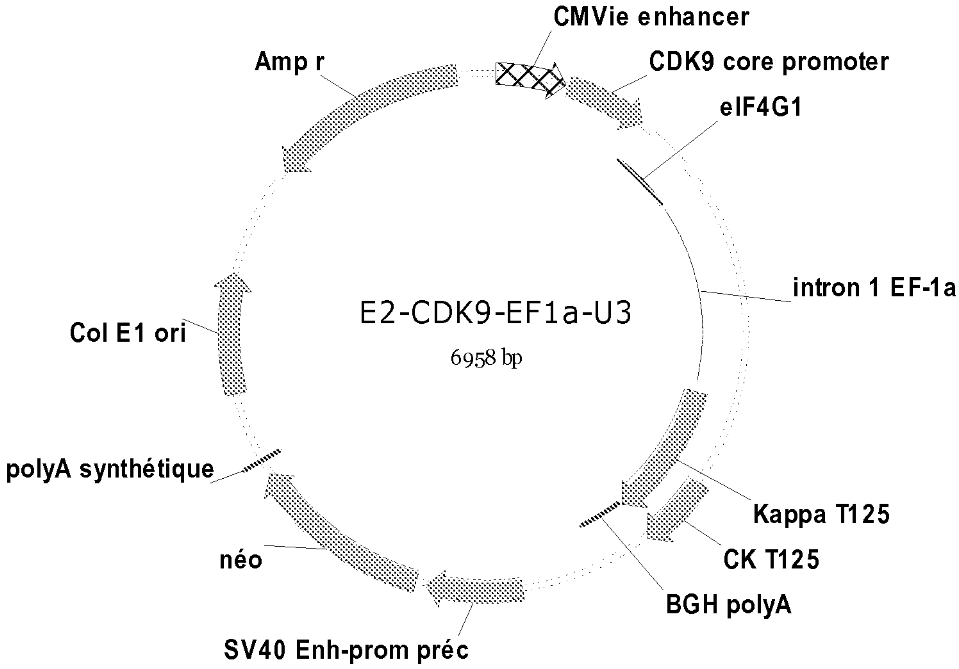


Figure 13

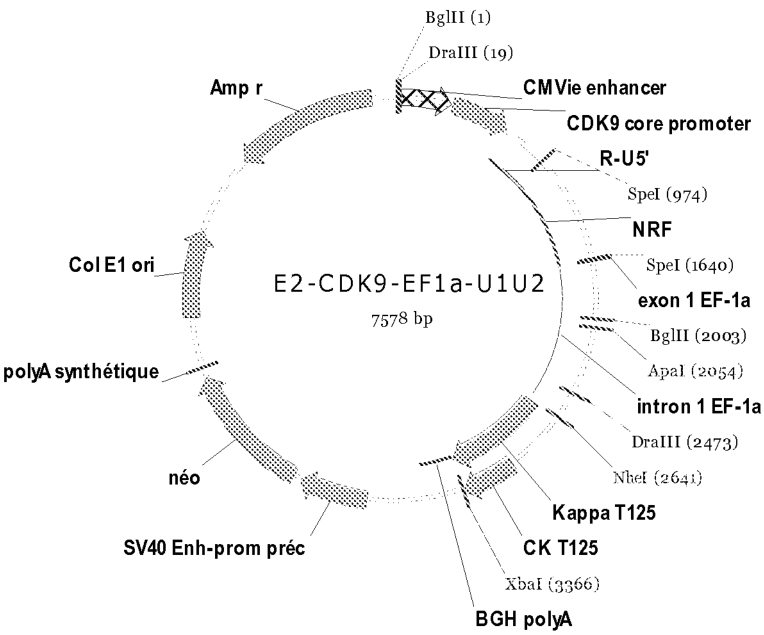

Figure 14

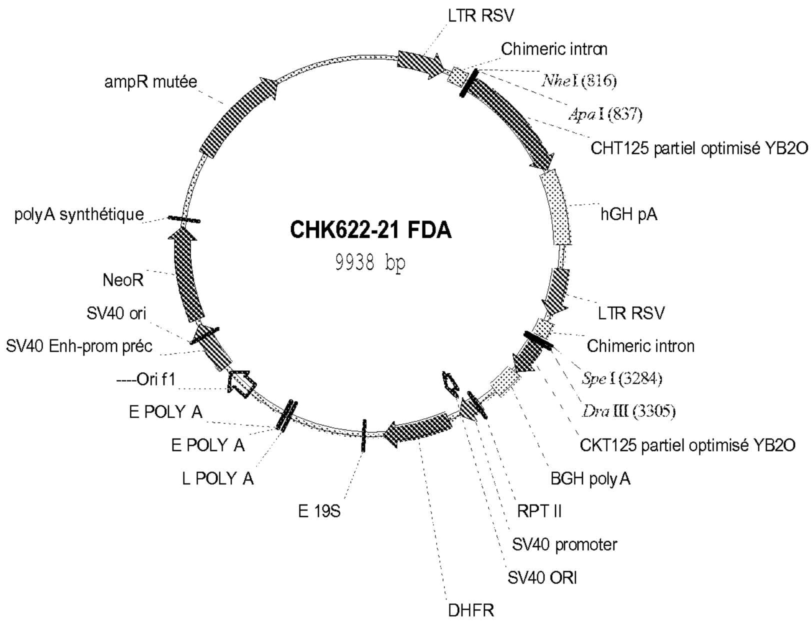
LTR RSV
Chimeric intron
NheI (816)
ApaI (837)
CHT125 partiel optimisé YB2O
hGH pA
LTR RSV
Chimeric intron
SpeI (3284)
DraIII (3305)
CKT125 partiel optimisé YB2O
BGH polyA
RPT II
SV40 promoter
SV40 ORI
DHFR
E 19S
L POLY A
E POLY A
E POLY A
----Ori f1
SV40 Enh-prom préc
SV40 ori
NeoR
polyA synthétique
ampR mutée
CHK622-21 FDA
9938 bp

Figure 15

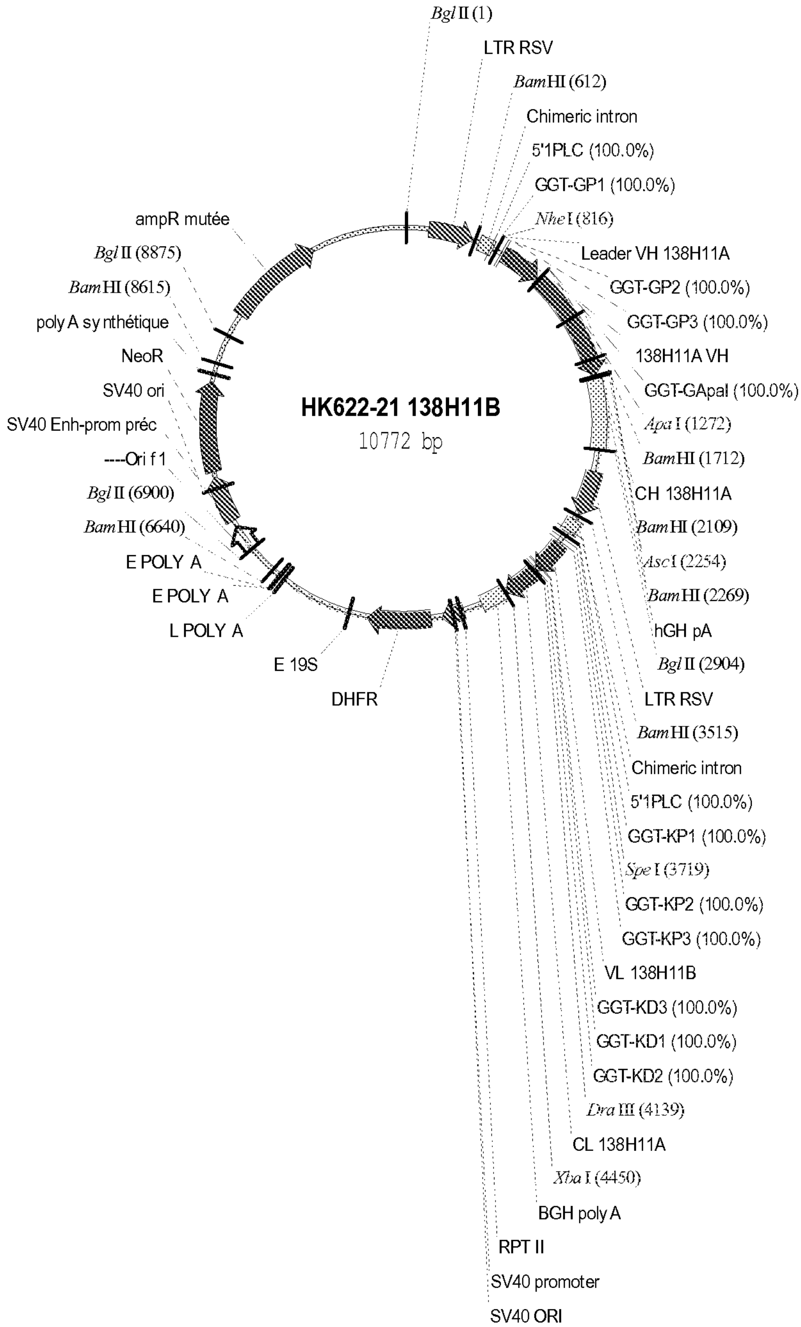
HK622-21 138H11B
10772 bp
BglII (1)
LTR RSV
BamHI (612)
Chimeric intron
5'1PLC (100.0%)
GGT-GP1 (100.0%)
NheI (816)
Leader VH 138H11A
GGT-GP2 (100.0%)
GGT-GP3 (100.0%)
138H11A VH
GGT-GApaI (100.0%)
ApaI (1272)
BamHI (1712)
CH 138H11A
BamHI (2109)
AscI (2254)
BamHI (2269)
hGH pA
BglII (2904)
LTR RSV
BamHI (3515)
Chimeric intron
5'1PLC (100.0%)
GGT-KP1 (100.0%)
SpeI (3719)
GGT-KP2 (100.0%)
GGT-KP3 (100.0%)
VL 138H11B
GGT-KD3 (100.0%)
GGT-KD1 (100.0%)
GGT-KD2 (100.0%)
DraIII (4139)
CL 138H11A
XbaI (4450)
BGH poly A
RPT II
SV40 promoter
SV40 ORI
DHFR
E 19S
L POLY A
E POLY A
E POLY A
BamHI (6640)
BglII (6900)
---Ori f1
SV40 Enh-prom préc
SV40 ori
NeoR
poly A synthétique
BamHI (8615)
BglII (8875)
ampR mutée

Figure 16

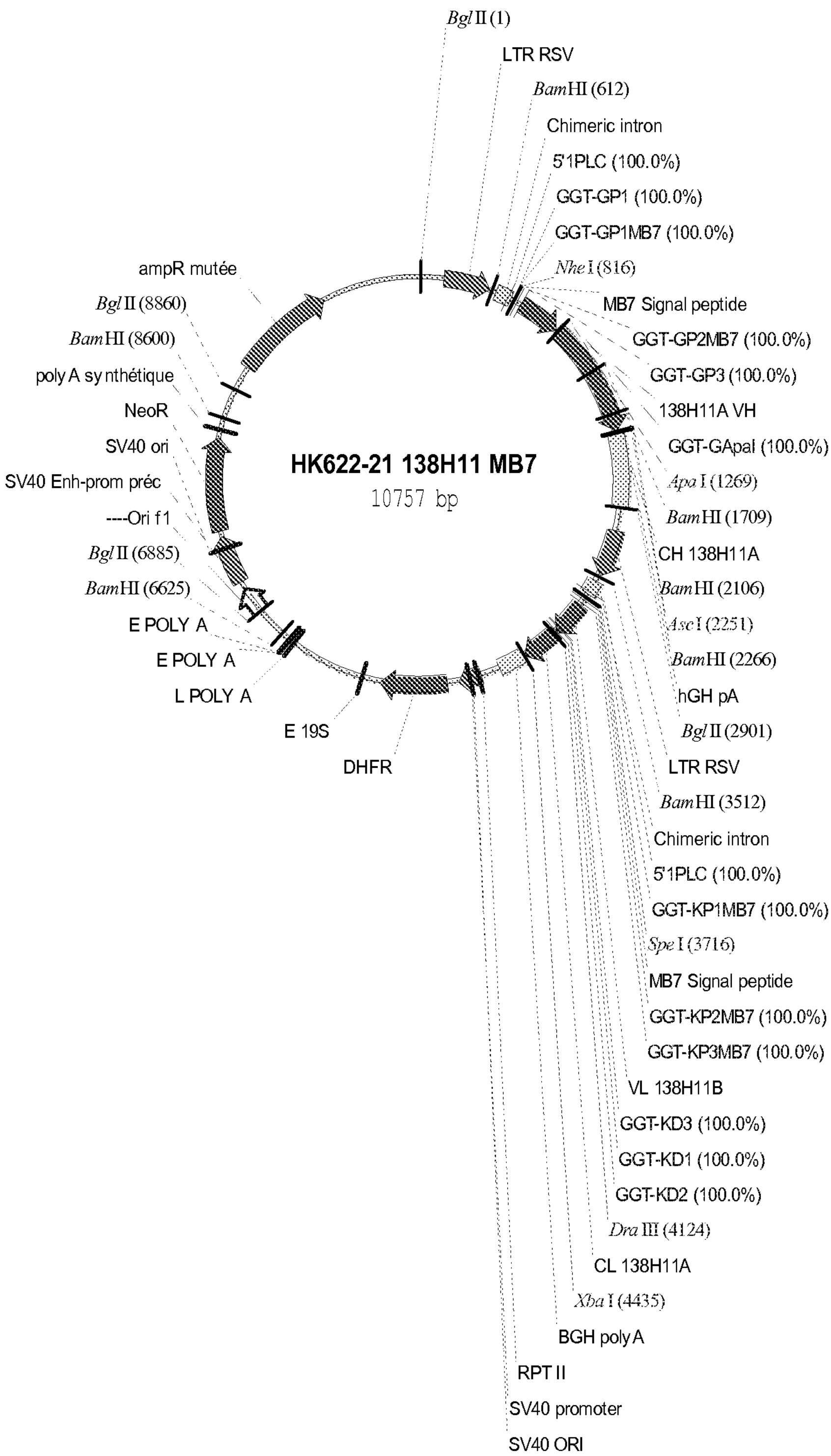
HK622-21 138H11 MB7
10757 bp
BglII (1)
LTR RSV
BamHI (612)
Chimeric intron
5'1PLC (100.0%)
GGT-GP1 (100.0%)
GGT-GP1MB7 (100.0%)
NheI (816)
MB7 Signal peptide
GGT-GP2MB7 (100.0%)
GGT-GP3 (100.0%)
138H11A VH
GGT-GApaI (100.0%)
ApaI (1269)
BamHI (1709)
CH 138H11A
BamHI (2106)
AscI (2251)
BamHI (2266)
hGH pA
BglII (2901)
LTR RSV
BamHI (3512)
Chimeric intron
5'1PLC (100.0%)
GGT-KP1MB7 (100.0%)
SpeI (3716)
MB7 Signal peptide
GGT-KP2MB7 (100.0%)
GGT-KP3MB7 (100.0%)
VL 138H11B
GGT-KD3 (100.0%)
GGT-KD1 (100.0%)
GGT-KD2 (100.0%)
DraIII (4124)
CL 138H11A
XbaI (4435)
BGH poly A
RPT II
SV40 promoter
SV40 ORI
ampR mutée
BglII (8860)
BamHI (8600)
poly A synthétique
NeoR
SV40 ori
SV40 Enh-prom préc
----Ori f1
BglII (6885)
BamHI (6625)
E POLY A
E POLY A
L POLY A
E 19S
DHFR

Figure 17

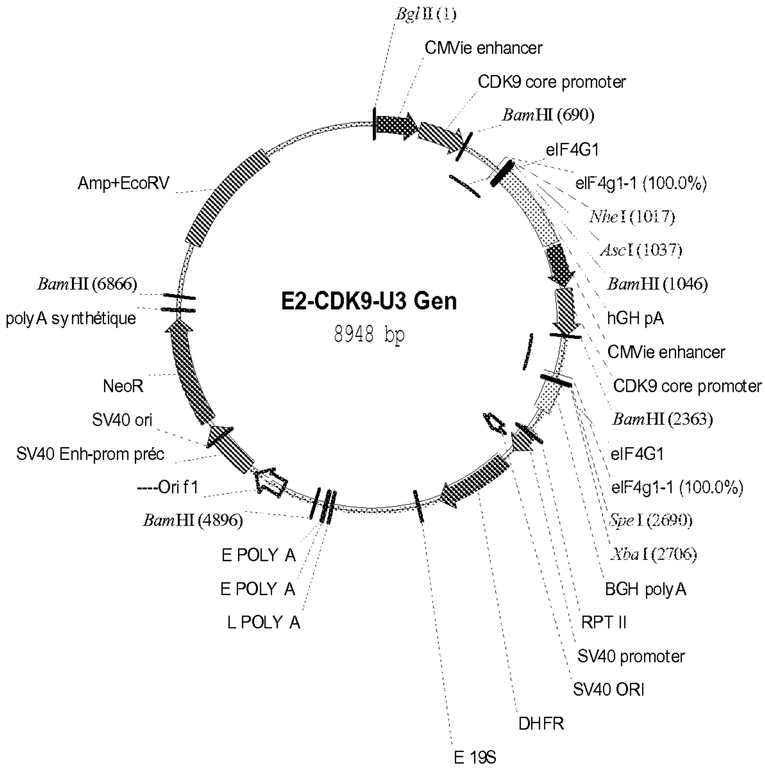
BglII (1)
CMVie enhancer
CDK9 core promoter
BamHI (690)
eIF4G1
eIF4g1-1 (100.0%)
NheI (1017)
AscI (1037)
BamHI (1046)
hGH pA
CMVie enhancer
CDK9 core promoter
BamHI (2363)
eIF4G1
eIF4g1-1 (100.0%)
SpeI (2690)
XbaI (2706)
BGH polyA
RPT II
SV40 promoter
SV40 ORI
DHFR
E 19S
L POLY A
E POLY A
E POLY A
BamHI (4896)
----Ori f1
SV40 Enh-prom préc
SV40 ori
NeoR
polyA synthétique
BamHI (6866)
Amp+EcoRV
E2-CDK9-U3 Gen
8948 bp

Figure 18

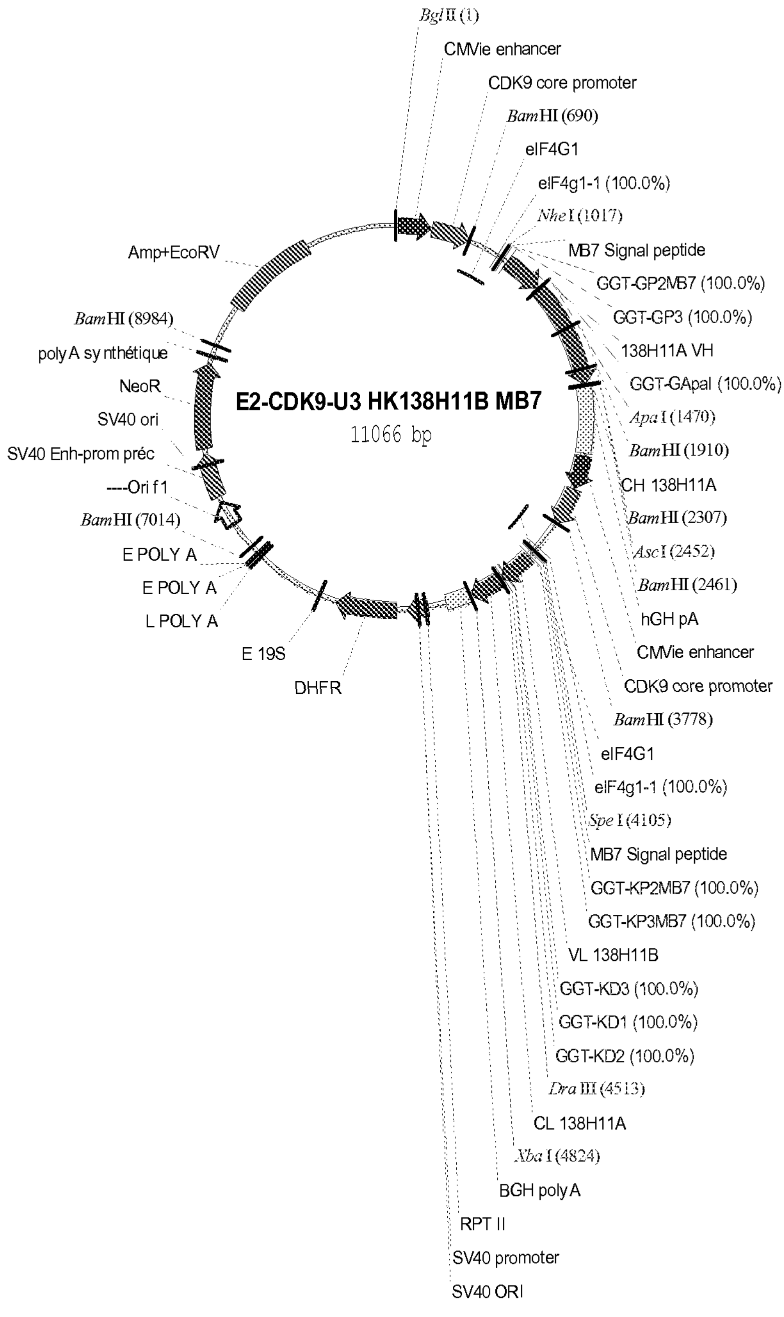
E2-CDK9-U3 HK138H11B MB7
11066 bp
BglII (1)
CMVie enhancer
CDK9 core promoter
BamHI (690)
eIF4G1
eIF4g1-1 (100.0%)
NheI (1017)
MB7 Signal peptide
GGT-GP2MB7 (100.0%)
GGT-GP3 (100.0%)
138H11A VH
GGT-GApaI (100.0%)
ApaI (1470)
BamHI (1910)
CH 138H11A
BamHI (2307)
AscI (2452)
BamHI (2461)
hGH pA
CMVie enhancer
CDK9 core promoter
BamHI (3778)
eIF4G1
eIF4g1-1 (100.0%)
SpeI (4105)
MB7 Signal peptide
GGT-KP2MB7 (100.0%)
GGT-KP3MB7 (100.0%)
VL 138H11B
GGT-KD3 (100.0%)
GGT-KD1 (100.0%)
GGT-KD2 (100.0%)
DraIII (4513)
CL 138H11A
XbaI (4824)
BGH poly A
RPT II
SV40 promoter
SV40 ORI
Amp+EcoRV
BamHI (8984)
poly A synthétique
NeoR
SV40 ori
SV40 Enh-prom préc
----Ori f1
BamHI (7014)
E POLY A
E POLY A
L POLY A
E 19S
DHFR

Figure 19

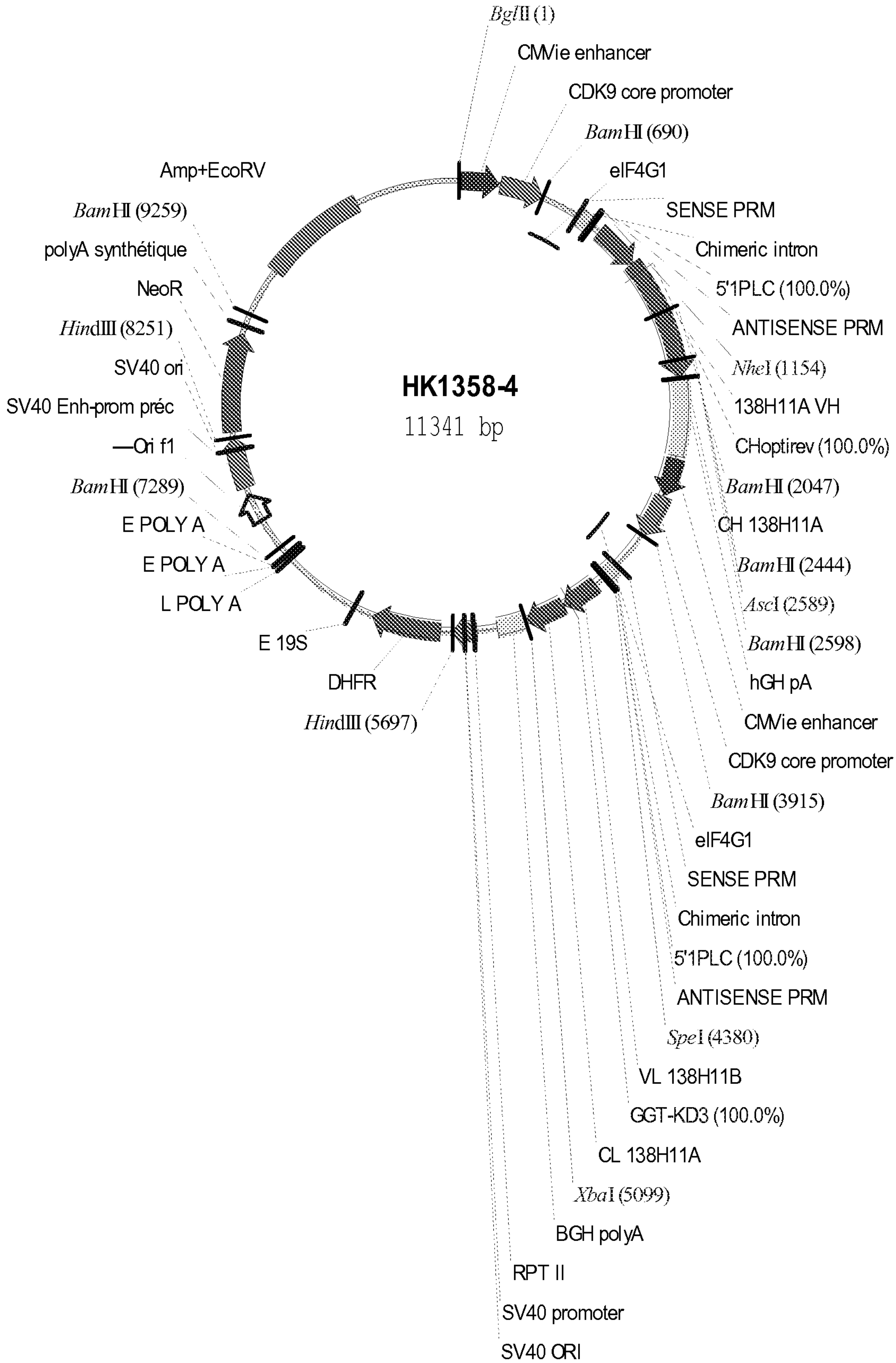
BglII (1)
CMVie enhancer
CDK9 core promoter
BamHI (690)
eIF4G1
SENSE PRM
Chimeric intron
5'1PLC (100.0%)
ANTISENSE PRM
NheI (1154)
138H11A VH
CHoptirev (100.0%)
BamHI (2047)
CH 138H11A
BamHI (2444)
AscI (2589)
BamHI (2598)
hGH pA
CMVie enhancer
CDK9 core promoter
BamHI (3915)
eIF4G1
SENSE PRM
Chimeric intron
5'1PLC (100.0%)
ANTISENSE PRM
SpeI (4380)
VL 138H11B
GGT-KD3 (100.0%)
CL 138H11A
XbaI (5099)
BGH polyA
RPT II
SV40 promoter
SV40 ORI
HindIII (5697)
DHFR
E 19S
L POLY A
E POLY A
E POLY A
BamHI (7289)
—Ori f1
SV40 Enh-prom préc
SV40 ori
HindIII (8251)
NeoR
polyA synthétique
BamHI (9259)
Amp+EcoRV
HK1358-4
11341 bp

Figure 20

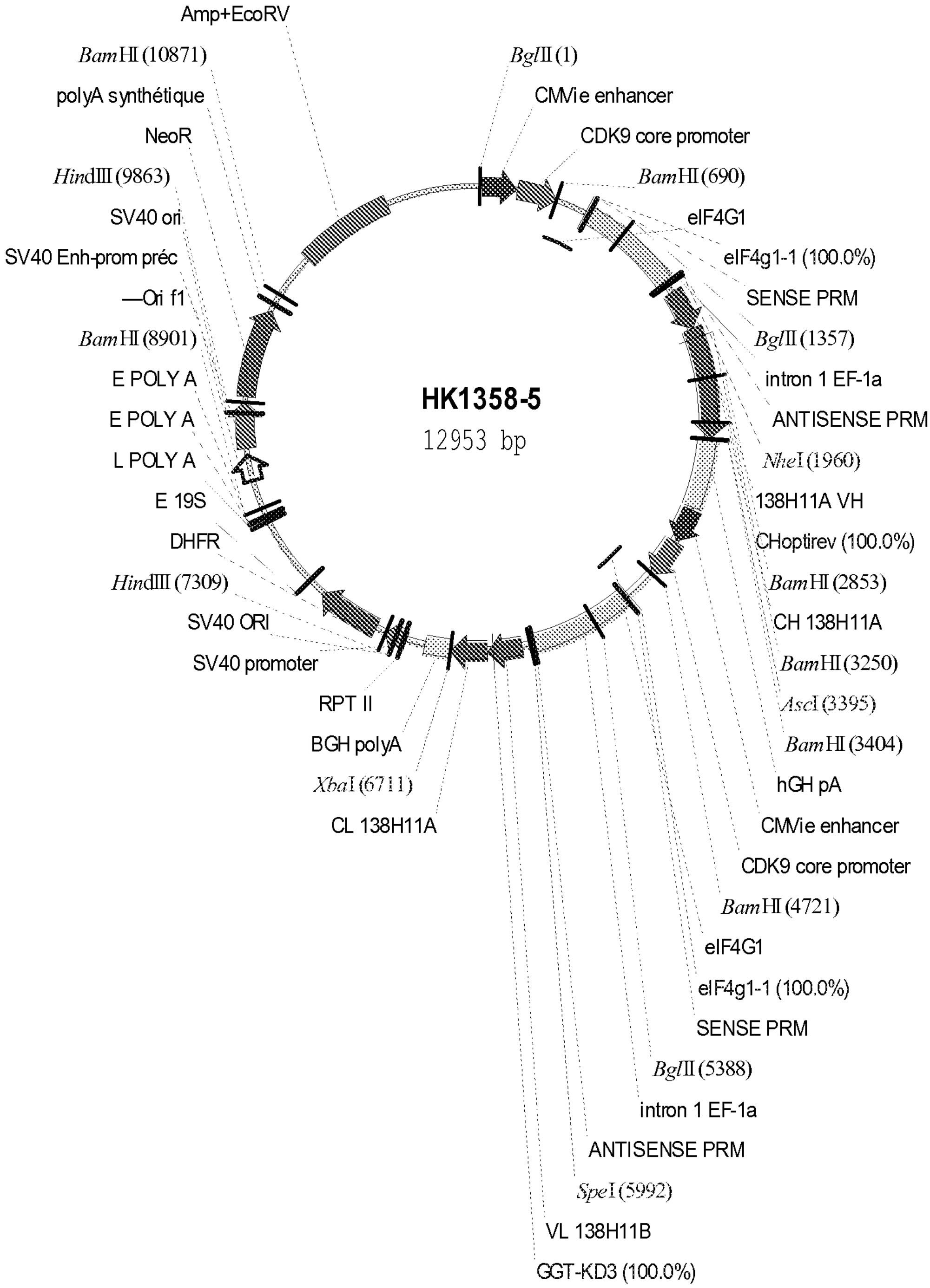
HK1358-5
12953 bp
Amp+EcoRV
BamHI (10871)
polyA synthétique
NeoR
HindIII (9863)
SV40 ori
SV40 Enh-prom préc
—Ori f1
BamHI (8901)
E POLY A
E POLY A
L POLY A
E 19S
DHFR
HindIII (7309)
SV40 ORI
SV40 promoter
RPT II
BGH polyA
XbaI (6711)
CL 138H11A
BglII (1)
CMVie enhancer
CDK9 core promoter
BamHI (690)
eIF4G1
eIF4g1-1 (100.0%)
SENSE PRM
BglII (1357)
intron 1 EF-1a
ANTISENSE PRM
NheI (1960)
138H11A VH
CHoptirev (100.0%)
BamHI (2853)
CH 138H11A
BamHI (3250)
AscI (3395)
BamHI (3404)
hGH pA
CMVie enhancer
CDK9 core promoter
BamHI (4721)
eIF4G1
eIF4g1-1 (100.0%)
SENSE PRM
BglII (5388)
intron 1 EF-1a
ANTISENSE PRM
SpeI (5992)
VL 138H11B
GGT-KD3 (100.0%)

Figure 21

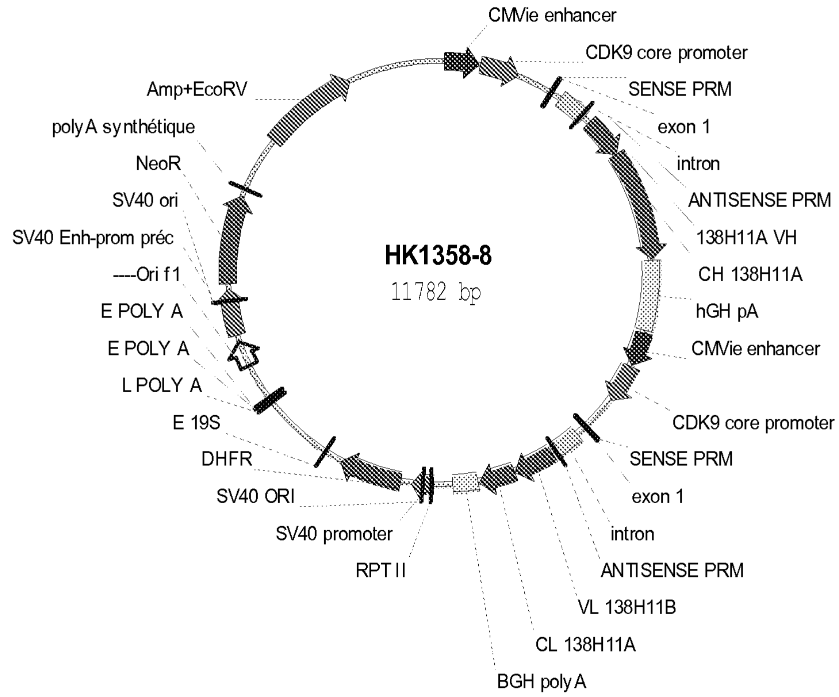


Figure 22

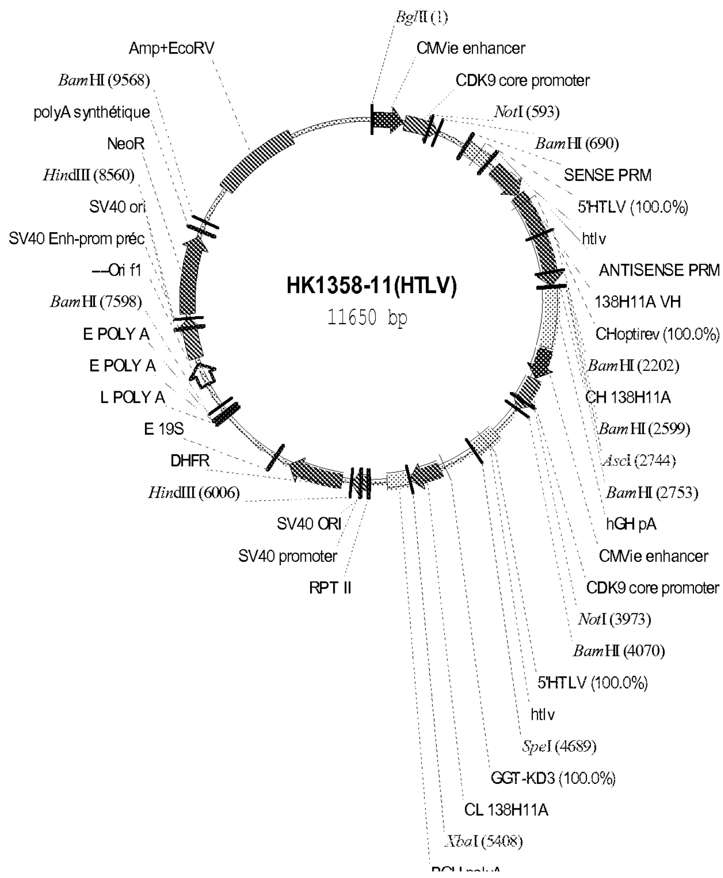

Figure 23

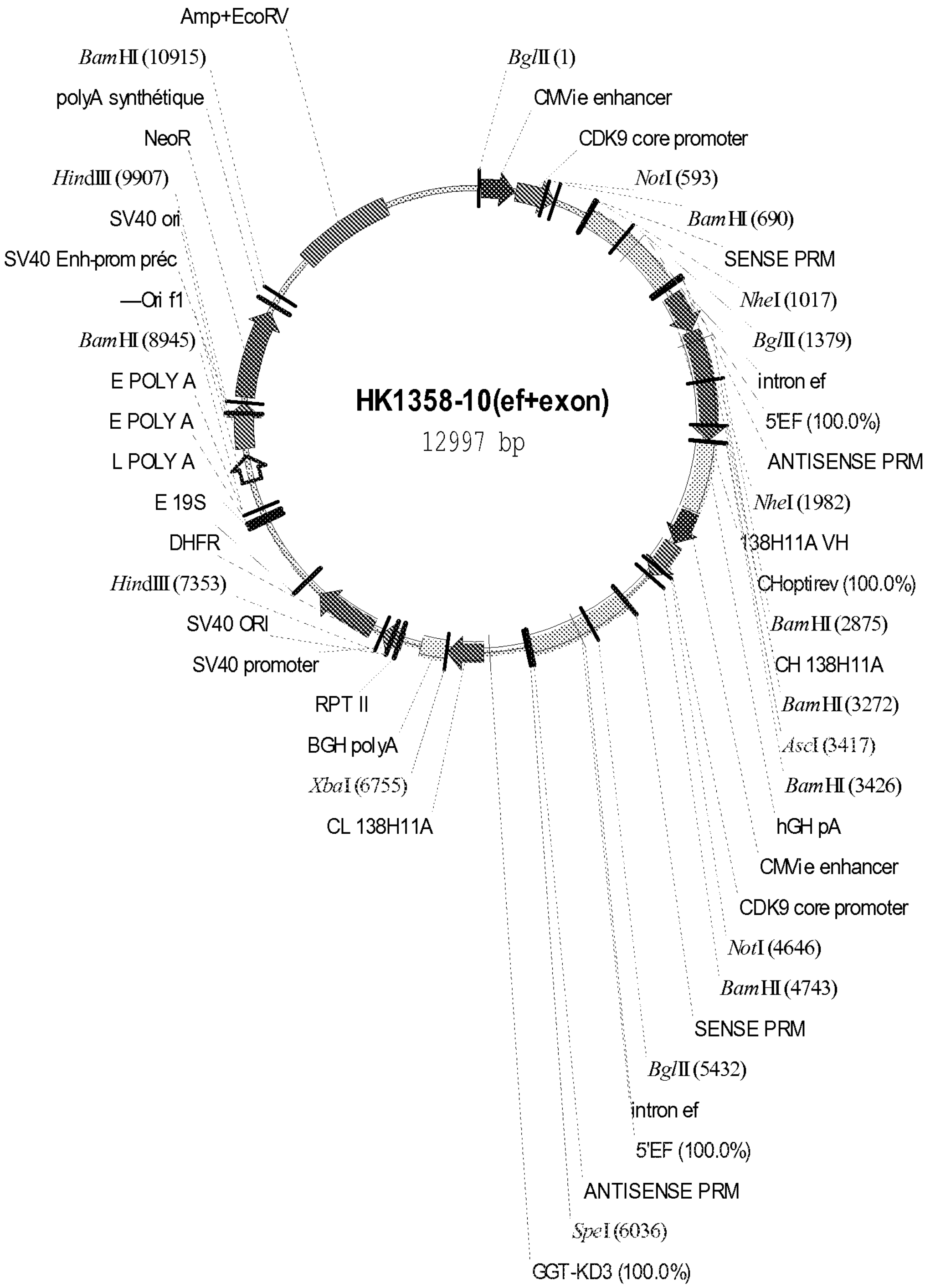
HK1358-10(ef+exon)
12997 bp
Amp+EcoRV
BamHI (10915)
polyA synthétique
NeoR
HindIII (9907)
SV40 ori
SV40 Enh-prom préc
—Ori f1
BamHI (8945)
E POLY A
E POLY A
L POLY A
E 19S
DHFR
HindIII (7353)
SV40 ORI
SV40 promoter
RPT II
BGH polyA
XbaI (6755)
CL 138H11A
BglII (1)
CMVie enhancer
CDK9 core promoter
NotI (593)
BamHI (690)
SENSE PRM
NheI (1017)
BglII (1379)
intron ef
5'EF (100.0%)
ANTISENSE PRM
NheI (1982)
138H11A VH
CHoptirev (100.0%)
BamHI (2875)
CH 138H11A
BamHI (3272)
AscI (3417)
BamHI (3426)
hGH pA
CMVie enhancer
CDK9 core promoter
NotI (4646)
BamHI (4743)
SENSE PRM
BglII (5432)
intron ef
5'EF (100.0%)
ANTISENSE PRM
SpeI (6036)
GGT-KD3 (100.0%)

Figure 24

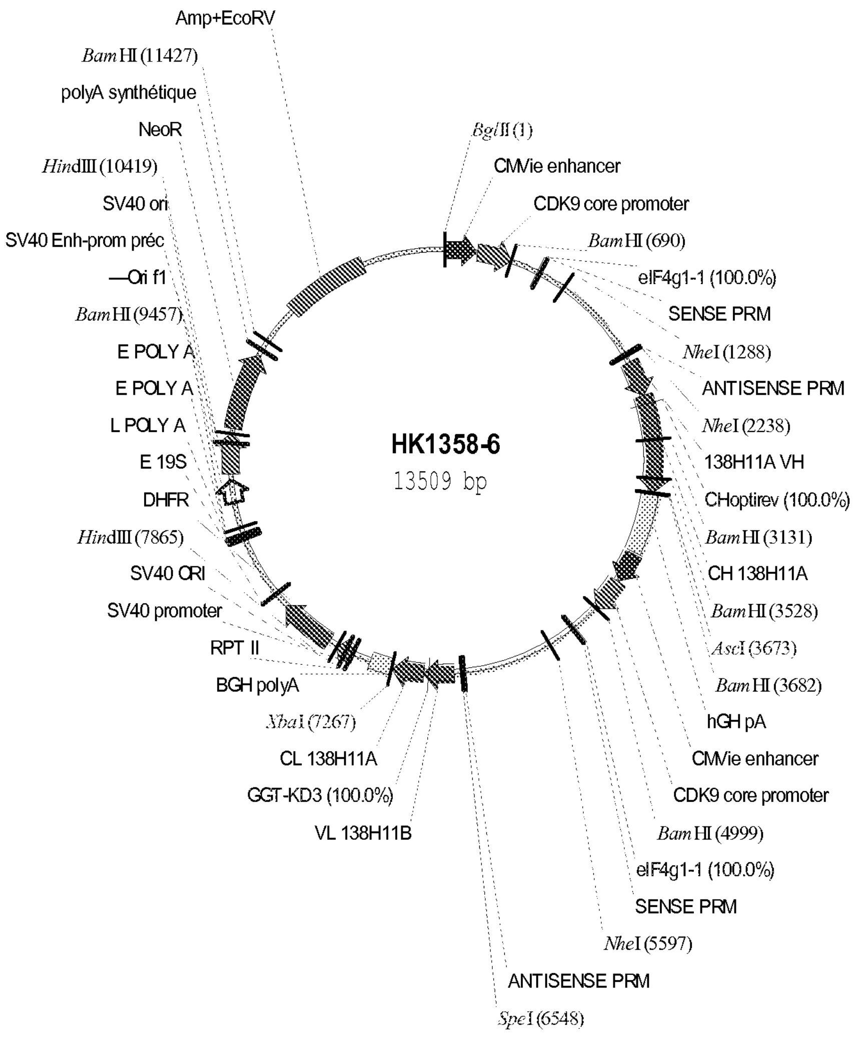
HK1358-6
13509 bp
Amp+EcoRV
BamHI (11427)
polyA synthétique
NeoR
HindIII (10419)
SV40 ori
SV40 Enh-prom préc
—Ori f1
BamHI (9457)
E POLY A
E POLY A
L POLY A
E 19S
DHFR
HindIII (7865)
SV40 ORI
SV40 promoter
RPT II
BGH polyA
XbaI (7267)
CL 138H11A
GGT-KD3 (100.0%)
VL 138H11B
BglII (1)
CMVie enhancer
CDK9 core promoter
BamHI (690)
eIF4g1-1 (100.0%)
SENSE PRM
NheI (1288)
ANTISENSE PRM
NheI (2238)
138H11A VH
CHoptirev (100.0%)
BamHI (3131)
CH 138H11A
BamHI (3528)
AscI (3673)
BamHI (3682)
hGH pA
CMVie enhancer
CDK9 core promoter
BamHI (4999)
eIF4g1-1 (100.0%)
SENSE PRM
NheI (5597)
ANTISENSE PRM
SpeI (6548)

Figure 25

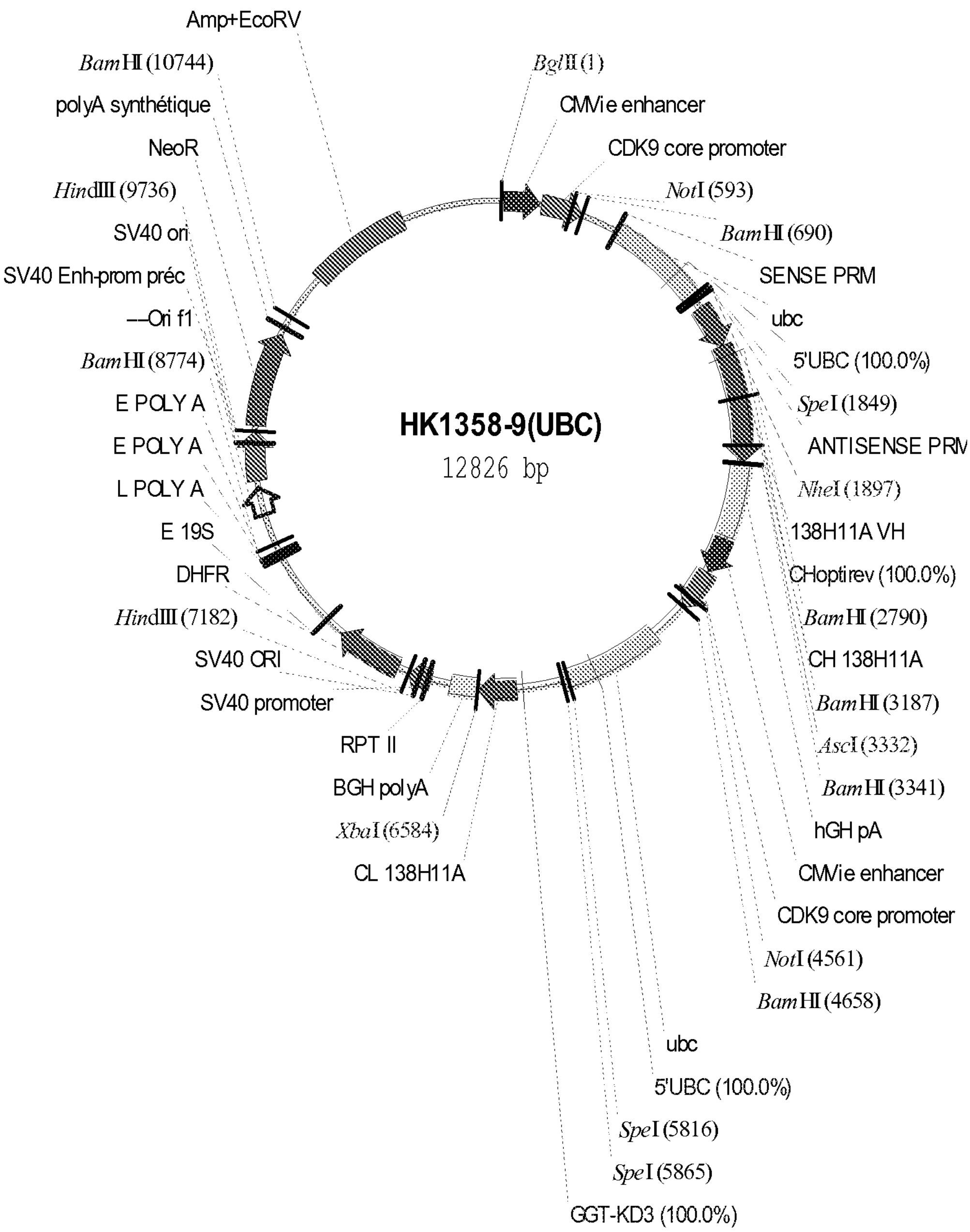
HK1358-9(UBC)
12826 bp
BglII (1)
CMVie enhancer
CDK9 core promoter
NotI (593)
BamHI (690)
SENSE PRM
ubc
5'UBC (100.0%)
SpeI (1849)
ANTISENSE PRM
NheI (1897)
138H11A VH
CHoptirev (100.0%)
BamHI (2790)
CH 138H11A
BamHI (3187)
AscI (3332)
BamHI (3341)
hGH pA
CMVie enhancer
CDK9 core promoter
NotI (4561)
BamHI (4658)
ubc
5'UBC (100.0%)
SpeI (5816)
SpeI (5865)
GGT-KD3 (100.0%)
CL 138H11A
XbaI (6584)
BGH polyA
RPT II
SV40 promoter
SV40 ORI
HindIII (7182)
DHFR
E 19S
L POLY A
E POLY A
E POLY A
BamHI (8774)
Ori f1
SV40 Enh-prom préc
SV40 ori
HindIII (9736)
NeoR
polyA synthétique
BamHI (10744)
Amp+EcoRV

Figure 26

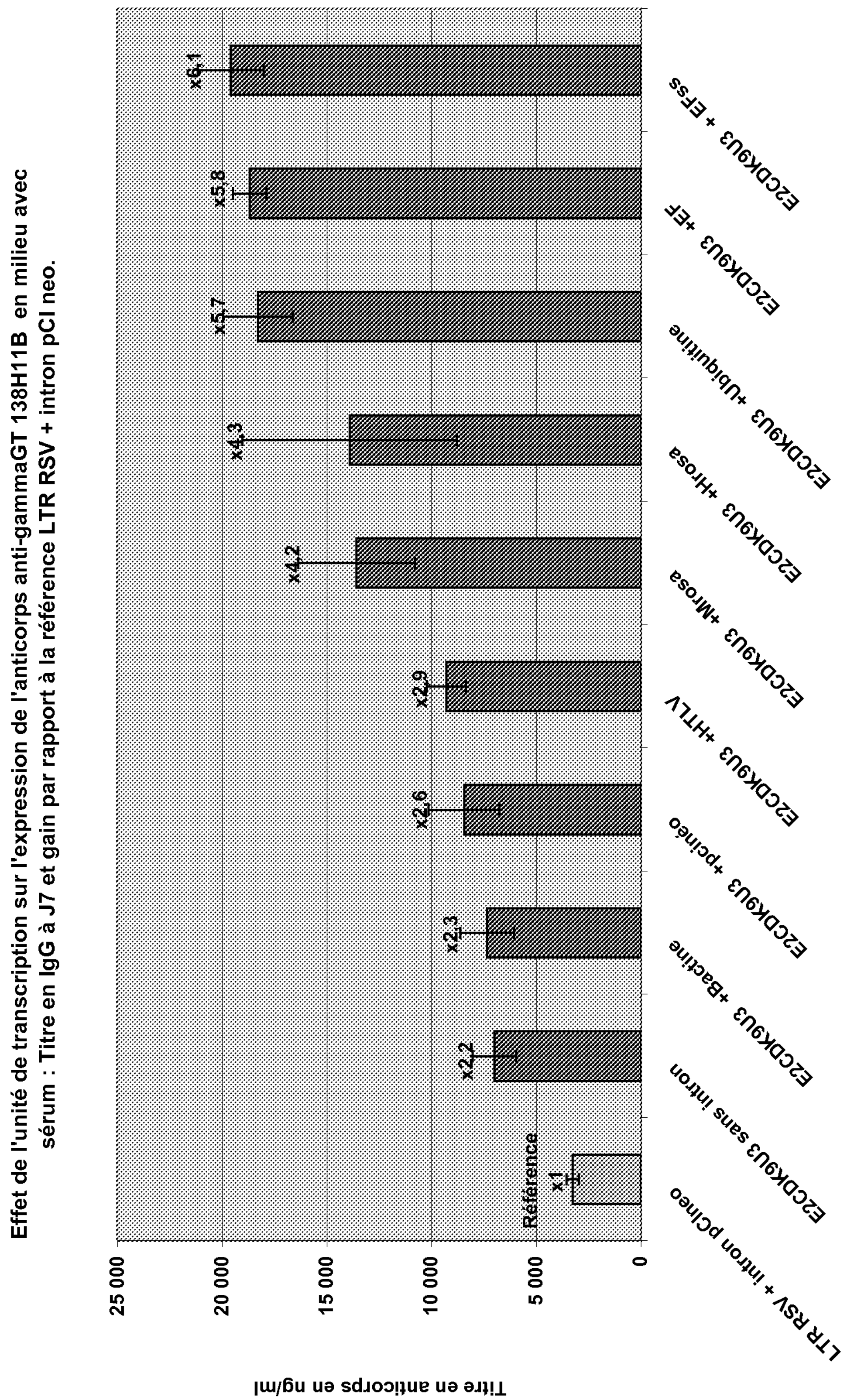

Effet de l'unité de transcription sur l'expression de l'anticorps anti-gammaGT 138H11B en milieu avec sérum : Titre en IgG à J7 et gain par rapport à la référence LTR RSV + intron pCI neo.
Titre en anticorps en ng/ml
25 000
20 000
15 000
10 000
5 000
0
Référence
x1
x2,2
x2,3
x2,6
x2,9
x4,2
x4,3
x5,7
x5,8
x6,1
LTR RSV + intron pCIneo
E2CDK9U3 sans intron
E2CDK9U3 +Bactine
E2CDK9U3 +pcineo
E2CDK9U3 +HTLV
E2CDK9U3 +Mrosa
E2CDK9U3 +Hrosa
E2CDK9U3 +Ubiquitine
E2CDK9U3 +EF
E2CDK9U3 + EFss

Figure 27

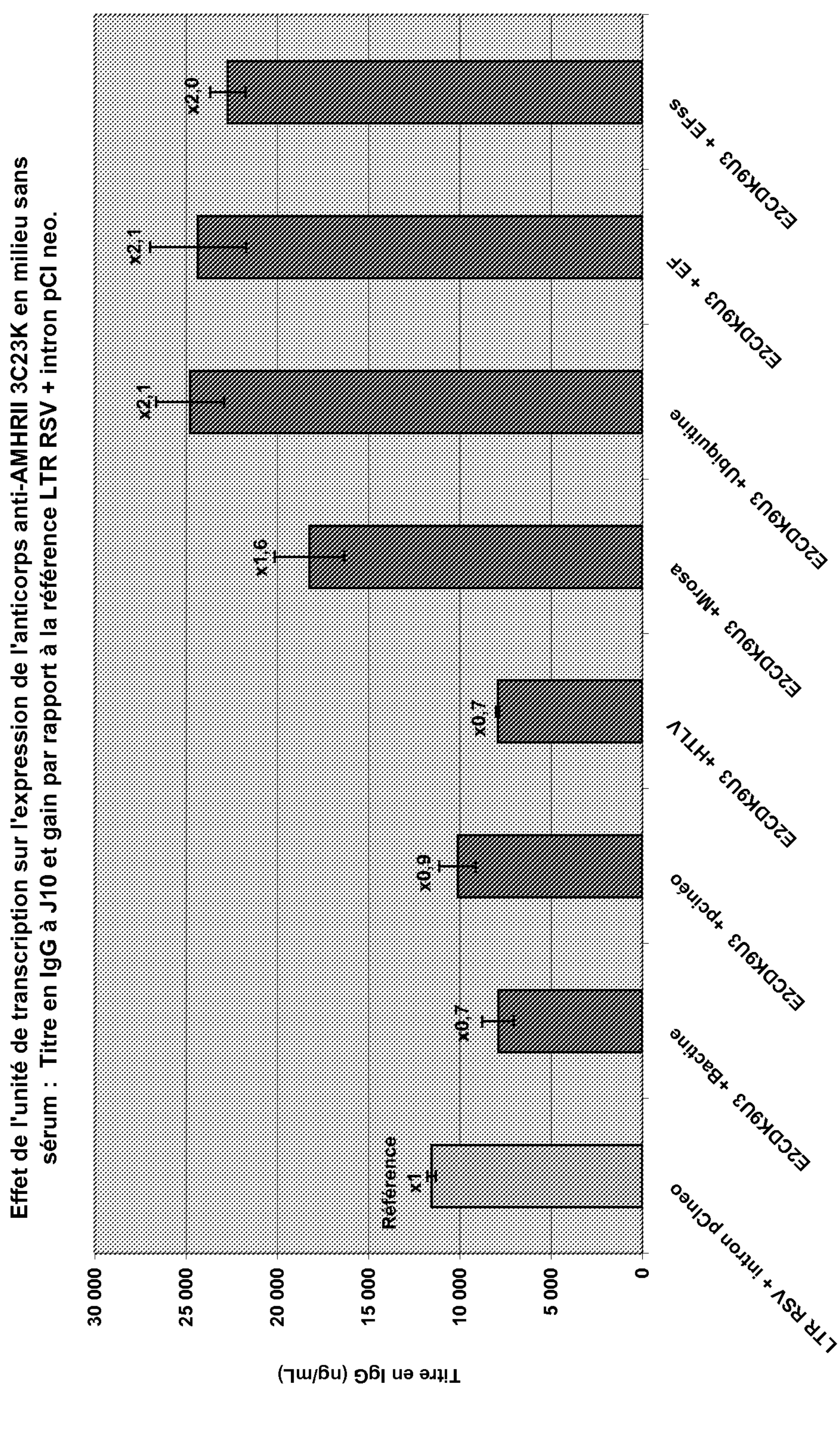
Effet de l'unité de transcription sur l'expression de l'anticorps anti-AMHRII 3C23K en milieu sans sérum : Titre en IgG à J10 et gain par rapport à la référence LTR RSV + intron pCI neo.
Titre en IgG (ng/mL)
30 000
25 000
20 000
15 000
10 000
5 000
0
Référence
x1
x0,7
x0,9
x0,7
x1,6
x2,1
x2,1
x2,0
LTR RSV + intron pCIneo
E2CDK9U3 +Bactine
E2CDK9U3 +pcineo
E2CDK9U3 +HTLV
E2CDK9U3 +Mrosa
E2CDK9U3 +Ubiquitine
E2CDK9U3 + EF
E2CDK9U3 + EFss

Figure 28

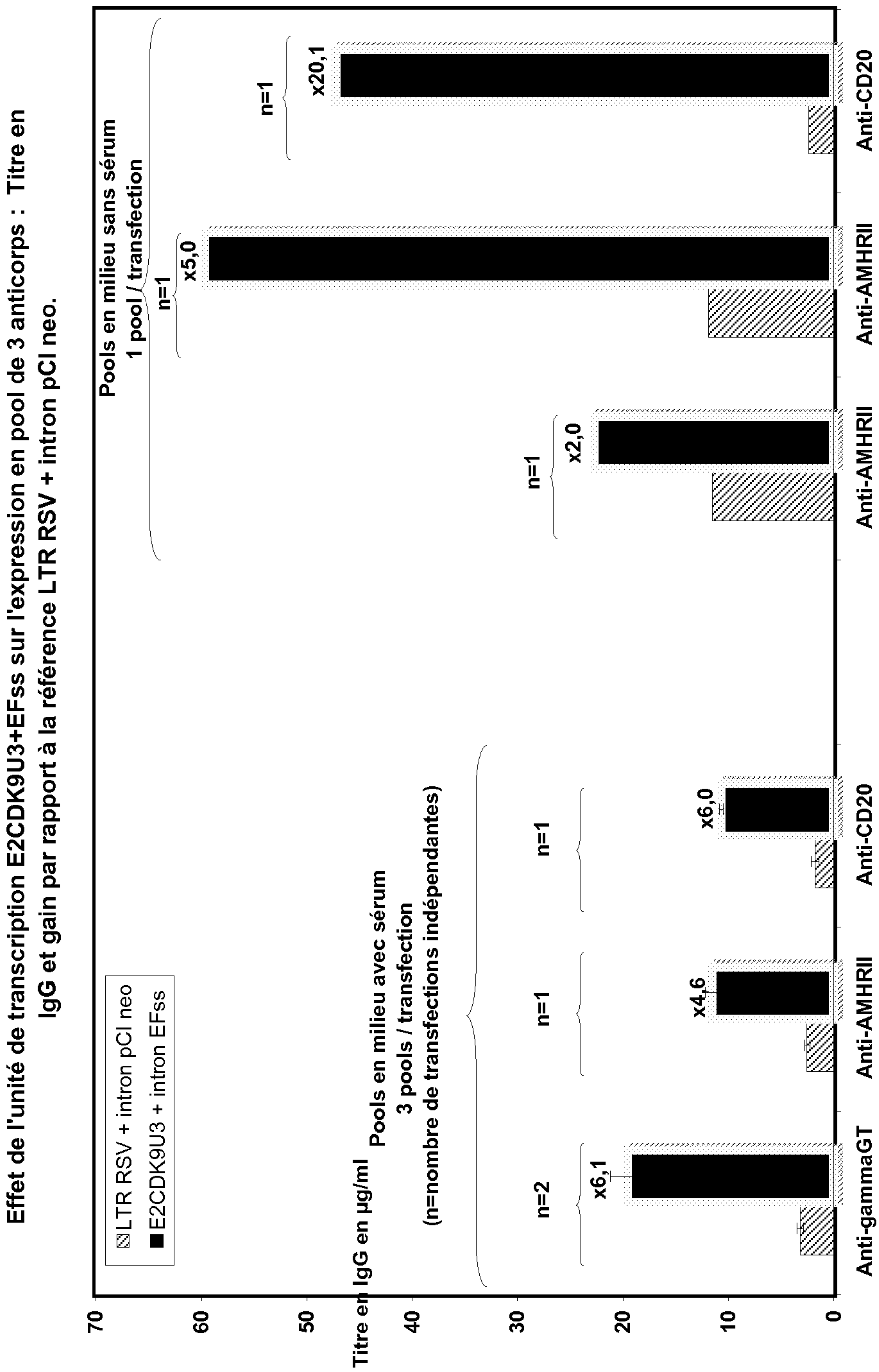

Effet de l'unité de transcription E2CDK9U3+EFss sur l'expression en pool de 3 anticorps : Titre en IgG et gain par rapport à la référence LTR RSV + intron pCI neo.
LTR RSV + intron pCI neo
E2CDK9U3 + intron EFss
Titre en IgG en µg/ml
0
10
20
30
40
50
60
70
Pools en milieu avec sérum
3 pools / transfection
(n=nombre de transfections indépendantes)
Pools en milieu sans sérum
1 pool / transfection
n=2
n=1
n=1
n=1
n=1
n=1
x6,1
x4,6
x6,0
x2,0
x5,0
x20,1
Anti-gammaGT
Anti-AMHRII
Anti-CD20
Anti-AMHRII
Anti-AMHRII
Anti-CD20

Figure 29

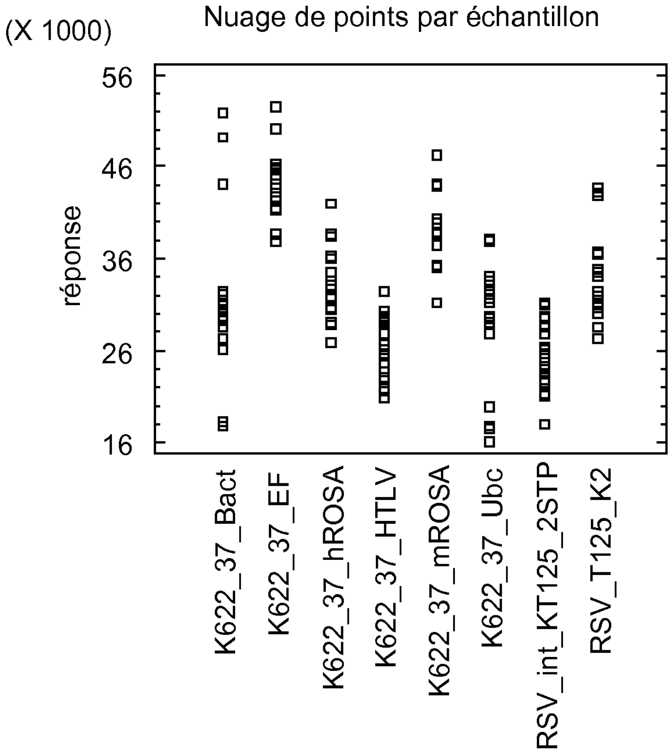


Figure 30

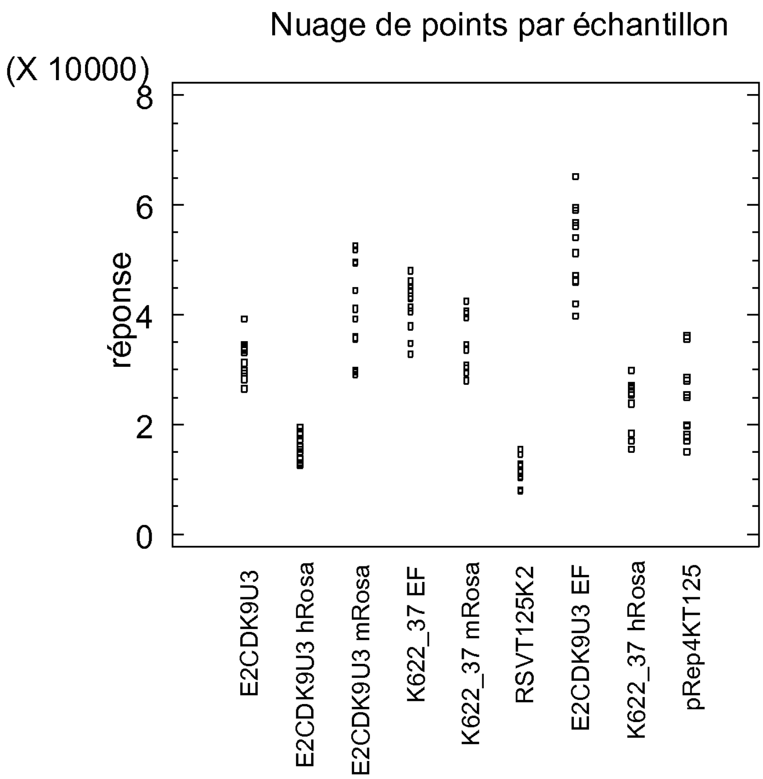

Figure 31

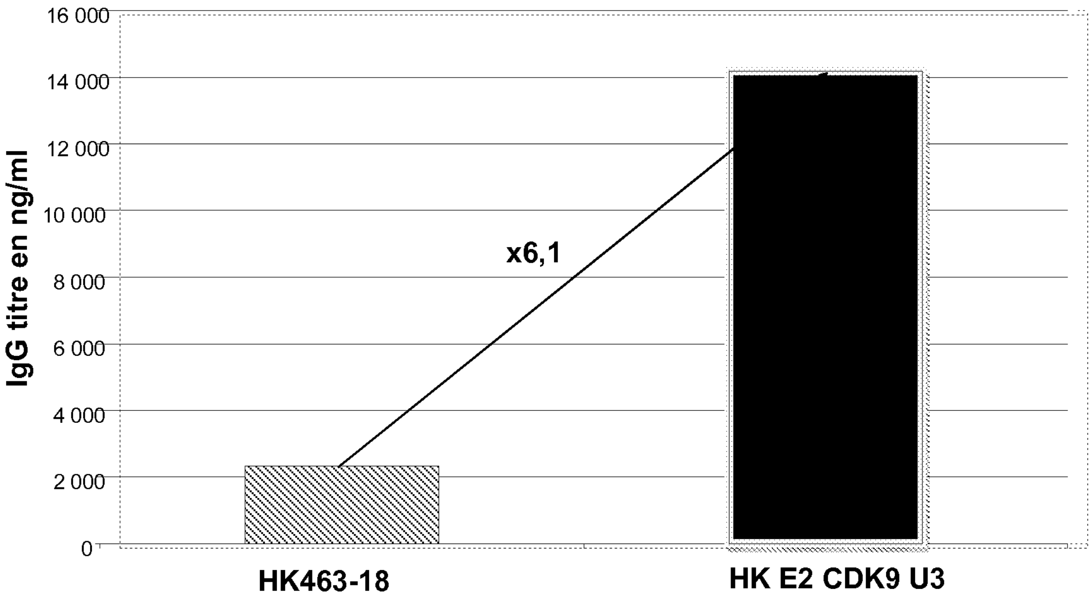


Figure 32

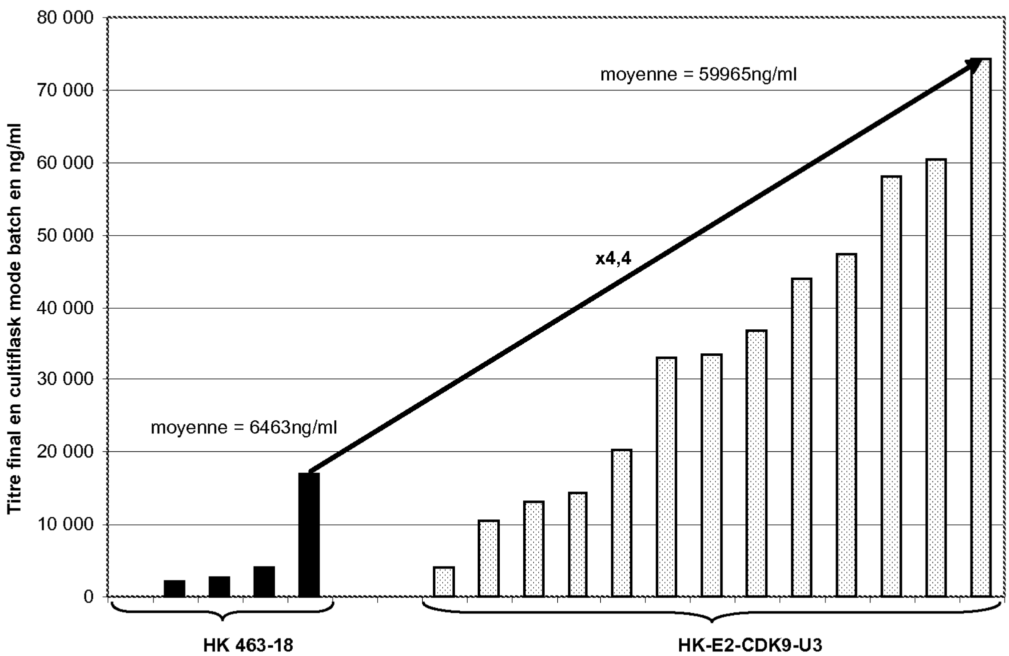

Figure 33

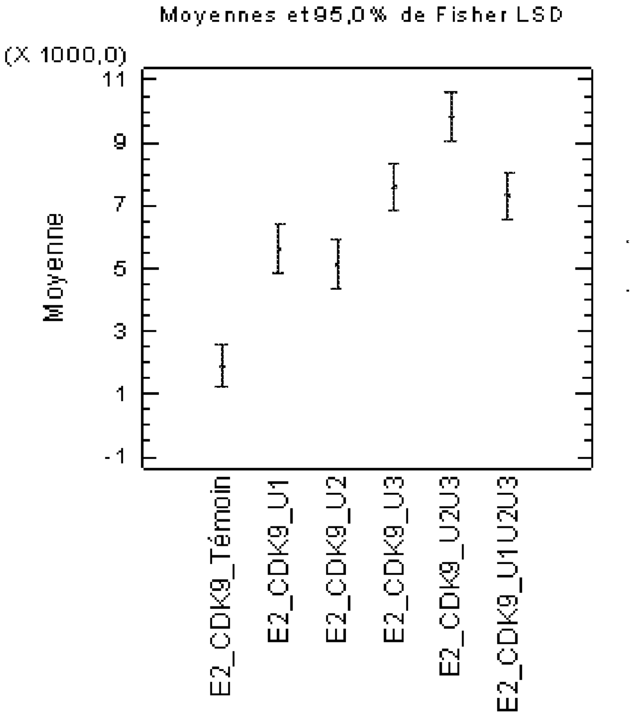


Figure 34

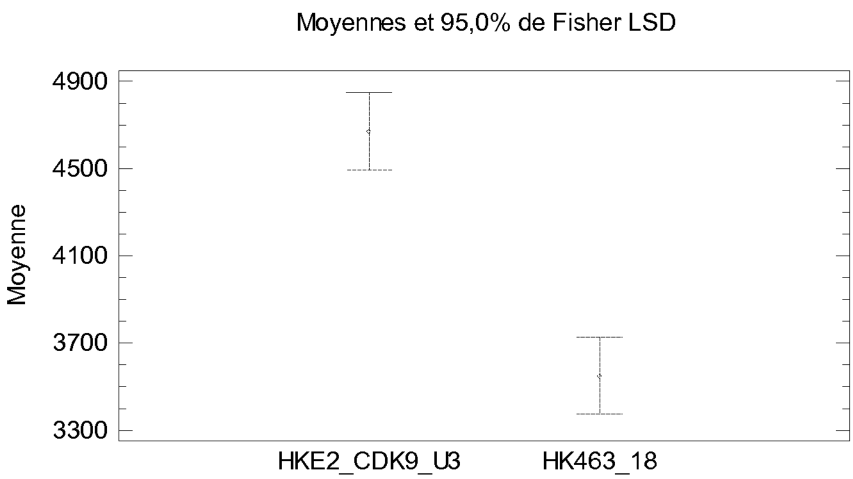

Figure 35

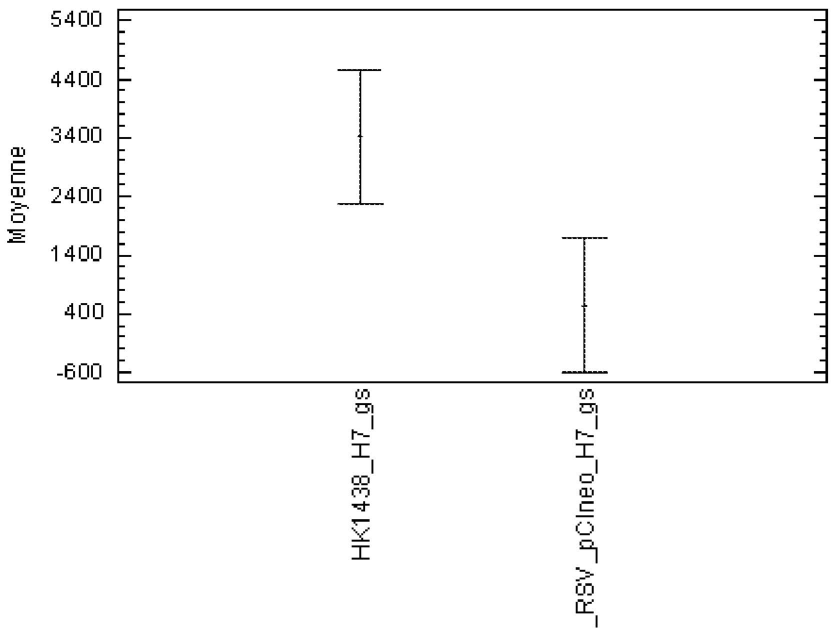
5400
4400
3400
2400
1400
400
-600
Moyenne
HK1438_H7_gs
_RSV_pCIneo_H7_gs

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 1159864


### Littérature non-brevet citée dans la description


- **LIU et al.** *Gene,* 2000, vol. 252, 51-59
- **FRITZ et al.** *Sci. STKE,* 05 Décembre 2000, vol. 2000 (61), 11
- **ROSS et al.** *Microbiol Rev.,* Septembre 1995, vol. 59 (3), 423-50